# FASCICULE DE BREVET EUROPEEN

(11) **EP 2 018 847 B1**
(45) Date de publication et mention de la délivrance du brevet: **17.09.2014**
(21) Numéro de dépôt: 08161083.4
(22) Date de dépôt: 24.07.2008
(51) Int. Cl.: A61K 8/46, A61K 8/49, A61Q 5/04, A61Q 5/06, A61Q 5/08

(54) **Composition capillaire comprenant au moins un colorant direct disulfure et au moins un agent alcalin hydroxyde et procédé de mise en forme et de coloration simultanées.**
Haarzusammensetzung, die mindestens ein Disulfid-Direktfärbemittel und mindestens eine Alkalihydroxidsubstanz enthält, und Verfahren zur gleichzeitigen Formgebung und Färbung
Hair composition comprising at least one direct disulfide dye and at least one alkali hydroxide agent and method of simultaneous styling and dyeing.

(30) Priorité: 24.07.2007 FR 0756706
(43) Date de publication de la demande: 28.01.2009
(73) Titulaire: L'Oréal, 75008 Paris (FR)
(72) Inventeur: Guerin, Frédéric, 75010, Paris (FR); De Boni, Maxime, 75020, Paris (FR)
(74) Mandataire: Dossmann, Gérard

(56) Documents cités:
- WO-A-2005/097051
- WO-A-2007/110542
- FR-A- 2 220 244
- FR-A- 2 876 576
- GB-A- 833 809
- US-A- 5 094 662
- US-A1- 2002 192 175

## Description

La présente invention a pour objet une composition capillaire de coloration et de mise en forme des fibres kératiniques comprenant un colorant direct disulfure et un agent alcalin hydroxyde.

Il existe divers traitements appliqués aux fibres kératiniques humaines dans le but d'en changer l'aspect. A titre d'exemples de procédé permettant de changer la couleur, on peut citer la décoloration ou la coloration des fibres. A titre d'exemple de procédé permettant de modifier la forme des fibres, on peut citer le défrisage ou décrêpage ou lissage.

Pour ce qui concerne la coloration, on connaît deux types de procédés, la coloration d'oxydation et la coloration directe.

La coloration d'oxydation consiste à mettre en oeuvre, en présence d'un agent oxydant, des bases d'oxydation (telles que par exemple des ortho- ou para-phénylènediamines, des ortho- ou para-aminophénols et des composés hétérocycliques), éventuellement associées à des coupleurs (comme notamment les méta-diamines aromatiques, les méta-aminophénols, les méta-diphénols et certains composés hétérocycliques).

La coloration directe ou semi-permanente consiste à appliquer des colorants directs, qui sont des molécules colorées et colorantes ayant une affinité pour les fibres, à laisser poser pour permettre aux molécules colorées de pénétrer par diffusion à l'intérieur de la fibre, puis à les rincer. On peut utiliser par exemple des colorants directs nitrés benzéniques, anthraquinoniques, nitropyridiniques, azoïques, xanthéniques, acridiniques, aziniques ou triarylméthaniques.

Il est aussi possible de colorer les fibres kératiniques à partir de colorants disulfures. De tels colorants sont par exemple décrits dans le document FR 2876576.

La décoloration est obtenue par l'emploi d'un agent oxydant, qui, en fonction de sa nature, permet de décolorer la fibre ou simplement de l'éclaircir. Ainsi, en utilisant du peroxyde d'hydrogène ou l'un de ses précurseurs en milieu en général alcalin, l'éclaircissement de la fibre est plutôt léger, et en utilisant des composés du type des sels peroxygénés (persulfate, perborate de métaux alcalins ou d'ammonium), en association avec du peroxyde d'hydrogène en milieu alcalin, la fibre est plus fortement décolorée.

Il est aussi possible d'observer un éclaircissement optique des cheveux foncés par l'application de colorants fluorescents. Cet éclaircissement optique ne met pas en oeuvre d'agent oxydant. Un tel effet est décrit par exemple dans la demande de brevet EP 1432390.

La mise en forme du cheveu peut être réalisée selon plusieurs méthodes. Une de ces méthodes consiste à mettre en contact les fibres avec une composition très fortement alcaline, comprenant des hydroxydes, et qui a pour résultat de transformer des ponts disulfures par lanthionisation. Ce type de procédé est en général employé pour défriser des cheveux.

Si l'on ne rencontre pas de problème particulier en mettant en oeuvre l'un des traitements précités, de manière isolée, il n'en est pas de même lorsque l'on souhaite combiner une mise en forme du cheveu avec une modification de la couleur. En effet, les étapes de mise en forme permanente, et plus particulièrement de défrisage, sont des procédés fragilisant les fibres et il est souvent difficilement envisageable de mettre en oeuvre par la suite, de manière rapprochée, une étape de coloration, et éventuellement un éclaircissement, sans risquer une dégradation très importante de la fibre.

Il est connu du document US 2002/192175 un procédé de défrisage et de coloration à partir de colorants cationiques, d'un défrisant, d'un émulsifiant, d'un hydratant et d'un agent conditionneur. Les résultats coloriels obtenus ne sont pas satisfaisants et les colorants décrits ne permettent pas d'obtenir un éclaircissement simultané des cheveux foncés.

Le document EP-A-1 464 318 décrit un procédé de coloration avec effet éclaircissant de fibres kératiniques ayant subi une déformation permanente, qui consiste à appliquer sur des fibres mises en forme antérieurement une composition comprenant un colorant fluorescent soluble. Ce procédé nécessitant deux étapes est contraignant et consommateur de temps. Les résultats coloriels ne sont pas toujours satisfaisants, notamment en terme d'intensité.

Le but de la présente invention est de pouvoir mettre en forme et colorer simultanément les fibres kératiniques, et éventuellement obtenir un éclaircissement des cheveux foncés, tout en limitant la dégradation des fibres kératiniques.

Ce but est atteint par la présente invention qui a pour objet une composition de traitement des fibres kératiniques, notamment la mise en forme, la coloration et/ou l'éclaircissement simultanés, comprenant dans un milieu cosmétiquement acceptable, * un ou plusieurs colorants directs disulfures, choisis parmi les composés de formules (I), (II) ou (III) selon la revendication 1
et
* un ou plusieurs agents alcalins de type hydroxyde minéral ou organique en quantité telle que le pH de la composition est compris entre 10 et 14.

L'invention a de même pour objet un procédé de mise en forme et coloration, et/ou éclaircissement simultanés des fibres kératiniques, dans lequel on applique la composition tinctoriale selon l'invention sur les fibres kératiniques pendant un temps suffisant pour développer la mise en forme, la coloration et/ou l'éclaircissement souhaités, puis on rince éventuellement les fibres kératiniques et on les lave au shampoing.

L'invention concerne aussi un dispositif à plusieurs compartiments comportant un premier compartiment comprenant un ou plusieurs agents alcalins de type hydroxyde minéral ou organique, et un deuxième compartiment comprenant une composition contenant un ou plusieurs colorants directs disulfures, le pH du mélange du contenu des différents compartiments étant compris entre 10 et 14.

L'invention concerne également un dispositif à plusieurs compartiments comportant un premier compartiment comprenant du carbonate de guanidine et un ou plusieurs colorants directs disulfures et un deuxième compartiment comprenant un ou plusieurs agents alcalins de type hydroxyde d'un métal alcalin ou alcalino-terreux, le pH du mélange du contenu des différents compartiments étant compris entre 10 et 14.

L'invention concerne enfin un dispositif à plusieurs compartiments comportant un premier compartiment comprenant du carbonate de guanidine, un deuxième compartiment comprenant un ou plusieurs agents alcalins de type hydroxyde d'un métal alcalin ou alcalino-terreux, et un troisième compartiment comprenant un ou plusieurs colorants directs disulfures, le pH du mélange du contenu des différents compartiments étant compris entre 10 et 14. La composition selon l'invention permet d'obtenir simultanément une mise en forme du cheveu, notamment un défrisage, et une coloration sans dégradation supplémentaire des fibres kératiniques. Lorsque ce procédé est mis en oeuvre sur cheveux foncés, on peut observer également, lorsque le colorant direct disulfure est fluorescent, une mise en forme et un éclaircissement simultanés des cheveux sans avoir à mettre en oeuvre un agent oxydant pour l'obtention de cet éclaircissement. Mais bien entendu, il n'est pas exclu que la composition selon l'invention comprenne un tel agent.

Il est ainsi possible d'effectuer une mise en forme des cheveux, telle qu'un défrisage, et une coloration et/ou un éclaircissement simultanés en utilisant une unique composition s'appliquant en une seule étape.

Par ailleurs, il a été également constaté que la présence de l'agent alcalin à pH élevé permettait une meilleure montée du colorant direct disulfure sur la fibre kératinique et un meilleur unisson, notamment en raison du gonflement des fibres dû audit agent alcalin.

Selon un mode de réalisation avantageux de l'invention, la composition est destinée à être appliquée sur des fibres kératiniques foncées. Plus particulièrement, les fibres kératiniques foncées sont des fibres pigmentées ou colorées artificiellement, dont la hauteur de ton est inférieure ou égale à 6 et de préférence inférieure ou égale à 4.

Il est rappelé que la notion de "ton" repose sur la classification des nuances naturelles, un ton séparant chaque nuance de celle qui la suit ou la précède immédiatement. Cette définition et la classification des nuances naturelles sont bien connues des professionnels de la coiffure et publiée dans l'ouvrage « Sciences des traitements capillaires » de Charles ZVIAK 1988, Ed.Masson, pp.215 et 278. Les hauteurs de ton s'échelonnent de 1 (noir) à 10 (blond clair clair), une unité correspondant à un ton ; plus le chiffre est élevé et plus la nuance est claire.

Il est à noter que la composition est appropriée pour le traitement de fibres kératiniques, quelle que soit leur coloration avant traitement et que celle-ci soit naturelle ou obtenue artificiellement.

L'effet de coloration et/ou éclaircissement est tenace, en particulier vis-à-vis des shampooings.

Au sens de la présente invention, et à moins qu'une indication différente ne soit donnée :
- un colorant direct disulfure est un composé comportant un ou plusieurs chromophores tels que définis ci-après, et comprenant une ou plusieurs liaisons disulfures S-S entre deux atomes de carbone, directement ou indirectement reliés au(x) chromophore(s) du composé, préférentiellement la liaison étant susceptible d'être réduite dans un milieu cosmétiquement acceptable ;
- les radicaux « aryles » ou « hétéroaryles » ou la partie aryle ou hétéroaryle d'un radical peuvent être substitués par au moins un substituant porté par un atome de carbone, choisi parmi :
   - un radical alkyle en C₁-C₁₆, de préférence en C₁-C₈, éventuellement substitué par un ou plusieurs radicaux choisis parmi les radicaux hydroxy, alcoxy en C₁-C₂, (poly)-hydroxyalcoxy en C₂-C₄, acylamino, amino substitué par deux radicaux alkyle, identiques ou différents, en C₁-C₄ éventuellement porteurs d'au moins un groupement hydroxyle, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
   - un atome d'halogène tel que chlore, fluor ou brome ;
   - un groupement hydroxyle ;
   - un radical alcoxy en C₁-C₂ ; un radical (poly)-hydroxyalcoxy en C₂-C₄ ;
   - un radical amino ;
   - un radical héterocycloalkyle à 5 ou 6 chaînons ;
   - un radical hétéroaryle à 5 ou 6 chaînons éventuellement cationique, préférentiellement imidazolium, et éventuellement substitué par un radical (C₁-C₄) alkyle, préférentiellement méthyle ;
   - un radical amino substitué par un ou deux radicaux alkyle, identiques ou différents, en C₁-C₆ éventuellement porteurs d'au moins :
      i) un groupement hydroxyle,
      ii) un groupement amino éventuellement substituté par un ou deux radicaux alkyle en C₁-C₃ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,
      iii) un groupement ammonium quaternaire -N⁺R'R"R'", M⁻ pour lequel R', R", R'", identiques ou différents représentent un atome d'hydrogène, ou un groupement alkyle en C₁-C₄; et M⁻ représente le contre-ion de l'acide organique, minéral ou de l'halogénure correspondant, ou
      iv) un radical hétéroaryle à 5 ou 6 chaînons éventuellement cationique, préférentiellement imidazolium, et éventuellement substitué par un radical (C₁-C₄) alkyle, préférentiellement méthyle ;
   - un radical acylamino (-NR-COR') dans lequel le radical R est un atome d'hydrogène, un radical alkyle en C₁-C₄ éventuellement porteur d'au moins un groupement hydroxyle et le radical R' est un radical alkyle en C₁-C₂ ; un radical carbamoyle ((R)₂N-CO-) dans lequel les radicaux R, identiques ou non, représentent un atome d'hydrogène, un radical alkyle en C₁-C₄ éventuellement porteur d'au moins un groupement hydroxyle ; un radical alkylsulfonylamino (R'SO₂-NR-) dans lequel le radical R représente un atome d'hydrogène, un radical alkyle en C₁-C₄ éventuellement porteur d'au moins un groupement hydroxyle et le radical R' représente un radical alkyle en C₁-C₄, un radical phényle ; un radical aminosulfonyle ((R)₂N-SO₂-) dans lequel les radicaux R, identiques ou non, représentent un atome d'hydrogène, un radical alkyle en C₁-C₄ éventuellement porteur d'au moins un groupement hydroxyle,
   - un radical carboxylique sous forme acide ou salifiée (de préférence avec un métal alcalin ou un ammonium, substitué ou non) ;
   - un radical nitro ;
   - un groupement cyano (CN) ;
   - un groupement polyhalogénoalkyle, préférentiellement le trifluorométhyle (CF3)
- la partie cyclique ou hétérocyclique d'un radical non aromatique peut être substituée par au moins un substituant porté par un atome de carbone choisi parmi les groupements :
   - hydroxyle,
   - alcoxy en C₁-C₄, (poly)hydroxyalcoxy en C₂-C₄,
   - alkylcarbonylamino ((RCO-NR'-) dans lequel le radical R' est un atome d'hydrogène, un radical alkyle en C₁-C₄ éventuellement porteur d'au moins un groupement hydroxyle et le radical R est un radical alkyle en C₁-C₂, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
   - alkylcarbonyloxy ((RCO-O-) dans lequel le radical R est un radical alkyle en C₁-C₄, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
   - alkcoxycarbonyle ((RO-CO-) dans lequel le radical R est un radical alkyle en C₁-C₄, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
- un radical cyclique, hétérocyclique, ou une partie non aromatique d'un radical aryle ou hétéroaryle, peut également être substitué par un ou plusieurs groupements oxo ;
- une chaîne hydrocarbonée est insaturée lorsqu'elle comporte une ou plusieurs liaisons doubles et/ou une ou plusieurs liaisons triples ;
- un radical « aryle » représente un groupement mono ou polycyclique, condensé ou non, comprenant de 6 à 22 atomes de carbones, et dont au moins un cycle est aromatique ; préférentiellement le radical aryle est un phényle, biphényle, naphtyle, indényle, anthracényle, ou tétrahydronaphtyle ;
- un « radical hétéroaromatique ou hétéroaryle » représente un groupement mono ou polycyclique, condensé ou non, éventuellement cationique, comprenant de 5 à 22 chaînons, de 1 à 6 hétéroatomes choisis parmi l'atome d'azote, d'oxygène, de soufre et de sélénium, et dont au moins un cycle est aromatique ; préférentiellement un radical hétéroaryle est choisis parmi acridinyle, benzimidazolyle, benzobistriazolyle, benzopyrazolyle, benzopyridazinyle, benzoquinolyle, benzothiazolyle, benzotriazolyle, benzoxazolyle, pyridinyle, tetrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle, xanthylyle et son sel d'ammonium ;
- un « radical cyclique » est un radical cycloalkyle non aromatique, mono ou polycyclique, condensé ou non, contenant de 5 à 22 atomes de carbone, pouvant comporter de 1 à plusieurs insaturations ;
- un « radical hétérocyclique » est un radical non aromatique mono ou polycyclique, condensé ou non, contenant de 5 à 22 chaînons, comportant de 1 à 6 hétéroatomes choisis parmi l'atome d'azote, d'oxygène, de soufre et de sélénium ;
- un « radical alkyle » est un radical hydrocarboné en C₁-C₁₆, linéaire ou ramifié, de préférence en C₁-C₈,
- l'expression « éventuellement substitué » attribué au radical alkyle sous entend que ledit radical alkyle peut être substitué par un ou plusieurs radicaux choisis parmi les radicaux i) hydroxy, ii) alcoxy en C₁-C₄, iii) acylamino, iv) amino éventuellement substitué par un ou deux radicaux alkyle, identiques ou différents, en C₁-C₄, lesdits radicaux alkyles pouvant former avec l'atome d'azote qui les portent un hétérocyle comprenant de 5 à 7 chaînons, comprenant éventuellement un autre hétéroatome différent ou non de l'azote ; v) ou un groupement ammonium quaternaire -N⁺R'R"R'", M⁻ pour lequel R', R", R'", identiques ou différents représentent un atome d'hydrogène, ou un groupement alkyle en C₁-C₄, ou alors -N'R'R"R'" forme un hétéroaryle tel que imidazolium éventuellement substitué par un groupement C₁-C₄ alkyle, et M⁻ représente le contre-ion de l'acide organique, minéral ou de l'halogénure correspondant,
- un « radical alcoxy » est un radical alkyl-oxy pour lequel le radical alkyle est un radical hydrocarboné, linéaire ou ramifié, en C₁-C₁₆ préférentiellement en C₁-C₈ ; lorsque le groupement alcoxy est éventuellement substitué, cela sous entend que le groupe alkyle est éventuellement substitué tel que défini supra.

De plus, sauf indication contraire, les bornes délimitant l'étendue d'une plage de valeurs sont comprises dans cette plage de valeurs.

Selon la présente invention, on entend par « chromophore » un radical issu d'un colorant, c'est-à-dire un radical issu d'une molécule absorbant dans le domaine visible du rayonnement (entre 400 et 800 nm).

En particulier, le colorant direct disulfure peut être un colorant direct disulfure fluorescent ou un colorant direct disulfure non-fluorescent.

Par composé fluorescent, on entend au sens de la présente invention un colorant qui est une molécule qui colore par elle-même, et donc absorbe la lumière du spectre visible, et éventuellement de l'ultraviolet, mais qui, contrairement à un colorant classique, transforme l'énergie absorbée en lumière fluorescente de plus grande longueur d'onde émise dans la partie visible du spectre. Ainsi, un composé fluorescent est capable d'absorber dans le rayonnement UV ou visible à une longueur d'onde λ_{abs} comprise entre 250 et 800 nm et capable de réémettre dans le domaine du visible à une longueur d'onde d'émission λ_{ém} comprise entre 400 et 800 nm.

De préférence les composés fluorescents sont des colorants capables d'absorber dans le visible à une longueur d'onde λ_{abs} comprise entre 400 et 800 nm, et de réémettre dans le visible à une longueur d'onde λ_{ém} comprise entre 400 et 800 nm. Plus préférentiellement les colorants fluorescents sont des colorants capables d'absorber à une longueur d'onde λ_{abs} comprise entre 420 nm et 550 nm et de réémettre dans le visible à une longueur d'onde λ_{ém} comprise entre 470 et 600 nm.

Un colorant fluorescent est à différencier d'un agent éclaircissant optique. Les agents éclaircissants optiques appelés généralement azurants optiques, ou "brighteners", ou "fluorescent brighteners", ou "fluorescent brightening agents", ou "fluorescent whitening agents", ou "whiteners", ou encore "fluorescent whiteners" en terminologie anglo-saxone, sont des composés transparents incolores, qui ne colorent pas, car ils n'absorbent pas dans la lumière visible, mais uniquement dans les Ultra Violets (longueurs d'onde allant de 200 à 400 nanomètres), et transforment l'énergie absorbée en lumière fluorescente de plus grande longueur d'onde émise dans la partie visible du spectre ; l'impression de couleur est alors uniquement engendrée par la lumière purement fluorescente à prédominante bleue (longueurs d'onde allant de 400 à 500 nanomètres).

Un colorant fluorescent est différent d'un pigment fluorescent qui lui, n'est pas soluble dans le milieu de la composition.

Plus particulièrement, un colorant fluorescent dans le cadre de la présente invention, éventuellement neutralisé, est soluble dans le milieu de la composition à au moins 0,001 g/l, plus particulièrement au moins 0,5 g/l, de préférence au moins 1 g/l et, selon un mode de réalisation encore plus préféré, au moins 5 g/l à une température comprise entre 15 et 25°C.

Les colorants directs disulfures de l'invention sont choisis parmi les composés de formules (I), (II) ou (III) suivantes : 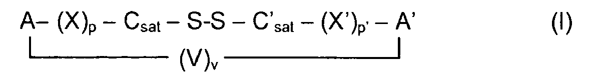 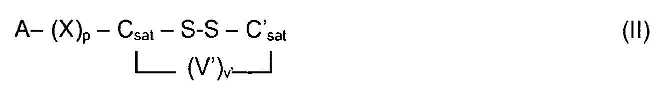 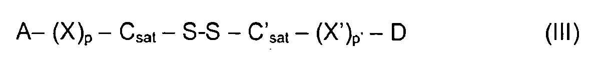 leurs sels, isomères optiques, isomères géométriques, et les solvates tels que hydrates,
formules dans lesquelles :
- A et A', identiques ou différents, représentent un radical contenant un ou plusieurs chromophores cationiques ou non;
- V et V', identiques ou différents représentent un groupe pontant
- v et v', identiques ou différents représentent 0 ou 1 ;
- X et X', identiques ou différents, représentent une chaîne hydrocarbonée en C₁-C₃₀, linéaire ou ramifiée, saturée ou insaturée, éventuellement interrompue et/ou éventuellement terminée à l'une ou deux de ses extrémités par un ou plusieurs groupes divalents ou leur combinaisons choisis parmi :
   - -N(R)-, -N⁺(R)(R')-, -O-, -S-, -CO-, -SO₂- avec R et R', identiques ou différents, choisis parmi un hydrogène, un radical alkyle en C₁-C₄, hydroxyalkyle, aminoalkyle
   - un radical (hétéro)cyclique aromatique ou non, saturé ou insaturé, condensé ou non comprenant éventuellement un ou plusieurs hétéroatomes identiques ou non, éventuellement substitué ;
- les coefficients p et p', identiques ou différents, représentent un entier égal à 0 ou 1 ;
- Cₛₐₜ et C'ₛₐₜ, identiques ou différents, représentent une chaîne alkylène en C₁-C₁₈, linéaire ou ramifiée, éventuellement substituée, éventuellement cyclique ;
- D correspond à un radical choisi parmi les radicaux hydroxy, hydroxyalkyle, alcoxy, carboxyliques, carboxylates, amino, alkylamino, dialkylamino.

### I. Colorants de formules (I), (II) et (III)

Les radicaux A et A' des formules (I), (II) et (III) peuvent contenir un ou plusieurs chromophores, fluorescents ou non, identiques ou différents.

### I.1. Chromophores

Au sens de la présente invention, les chromophores sont dits différents lorsqu'ils diffèrent par leur structure chimique. De tels chromophores peuvent être des chromophores issus de familles différentes ou d'une même famille à la condition de présenter des structures chimiques différentes. Par exemple, les chromophores peuvent être choisis dans la famille des colorants azoïques ou poly(méthiniques) mais différer par la structure chimique des radicaux le constituant ou par la position respective de ces radicaux.

A titre de chromophores utiles dans la présente invention, on peut citer les radicaux issus des colorants acridines, acridones, anthranthrones, anthrapyrimidines, anthraquinones, azines, azoïques, azométhines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines (comme les azacarbocyanines, diazacarbocyanines, diazahémicyanines, hémicyanines, tétraazacarbocyanines), diazines, dicétopyrrolopyrroles, difluoro{2-[(2H-pyrrol-2-ylidene-kN)methyl]-1H-pyrrolato-kN}bores (BODIPY ®), dioxazines, diphénylamines, diphénylméthanes, dithiazines, flavonoïdes tels que flavanthrones et flavones, fluorindines, formazans, hydrazones, en particulier arylhydrazones, hydroxycétones, indamines, indanthrones, indigoides et pseudo-indigoïdes, indophénols, indoanilines, isoindolines, isoindolinones, isoviolanthrones, lactones, méthines, (poly)méthines (notamment cyanines et styryles/hémicyanines), naphthalimides, naphthanilides, naphthylamine (comme les dansyles), naphtholactames, naphthoquinones, nitro, notamment les nitro(hétéro)aromatiques, oxadiazoles, oxazines, périlones, périnones, pérylènes, phénazines, phénothiazines, phthalocyanine, polyènes/caroténoides, porphyrines, pyranthrones, pyrazolanthrones, pyrazolones, pyrimidinoanthrones, pyronines, quinacridones, quinolines, quinophthalones, squaranes, stilbènes, tétrazoliums, thiazines, thioindigo, thiopyronines, triarylméthanes, xanthènes.

Parmi les chromophores nitro utilisables selon l'invention, on peut citer de manière non limitative les radicaux issus des colorants suivants:
- 1,4-diamino-2-nitrobenzène
- 1-amino-2 nitro-4- β - hydroxyéthylaminobenzène
- 1-amino-2 nitro-4-bis(β -hydroxyéthyl)-aminobenzène
- 1,4-Bis(β -hydroxyéthylamino)-2-nitrobenzène
- 1- β -hydroxyéthylamino-2-nitro-4-bis-( β -hydroxyéthylamino)-benzène
- 1- β -hydroxyéthylamino-2-nitro-4-aminobenzène
- 1- β -hydroxyéthylamino-2-nitro-4-(éthyl)( β -hydroxyéthyl)-aminobenzène
- 1-amino-3-méthyl-4- β -hydroxyéthylamino-6-nitrobenzène
- 1-amino-2-nitro-4- β -hydroxyéthylamino-5-chlorobenzène
- 1,2-Diamino-4-nitrobenzène
- 1-amino-2- β -hydroxyéthylamino-5-nitrobenzène
- 1,2-Bis-( β -hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1- β -hydroxyéthyloxy-2- β -hydroxyéthylamino-5-nitrobenzène
- 1-Méthoxy-2- β -hydroxyéthylamino-5-nitrobenzène
- 1- β -hydroxyéthyloxy-3-méthylamino-4-nitrobenzène
- 1- β.γ -dihydroxypropyloxy-3-méthylamino-4-nitrobenzène
- 1- β -hydroxyéthylamino-4- β,γ-dihydroxypropyloxy-2-nitrobenzène
- 1- β.γ -dihydroxypropylamino-4-trifluorométhyl-2-nitrobenzène
- 1- β -hydroxyéthylamino-4-trifluorométhyl-2-nitrobenzène
- 1- β -hydroxyéthylamino-3-méthyl-2-nitrobenzène
- 1- β -aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-( β -hydroxyéthyl)-amino-3-nitrobenzène
- 1- β -hydroxyéthylamino-2-nitrobenzène
- 1-Hydroxy-4- β -hydroxyéthylamino-3-nitrobenzène.

Parmi les chromophores azoïques, azométhiniques ou méthiniques utilisables selon l'invention, on peut citer les radicaux issus des colorants azoïques, azométhiniques ou méthiniques cationiques décrits dans les demandes de brevets WO 95/15144, WO 95/01772 et EP-714954, et en particulier le Basic Red 59, le Basic Orange 31 et le Basic Yellow 87.

On peut également citer parmi les chromophores azoïques ceux décrits dans le Colour Index International 3e édition, et notamment les composés suivants :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-( β -hydroxyéthyl) aminobenzène et l'acide 4-hydroxy-3-(2-méthoxyphénylazo)-1-naphtalène sulfonique.

Parmi les chromophores quinoniques, ceux mentionnés dans le Colour Index International précité conviennent, et parmi ceux-ci, on peut citer entre autres, les radicaux issus des colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
-1-N-méthylmorpholiniumpropylamino-4-hydroxyanthraquinone
-1-Aminopropylamino-4-méthylaminoanthraquinone
-1-Aminopropylaminoanthraquinone
-5- β -hydroxyéthyi-1,4-diaminoanthraquinone
-2-Aminoéthylaminoanthraquinone
-1,4-Bis-( β.γ-dihydroxypropylamino)-anthraquinone.

Parmi les chromophores aziniques, conviennent ceux listés dans le Colour Index International et par exemple les radicaux issus des colorants suivants :
- Basic Blue 17
- Basic Red 2.

Parmi les chromophores triarylméthaniques utilisables selon l'invention, on peut citer, outre ceux listés dans le Colour Index, les radicaux issus des colorants suivants :
- Basic Green 1
- Acid blue 9
- Basic Violet 3
- Basic Violet 14
- Basic Blue 7
- Acid Violet 49
- Basic Blue 26
- Acid Blue 7

Parmi les chromophores indoaminiques utilisables selon l'invention, on peut citer les radicaux issus des colorants suivants :
- 2-β-hydroxyéthylamino-5-[bis-(β-4'-hydroxyéthyl)amino]anilino-1,4-benzoquinone
- 2- β -hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

On peut aussi citer les chromophores décrits dans les documents US 5888252, EP 1133975, WO 03/029359, EP 860636. On peut aussi citer ceux listés dans l'encyclopédie "The chemistry of synthetic dye" de K. VENKATARAMAN, 1952, Academic press vol 1 à 7, dans l'encyclopédie "Kirk Othmer" "Chemical technology", chapitre "dyes and Dye intermediate", 1993, Wiley and sons, et dans divers chapitre de l'encyclopédie "ULLMANN's ENCYCLOPEDIA of Industrial chemistry" 7th édition, Wiley and sons.

De préférence, dans les formules (I), (II) ou (III), A et A' représentent un radical comprenant un chromophore choisi parmi les chromophores de type azoïque, anthraquinone et hydrazone.

On peut également citer les colorants fluorescents décrits dans les documents EP 1133975, WO 03/029359, EP 860636, WO 95/01772, WO 95/15144, EP 714954 et ceux listés dans l'encyclopédie "The chemistry of synthetic dye" de K. VENKATARAMAN, 1952, Academic press vol 1 à 7, dans l'encyclopédie "Kirk Othmer" "Chemical technology", chapitre "dyes and Dye intermediate", 1993, Wiley and sons, et dans divers chapitres de l'encyclopédie "ULLMANN's ENCYCLOPEDIA of Industrial chemistry" 7th édition, Wiley and sons, dans The Handbook - A Guide to Fluorescent Probes and Labeling Technologies, 10th Ed Molecular Probes/Invitrogen - Oregon 2005 diffusé par Internet ou dans les éditions précédentes imprimées.

Selon une variante, les radicaux A et/ou A' des formules (I), (II) ou (III) contiennent ou non un radical cationique porté par ou inclus dans au moins un des chromophores.

De préférence, le radical cationique est un ammonium quaternaire.

Ces radicaux cationiques sont par exemple un radical alkylammonium, acridinium, benzimidazolium, benzobistriazolium, benzopyrazolium, benzopyridazinium, benzoquinolium, benzothiazolium, benzotriazolium, benzoxazolium, bi-pyridinium, bis-tétrazolium, dihydrothiazolium, imidazopyridinium, imidazolium, indolium, isoquinolium, naphthoimidazolium, naphthooxazolium, naphthopyrazolium, oxadiazolium, oxazolium, oxazolopyridinium, oxonium, phénazinium, phénooxazolium, pyrazinium, pyrazolium, pyrazoyltriazolium, pyridinium, pyridinoimidazolium, pyrrolium, pyrylium, quinolium, tétrazolium, thiadiazolium, thiazolium, thiazolopyridinium, thiazoylimidazolium, thiopyrylium, triazolium ou xanthylium.

De préférence, les chromophores fluorescents sont choisis parmi ceux issus de colorants de type coumarines, (poly)méthines (notamment cyanines et styryles/hémicyanines) et naphthalimides.

Selon une variante, les radicaux A et/ou A' des formules (I), (II) ou (III), identiques ou différents, représentent un radical contenant un ou plusieurs chromophores fluorescents cationiques ou non. De préférence, les radicaux A et/ou A', identiques ou différents, contiennent un chromophore styryle.

Selon un mode de réalisation particulier, les radicaux A, A' dans les formules (I), (II), ou (III) comprennent un ou plusieurs chromophores azoïques cationiques, décrits par exemple dans EP 850636, FR 2788433, EP 920856, WO 9948465, FR 2757385, EP 850637, EP 918053, WO 9744004, FR 2570946, FR 2285851, DE 2538363, FR 2189006, FR 1560664, FR 1540423, FR 1567219, FR 1516943, FR 1221122, DE 4220388, DE 4137005, WO 0166646, US 5708151, WO 9501772, WO 515144, GB 1195386, US 3524842, US 5879413, EP 1062940, EP 1133976, GB 738585, DE 2527638, FR 2275462, GB 1974-27645, Acta Histochem. (1978), 61(1), 48-52; Tsitologiya (1968), 10(3), 403-5 ; Zh. Obshch. Khim. (1970), 40(1), 195-202; Ann. Chim. (Rome) (1975), 65(5-6), 305-14 ; Journal of the Chinese Chemical Society (Taipei) (1998), 45(1), 209-211 ; Rev. Roum. Chim. (1988), 33(4), 377-83; Text. Res. J. (1984), 54(2), 105-7 ; Chim. Ind. (Milan) (1974), 56(9), 600-3 ; Khim. Tekhnol. (1979), 22(5), 548-53 ; Ger. Monatsh. Chem. (1975), 106(3), 643-8 ; MRL Bull. Res. Dev. (1992), 6(2), 21-7 ; Lihua Jianyan, Huaxue Fence (1993), 29(4), 233-4 ; Dyes Pigm. (1992), 19(1), 69-79 ; Dyes Pigm. (1989), 11 (3), 163-72.

### I.2. Cₛₐₜ et C'ₛₐₜ :

Comme indiqué auparavant, dans les formules (I), (II) ou (III), Cₛₐₜ et C'ₛₐₜ, indépendamment l'un de l'autre, représentent une chaîne alkylène en C₁-C₁₈, linéaire ou ramifiée, éventuellement substituée, éventuellement cyclique.

A titre de substituant, on peut citer les groupements amino, (C₁-C₄)alkylamino, (C₁-C₄)dialkylamino, ou le groupement R^{a}-Z^{a}-C(Z^{b})- (dans laquelle Z^{a}, Z^{b}, identiques ou différents, représentent un atome d'oxygène, de soufre, ou un groupe NR^{a}', et R^{a}, représente un métal alcalin, un atome d'hydrogène, ou un groupement C₁-C₄ alkyle et R^{a}' représente un atome d'hydrogène ou un groupement C₁-C₄ alkyle) présents de préférence sur le carbone en position béta ou gamma des atomes de soufre.

De préférence, dans le cas des formules (I) ou (III), Cₛₐₜ et C'ₛₐₜ, représentent une chaîne -(CH₂)ₖ- avec k entier, compris inclusivement entre 1 et 8.

De préférence, dans le cas de la formule (II), C'ₛₐₜ représente un radical -(CH₂)ₖ-, Cₛₐₜ représente un radical -(CH₂)ₖ-CH-, k ayant la même signification que précédemment.

### 1. 3. X et X':

Conformément à un autre mode de réalisation de l'invention, dans les formules (I), (II) ou (III), lorsque p et/ou p' respectivement, sont égaux à 1, X et/ou X' respectivement, représentent la séquence suivante :

- (T)ₜ- (Y)_{y}- (Z)_{z}-

ladite séquence étant reliée dans les formules (I), (II) ou (III), comme suit :
- Cₛₐₜ (ou C'ₛₐₜ respectivement) - (T)ₜ- (Y)_{y}- (Z)_{z}- A (ou A' respectivement) ; dans laquelle :
   T représente un ou plusieurs radicaux ou leurs combinaisons choisis parmi -SO₂-, -O-, -S-, -N(R)-, -N⁺(R)(R'), -CO-, où R et R', identiques ou différents, représentent un atome d'hydrogène, un radical alkyle en C₁-C₄, ou hydroxyalkyle en C₁-C₄;
   le coefficient t vaut 0 ou 1 ;
   Y représente :
- un radical choisi parmi -(CH₂)₂-SO₂- ; -CH₂-CHR-CO-NR'- avec R et R', identiques ou non, représentant un atome d'hydrogène, un radical alkyle en C₁-C₄.
- un groupement de formule (a), (a') ou (a") : 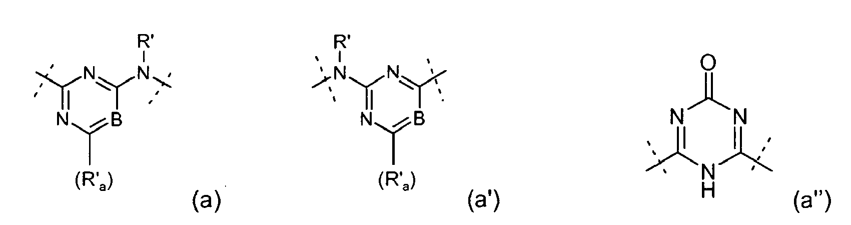 dans laquelle
   - B représente -N-, -CRₐ avec Rₐ représentant un atome d'hydrogène, un atome d'halogène choisi parmi le chlore ou le fluor, un groupement nitro, un groupement pyridinium éventuellement substitué;
   - R' a la même définition que précédemment
   - R'ₐ représente :
      - un atome d'hydrogène
      - un atome de chlore ou un atome de fluor
      - un groupement pyridinium éventuellement substitué par au moins un groupement R_{c}, R_{c} prouvant être un groupement alkyle en C₁-C₄, un atome d'halogène, un groupement carboxylique -COOM, (avec M représentant un atome d'hydrogène, un métal alcalin, un groupement ammonium ou un groupement ammonium substitué par un ou plusieurs radicaux alkyle, identiques ou non, linéaires ou ramifiés, en C₁-C₁₈, éventuellement porteurs d'au moins un hydroxyle) ; un groupement ester
      - COOR_{d} avec R_{d} représentant un radical alkyle en C₁-C₄ ; un groupement amide -CON(R_{d})₂ avec R_{d} identiques ou non, représentant un atome d'hydrogène ou un radical alkyle en C₁-C₄ ;
      - un groupement hydroxyle
      - un groupement amino, alkylamino ou dialkylamino, les groupements alkyles identiques ou différents, en C₁-C₁₈, étant linéaires ou ramifiés, éventuellement interrompus par un hétéroatome choisi parmi N, O, éventuellement substitués par un ou plusieurs groupements hydroxyles
      - un groupement NHNHCOR où R représente un groupement alkyle linéaire ou ramifié en C₁-C₁₀
- un groupement de formule (b) suivante : 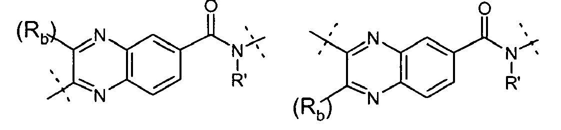 dans laquelle
   - R' a la même définition que précédemment
   - R_{b} représente
      - un atome de chlore
      - un groupement amino, alkylamino ou dialkylamino, les groupements alkyles identiques ou différents, en C₁-C₁₈, étant linéaires ou ramifiés, éventuellement interrompus par un hétéroatome choisi parmi N, O, S, éventuellement substitués par un ou plusieurs hydroxyles
      - un hétérocycle azoté, saturé ou insaturé, pouvant être substitué
      - un groupement arylamino, dans lequel de préférence le radical aryle est en C₆ ;
   y vaut 0 ou 1 ;
   Z représente :
- -(CH₂)ₘ- avec m entier compris entre 1 et 8
- -(CH₂CH₂O)_{q}- ou -(OCH₂CH₂)_{q}- dans lesquelles q est un entier compris entre 1 et 15
- un radical aryle, alkylaryle ou arylalkyle dont le radical alkyle est en C₁-C₄ et le radical aryle est de préférence en C₆, étant éventuellement substitué par au moins un groupement SO₃M avec M représentant un atome d'hydrogène, un métal alcalin ou un groupement ammonium ammonium substitué par un ou plusieurs radicaux alkyle, identiques ou non, linéaires ou ramifiés, en C₁-C₁₈ éventuellement porteurs d'au moins un hydroxyle ;
   z vaut 0 ou 1.

Avantageusement, Y représente l'un des groupements ci-dessous : 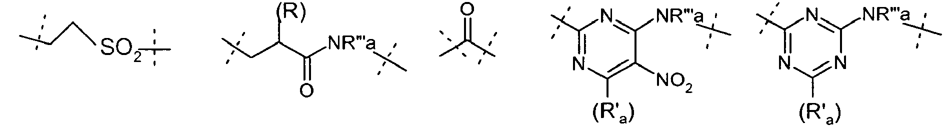 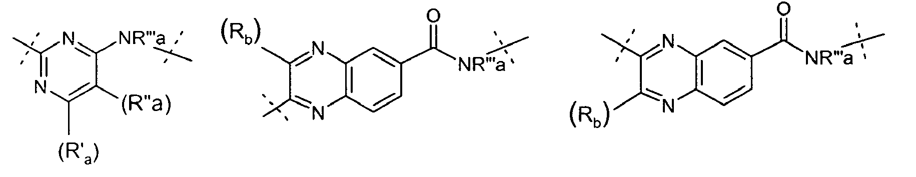 dans lesquels les radicaux R, R'ₐ et R_{b} sont définis comme précédemment ; R"ₐ a la même définition que R'ₐ, indépendamment les uns des autres ; R'"ₐ représente un atome d'hydrogène ou un radical alkyle.

Par ailleurs, selon un mode de réalisation particulier de l'invention, Z représente : 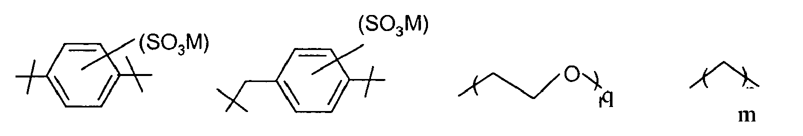 M, q et m ayant la même signification que précédemment.

Selon un mode de réalisation préféré, X et X', identiques ou différents, représentent un groupement -N(R)-, dans lequel R a la signification indiquée ci-dessus et est de préférence un atome d'hydrogène.

### I.4. Colorants directs disulfures:

Comme indiqué auparavant, dans les formules (I) et (II), V représente un groupe pontant les deux radicaux A et A', identiques ou différents, v pouvant être égal à 0 ou 1, et V' représente un groupe pontant les deux radicaux Cₛₐₜ et C'ₛₐₜ, identiques ou différents, v' pouvant être égal à 0 ou 1

Lorsque v ou v' est égal à 1, le groupe V ou V' pontant respectivement les deux chromophores A et A' ou les deux radicaux Cₛₐₜ et C'ₛₐₜ, représentent un radical alkyle en C₁-C₈, éventuellement terminé à l'une ou ses deux extrémités par un groupement choisi parmi amine, amide ou ester.

Conformément à un mode de réalisation particulier de l'invention, le colorant direct disulfure de formule (I) est tel que v est égal à 0.

A titre d'exemples particuliers, le colorant direct disulfure est choisi parmi : 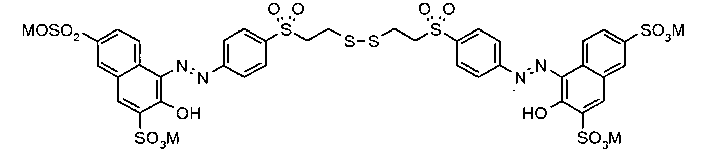 ainsi que les composés suivants, sous forme acide, basique ou neutralisée :

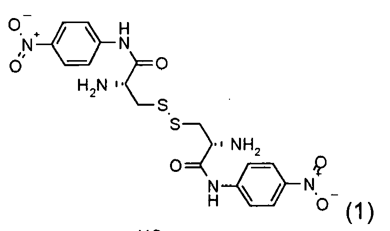

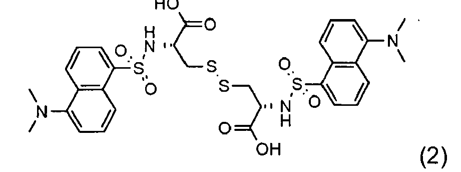

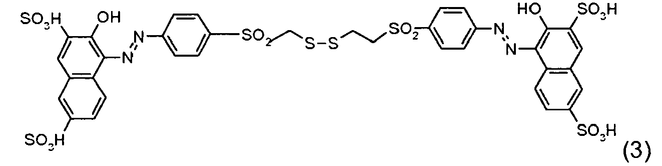

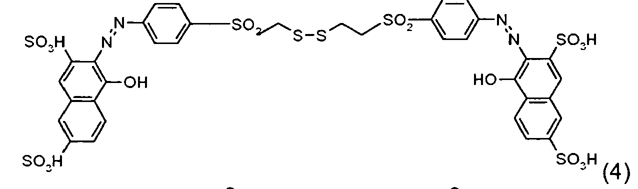

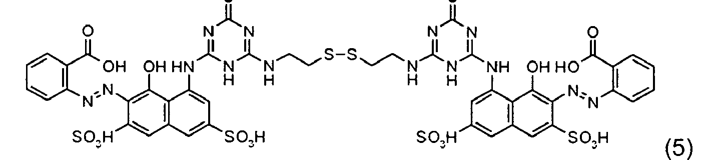

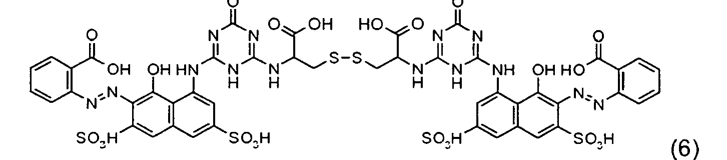

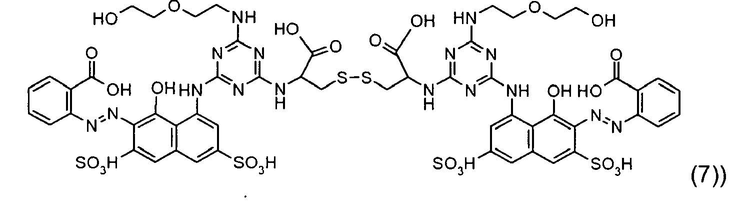

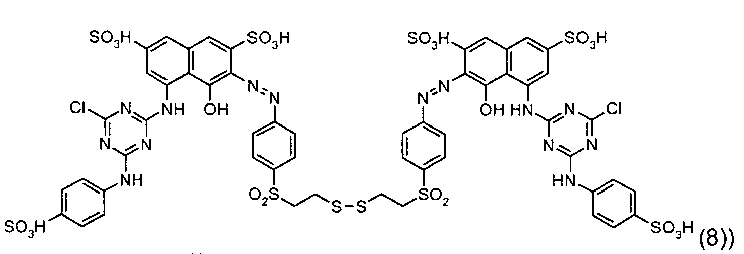

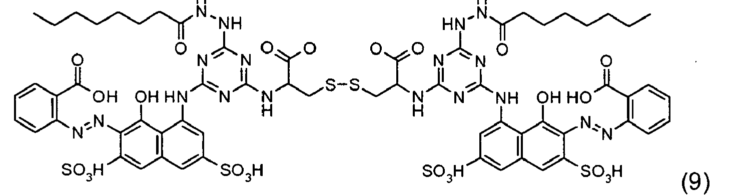

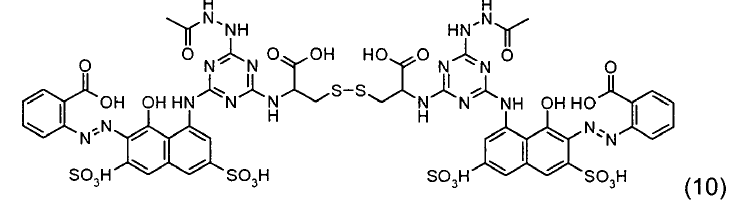

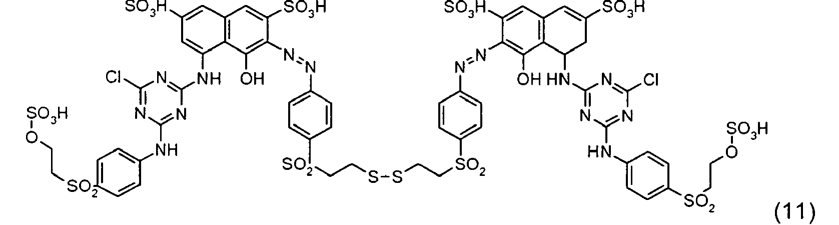

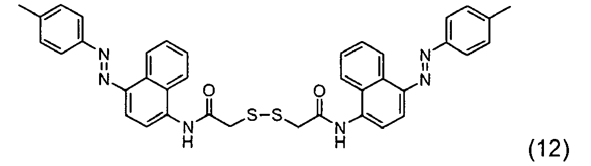

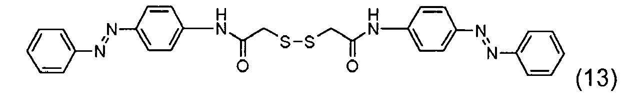

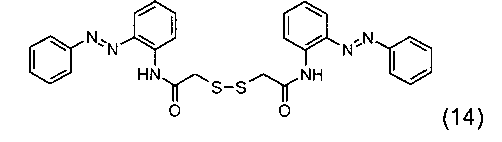

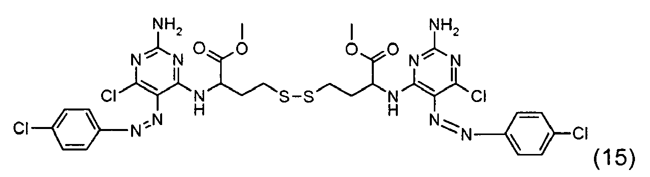

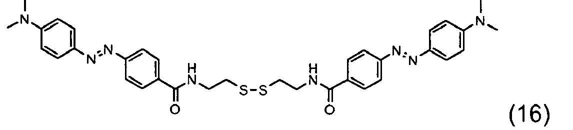

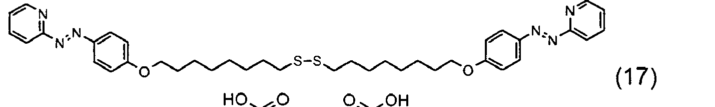

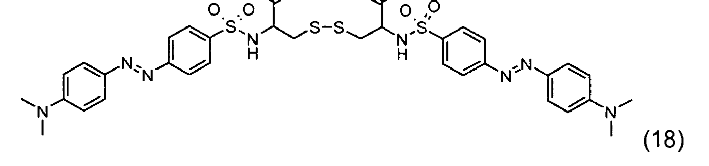

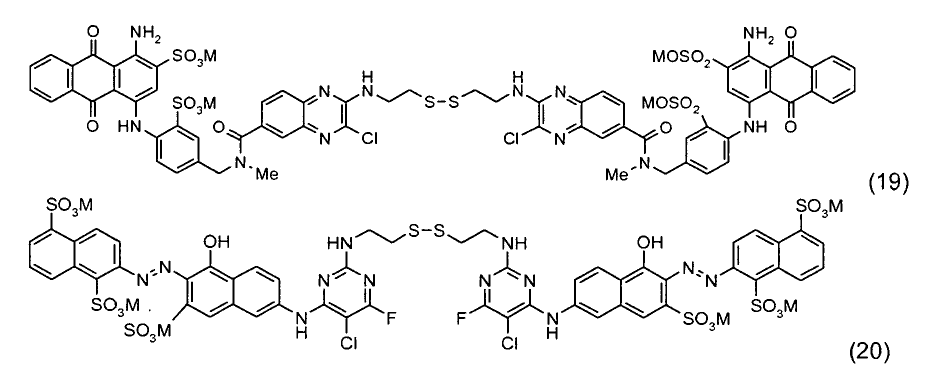

M représentant un atome d'hydrogène, un métal alcalin ou un groupement ammonium ou un groupement ammonium substitué par un ou plusieurs radicaux alkyle, identiques ou non, linéaires ou ramifiés, en C₁-C₁₀ éventuellement porteurs d'au moins un hydroxyle ;

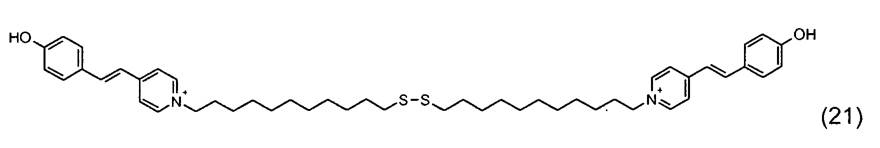

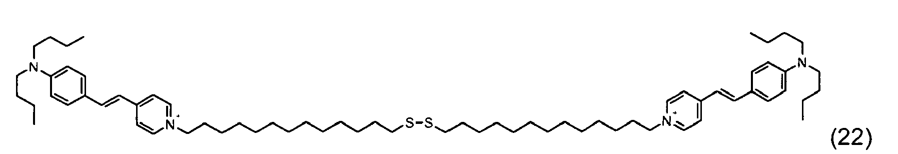

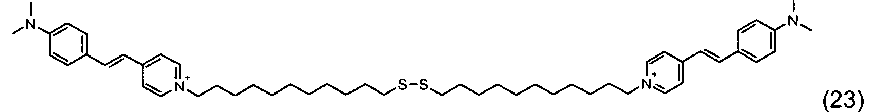

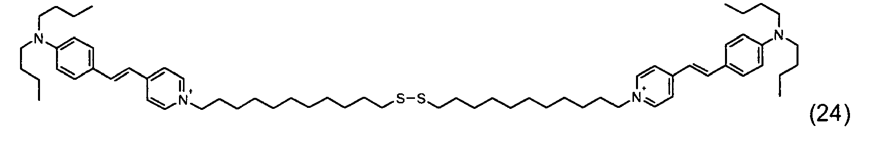

Ces composés particuliers, indépendamment de tout choix de forme libre ou salifiée, ainsi que leur mode de préparation sont connus de la technique.

Conformément à un mode préféré de l'invention, dans les formules (I), (II) ou (III), A et/ou A' comprennent un chromophore cationique.

A titre d'exemples, on peut citer les composés de formule (21) à (24) ci-dessus, leurs sels, hydrates ou solvates.

Selon une autre variante préférée de l'invention, le colorant direct disulfure est un colorant, fluorescent ou non, cationique comprenant au moins un radical ammonium quaternaire et tel que, dans la formule (I) avec p et p' égaux à 1 :
A et A', identiques ou différents, plus préférentiellement identiques, représentent W-N=N-Ar- ou -W-N=N-Ar ou W-C(R^{c})=C(R^{d})-Ar- ou -W-C(R^{c})=C(R^{d})-Ar, avec W représentant un hétérocycle, aromatique ou non, condensé ou non, comprenant un ammonium quaternaire ; Ar représente un radical (hétéro)aryle à 5 ou 6 chaînons, ou un bicycle (hétéro)aromatique de type naphtyle, benzopyridinium, indolinyle ou benzoindolinyle, éventuellement substitués par un ou plusieurs atomes d'halogène ; par un ou plusieurs groupements alkyle ; par un ou plusieurs groupements hydroxyle ; par un ou plusieurs groupements alcoxy, par un ou plusieurs groupements hydroxyalkyle, par un ou plusieurs groupements amino ou (di)alkylamino, par un ou plusieurs groupements acylamino ; par un ou plusieurs groupements hétérocycloalkyle ou hétéroaryle à 5 ou 6 chaînons ; R^{c}, R^{d}, identiques ou différents, représentent un atome d'hydrogène ou un groupement alkyle en C₁-C₄.

Selon une variante préférée p=p'=1 ; y=z=0 ; t=1 et T représente -N(R)-, de préférence en position para sur Ar par rapport à la fonction azoïque.

De préférence, W est un imidazolium, pyridinium, benzopyridinium, benzimidazolium, quinolinium, pyrazolium, et benzothiazolium éventuellement substitués par un ou plusieurs radicaux alkyles, identiques ou non, en C₁-C₄.

Parmi les colorants directs disulfures préférés de la présente invention, on peut citer notamment les composés suivants : 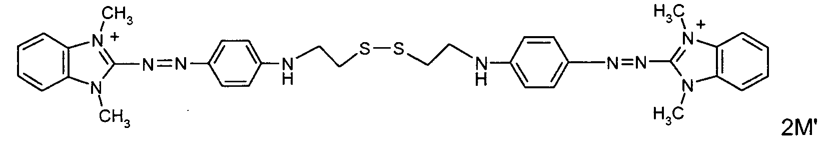 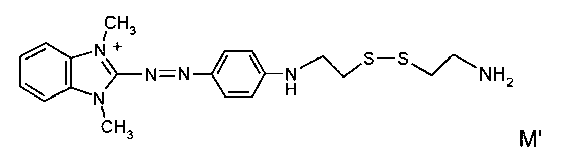 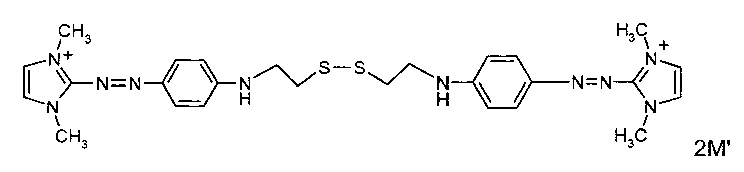 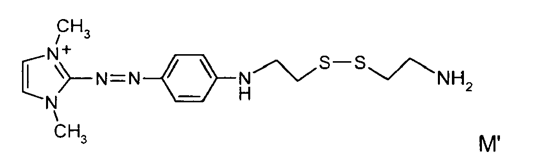 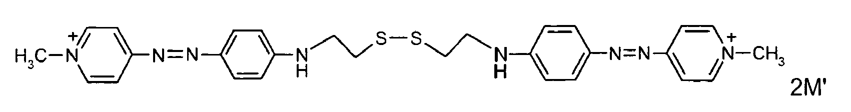 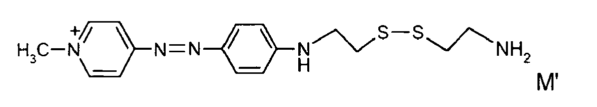 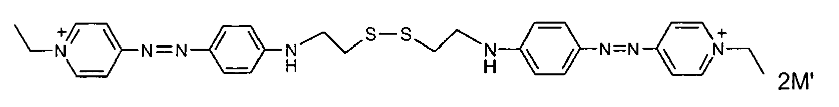 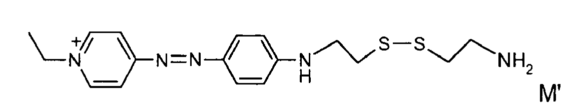 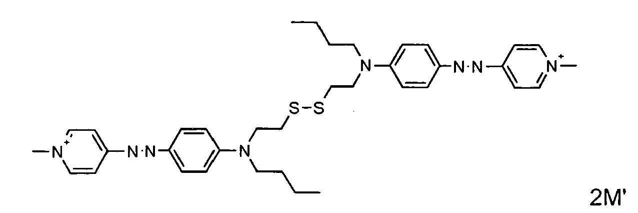 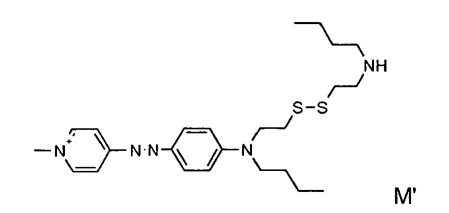 avec M' représentant un sel d'acide organique ou minéral.

Selon une autre variante préférée, le colorant direct disulfure est un colorant fluorescent cationique comprenant au moins un radical ammonium quaternaire et tel que, dans la formule (I) avec p égal à 1 et v égal à 0 :
A représente un radical naphthalimidyle de formule : 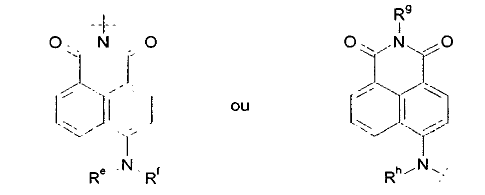 avec 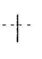 représentant la liaison avec le groupement X ou X', Cₛₐₜ ou C'ₛₐₜ
dans lequel R^{e}, R^{f}, R^{g},et R^{h}, identiques ou différents, représentent un atome d'hydrogène ou un groupement alkyle en C₁-C₆ éventuellement substitué.

Selon une autre variante préférée, le colorant direct disulfure est un colorant fluorescent choisi parmi : 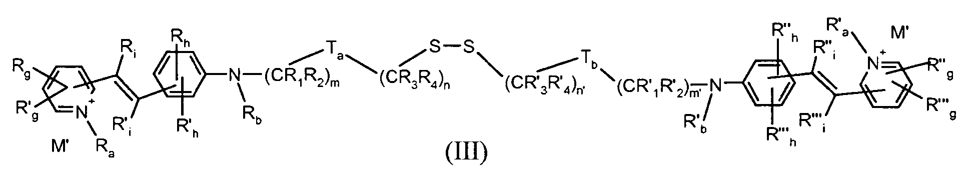 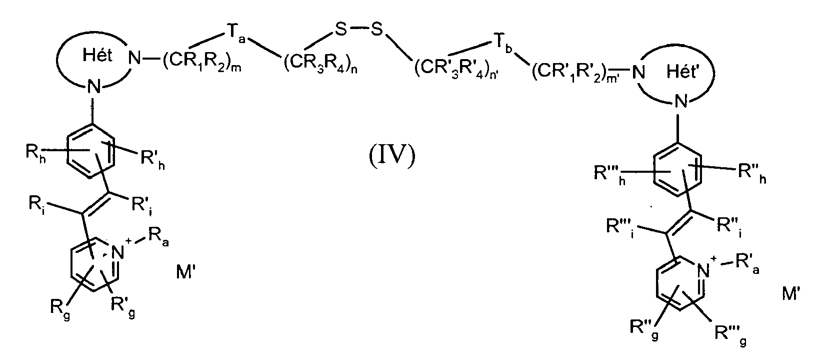 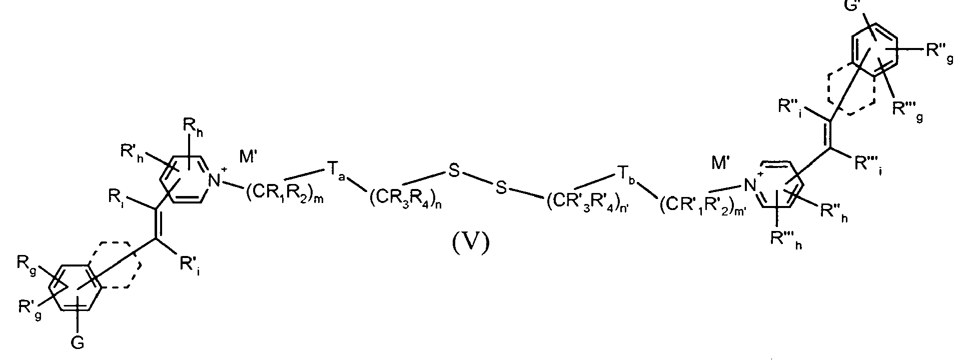 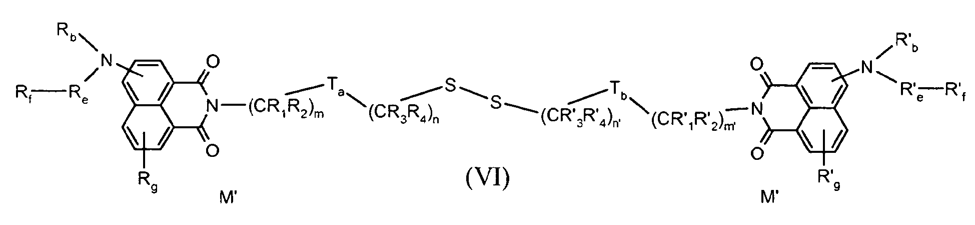 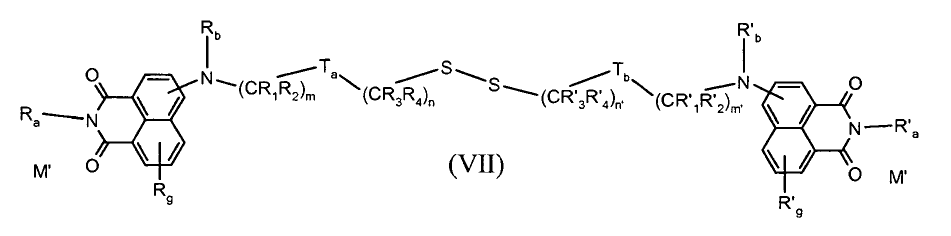 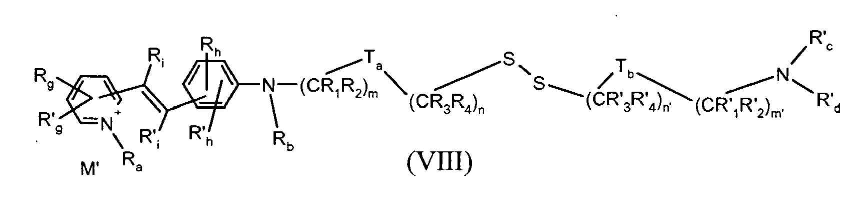 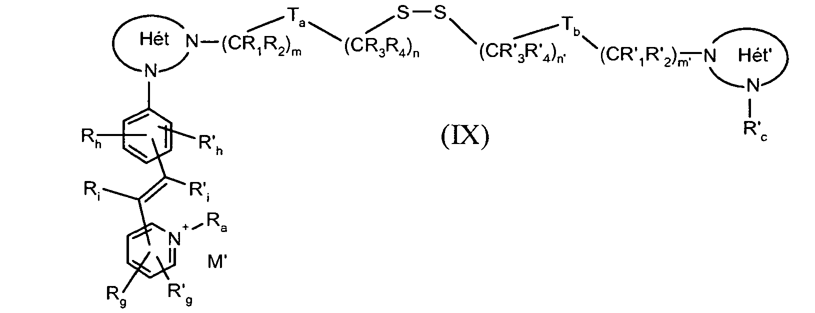 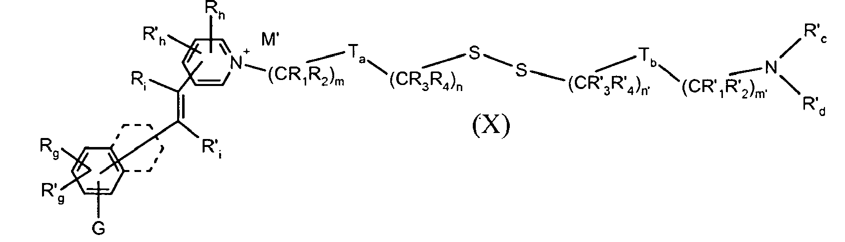 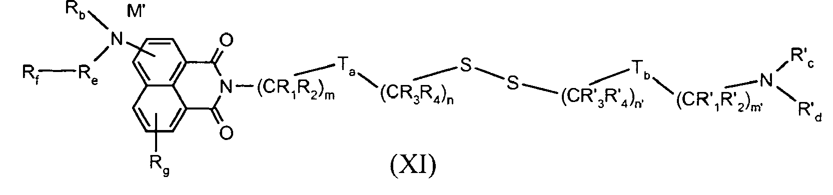 et 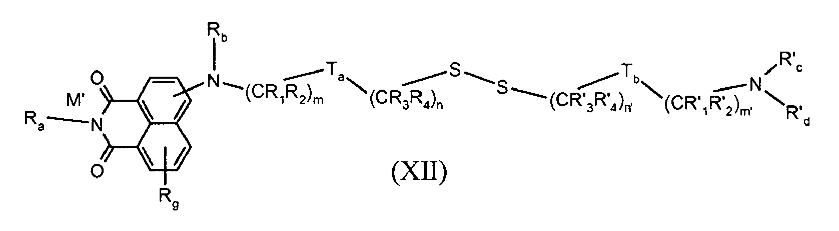 dans lequel
- G et G', identiques ou différents, représentent un groupement -NR_{c}R_{d}, -NR'_{c}R'_{d}, ou C₁-C₆ alkoxy éventuellement substitué, préférentiellement non substitué ; préférentiellement G et G' représentent un groupement -NR_{c}R_{d} et -NR'_{c}R'_{d} respectivement ;
- Rₐ et R'ₐ, identiques ou différents, un groupement aryl(C₁-C₄)alkyle ou un groupement C₁-C₆ alkyle éventuellement substitué par un groupement hydroxyle ou amino, C₁-C₄ alkylamino, C₁-C₄ dialkylamino, lesdits radicaux alkyles pouvant former avec l'atome d'azote qui les portent un hétérocyle comprenant de 5 à 7 chaînons, comprenant éventuellement un autre hétéroatome différent ou non de l'azote ; préférentiellement Rₐ et R'ₐ représentent un groupement C₁-C₃ alkyle éventuellement substitué par un groupement hydroxy, ou un groupement benzyle ;
- R_{b,} R'_{b}, identiques ou différents, représentent un atome d'hydrogène, un groupement aryl(C₁-C₄)alkyle ou un groupement C₁-C₆ alkyle éventuellement substitué; préférentiellement R_{b,} R'_{b} représentent un atome d'hydrogène ou un groupement C₁-C₃ alkyle ou benzyle ;
- R_{c}, R'_{c}, R_{d} et R'_{d}, identiques ou différents, représentent un atome d'hydrogène, un groupement aryl(C₁-C₄)alkyle, C₁-C₆ alcoxy ou un groupement C₁-C₆ alkyle éventuellement substitué ; R_{c}, R'_{c}, R_{d} et R'_{d} représentent préférentiellement un atome d'hydrogène, un groupement hydroxy, C₁-C₃ alcoxy, amino, C₁-C₃ (di)alkylamino, ou un groupement C₁-C₃ alkyle éventuellement substitué par i) un groupement hydroxy, ii) amino, iii) C₁-C₃ (di)alkylamino, ou iv) ammonium quaternaire (R")(R'")(R"")N⁺-;
   ou alors deux radicaux adjacents R_{c} et R_{d}, R'_{c} et R'_{d} portés par le même atome d'azote forment ensemble un groupe hétérocyclique ou hétéroaryle ; préférentiellement l'hétérocycle ou l'hétéroaryle est monocyclique et comprend entre 5 et 7 chaînons ; plus préférentiellement les groupements sont choisis parmi l'imidazolyle et le pyrrolidinyle ;
- Rₑ et R'ₑ, identiques ou différents, représentent une chaîne hydrocarbonée en C₁-C₆ alkylènyle divalente, linéaire ou ramifiée, éventuellement insaturée ;
- R_{f} et R'_{f}, identiques ou différents, représentent un groupement ammonium quaternaire (R")(R'")(R"")N⁺- où R", R"' et R"", identiques ou différents, représentent un atome d'hydrogène ou un groupement C₁-C₄ alkyle ou alors (R")(R'")(R"")N⁺- représente un groupement hétéroaryle cationique éventuellement substitué préférentiellement un groupement imidazolinium éventuellement substitué par un groupement C₁-C₃ alkyle ;
- Rg, R'g, R"_{g,} R'"_{g,} R_{h,} R'_{h,} R"_{h,} et R'"_{h,} identiques ou différents, représentent un atome d'hydrogène, un atome d'halogène, un groupement amino, C₁-C₄ alkylamino, C₁-C₄ dialkylamino, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle , alkylsulfonylamino, un radical amino-sulfonyle, ou un radical C₁-C₁₆ alkyle éventuellement substitué par un groupement choisi parmi C₁-C₁₂ alcoxy, hydroxy, cyano, carboxy, amino, C₁-C₄ alkylamino et C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ; préférentiellement Rg, R'g, R"_{g,} R'"_{g,} R_{h,} R'_{h,} R"_{h,} et R'"ₕ représentent un atome d'hydrogène, d'halogène ou un groupement C₁-C₃ alkyle ;
- ou alors deux groupements Rg et R'g ; R"g et R"'g ; Rₕ, et R'ₕ ; R"ₕ et R'"ₕ portés par deux atomes de carbone adjacents, forment ensembles une cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, C₁-C₄ alkylamino, C₁-C₄ dialkylamino, nitro, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle, alkylsulfonylamino, un radical amino-sulfonyle, ou un radical C₁-C₁₆ alkyle éventuellement substitué par : un groupement choisi parmi C₁-C₁₂ alcoxy, hydroxy, cyano, carboxy, amino, C₁-C₄ alkylamino, C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ; préférentiellement Rg et R'g; R"g et R"'g forment ensemble un groupement benzo ;
- ou alors lorsque G représente -NR_{c}R_{d} et G' représente -NR'_{c}R'_{d} deux groupements R_{c} et R'g ; R'_{c} et R"g ; R_{d} et Rg ; R'_{d} et R"'g forment ensemble un hétéroaryle ou hétérocycle saturé, éventuellement substitué par un ou plusieurs groupement C₁-C₆ alkyle, préférentiellement un l'hétérocycle contenant un ou deux hétéroatomes choisis parmi l'azote et l'oxygène et comprenant entre 5 et 7 chaînons ; plus préférentiellement l'hétérocycle est choisi parmi les groupement morpholinyle, pypérazinyle, pypéridinyle et pyrrolidinyle ;
- R_{i,} R'_{i,} R"_{i,} et R'"ᵢ, identiques ou différents, représentent un atome d'hydrogène, ou un groupement C₁-C₄ alkyle ;
- R₁, R₂, R₃, R₄, R'₁, R'₂, R'₃, et R'₄, identiques ou différents, représentent un atome d'hydrogène ou un groupement C₁-C₄ alkyle, C₁-C₁₂ alcoxy, hydroxy, cyano, carboxy, amino, C₁-C₄ alkylamino, C₁-C₄ dialkylamino, lesdits radicaux alkyles pouvant former avec l'atome d'azote qui les portent un hétérocyle comprenant de 5 à 7 chaînons, comprenant éventuellement un autre hétéroatome différent ou non de l'azote ; préférentiellement R₁, R₂, R₃, R₄, R'₁, R'₂, R'₃, et R'₄ sont des atomes d'hydrogène ou un groupement amino ; plus préférentiellement R₁, R₂, R₃, R₄, R'₁, R'₂, R'₃, et R'₄ représentent un atome d'hydrogène;
- Tₐ, T_{b}, identiques ou différents, représentent i) soit une liaison covalente σ, ii) soit un ou plusieurs radicaux ou leurs combinaisons choisis parmi -SO₂-, -O-, -S-, -N(R)-, -N⁺(R)(R°)-, -CO-, avec R, R°, identiques ou différents, représentant un atome d'hydrogène, un radical alkyle en C₁-C₄, hydroxyalkyle en C₁-C₄; ou un aryl(C₁-C₄)alkyle, préférentiellement Tₐ est identique à T_{b} et représentent une liaison covalente σ ou un groupement choisi parmi -N(R)-, -C(O)-N(R)-, - N(R)-C(O)-, -O-C(O)-, -C(O)-O- et -N⁺(R)(R°)-, avec R, R° identiques ou différents représentant un atome d'hydrogène ou un groupement C₁-C₄ alkyle ; plus préférentiellement Tₐ et T_{b} représentent une liaison σ ; iii) soit un radical hétérocycloalkyle ou hétéroaryle, cationique ou non, préférentiellement monocycliques, préférentiellement identiques, contenant préférentiellement deux hétéroatomes (plus préférentiellement deux atomes d'azote) et comportant préférentiellement de 5 à 7 chaînons tel que l'imidazolium;
- 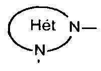 ou 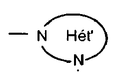 identiques ou différents, représentent une groupement hétérocyclique éventuellement substitué ; préférentiellement les hétérocycles sont identiques, monocycliques, saturés, et comprennent au total deux atomes d'azote et de 5 à 8 chaînons ;
- représentent une groupement aryle ou hétéroaryle fusionné au cycle phényle ; ou alors est absent du cycle phényle; préférentiellement lorsque le cycle est présent le cycle est un benzo ;
- m, m', n et n', identiques ou différents, représentent un entier compris inclusivement entre 0 et 6 avec m+n, m'+n', identiques ou différents, représente un entier compris inclusivement entre 1 et 10; préférentiellement m+n=m'+n'=un entier compris inclusivement entre 2 et 4 ; plus préférentiellement m+n=m'+n'= un entier égal à 2;
M' représentant un sel d'acide organique ou minéral.

A titre d'exemples de colorants directs disulfures fluorescents, on peut encore citer notamment les composés suivants : 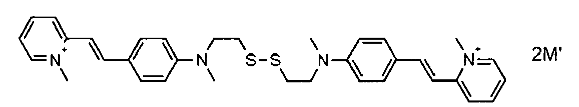 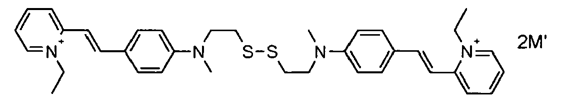 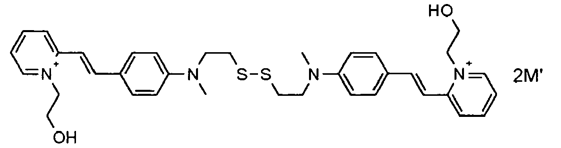 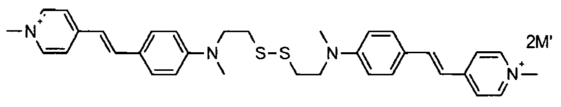 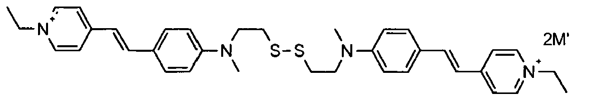 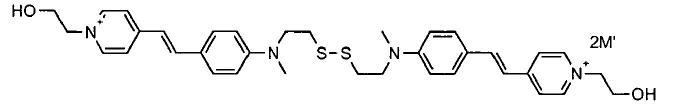 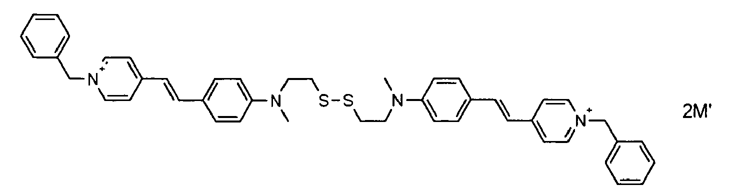 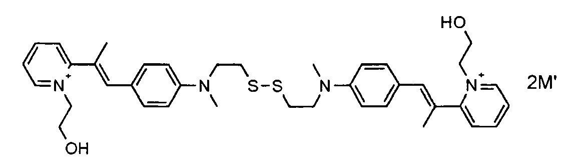 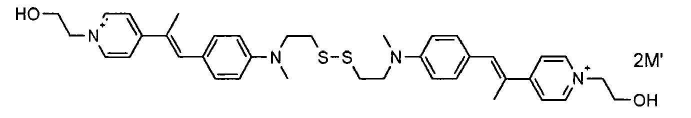 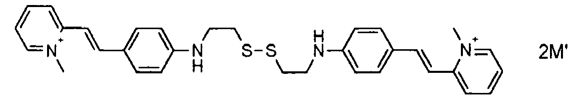 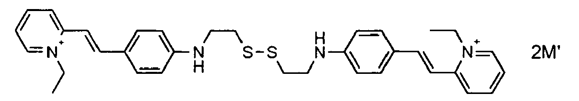 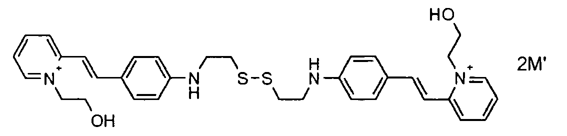 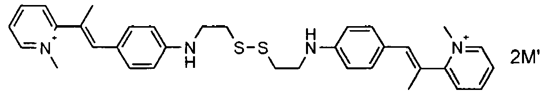 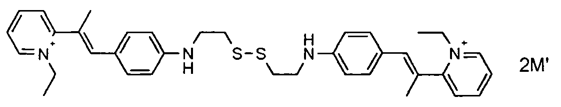 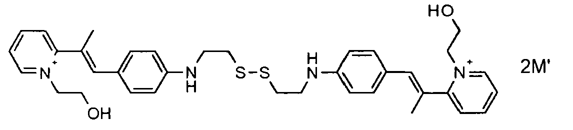 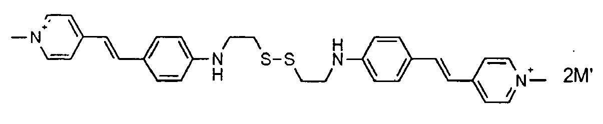 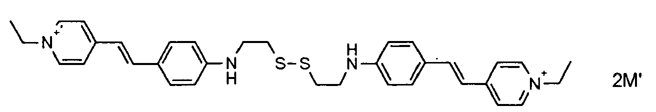 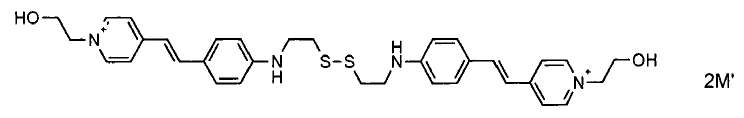 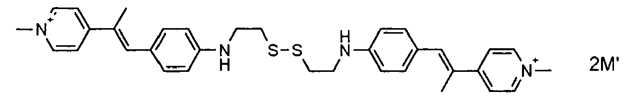 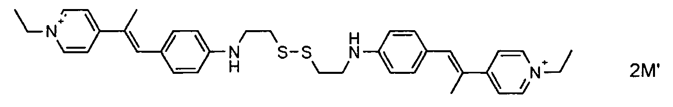 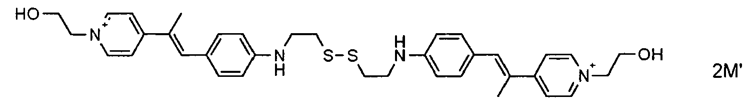 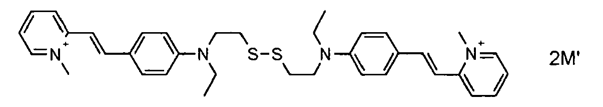 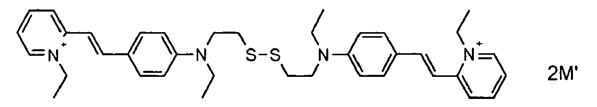 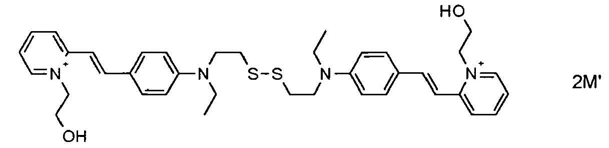 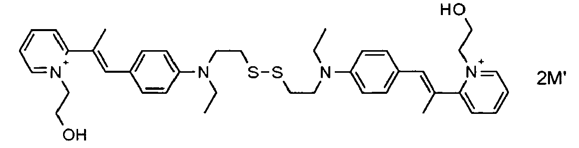 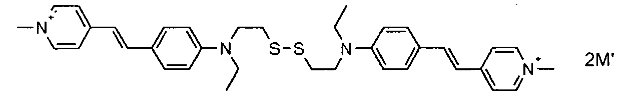 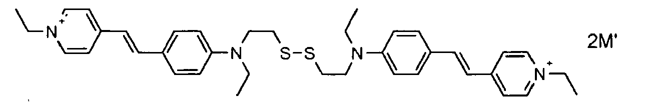 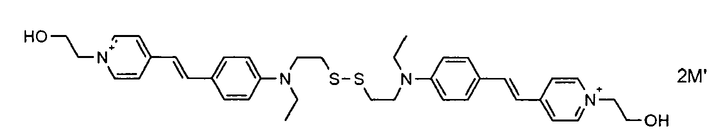 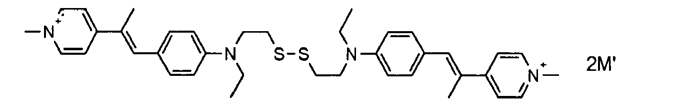 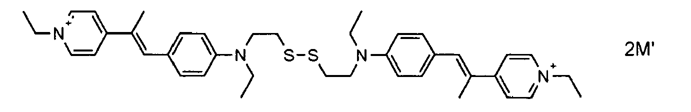 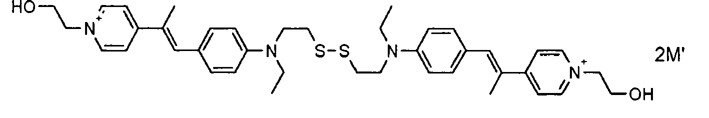 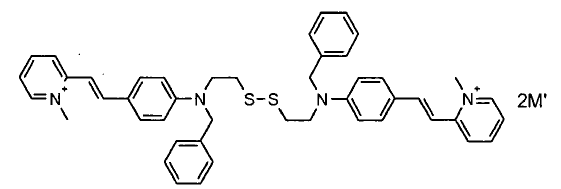 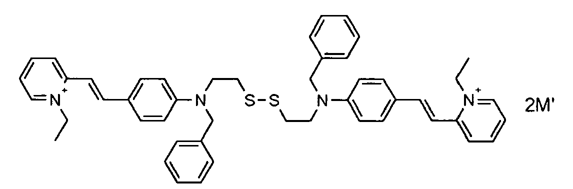 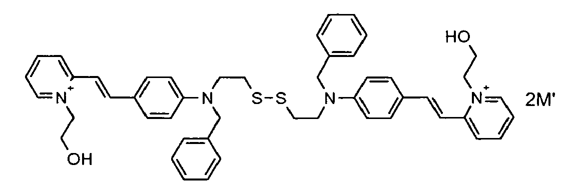 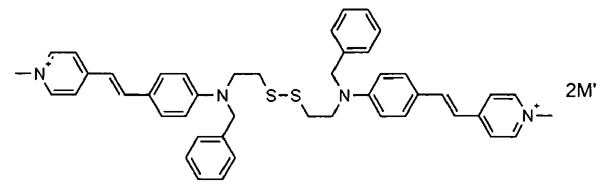 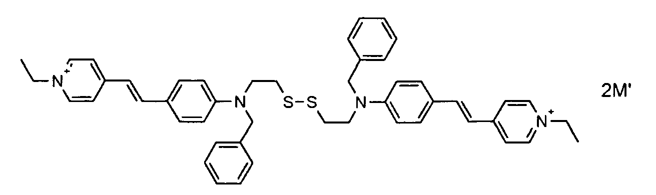 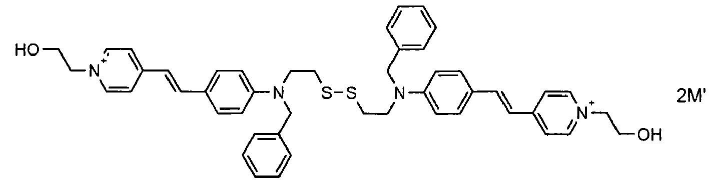 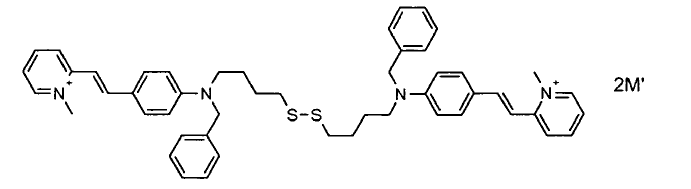 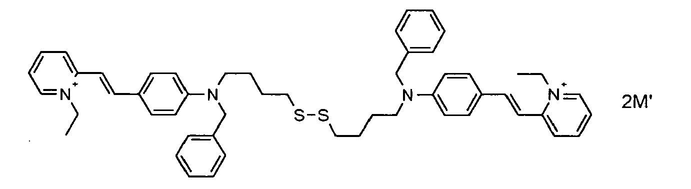 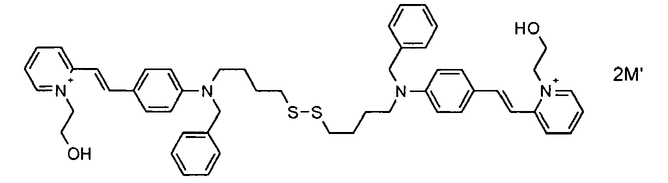 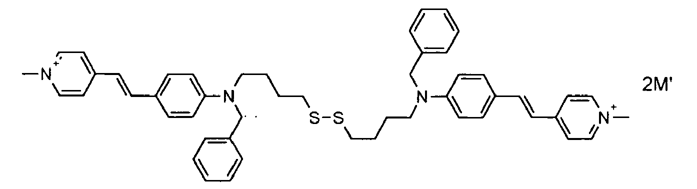 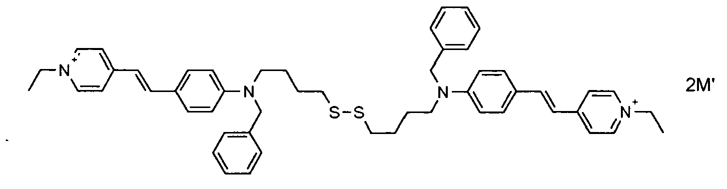 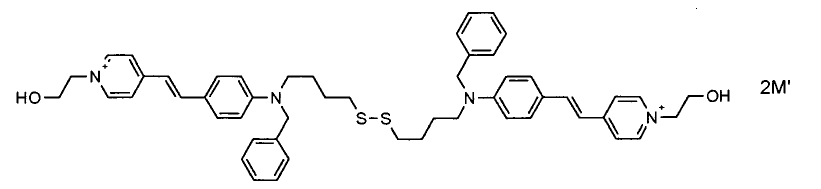 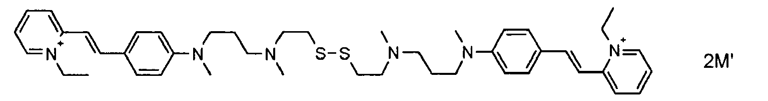 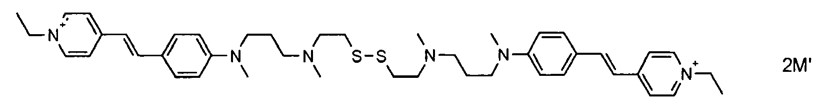 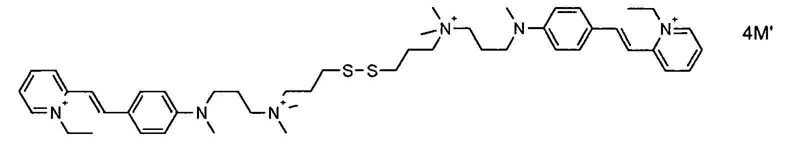 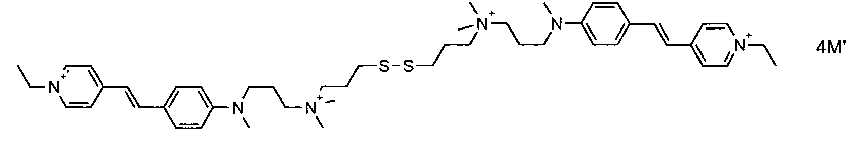 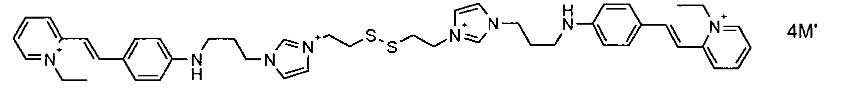 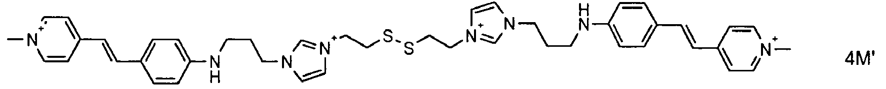 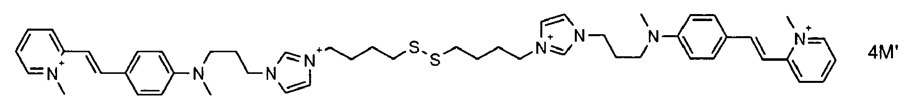 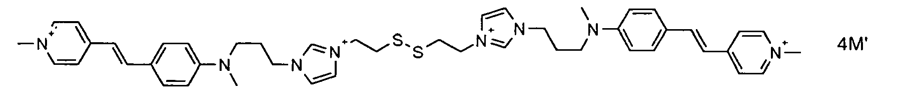 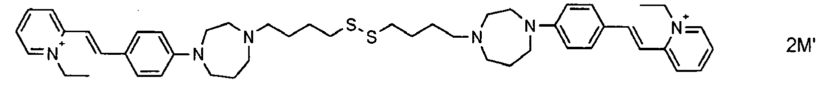 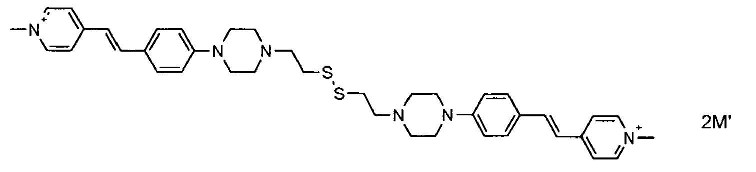 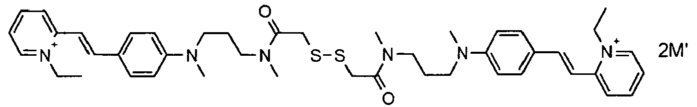 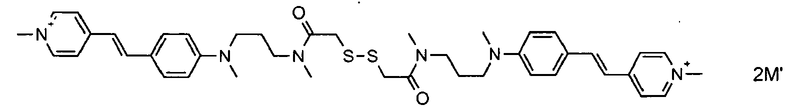 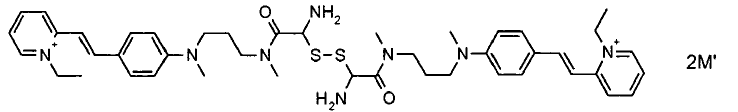 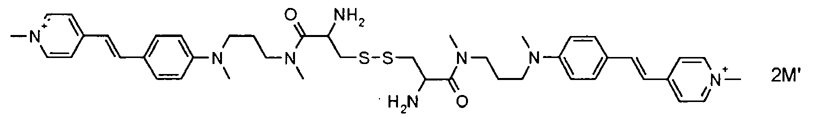 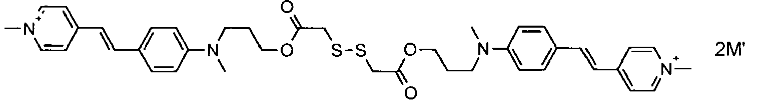 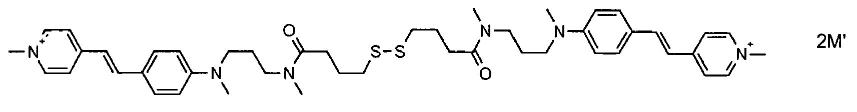 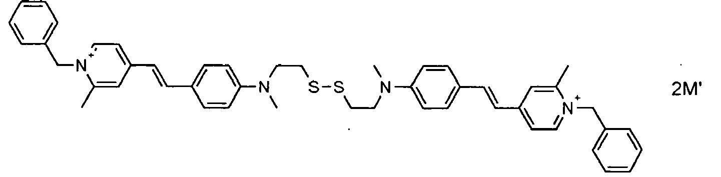 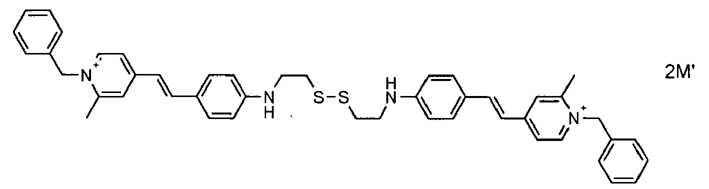 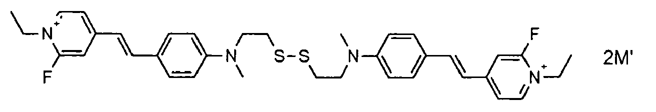 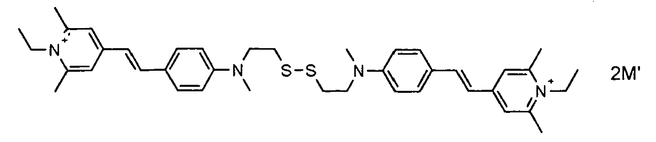 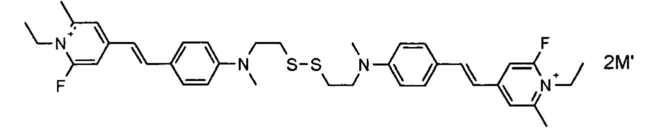 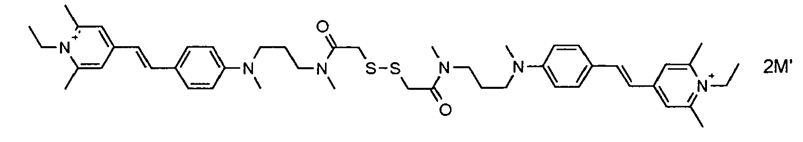 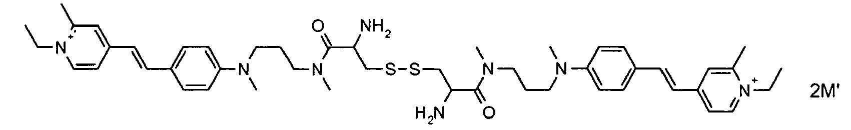 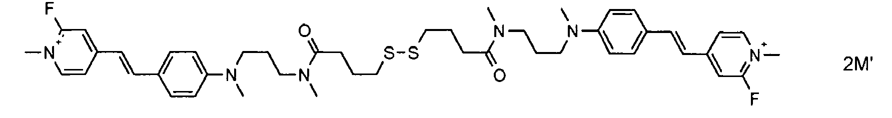 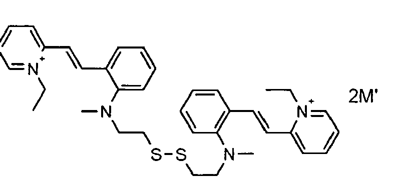 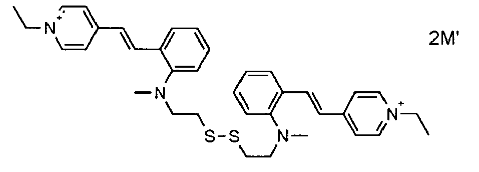 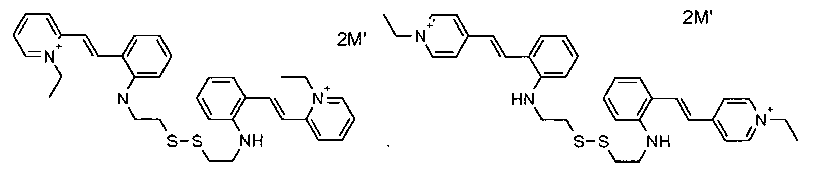 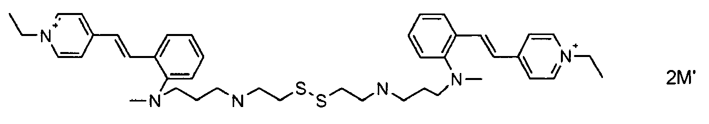 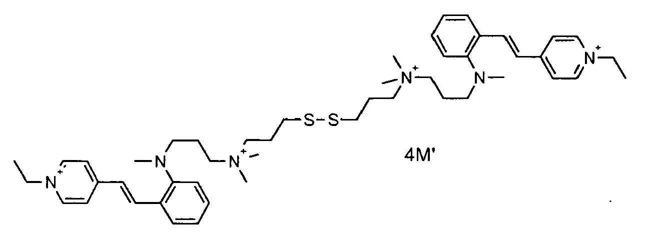 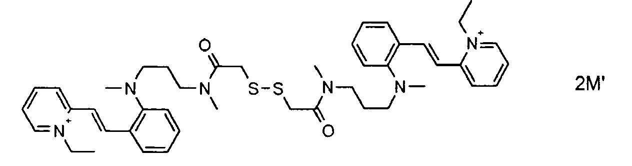 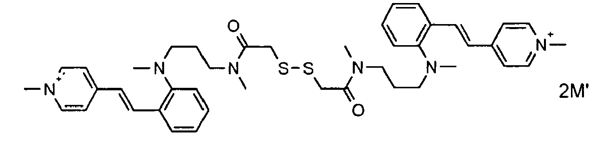 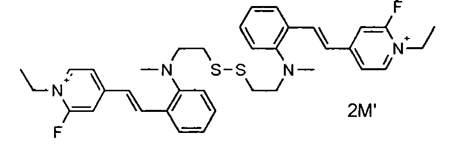 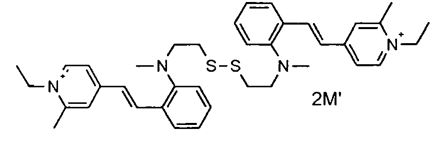 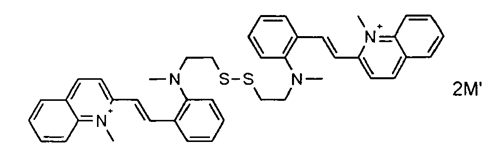 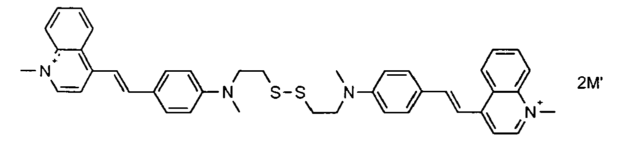 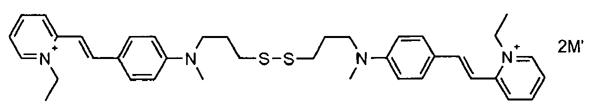 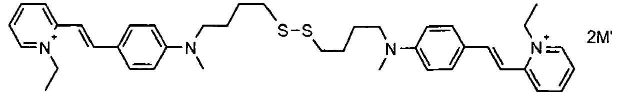 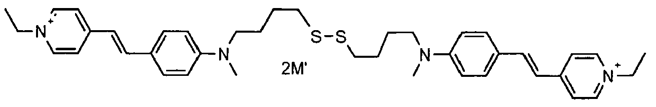 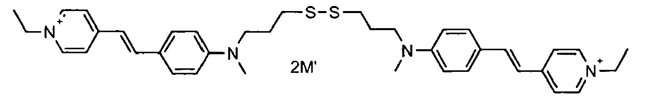 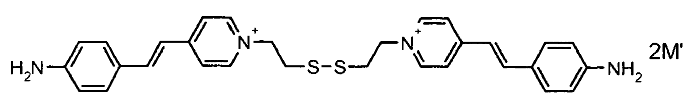 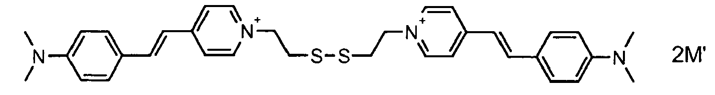 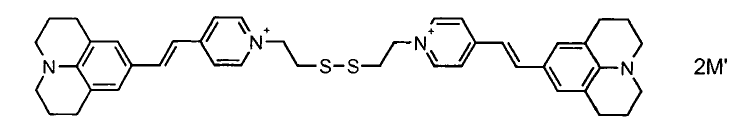 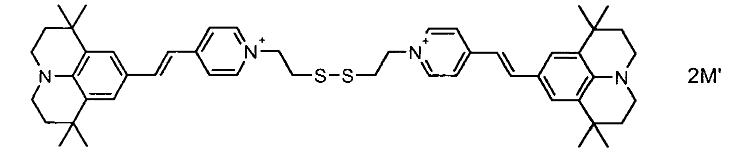 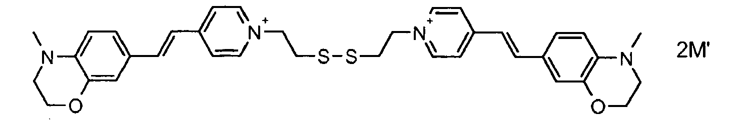 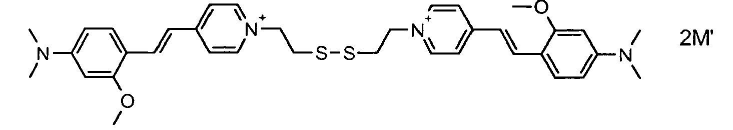 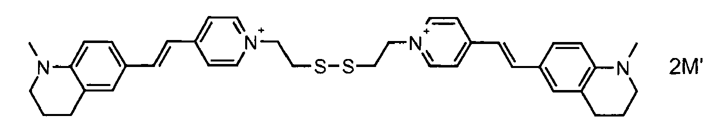 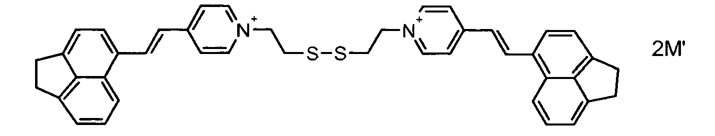 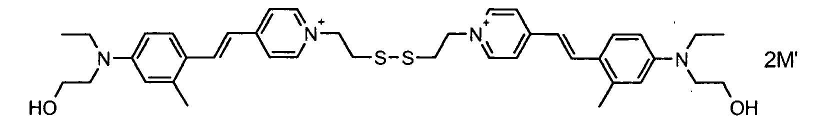 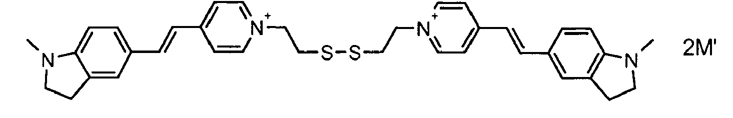 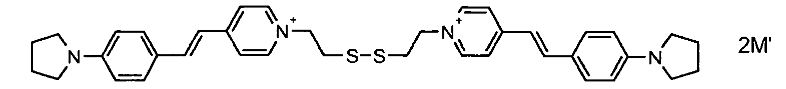 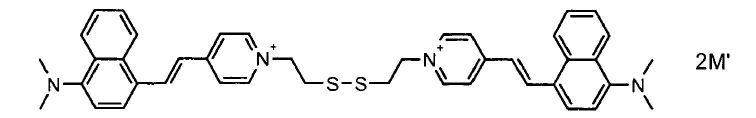 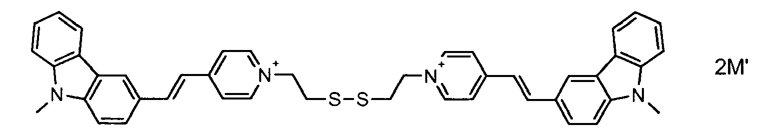 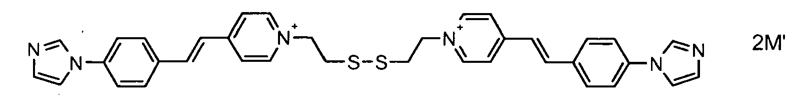 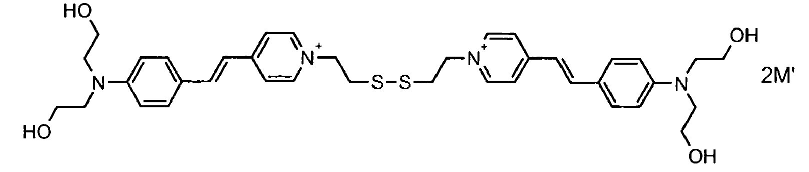 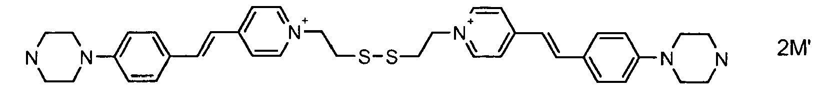 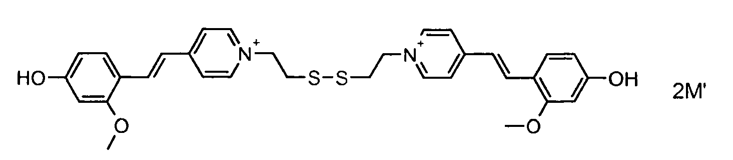 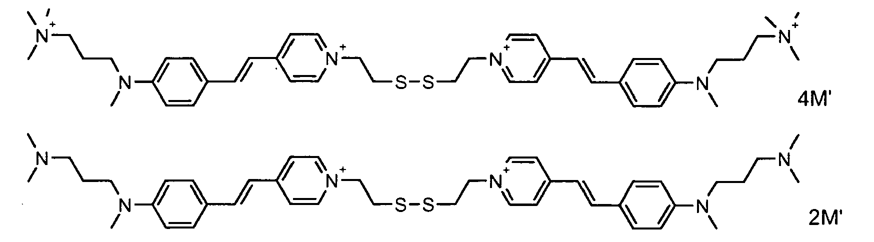 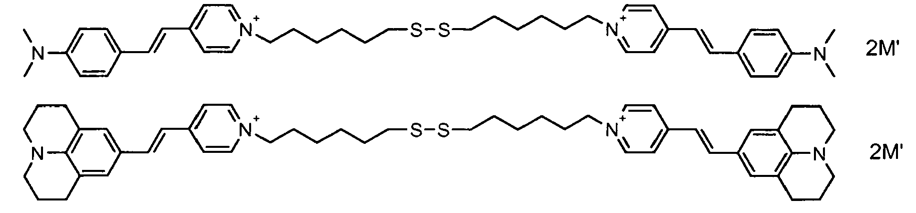 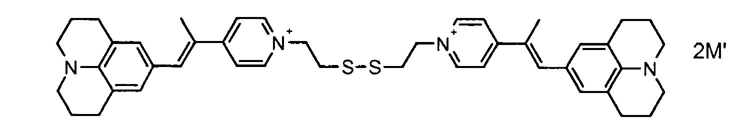 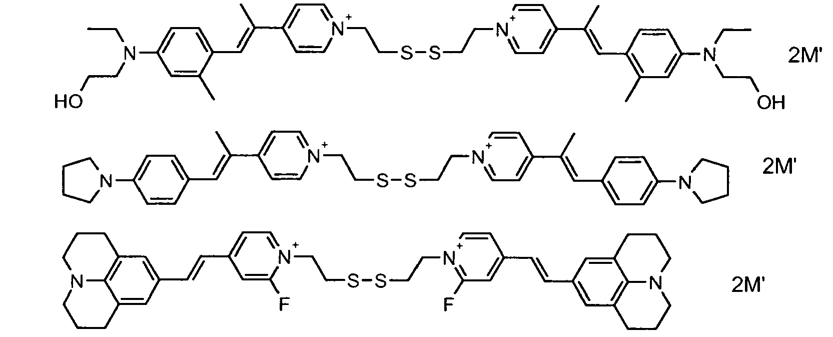 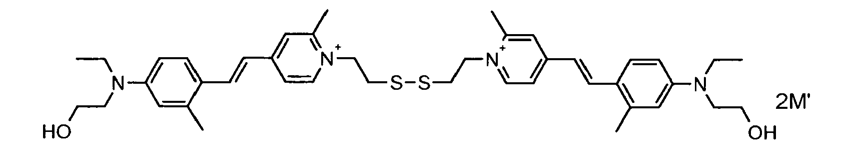 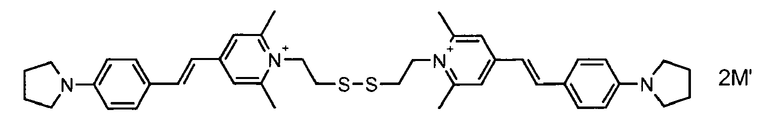 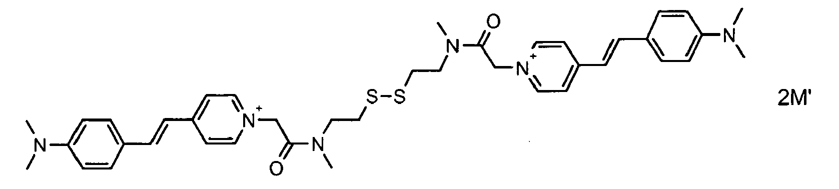 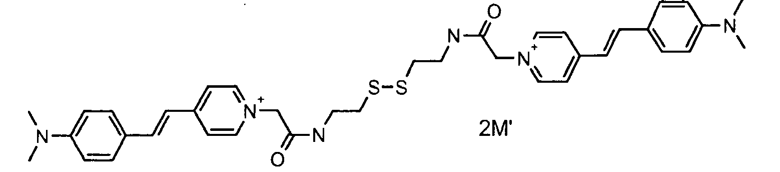 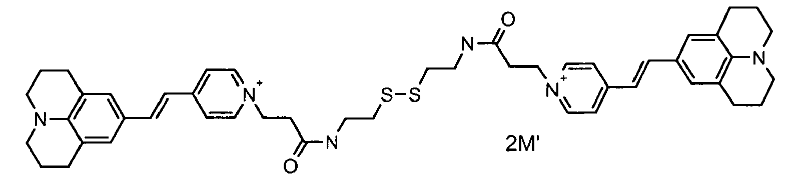 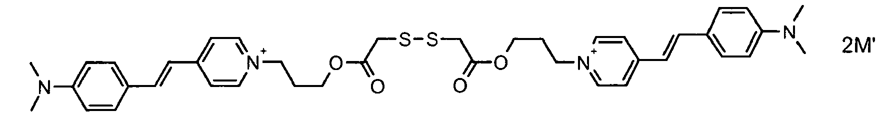 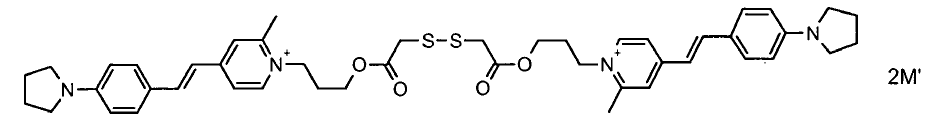 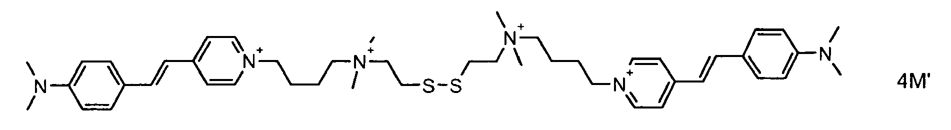 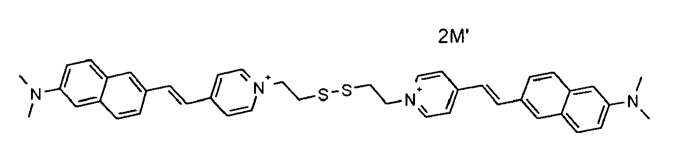 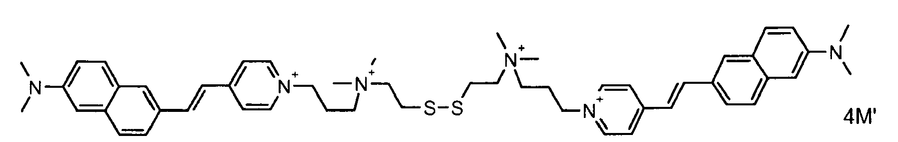 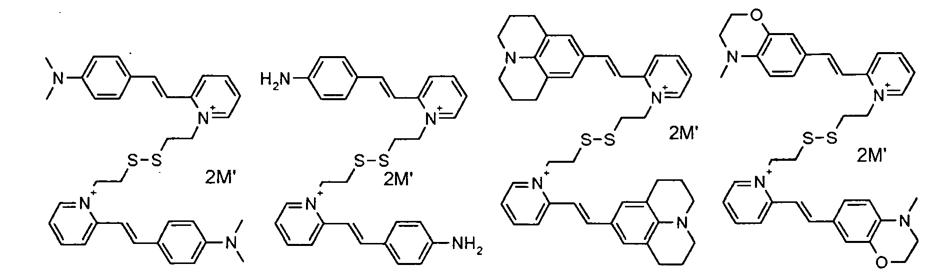 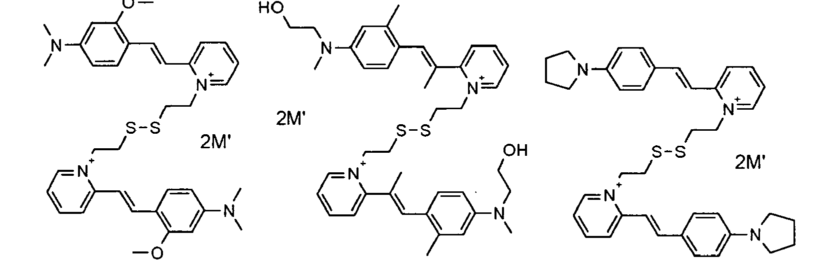 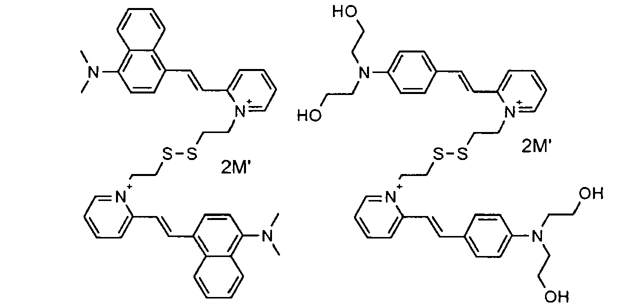 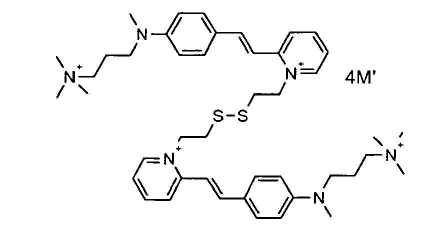 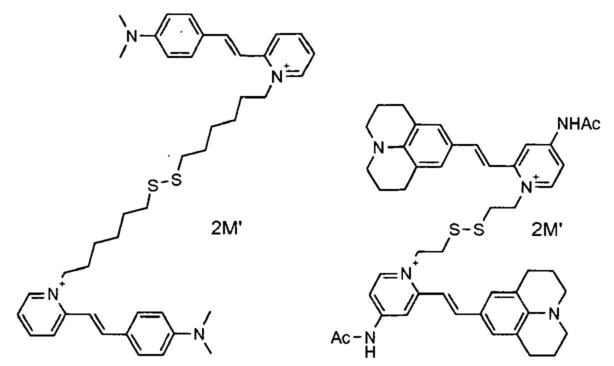 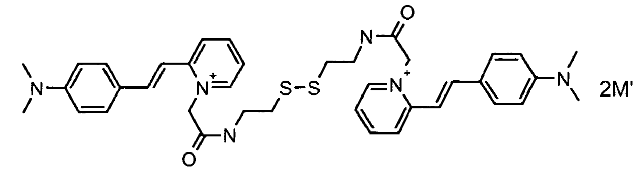 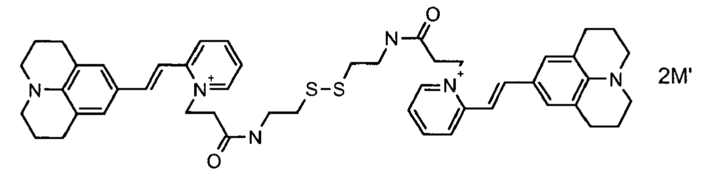 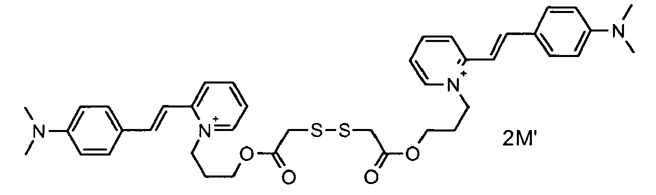 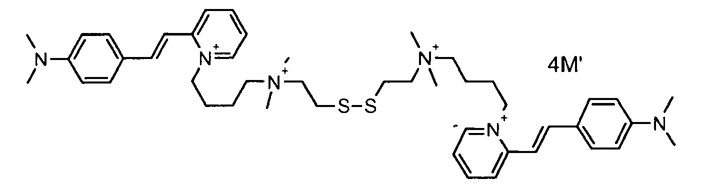 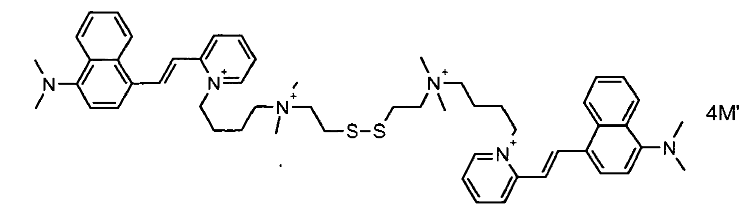 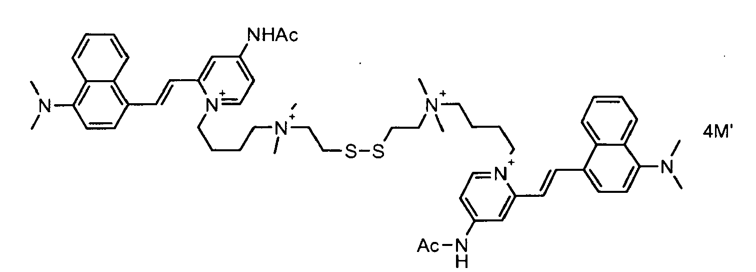 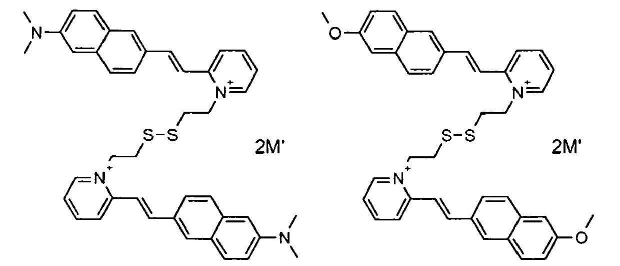 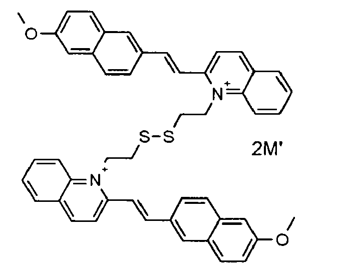 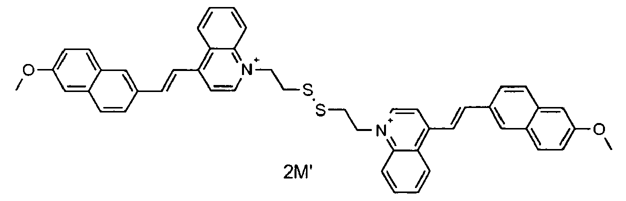 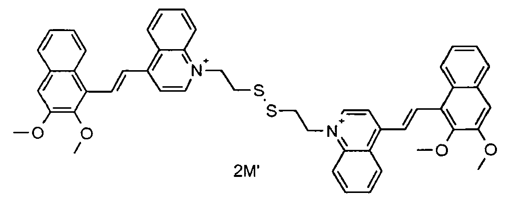 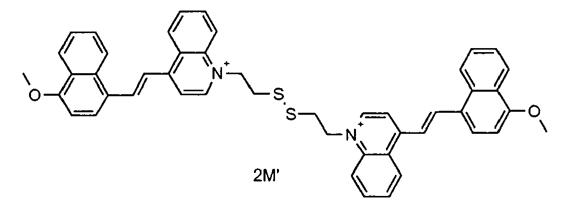 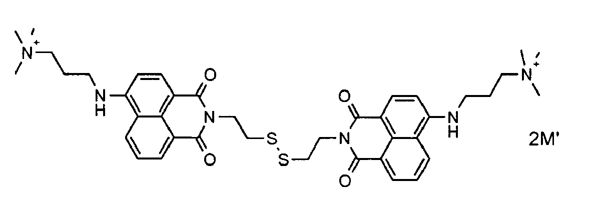 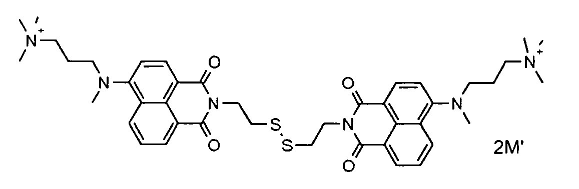 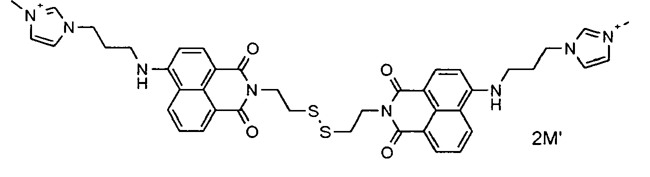 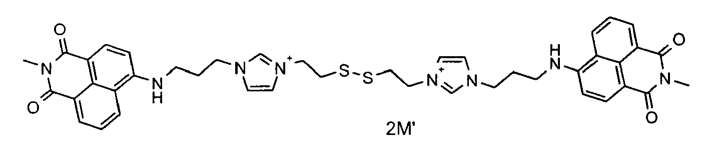 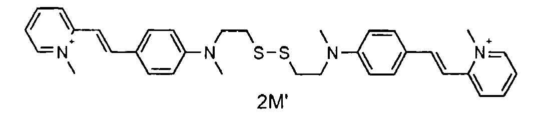 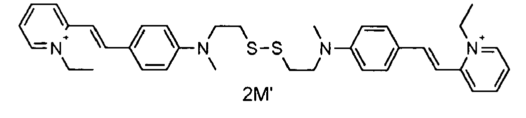 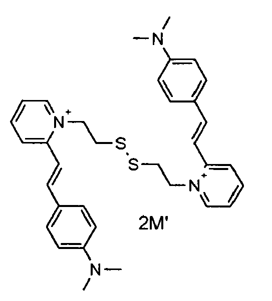 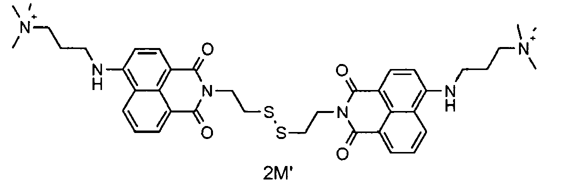 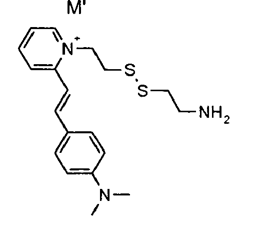 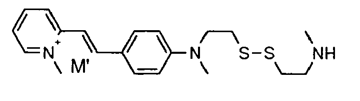 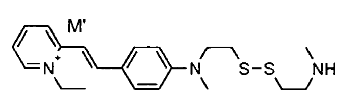 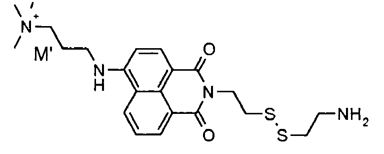 et 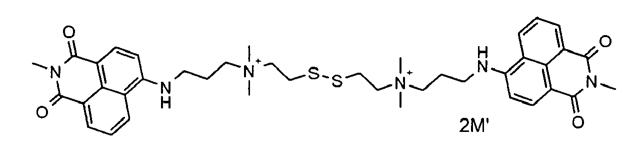 avec M' représentant un sel d'acide organique ou minéral.

Selon une seconde variante préférée, à titre de colorants directs disulfures, on peut utiliser ceux décrits dans les demandes de brevet GB 2412916, WO 05097051, WO 06134043, WO 06134051, WO 06136516, WO 06136518, WO 06136617.

### I.5. Le sel d'acide organique ou minéral:

Le sel d'acide organique ou minéral est plus particulièrement choisi parmi les chlorhydrates, les bromhydrates, les sulfates, parmi lesquels le méthylsulfate et l'éthylsulfate, les citrates, les succinates, les tartrates, les lactates, les méthosulfates, les tosylates, les benzènesulfonates, les phosphates, les acétates, triflates et tétrafluoroborates.

### I.6. Préparation des colorants directs disulfures, fluorescents ou non :

Les colorants directs disulfures peuvent être préparés selon des méthodes connues de l'homme de l'art.

Selon une première possibilité, on peut faire réagir un composé disulfure comprenant deux fonctions amine, de préférence primaire ou secondaire avec une quantité suffisante d'un "chromophore réactif fluorescent ou non" ou d'un composé comprenant un tel "chromophore réactif fluorescent ou non ", en d'autres termes comprenant une fonction électrophile.

Parmi les "chromophores réactifs", on peut citer les colorants réactifs comportant notamment une fonction vinylsulfone, sulfatoéthylsulfone, mono- di-chlorotriazine, mono- di-chloro pyrimidine, difluoro chloro pyrimidine, dichloroquinoxaline, bromo-vinylsulfone.

Conviennent aussi, en tant que chromophores réactifs, les composés chromophores comprenant au moins un groupement susceptible de réagir avec une fonction amine pour donner un groupement sulfamide (-SO₂-NR-), amide (-CO-NR-). Par exemple, on peut mentionner les groupes -SO₃W', -COOW' (avec W' représentant un atome d'hydrogène, un métal alcalin, comme le sodium, le potassium, un groupement ammonium, un groupement ammonium substitué par un ou plusieurs groupement alkyle, identiques ou non, linéaires ou ramifiés, en C₁-C₁₀, éventuellement porteurs d'au moins un hydroxyle), que l'on peut activer préalablement, selon des méthodes connues, respectivement en groupement -S0₂Cl, -COCl.

Il est ainsi envisageable de mettre en oeuvre, à titre de chromophore réactif, les colorants acides du Colour Index répertoriés comme tels.

On pourra se référer notamment à l'ouvrage Advanced Organic Chemistry, March, 4ème Ed, pour avoir plus de détails sur les conditions opératoires mises en oeuvre.

Toujours dans le cadre de cette première possibilité, on peut mettre en oeuvre des chromophores comprenant un groupement labile directement lié ou non au chromophore et susceptible d'être substitué par un groupement amine, tel que Cl, Br, F, O-alkyle (par exemple O-Me), O-aryle, O-alkylaryle (par exemple O-benzyle).

Les colorants directs disulfures peuvent aussi être obtenus, dans le cadre de cette possibilité, en utilisant des chromophores possédant une fonction acrylate (-OCO-C=C-) sur laquelle on effectue une réaction d'addition.

Conformément à une autre possibilité, les colorants directs disulfures peuvent être obtenus en faisant réagir un composé disulfure avec un composé portant deux fonctions acide carboxylique activées selon les méthodes classiques (par exemple réaction avec un carbodiimide ou avec le chlorure de thionyle). Le produit résultant est ensuite mis à réagir avec un chromophore porteur d'une fonction nucléophile, par exemple de type amine primaire ou secondaire, ou de type alcool aliphatique ou aromatique comme le phénol.

Là encore, on pourra se référer à l'ouvrage Advanced Organic Chemistry, March, 4ème Ed, pour avoir plus de détails sur les conditions opératoires mises en oeuvre.

Conformément à une troisième possibilité, les colorants directs disulfures peuvent être obtenus par réaction d'un composé comprenant un groupement disulfure et deux groupements hydroxyle activés préalablement en groupements partants (par exemple mésylate, tosylate) avec un chromophore portant une fonction nucléophile, avantageusement de type amine primaire, secondaire ou tertiaire, hétéroaromatique ou non, par exemple de type pyridine, imidazole, benzimidazole.

Conformément à une quatrième possibilité, les colorants directs disulfures peuvent être obtenus par oxydation ménagée de colorants portant une fonction SH.

Conformément à une cinquième possibilité, et notamment pour la préparation des composés répondant à la formule (II) et (III), les colorants directs disulfures peuvent être obtenus par une variante des possibilités une, deux ou trois décrites ci-dessus, en utilisant une quantité molaire de réactif disulfure supérieure ou égale à la quantité molaire de réactif contenant le groupement chromophore.

La préparation des colorants directs disulfures répondant à la formule (I) pour lesquels A et A' sont identiques est en revanche facilitée par l'emploi d'une quantité molaire de réactif contenant le groupement chromophore de préférence supérieure ou égale à deux fois la quantité de réactifs disulfures.

Conformément à une sixième possibilité, et notamment pour la préparation des composés répondant à la formule (I) dans laquelle v=0 et les deux groupements A, A' d'une part et X et X' d'autre part, sont différents, les composés disulfures peuvent être obtenus à partir de composés disulfures répondant à la formule (III).

### II. Composition tinctoriale:

### II.1. Colorants :

La composition tinctoriale selon l'invention peut contenir un ou plusieurs colorants directs disulfures. La composition contient de préférence une quantité de colorant direct disulfure comprise entre 0,001 et 10% en poids et encore plus préférentiellement entre 0,005 et 5% en poids par rapport au poids total de la composition.

La composition tinctoriale peut en outre contenir des colorants directs additionnels, exempts de liaison disulfure, de nature non ionique, cationique ou anionique ou leurs mélanges.

Le ou les colorants directs additionnels présents dans la composition tinctoriale sont en général présents à une teneur comprise entre 0,0005 et 12 % en poids, de préférence entre 0,005 et 6 % en poids, par rapport au poids total de la composition.

Le milieu approprié pour la teinture, appelé aussi support de teinture, est un milieu cosmétique généralement constitué par de l'eau ou par un mélange d'eau et d'au moins un solvant organique. A titre de solvant organique, on peut par exemple citer les alcanols inférieurs en C₁-C₄, tels que l'éthanol et l'isopropanol, ainsi que les alcools aromatiques comme l'alcool benzylique ou le phénoxyéthanol, les polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore le glycérol. On peut également utiliser comme solvants les polyéthylèneglycols et les polypropylèneglycols, et les mélanges de ces composés. On peut aussi utiliser le carbonate de propylène

Les solvants organiques, lorsqu'ils sont présents sont, de préférence présents dans des proportions de préférence comprises entre 1 et 40 % en poids environ par rapport au poids total de la composition tinctoriale, et encore plus préférentiellement entre 5 et 30 % en poids environ.

### II.2. Agent alcalin :

La composition selon l'invention comprend en outre un ou plusieurs agents alcalins de type hydroxyde minéral ou organique en quantité telle que le pH de la composition est compris entre 10 et 14, de préférence entre 12 et 14.

Plus particulièrement, l'agent alcalin de type hydroxyde est choisi parmi les hydroxydes de métal alcalin, alcalino-terreux, de métaux de transition, en particulier des groupes IIIB, IVB, VB et VIB, de lanthanides ou d'actinides, les hydroxydes d'ammonium, l'hydroxyde de guanidine, ou leurs mélanges.

A titre d'exemples de tels composés, on peut citer l'hydroxyde de sodium, l'hydroxyde de potassium, l'hydroxyde de lithium, l'hydroxyde de rubidium, l'hydroxyde de césium, l'hydroxyde de francium, l'hydroxyde de magnésium, l'hydroxyde de calcium, l'hydroxyde de strontium, l'hydroxyde de baryum, l'hydroxyde de manganèse, l'hydroxyde de zinc, l'hydroxyde de cérium, l'hydroxyde de lanthane, l'hydroxyde d'actinium, l'hydroxyde de thorium, l'hydroxyde d'aluminium, l'hydroxyde de guanidine et les hydroxydes d'ammonium quaternaires.

Il est à noter que certains hydroxydes, et plus particulièrement l'hydroxyde de guanidine, peuvent se trouver sous la forme de précurseurs, c'est-à-dire d'au moins deux composés qui mis en présence, conduisent par une réaction chimique, à l'hydroxyde de guanidine. A titre d'exemple, on peut donc citer l'association d'un hydroxyde de métal alcalino-terreux, comme par exemple le calcium, avec le carbonate de guanidine.

De préférence, l'agent alcalin de type hydroxyde minéral ou organique est choisi parmi les hydroxydes de sodium, de potassium, de calcium, de guanidine ou leurs mélanges.

De manière encore plus préférée, l'agent alcalin hydroxyde minéral ou organique est choisi parmi les hydroxydes de sodium, de guanidine ou leurs mélanges.

Avantageusement, la quantité d'agent alcalin hydroxyde est comprise entre 0,5 et 10 % en poids, et de préférence entre 1 et 8 % en poids par rapport au poids de la composition.

### II.3. Adjuvants:

La composition peut également comprendre des tensioactifs non-ioniques, anioniques, cationiques ou leurs mélanges. Les tensioactifs utilisés comprennent de préférence une chaîne alkyle ou acyle en C₁₀-C₂₄, plus particulièrement en C₁₂-C₂₄, et de préférence en C₁₂-C₂₂, éventuellement mono- ou poly- oxyalkyléné ou mono- ou poly- glycérolé.

En ce qui concerne les tensioactifs anioniques, on utilise habituellement les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants, seuls ou en mélange :
- les alkylsulfates, les alkyléthersulfates, les alkylamidoéthersulfates, les alkylarylpolyéthersulfates ;
- les alkylsulfonates, les alkylamidesulfonates, les alkyl-arylsulfonates ;
- les alkylsulfosuccinates, les alkyléthersulfosuccinates, les alkylamide-sulfosuccinates ;
- les alkylsulfoacétates ;
- les acylsarconisates ; et les acylglutamates :
- les esters d'alkyle et d'acides polyglycoside-carboxyliques tels que les glucoside-citrates d'alkyle, les polyglycoside-tartrates d'alkyle, et les polyglycoside-sulfosuccinates d'alkyle ;
- les alkylsulfosuccinamates ;
- les acyliséthionates, les N-acyltaurates ; les acyllactylates ;
- les acides d'alkyl-D-galactoside uroniques ;
- les acides alkyléther-carboxyliques polyoxyalkylénés, les acides alkylaryléther-carboxyliques polyoxyalkylénés, les acides alkylamidoéther carboxyliques polyoxyalkylénés ;
le groupe alkyle ou acyle (RCO-) de ces composés comportant de 10 à 24 atomes de carbone et le groupe aryle désignant de préférence un groupe phényle ou benzyle ; le nombre de groupements oxyalkylénés, et de préférence oxyéthylénés, est compris entre 2 et 50.

Pour ce qui concerne les tensioactifs non ioniques, ces derniers peuvent être avantageusement choisis parmi les composés suivants, seuls ou en mélange :
- les alcools gras polyéthoxylés, polypropoxylés ou polyglycérolés,
- les alpha-diols polyéthoxylés, polypropoxylés ou polyglycérolés, le nombre de groupements oxyde d'éthylène ou oxyde de propylène allant de 2 à 50 ; le nombre de groupements glycérol allant de 2 à 30 ;
- les copolymères d'oxyde d'éthylène et d'oxyde de propylène,
- les condensats d'oxyde d'éthylène et d'oxyde de propylène sur des alcools gras ;
- les alcanolamides gras polyéthoxylés ayant de 2 à 30 moles d'oxyde d'éthylène ;
- les amides gras polyglycérolés comportant de 1 à 5 groupements glycérol ;
- les esters éthoxylés d'acides gras du sorbitane ayant de 2 à 30 moles d'oxyde d'éthylène, les esters d'acides gras du saccharose, les esters d'acide gras du polyéthylèneglycol ;
- les alkylpolyglucosides, les dérivés de N-alkylglucamine ;
ces composés comprenant une chaîne alkyle ou acyle comprenant 10 à 24 atomes de carbone.

Les tensioactifs cationiques entrant dans la composition selon l'invention, peuvent notamment être choisis parmi les composés suivants, seuls ou en mélange :
- les sels d'amines grasses primaires, secondaires ou tertiaires, éventuellement polyoxyalkylénées,
- les sels d'ammonium quaternaire tels que les chlorures ou les bromures de tétraalkylammonium, d'alkylamidoalkyltrialkylammonium, de trialkylbenzyl-ammonium, de trialkylhydroxyalkylammonium, d'alkylpyridinium,
- les dérivés d'alkyl-imidazoline ;
ces composés comprenant au moins une chaîne alkyle comprenant 10 à 24 atomes de carbone.

Enfin, les tensioactifs amphotères peuvent être choisis parmi les composés suivants, seuls ou en mélange :
- les dérivés d'amines aliphatiques secondaires ou tertiaires, dans lesquels le groupe aliphatique est une chaîne linéaire ou ramifiée comportant 10 à 24 atomes de carbone et comprenant au moins un groupe anionique hydrosolubilisant tel que, par exemple, un groupe carboxylate, sulfonate, sulfate, phosphate ou phosphonate,
- les alkylbétaïnes, les sulfobétaïnes, les alkylamidoalkyl(C6-C8)bétaïnes, les alkylamidoalkyl(C6-C8)sulfobétaïnes ;
   ces composés comprenant une chaîne alkyle comprenant de 10 à 24 atomes de carbone.

Parmi les tensio-actifs, on préfère utiliser les alkylsulfates, les alkylbenzènesulfates, les alkyléthersulfates, les alkylsulfonates, les sels d'ammonium quaternaire, les alkylbétaïnes, les alcanolamides d'acides gras, les esters d'acides gras oxyéthylénés seuls ou en mélanges.

Lorsque les compositions selon l'invention contiennent un ou plusieurs agents tensioactifs, le ou les tensioactifs sont généralement présents à une concentration de 0,5 à 30 % en poids, et de préférence, comprise entre 0,5 et 10 % en poids par rapport au poids total de la composition.

Dans le but d'améliorer les propriétés cosmétiques des fibres kératiniques traitées ou encore d'en atténuer ou d'en éviter la dégradation, la composition selon l'invention peut également comprendre un ou plusieurs agents traitants de nature cationiques, anioniques, non ioniques ou amphotères.

On peut notamment utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2.535.730, les polyorganosiloxanes à groupements aminoalkyles modifiés par des groupements alcoxycarbonyalkyles tels que ceux décrits dans le brevet US n° 4.749.732, des polyorganosiloxanes tels que le copolymère polydiméthylsiloxane-polyoxyalkyle du type Diméthicone Copolyol, un polydiméthylsiloxane à groupements terminaux stéaroxy (stéaroxydiméthicone), un copolymère polydiméthylsiloxane-dialkylammonium acétate ou un copolymère polydiméthyl-siloxane polyalkylbétaïne décrits dans le brevet britannique n° 2.197.352, des polysiloxanes organo modifiés par des groupements mercapto ou mercaptoalkyles tels que ceux décrits dans le brevet français n° 1.530.369 et dans la demande de brevet européen n° 295.780, ainsi que des silanes tels que le stéaroxytriméthylsilane.

Les compositions selon l'invention peuvent également contenir d'autres ingrédients traitants tels que des polymères cationiques tels que ceux utilisés dans les compositions des brevets français n° 79.32078 (2.472.382) et 80.26421 (2.495.931), ou encore des polymères cationiques du type ionène tels que ceux utilisés dans les compositions du brevet luxembourgeois n° 83703, des aminoacides basiques (tels que la lysine, l'arginine) ou acides (tels que l'acide glutamique, l'acide aspartique), des peptides et leurs dérivés, des hydrolysats de protéines, des cires dont les céramides, des alcools gras, des dérivés de la lanoline.

Les compositions selon l'invention peuvent contenir, en outre, un ou plusieurs adjuvants choisis parmi les vitamines et pro-vitamines dont le panthénol, les filtres solaires hydrosolubles et liposolubles, siliconés ou non siliconés, les agents nacrants et opacifiants, les agents séquestrants, les agents plastifiants, les agents filmogènes, les céramides, les agents stabilisants, les agents de conditionnement, les agents épaississants minéraux et organiques différents des (co)polymères carboxyvinyliques, les agents antioxydants, les agents de pénétration, les parfums et les agents conservateurs.

Les adjuvants ci dessus sont en général présents en quantité comprise pour chacun d'eux entre 0,01 et 20 % en poids par rapport au poids de la composition.

La composition peut aussi comprendre au moins un autre composé disulfure additionnel différent des colorants directs disulfures de l'invention. A titre indicatif, le disulfure peut être choisi parmi les composés comprenant au moins une chaîne grasse, plus particulièrement au moins une chaîne hydrocarbonée en C₅-C₃₀, linéaire ou ramifiée, saturée ou non, éventuellement substituée par un hétéroatome, éventuellement interrompue par un groupement carboxylique, neutralisé ou non. A titre d'exemple de composés de ce type, on peut citer les dimères de l'acide thioglycolique et ses dérivés du type CH₃-(CH₂)₁₇-S-S-(CH₂)₁₇-CH₃ ou CH₃-(CH₂) -S-S-(CH₂)₁₀-CH₃

S'il est présent, la teneur en ce composé est comprise entre 0,001 et 10 % en poids par rapport au poids de la composition.

Bien entendu, l'homme de l'art veillera à choisir ce ou ces éventuels composés complémentaires de manière telle que les propriétés avantageuses attachées intrinsèquement à la composition capillaire conforme à l'invention ne soient pas, ou substantiellement pas, altérées par la ou les adjonctions envisagées.

### II.4. Formes de la composition :

La composition tinctoriale peut se présenter sous des formes diverses, telles que sous forme de liquide, de lotion, de crème, de gel, ou sous toute autre forme appropriée pour réaliser une teinture des fibres kératiniques, et notamment des cheveux. Elle peut également être conditionnée sous pression en flacon aérosol en présence d'un agent propulseur et former une mousse.

Préférentiellement, les compositions selon l'invention se présentent sous forme d'une crème épaissie de façon à maintenir les cheveux aussi raides que possible. On réalise ces crèmes, sous forme d'émulsions "lourdes", par exemple à base d'une phase liquide inerte.

A titre d'exemple de phase liquide inerte, on peut citer les polydécènes de formule C₁₀ₙH_{[(20n)+2]} dans laquelle n varie de 3 à 9 et de préférence de 3 à 7, les esters et en particulier les esters d'alcools gras ou d'acides gras, les esters ou di-esters de sucres d'acides gras en C₁₂-C₂₄, les éthers cycliques ou les esters cycliques, les huiles de silicone, les huiles minérales ou les huiles végétales, ou leurs mélanges.

Les composés de formule C₁₀ₙH_{[(20n)+2]} avec n variant de 3 à 9 répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes.

Parmi ces composés, on préfère selon l'invention ceux pour lesquels dans la formule, n varie de 3 à 7.

On peut citer à titre d'exemple le produit vendu sous la dénomination Silkflo® 366 NF Polydecene par la société Amoco Chemical, ceux vendus sous la dénomination Nexbase® 2002 FG, 2004 FG, 2006 FG et 2008 FG par la société Fortum.

En ce qui concerne les esters, on peut citer à titre d'exemple :
- les esters de monoalcools inférieurs saturés linéaires ou ramifiés en C₃-C₆, avec des acides gras monofonctionnels en C₁₂-C₂₄, ces derniers pouvant être linéaires ou ramifiés, saturés ou insaturés et choisis notamment parmi les oléates, laurates, palmitates, myristates, béhénates, cocoates, stéarates, linoléates, linolénates, caprates, arachidonates, ou leurs mélanges comme notamment les oléo-palmitates, oléo-stéarates, palmito-stéarates. Parmi ces esters, on préfère plus particulièrement utiliser le palmitate d'isopropyle, le myristate d'isopropyle et le stéarate d'octyl dodécyle.
- les esters de monoalcools linéaires ou ramifiés en C₃-C₈, avec des acides gras bifonctionnels en C₈-C₂₄, ces derniers pouvant être linéaires ou ramifiés, saturés ou insaturés, comme par exemple le di-ester isopropylique de l'acide sébacique, appelé aussi sébaçate de di-isopropyle,
- les esters de monoalcools linéaires ou ramifiés en C₃-C₈, avec des acides gras bifonctionnels en C₂-C₈, ces derniers pouvant être linéaires ou ramifiés, saturés ou insaturés, comme par exemple l'adipate de di-octyle et le maléate de di-caprylyle,
- l'ester d'un acide trifonctionnnel comme le citrate de tri-éthyle.

En ce qui concerne les esters et di-esters de sucres d'acides gras en C₁₂-C₂₄, on entend par "sucre" des composés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres utilisables selon l'invention, on peut citer par exemple le sucrose (ou saccharose), le glucose, le galactose, le ribose, le fuctose, le maltose, le fructose, le mannose, l'arabinose, le xylose, le lactose, et leurs dérivés notamment alkylés, tels que les dérivés méthylés comme le méthylglucose.

Les esters de sucres et d'acides gras utilisables selon l'invention peuvent être choisis notamment dans le groupe comprenant les esters ou mélanges d'esters de sucres décrits ci-avant et d'acides gras en C₁₂-C₂₄, linéaires ou ramifiés, saturés ou insaturés.

Les esters peuvent être choisis parmi les mono-, di-, tri- et tétra-esters, les polyesters et leurs mélanges.

Ces esters peuvent être par exemple choisis parmi les oléates, laurates, palmitates, myristates, béhénates, cocoates, stéarates, linoléates, linolénates, caprates, arachidonates, ou leurs mélanges comme notamment les esters mixtes oléo-palmitates, oléo-stéarates, palmito-stéarates.

Plus particulièrement, on préfère utiliser les mono- et di- esters et notamment les mono- ou di- oléates, stéarates, béhénates, oléopalmitates, linoléates, linolénates, oléostéarates, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

En ce qui concerne les ethers cycliques et esters cycliques, conviennent notamment la γ-butyrolactone, le diméthyl isosorbide, ou le diisopropyl isosorbide.

Les huiles de silicone peuvent aussi être employées comme phase liquide organique inerte.

Plus particulièrement, les huiles de silicone convenables sont des fluides de silicones liquides et non volatiles de viscosité inférieure ou égale à 10 000 mPa.s à 25 °C, la viscosité des silicones étant mesurée selon la norme ASTM 445 Appendice C.

Les huiles de silicone sont définies plus en détail dans l'ouvrage de Walter NOLL "Chemistry and Technology of Silicones" (1968) - Academic Press.

Parmi les huiles de silicone utilisables selon l'invention, on peut citer notamment les huiles de silicones vendues sous les dénominations DC-200 fluid - 5 mPa.s, DC-200 fluid - 20 mPa.s, DC-200 fluid - 350 mPa.s, DC-200 fluid - 1000 mPa.s, DC-200 fluid - 10 000 mPa.s par la société Dow Corning.

Les huiles minérales peuvent aussi être utilisées comme phase liquide inerte organique, comme par exemple l'huile de paraffine.

Les huiles végétales peuvent aussi convenir, et notamment l'huile d'avocat, l'huile d'olive ou la cire liquide de jojoba.

De préférence, la phase liquide inerte organique est choisie parmi les polydécènes de formule C₁₀ₙH_{[(20n)+2]} dans laquelle n varie de 3 à 9 et de préférence de 3 à 7, les esters d'alcools gras ou d'acides gras, et leurs mélanges.

La concentration en phase liquide inerte organique représente de 5 à 60% en poids total de la composition.

On peut également utiliser des liquides ou des gels contenant des agents épaississants tels que des polymères ou des copolymères carboxyvinyliques qui "collent" les cheveux et les maintiennent dans la position lisse pendant le temps de pose.

### III. Procédé de coloration:

Un autre objet de l'invention concerne un procédé de mise en forme telle que le défrisage des fibres kératiniques mettant en oeuvre une composition cosmétique comprenant, dans un milieu cosmétiquement acceptable, un ou plusieurs colorants directs disulfures et un ou plusieurs agents alcalins de type hydroxyde organique ou minéral tel que défini précédemment, ledit ou lesdits agents étant présents dans la composition en quantité telle que le pH de la composition est compris entre 10 et 14, de préférence entre 12 et 14.

Selon le procédé de mise en forme et de coloration, et éventuellement d'éclaircissement, simultanés des fibres kératiniques, on applique la composition tinctoriale sur les fibres kératiniques pendant un temps suffisant pour obtenir la mise en forme et la coloration, et éventuellement l'éclaircissement, souhaités. On rince ensuite éventuellement les fibres kératiniques et on les lave au shampooing.

Avantageusement, dans le procédé selon l'invention, la durée de l'application est inférieure à 40 minutes, et plus avantageusement inférieure à 30 minutes.

Selon un mode de réalisation préféré du procédé selon l'invention, on applique sur lesdites fibres la composition selon l'invention, puis l'on soumet les cheveux à une déformation mécanique permettant de leur conférer une nouvelle forme, notamment par une opération de lissage des cheveux avec un peigne à larges dents, avec le dos d'un peigne ou à la main. Après un temps de pose, plus particulièrement de 5 à 60 minutes, de préférence, de 10 à 30 minutes, on procède alors éventuellement à un nouveau lissage, puis on rince les cheveux.

La composition selon l'invention, lorsqu'elle contient un colorant fluorescent, est avantageusement appliquée sur des cheveux présentant une hauteur de ton inférieure ou égale à 6 et de préférence inférieure ou égale à 4.

La température d'application est généralement comprise entre la température ambiante (15 à 25°C) et 80°C et plus particulièrement entre 15 et 40°C. Ainsi, on peut, avantageusement, après application de la composition selon l'invention, soumettre la chevelure à un traitement thermique par chauffage à une température comprise entre 30 et 60°C. Dans la pratique, cette opération peut être conduite au moyen d'un casque de coiffure, d'un sèche-cheveux, d'un dispensateur de rayons infrarouges et d'autres appareils chauffants classiques.

On peut utiliser, à la fois comme moyen de chauffage et de lissage de la chevelure, un fer chauffant à une température comprise entre 60 et 220°C et de préférence entre 120 et 200°C.

Selon une variante, les fibres kératiniques humaines sont pigmentées ou colorées artificiellement.

Lorsque la composition selon l'invention est utilisée pour éclaircir des fibres kératiniques foncées, telles que des cheveux châtains par exemple, la réflectance mesurée avant et après application permet d'évaluer l'éclaircissement obtenu. Si l'on mesure la réflectance des cheveux lorsqu'on les irradie avec de la lumière visible dans la gamme de longueurs d'onde allant de 400 à 700 nanomètres, et que l'on compare les courbes de réflectance en fonction de la longueur d'onde, des cheveux traités avec la composition de l'invention et des cheveux non traités, on constate que la courbe de réflectance correspondant aux cheveux traités, dans une gamme de longueurs d'onde allant de 500 à 700 nanomètres, est supérieure à celle correspondant aux cheveux non traités.

Cela signifie que, dans la gamme de longueurs d'onde allant de 500 à 700 nanomètres, et de préférence de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%.

Il est précisé toutefois qu'il peut exister dans la gamme de longueurs d'onde allant de 500 à 700 nanomètres, et de préférence de 540 à 700 nanomètres, une ou plusieurs plages où la courbe de réflectance correspondant aux fibres traitées est soit superposable, soit inférieure à la courbe de réflectance correspondant aux fibres non traitées.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

### III. Dispositif ou « kit » de teinture:

L'invention a aussi pour objet un dispositif à plusieurs compartiments ou "kit" de teinture dans lequel un premier compartiment (i) comprend un ou plusieurs agents alcalins de type hydroxyde minéral ou organique, et un deuxième compartiment (ii) comprend une composition contenant un ou plusieurs colorants directs disulfures, le pH du mélange du contenu des différents compartiments étant compris entre 10 et 14.

Il est à noter que dans le cas particulier d'un agent alcalin de type hydroxyde mis en oeuvre sous forme d'un précurseur, les composants précurseurs ne sont pas stockés dans le même compartiment. Ainsi, plus particulièrement dans le cas de la guanidine, le carbonate de guanidine est avantageusement stocké avec le colorant direct disulfure, et l'hydroxyde de métal alcalin ou alcalino-terreux dans un deuxième compartiment. On peut aussi avoir trois compartiments, le premier contenant le carbonate de guanidine, le second l'hydroxyde de métal alcalin ou alcalino-terreux et le troisième le colorant direct disulfure.

Selon une première variante, le dispositif selon l'invention, comprend, en outre, une composition supplémentaire (iii) comprenant un ou plusieurs agents traitants et/ou un ou plusieurs adjuvants, tels que décrits précédemment.

Selon une deuxième variante, le ou les agents traitant et/ou le ou les adjuvants précités sont présents dans l'un et/ou l'autre des compartiments (i) ou (ii) et de préférence dans le compartiment (ii).

L'un de ces compartiments peut en outre contenir un ou plusieurs autres colorants directs.

Les compositions du dispositif selon l'invention sont conditionnées dans des compartiments ou récipients ou dispositifs distincts, accompagnés, éventuellement, de moyens d'application appropriés, identiques ou différents, tels que les pinceaux, les brosses, les éponges. Le dispositif mentionné ci-dessus peut également être équipé d'un moyen permettant de délivrer sur les cheveux le mélange souhaité, par exemple tel que les dispositifs décrits dans le brevet FR 2 586 913.

Les exemples qui suivent servent à illustrer l'invention sans toutefois présenter un caractère limitatif. Les colorants fluorescents disulfures des exemples ci-après ont été entièrement caractérisés par les méthodes spectroscopiques et spectrométriques classiques.

### EXEMPLES

### EXEMPLES DE SYNTHESE

### Exemple 1

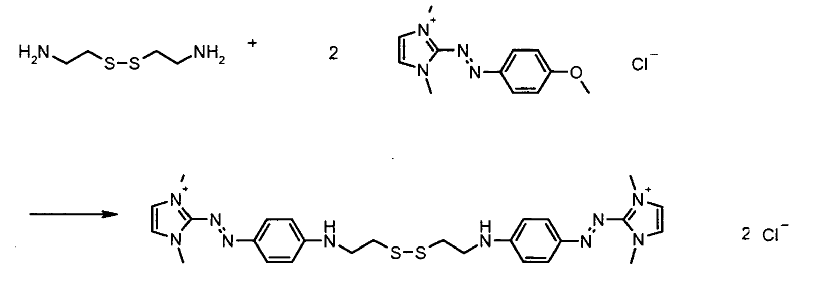

La cystamine base (552.2 mg ; 3.62 mmol) obtenue à partir de dichlorhydrate de cystamine par addtion de soude et extraction par l'acétate d'éthyle, est solubilisée dans 2 ml de pentanol. On ajoute le chlorure de 2-[(4-methoxyphenyl)diazenyl]-1,3-dimethyl-1 H-imidazol-3-ium (2.42 m ; 9.1 mmol), en suspension dans 80 ml de dichlorométhane. Le mélange est porté à 50°C et maintenu agité 1 heure. Il est concentré sous vide (élimination du dichlorométhane), on ajoute 20 ml d'eau et le mélange réactionnel est maintenu une heure supplémentaire à 50°C. Il est ensuite refroidi et versé sur 50mL de pentanol ; un précipité rouge apparaît, qui est filtré et lavé par de l'acétone puis séché sous vide. On obtient ainsi 1.1g de poudre rouge foncée conforme à la structure ci-dessus.

### Exemple 2

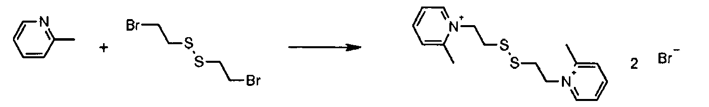

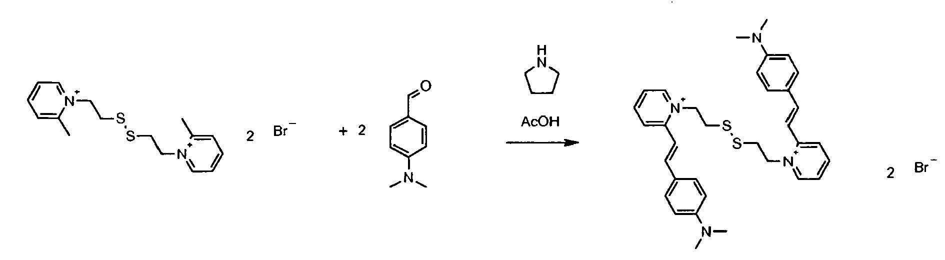

### Etape 1 : dibromure de 1,1'-(disulfanediyldiéthane-2,1-diyl)bis(2-méthyl-pyridinium)

Un mélange de 56 g de 1-bromo-2-[(2-bromoethyl)disulfanyl]ethane et de 15 mL de *N*-Méthylpyrrolidone (NMP) est versé goutte à goutte sur 35 g de 2-picoline sous agitation, à 80°C. Le mélange (suspension blanche) est maintenu agité 30 min à 80°C, 100 mL d'acétonitrile sont ajoutés, l'agitation est maintenue à 80°C pendant 90 min.

Après refroidissement, le solide obtenu est filtré, lavé par 100 mL d'acétonitrile puis séché.

On recueille 56,2 g de poudre brune. 45 g de cette poudre sont mis en suspension dans 300 mL d'isopropanol, à reflux. Une fois la température baissée à 40°C, le solide est filtré, lavé par 3 fois 100 mL d'isopropanol et séché sous vide. Produit beige clair, 40,56 g. Analyses en conformité avec la structure attendue.

### Etape 2 : dibromure de 1,1'-(disulfanediyldiéthane-2,1-diyl)bis(2-{(E)-2-[4-(diméthylamino)phényl]vinyl}pyridinium)

150 mg de pyrrolidine puis 129 mg d'acide acétique sont ajoutés à une solution de 297 mg de 4-diméthylaminobenzaldéhyde dans 2 mL de méthanol. Après 18 h d'agitation à température ambiante, 495 mg de dibromure de 1'-(disulfanediyldiéthane-2,1-diyl)bis(2-méthylpyridinium) sont ajoutés au mélange et l'agitation est maintenue à température ambiante pendant 7 j. Après filtration, lavage par du méthanol et séchage sous vide, 312 mg de poudre orange sont recueillis. Analyses en conformité avec la structure attendue. RMN ¹H (400 MHz, MeOH-*d*₄) : 3.02 (s ,6H), 3.22 (t, 2H), 5 (t, 2H), 6.72 (m, 2H), 7.19 (d, 1H), 7.63 (m, 3H), 7.76 (d, 1H), 8.3 (m, 2H), 8.59 (m, 1H).

### Exemple 3

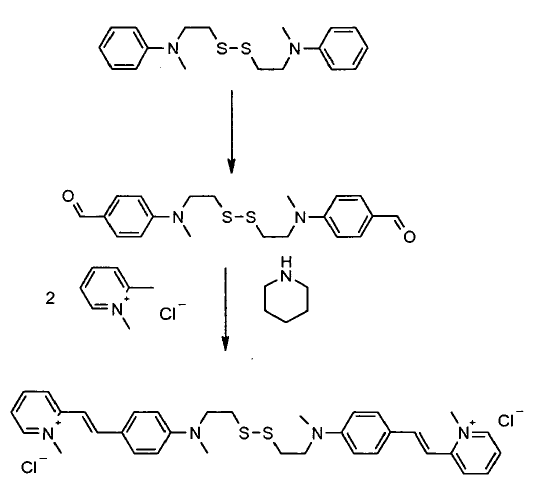

### Etape 1 : 4,4'-{disulfanediylbis[éthane-2,1-diyl(méthylimino)]}dibenzaldéhyde

82,3 g d'oxychlorure de phosphore sont ajoutés à 500 mL de DMF à 0°C. Après 30 min d'agitation à 0°C une solution de 47g de *N*,*N*'-(disulfanediyldiéthane-2,1-diyl)bis(N-méthylaniline) sont ajoutés goutte à goutte. Le mélange est agité 90 min. à 0°C puis 75 min. à 10°C et 105 min. à 40°C. Il est ensuite versé sur 2,5 L d'eau glacée, 700 mL de soude 5N sont ajoutés. Le précipité jaune obtenu est filtré sur célite, solubilisé dans 200 mL de dichlorométhane et la solution obtenue est lavée par 200 mL de solution aqueuse saturée en chlorure de sodium. Après séchage sur sulfate de magnésium et évaporation du dichlorométhane, le résidu jaune (80g) est purifié par chromatographie sur gel de silice.
Après séchage, une poudre jaune clair est recueillie. Les analyses indiquent que le produit est conforme à la structure attendue.

### Etape 2 : dichlorure de 2,2'-{disulfanediylbis[éthane-2,1-diyl(méthylimino)-4,1-phénylène(E)éthène-2,1-diyl]}bis(1-méthylpyridinium)

25 g de 4,4'-{disulfanediylbis[éthane-2,1-diyl(méthylimino)]}dibenzaldehyde et 18.5 g de chlorure de N-méthylpicolinium sont dissous dans 300 mL de méthanol. 12,7 mL de pipéridine sont ajoutés au mélange. Le tout est chauffé, sous agitation à 55°C pendant 11 h. Le méthanol est éliminé sous vide à 40°C. Le solide est mélangé à 300 mL d'isopropanol. Après un nouveau séchage par évaporation on introduit 200 mL d'isopropanol. Le mélange prend en masse, il est étendu par addition de 100 mL d'iospropanol et essoré sur verre fritté. Le solide recueilli est lavé par de l'isopropanol puis de l'acétone, puis séché sous vide. Après séchage, 36,7g de poudre orange sont recueillis. Par recristallisation dans l'isopropanol, 27 g de poudre rouge orange de pureté supérieure sont recueillis. Les analyses indiquent que le produit est conforme et pur. RMN ¹H (400 MHz, MeOH-*d*₄) 2.99 (t, 4H), 3.81 (t, 4H), 4.31 (s, 6H), 6.86 (d, 4H), 7.22 (d, 2H), 7.63 (m, 2H), 7.69 (d, 4H), 7.83 (d, 2H), 8.29 (m, 2H), 8.36 (m, 2H), 8.61 (m, 2H).

### Exemple 4 :

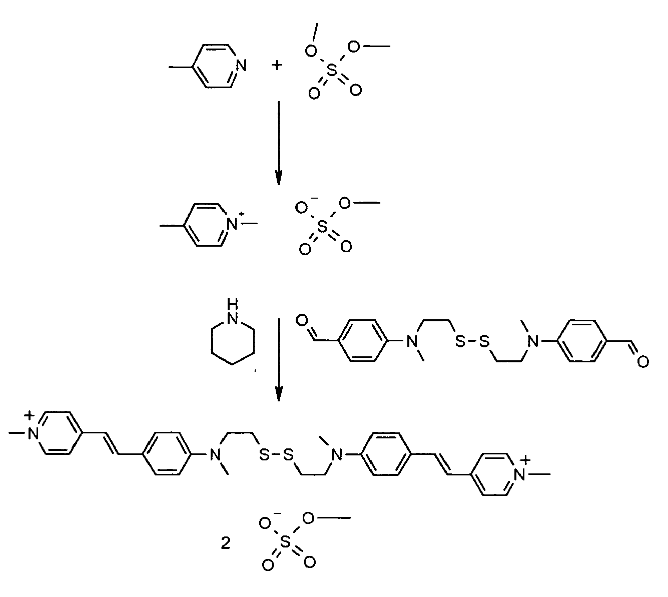

### Diméthoxysulfate de 4,4'-{disulfanediylbis[éthane-2,1-diyl(méthylimino)-4,1-phénylène(E)éthène-2,1-diyl]}bis(1-méthylpyridinium)

2,62 g de 4-picoline sont dilués dans 25 mL de dichlorométhane, 3 mL de sulfate de diméthyle sont ajoutés à la solution, dont la température s'élève au reflux (40°C). Après 40 min. d'agitation, 50 mL d'isopropanol sont ajoutés, et le mélange est concentré par distillation du dichlorométhane (mélange chauffé à 60°C). 1,83 g de pyrrolidine sont introduits dans le mélange suivis de 4,99 g de 4,4'-{disulfanediylbis[éthane-2,1-diyl(méthylimino)]}dibenzaldéhyde. Après 2 h d'agitation à 65°C, le mélange réactionnel est refroidi à température ambiante, le précipité formé est filtré, lavé par 3 fois 100 mL d'isopropanol. La pâte rouge obtenue est dispersée dans 200 mL d'isopropanol, le mélange ainsi obtenu est porté à reflux puis refroidi. Le précipité rouge formé est filtré puis séché. 8,94 g de poudre rouge sont recueillis. Les analyses indiquent que le produit est conforme et pur. RMN ¹ H (400 MHz, DMSO-*d*₆) 2.96 (t, 4 H), 3.02 (s, 6 H), 3.36 (s, 6 H), 3.72 (t, 4 H), 4.16 (s, 6 H), 6.81 (d, 4 H), 7.15 (d, 2 H), 7.57 (d, 4 H), 7.87 (d, 2 H), 8.02 (d, 4 H), 8.66 (d, 4 H).

### Exemple 5

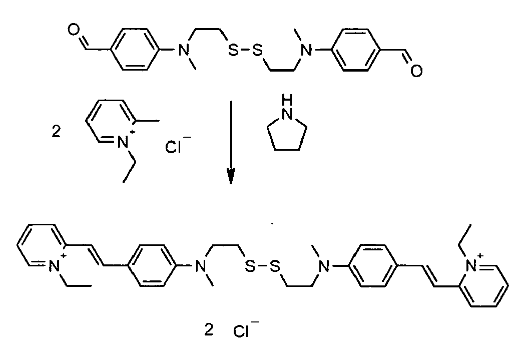

### Dichlorure de 2,2'-{disulfanediylbis[éthane-2,1-diyl(méthylimino)-4,1-phénylène(E)éthène-2,1-diyl]}bis(1-éthylpyridinium)

10 g de 4,4'-{disulfanediylbis[éthane-2,1-diyl(méthylimino)]}dibenzaldéhyde et 8,1 g de chlorure de N-éthylpicolinium sont dissous dans 100 mL d'isopropanol. 1,3 g de pipéridine sont ajoutés au mélange. Le tout est chauffé, sous agitation à reflux pendant 5 h. L'isopropanol est éliminé sous vide à 50°C. La gomme obtenue est triturée avec de l'acétone. 18 g de solide sont recueillis et traités au noir de charbon. 7,1 g de produit sont collectés et 4 g sont purifiés par chromatographie liquide liquide

(eau/BuOH). Après séchage 1,65 g de poudre rouge sont recueillis. Les analyses indiquent que le produit est conforme et pur. RMN ¹H (400 MHz, MeOH-*d*₄) 1.57 (t, 6 H), 2.98 (t, 4 H), 3.11 (s, 6 H), 3.8 (t, 4 H), 4.73 (q, 4 H), 6.85 (m, 4 H), 7.23 (d, 2 H), 7.69 (m, 6 H), 7.85 (d, 2 H), 8.29 (m, 2 H), 8.38 (m, 2 H), 8.67 (m, 2 H).

### Exemple 6

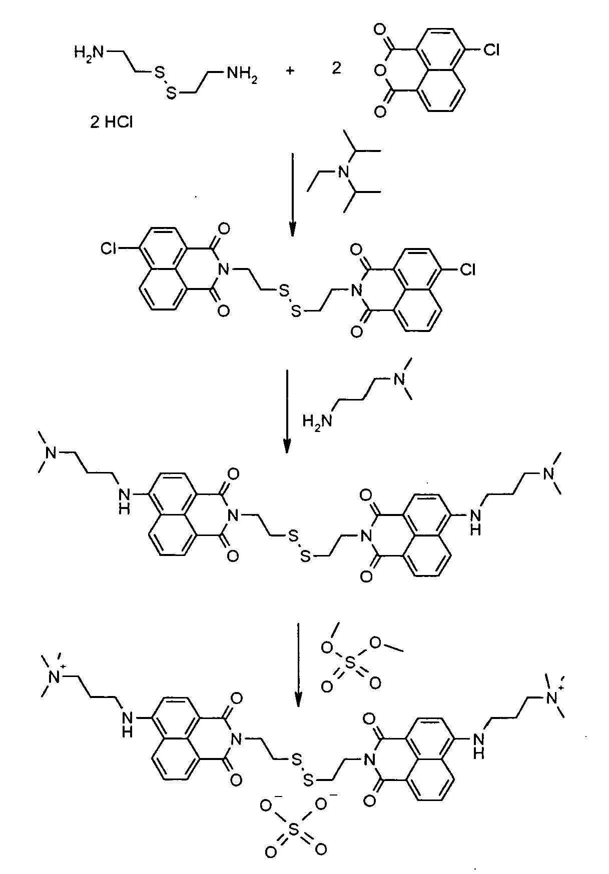

### Etape 1 : 2,2'-(disulfanediyldiéthane-2,1-diyl)bis(6-chloro-1H-benzo[de]-isoquinoline-1,3(2H)-dione)

9,30 g de 6-chloro-1 H,3H-benzo[de]isochromene-1,3-dione et 4,46 g de chlorhydrate de cystamine chlorhydratée sont mis en suspension dans 50ml de N-méthylpyrrolidone (NMP). 5,5 g de diisopropylethylamine sont ajoutés et le mélange est chauffé, sous agitation à 120°C. Après deux heures 50 mL de NMP sont ajoutés et le mélange est

maintenu agité à 120°C pendant 3 h. Après refroidissement, le produit précipité est recueilli, la solution filtrée est étendue par ajout de 200 mL d'eau et un deuxième précipité est recueilli. Les précipités sont lavés à l'eau et séchés. 11,46 g de poudre blanche sont recueillis. Les analyses montrent que le produit est conforme à l'attendu

### Etape 2 : 2,2'-(disulfanediyldiéthane-2,1-diyl)bis[6-{[3-(diméthylamino)propyl]-amino}-1H-benzo[de]isoquinoline-1,3(2H)-dione]

4 g de (6-chloro-2-(2-{[2-(6-chloro-1,3-dioxo-1H-benzo[de]isoquinolin-2(3H)-yl)éthyl]-disulfanyl}éthyl)-1H-benzo[de]isoquinoline-1,3(2H)-dione sont mis en suspension 40 mL de N,N-dimethylpropane-1,3-diamine. Le mélange est chauffé sous agitation à 110°C pendant 12 h. Après refroidissement, un précipité jaune est collecté, 500 mL de mélange éthanol/eau : 1/1 sont ajoutés goutte à goutte au filtrat. La pâte jaune obtenue est isolée et triturée avec 200 mL d'acétone. Les solides obtenus sont lavés par 300 mL d'eau et séchés. 4,5 g de poudre jaune sont recueillis. Les analyses montrent que le produit est conforme à l'attendu.

### Etape 3 : sulfate de 3,3'-{disulfanediylbis[ethane-2,1-diyl(1,3-dioxo-1H-benzo[de]isoquinoline-2,6(3H)-diyl)imino]}bis(N,N,N-triméthylpropan-1-aminium)

4 g de 6-{[3-(diméthytamino)propyt]amino}-2-[2-({2-[6-{[3-(diméthylamino)propyl]-amino}-1,3-dioxo-1H-benzo[de]isoquinolin-2(3H)-yl]éthyl}disulfanyl)éthyl]-1H-benzo-[de]isoquinoline-1,3(2H)-dione sont mis en suspension dans 50 mL de diméthylformamide 4 mL de diméthylsulfate sont ajoutés et le mélange est maintenu sous agitation à température ambiante pendant 4 h. Le mélange réactionnel est versé dans 500 mL d'acétate d'éthyle. Le précipité est filtré, lavé par 4 fois 100 mL d'acétate d'éthyle et séché sous vide. On recueille 5,9 g de poudre jaune. Les analyses indiquent que le produit est conforme à l'attendu. RMN ¹H (400 MHz, DMSO-*d*₆) : 2.13 (m, 4 H), 3.06 (m, 4 H), 3.09 (s, 18 H), 3.46 (m, 4 H), 4.36 (m, 4 H), 6.85 (d, 2 H), 7.71 (m, 2 H), 7.82 (t, 2 H), 8.28 (d, 2 H), 8.29 (dd, 2 H), 8.45 (dd, 2 H).

### Exemple 7

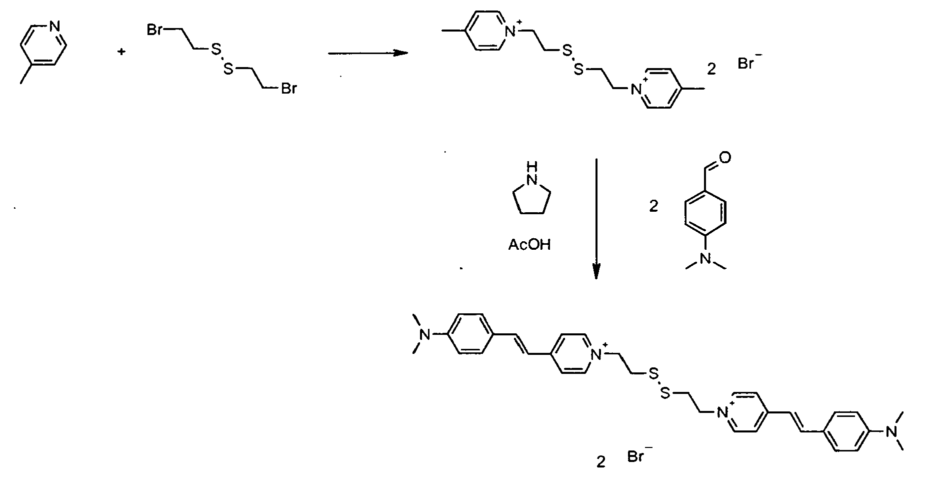

### Etape 1 : dibromure de 1,1'-(disulfanediyldiéthane-2,1-diyl)bis(4-méthyl-pyridinium)

67 g de 4-picoline sont dilués dans 100 mL d'acétonitrile et le mélange est porté à 80°C. Un mélange de 60 g de 1-bromo-2-[(2-bromoethyl)disulfanyl]ethane et 15 mL de N-méthylpyrrolidone (NMP) est ajouté en 5 min. Après 4 h d'agitation à 85°C, le mélange est refroidi. Le solide obtenue est filtré, rincé par 3 x 200 mL d'acétonitrile puis dissout dans 800 mL d'isopropanol (à reflux). Après refroidissement, 1 L d'éther éthylique est ajouté. Le précipité formé est filtré, rincé par 3 x 200 mL d'éther éthylique puis séché. La poudre blanc cassée obtenue (73,77g) contient très majoritairement (>90%) le produit attendu qui est utilisé tel quel pour l'étape suivante.

### Etape 2 : dibromure de 1,1'-(disulfanediyldiéthane-2,1-diyl)bis(4-{(E)-2-[4-(diméthylamino)phényl]vinyl}pyridinium)

13,2 g de 4-diméthylaminobenzaldéhyde sont mis en suspension dans 100 mL de méthanol. 6,2 g de pyrrolidine puis 5,3 g d'acide acétique dilués dans 20 mL de méthanol sont ajoutés au mélange (pH final 5/6). 20 g de dibromure de 1,1'-(disulfanediyldiéthane-2,1-diyl)bis(4-méthylpyridinium) obtenus à l'étape précédente, solubilisés dans 80 mL de méthanol sont introduits, puis le mélange réactionnel est dilué par ajout de 100 mL de méthanol. Après 21 h d'agitation à température ambiante, un premier précipité est recueilli, lavé par 3 x 100 mL d'éthanol puis 3 x 200 mL d'acétate d'éthyle et séché (poudre rouge, 7,4 g), puis un second précipité formé dans le filtrat est également recueilli et séché (poudre rouge, 11,44 g). Les analyses indiquent que les deux fractions sont conformes à la structure attendue. RMN ¹H (400 MHz, MeOH-*d*₄) : 3.02 (s, 12 H), 3.42 (t, 4 H), 4.74 (t, 4 H), 6.77 (d, 4 H), 7.19 (d, 2 H), 7.6 (d, 4 H), 7.97 (d, 2 H), 8.1 (d, 4 H), 8.79 (d, 4 H).

### EXEMPLES DE MISE EN OEUVRE:

### EXEMPLE 1

On a préparé les compositions suivantes (teneurs exprimées en grammes de matière active) :
(*) Colorant direct disulfure : 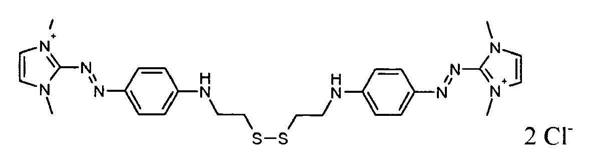 Composition (i)

| | |
|---|---|
| Propylène glycol | 50,5g |
| Silice | 3g |
| Hydroxyde de calcium | 45g |
| Dioxyde de titane | 1,5g |

Composition (ii)

| | |
|---|---|
| Colorant direct disulfure (*) | 0,62g |
| Guanidine carbonate | 7g |
| Huile minérale | 13,5g |
| Huile de paraffine | 16,5g |
| Alcool cétéarylique, PEG-75 lanoline, béhétrimonium méthosulfate | 10g |
| Agent alcalin | qsp pH=11 |
| Eau déminéralisée | qsp 100g |

Au moment de l'emploi, on mélange 30g de la composition (i) et 235g de la composition (ii), puis on applique le mélange obtenu sur cheveux frisés à 90% blancs pendant 15 minutes à température ambiante.

Après rinçage, lavage au shampooing, rinçage et séchage, les cheveux sont lissés au peigne et teints dans une nuance rouge fuchsia très intense. La coloration est très tenace aux lavages.

### EXEMPLE 2

On a préparé la composition suivante (teneurs exprimées en grammes de matière active) :

| | |
|---|---|
| Colorant direct disulfure (*) | 0,62g |
| Propylène glycol | 5,5g |
| Huile de vaseline | 13,5g |
| Vaseline | 23g |
| Alcool cétylstéarylique/mono-stéarate de polyéthylène glycol (23OE) | 10g |
| Alcool de lanoline oxyéthylénée (15OE) | 1g |
| Lanoline oxyéthylénée | 0,5g |
| Alcool cétylique | 1g |
| Hydroxyde de sodium | 1,99g |
| Eau déminéralisée | qsp 100g |

On applique cette composition sur cheveux frisés à 90% blancs pendant 15 minutes à température ambiante.

Après rinçage, lavage au shampooing, rinçage et séchage, les cheveux sont lissés au peigne et teints dans une nuance rouge fuchsia très intense. La coloration est très tenace aux lavages.

### EXEMPLE 3

On a préparé les compositions suivantes (teneurs exprimées en grammes de matière active) :
Composition (i)

| | |
|---|---|
| Propylène glycol | 50,5g |
| Silice | 3g |
| Hydroxyde de calcium | 45g |
| Dioxyde de titane | 1,5g |

Composition (ii)

| | |
|---|---|
| Colorant direct disulfure fluorescent (**) | 10g |
| Eau déminéralisée | qsp 100g |

| | |
|---|---|
| (**) Colorant direct disulfure fluorescent : 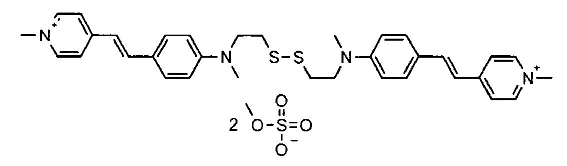 | |

Composition (iii)

| | |
|---|---|
| Guanidine carbonate | 7g |
| Huile minérale | 13,5g |
| Huile de paraffine | 16,5g |
| Alcool cétéarylique, PEG-75 lanoline, béhétrimonium méthosulfate | 10g |
| Agent alcalin | qsp pH=13 |
| Eau déminéralisée | qsp 100g |

Au moment de l'emploi, on mélange 30g de la composition (i), 12g de la composition (ii) et 235g de la composition (iii), puis on applique le mélange obtenu sur cheveux bruns (hauteur de ton 2) frisés pendant 20 minutes à température ambiante.

Après rinçage, lavage au shampooing, rinçage et séchage, les cheveux sont lissés au peigne et teints dans une nuance avec une hauteur de ton 4 (châtain) et un reflet acajou très visible. La coloration est très tenace aux lavages.

## Revendications

1. Composition de traitement des fibres kératiniques, comprenant dans un milieu cosmétiquement acceptable,
* un ou plusieurs colorants directs disulfures choisis parmi les composés de formules (1), (II) ou (III) suivantes : 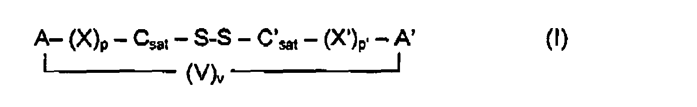 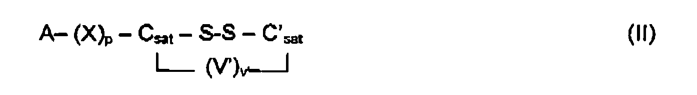 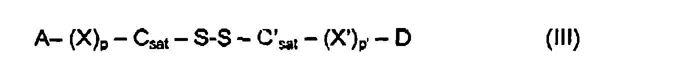 leurs sels, isomères optiques, isomères géométriques, et les solvates tels que hydrates,
formules dans lesquelles :
• A et A', identiques ou différents, représentent un radical contenant un ou plusieurs chromophores cationiques ou non;
• V et V', identiques ou différents représentent un groupe pontant
• v et v', identiques ou différents représentent 0 ou 1 ;
• les coefficients p et p', identiques ou différents, représentent un entier égal à 0 ou 1 ;
• X et X', identiques ou différents, représentent :
1) une chaîne hydrocarbonée en C₁-C₃₀, linéaire ou ramifiée, saturée ou insaturée, éventuellement interrompue et/ou éventuellement terminée à l'une ou deux de ses extrémités par un ou plusieurs groupes divalents ou leur combinaisons choisis parmi :
-N(R)-, -N⁺(R)(R')-, -O-, -S-, -CO-, -SO₂- avec R et R', identiques ou différents, choisis parmi un hydrogène, un radical alkyle en C₁-C₄, hydroxyalkyle, aminoalkyle,
un radical (hétéro)cyclique aromatique ou non, saturé ou insaturé, condensé ou non comprenant éventuellement un ou plusieurs hétéroatomes identiques ou non, éventuellement substitué ; ou
2) la séquence suivante :
- (T)ₜ- (Y)_{y}- (Z)_{z}-
ladite séquence étant reliée dans les formules (I), (II) ou (III), comme suit :
- Cₛₐₜ (ou C'ₛₐₜ respectivement) - (T)ₜ - (Y)_{y} - (Z)_{z} - A (ou A' respectivement) ;
dans laquelle :
(i) T représente un ou plusieurs radicaux ou leurs combinaisons choisis parmi -SO₂-, -O-, -S-, -N(R)-, -N*(R)(R'), -CO-, où R et R', identiques ou différents, représentent un atome d'hydrogène, un radical alkyle en C₁-C₄, ou hydroxyalkyle en C₁-C₄; le coefficient t vaut 0 ou 1 ;
(ii) Y représente :
- un radical choisi parmi -CO-, -(CH₂)-SO₂, -(CH₂)₂-SO₂- ; - CH₂-CHR-CO-NR'- avec R et R', identiques ou non, représentant un atome d'hydrogène, un radical alkyle en C₁-C₄;
- un groupement de formule (a), (a') ou (a") : 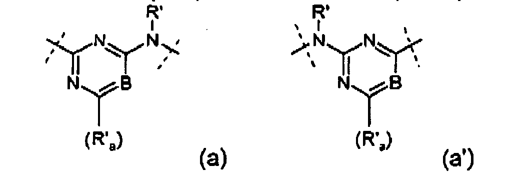 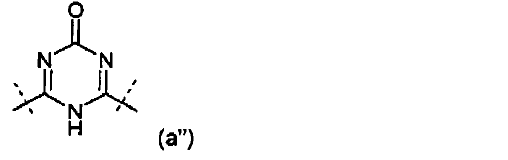 dans laquelle :
• B représente -N-, -CRₐ avec
Rₐ représentant
- un atome d'hydrogène,
- un atome d'halogène choisi parmi le chlore ou le fluor,
- un groupement nitro, substitué,
- un groupement pyridinium éventuellement substitué,
- un groupement hydroxyle,
- un groupement amino, alkylamino ou dialkylamino, les groupements alkyles identiques ou différents, en C₁-C₁₈, étant linéaires ou ramifiés, éventuellement interrompus par un hétéroatome choisi parmi N, O, éventuellement substitués par un ou plusieurs groupements hydroxyles
- un groupement NHNHCOR où R représente un groupement alkyle linéaire ou ramifié en C₁-C₁₀ ;
• R' a la même définition que précédemment
• R'ₐ représente :
- un atome d'hydrogène
- un atome de chlore ou un atome de fluor
- un groupement pyridinium éventuellement substitué par au moins un groupement R_{c}, R_{c} prouvant être un groupement alkyle en C₁-C₄, un atome d'halogène, un groupement carboxylique -COOM, (avec M représentant un atome d'hydrogène, un métal alcalin, un groupement ammonium ou un groupement ammonium substitué par un ou plusieurs radicaux alkyle, identiques ou non, linéaires ou ramifiés, en C₁-C₁₈, éventuellement porteurs d'au moins un hydroxyle) ; un groupement ester -COOR_{d} avec R_{d} représentant un radical alkyle en C₁-C₄ : un groupement amide -CON(R_{d})₂ avec R_{d} identiques ou non, représentant un atome d'hydrogène ou un radical alkyle en C₁-C₄ ;
- un groupement hydroxyle
- un groupement amino, alkylamino ou dialkylamino, les groupements alkyles identiques ou différents, en C₁-C₁₈, étant linéaires ou ramifiés, éventuellement interrompus par un hétéroatome choisi parmi N, O, éventuellement substitués par un ou plusieurs groupements hydroxyles
- un groupement NHNHCOR où R représente un groupement alkyle linéaire ou ramifié en C₁-C₁₀
- un groupement de formule (b) suivante : 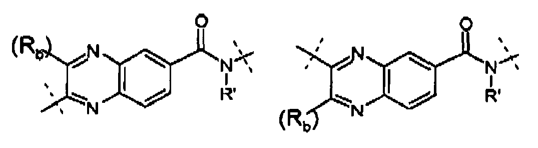 dans laquelle
• R' a la même définition que précédemment
• R_{b} représente
- un atome de chlore
- un groupement amino, alkylamino ou dialkylamino, les groupements alkyles identiques ou différents, en C₁-C₁₈, étant linéaires ou ramifiés, éventuellement interrompus par un hétéroatome choisi parmi N, O, S, éventuellement substitués par un ou plusieurs hydroxyles
- un hétérocycle azoté, saturé ou insaturé, pouvant être substitué
- un groupement arylamino, dans lequel de préférence le radical aryle est en C₆;
y vaut 0 ou 1 ;
(iii) Z représente
- -(CH₂)ₘ- avec m entier compris entre 1 et 8
- -(CN₂CH₂O)_{q}- ou -(OCH₂CH₂)_{q}- dans lesquelles q est un entier compris entre 1 et 15
- un radical aryle, alkylaryle ou arylalkyle dont le radical alkyle est en C₁-C₄ et le radical aryle est de préférence en C₆, étant éventuellement substitué par au moins un groupement SO₃M avec M représentant un atome d'hydrogène, un métal alcalin ou un groupement ammonium substitué par un ou plusieurs radicaux alkyle, identiques ou non, linéaires ou ramifiés, en C₁-C₁₈ éventuellement porteurs d'au moins un hydroxyle
z vaut 0 ou 1 ;
• Cₛₐₜ et C'ₛₐₜ, identiques ou différents, représentent une chaîne alkylène en C₁-C₁₈, linéaire ou ramifiée, éventuellement substituée, éventuellement cyclique ;
• D correspond à un radical choisi parmi les radicaux hydroxy, hydroxyalkyle, alcoxy, carboxyliques, carboxylates, amino, alkylamino, dialkylamino, et
* un ou plusieurs agents alcalins de type hydroxyde minéral ou organique en quantité telle que le pH de la composition est compris entre 10 et 14.

2. Composition selon la revendication 1, **caractérisée en ce que**, dans les formules (I), (II) ou (III), lorsque p et/ou p' respectivement, sont égaux à 1, X et/ou X' respectivement, représentent la séquence suivante :
- (T)ₜ-(Y)_{y}-(Z)_{z}-
ladite séquence étant reliée dans les formules (I), (II) ou (III), comme suit :
- Cₛₐₜ (ou C'ₛₐₜ respectivement) - (T)ₜ - (Y)_{y} - (Z)_{z} - A (ou A' respectivement) ;
dans laquelle :
T représente un ou plusieurs radicaux ou leurs combinaisons choisis parmi -SO₂, -O-, -S-, -N(R)-, -N⁺(R)(R'), -CO-, où R et R', identiques ou différents, représentent un atome d'hydrogène, un radical alkyle en C₁-C₄, ou hydroxyalkyle en C₁-C₄;
le coefficient t vaut 0 ou 1 ;
Y représente :
- un radical choisi parmi -CO-, -(CH₂)-SO₂, -(CH₂)₂-SO₂- ; -CH₂-CHR-CO-NR'-avec R et R', identiques ou non, représentant un atome d'hydrogène, un radical alkyle en C₁-C₄ ;
- un groupement de formule (a), (a') ou (a") : 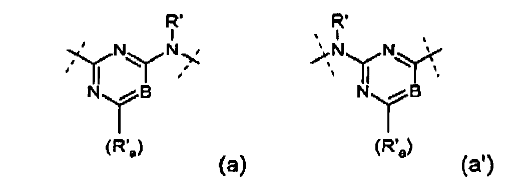 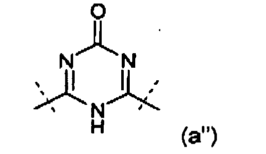 dans laquelle :
• B représente -N-, -CRₐ avec
Rₐ représentant
- un atome d'hydrogène,
- un atome d'halogène choisi parmi le chlore ou le fluor,
- un groupement nitro,
- un groupement pyridinium éventuellement substitué,
- un groupement hydroxyle,
- un groupement amino, alkylamino ou dialkylamino, les groupements alkyles identiques ou différents, en C₁-C₁₈, étant linéaires ou ramifiés, éventuellement interrompus par un hétéroatome choisi parmi N, O, éventuellement substitués par un ou plusieurs groupements hydroxyles
- un groupement NHNHCOR où R représente un groupement alkyle linéaire ou ramifié en C₁-C₁₀ ;
• R' a la même définition que précédemment
• R'ₐ représente :
- un atome d'hydrogène
- un atome de chlore ou un atome de fluor
- un groupement pyridinium éventuellement substitué par au moins un groupement R_{c}, R_{c} prouvant être un groupement alkyle en C₁-C₄, un atome d'halogène, un groupement carboxylique -COOM, (avec M représentant un atome d'hydrogène, un métal alcalin, un groupement ammonium ou un groupement ammonium substitué par un ou plusieurs radicaux alkyle, identiques ou non, linéaires ou ramifiés, en C₁-C₁₈, éventuellement porteurs d'au moins un hydroxyle) ; un groupement ester -COOR_{d} avec R_{d} représentant un radical alkyle en C₁-C₄ ; un groupement amide -CON(R_{d})₂ avec R_{d} identiques ou non, représentant un atome d'hydrogène ou un radical alkyle en C₁-C₄ :
- un groupement hydroxyle
- un groupement amino, alkylamino ou dialkylamino, les groupements alkyles identiques ou différents, en C₁-C₁₈, étant linéaires ou ramifiés, éventuellement interrompus par un hétéroatome choisi parmi N, O, éventuellement substitués par un ou plusieurs groupements hydroxyles
- un groupement NHNHCOR où R représente un groupement alkyle linéaire ou ramifié en C₁-C₁₀
- un groupement de formule (b) suivante : 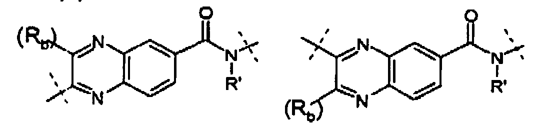 dans laquelle
• R' a la même définition que précédemment
• R_{b} représente
- un atome de chlore
- un groupement amino, alkylamino ou dialkylamino, les groupements alkyles identiques ou différents, en C₁-C₁₈, étant linéaires ou ramifiés, éventuellement interrompus par un hétéroatome choisi parmi N, O, S, éventuellement substitués par un ou plusieurs hydroxyles
- un hétérocycle azoté, saturé ou insaturé, pouvant être substitué
- un groupement arylamino, dans lequel de préférence le radical aryle est en C₆ ;
y vaut 0 ou 1 ;
Z représente
- -(CH₂)ₘ- avec m entier compris entre 1 et 8
- -(CH₂CH₂O)_{q}- ou -(OCH₂CH₂)_{q}- dans lesquelles q est un entier compris entre 1 et 15
- un radical aryle, alkylaryle ou arylalkyle dont le radical alkyle est en C₁-C₄ et le radical aryle est de préférence en C₆, étant éventuellement substitué par au moins un groupement SO₃M avec M représentant un atome d'hydrogène, un métal alcalin ou un groupement ammonium ammonium substitué par un ou plusieurs radicaux alkyle, identiques ou non, linéaires ou ramifiés, en C₁-C₁₈ éventuellement porteurs d'au moins un hydroxyle
z vaut 0 ou 1 ;
• Cₛₐₜ et C'ₛₐₜ, identiques ou différents, représentent une chaîne alkylène en C₁-C₁₈, linéaire ou ramifiée, éventuellement substituée, éventuellement cyclique:
• D correspond à un radical choisi parmi les radicaux hydroxy, hydroxyalkyle, alcoxy, carboxyliques, carboxylates, amino, alkylamino, dialkylamino.

3. Composition selon la revendication 1 ou 2 **caractérisée en ce que**, dans les formules (I), (II) ou (III), A et/ou A' représentent un radical comprenant au moins un chromophore issu de colorants de type acridines, acridones, anthranthrones, anthrapyrimidines, anthraquinones, azines, azoïques, azométhines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines, diazines, dicétopyrrolopyrroles, dioxazines, diphénylamines, diphénylméthanes, dithiazines, flavonoïdes, fluorindines, formazans, hydrazones, hydroxycétones, indamines, indanthrones, indigoides et pseudo-indigoïdes, indophénols, indoanilines, isoindolines, isoindolinones, isoviolanthrones, lactones, méthines, naphthalimides, naphthanilides, naphtholactames, naphthoquinones, nitro, oxadiazoles, oxazines, périlones, périnones, pérylènes, phénazines, phénothiazines, phthalocyanine, polyèneslcaroténoides, porphyrines, pyranthrones, pyrazolanthrones, pyrazolones, pyrimidinoanthrones, pyronines, quinacridones, quinolines, quinophthalones, squaranes, stilbènes, tétrazoliums, thiazines, thioindigo, ttliopyronines, triarylméthanes, xanthènes.

4. Composition selon l'une quelconque des revendications 1 à 3, dans laquelle le colorant direct disulfure est choisi parmi 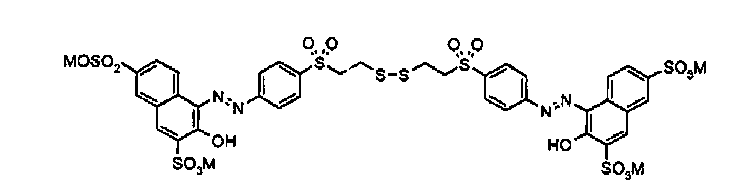 ainsi que les composés suivants, sous forme acide, basique ou neutralisée : 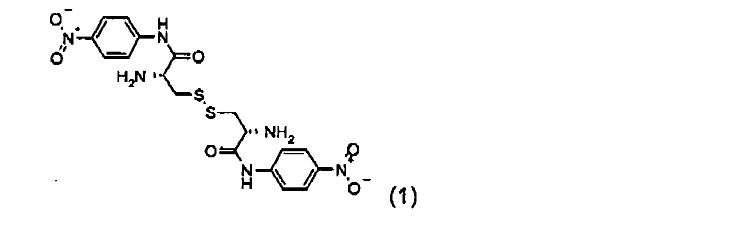 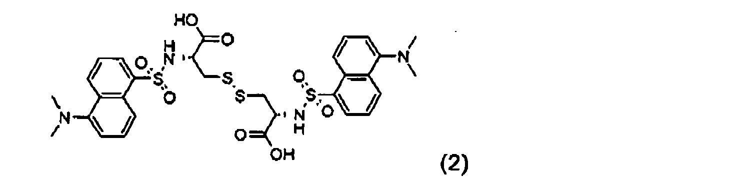 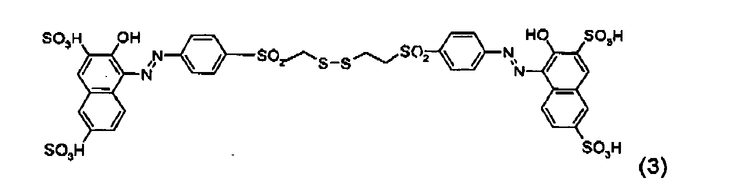 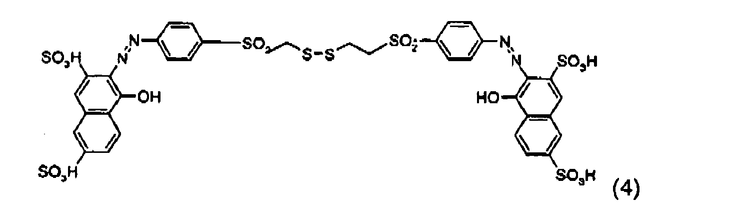 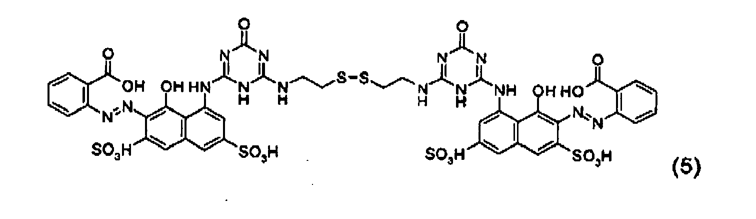 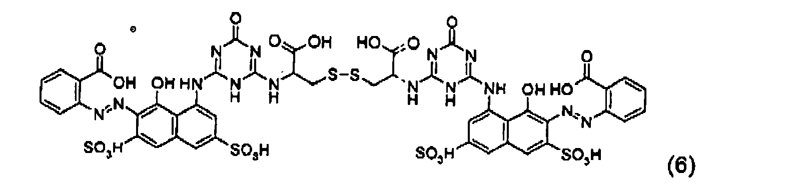 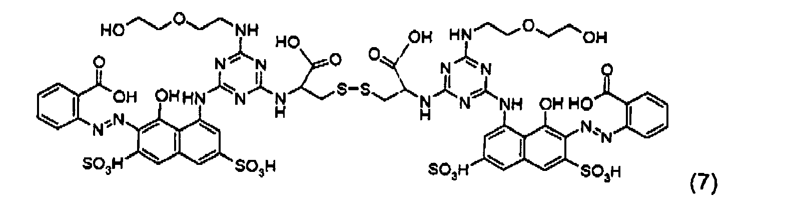 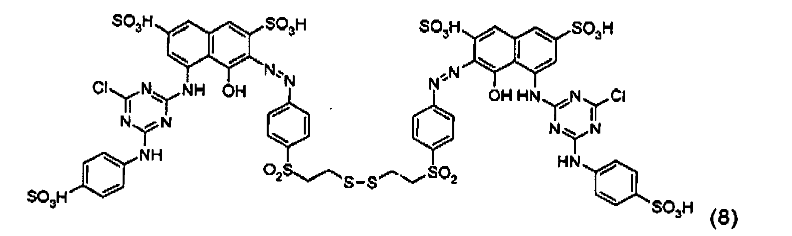 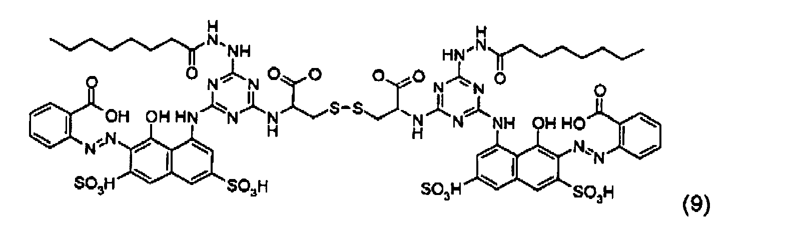 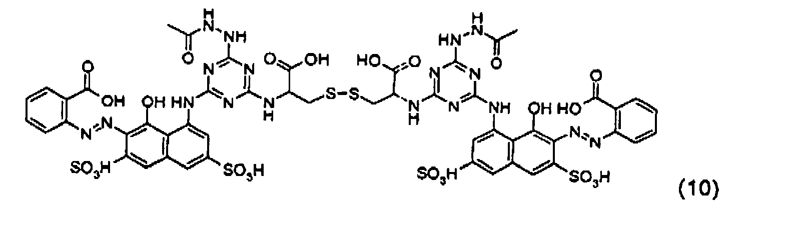 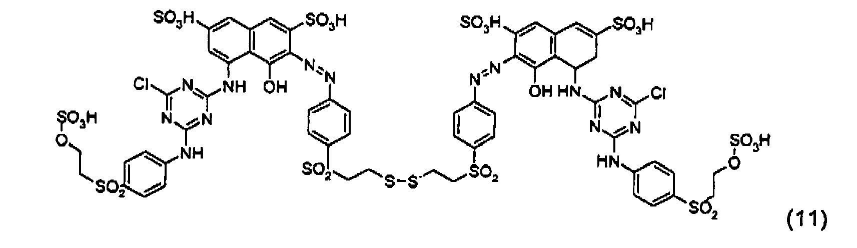 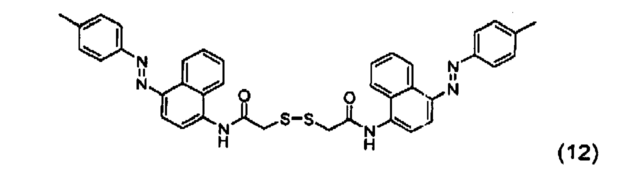 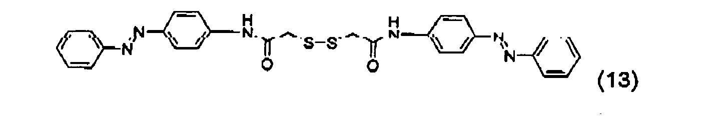 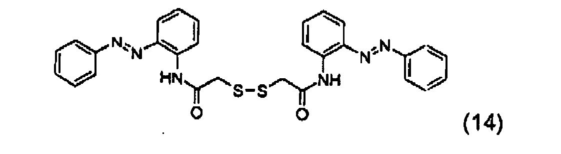 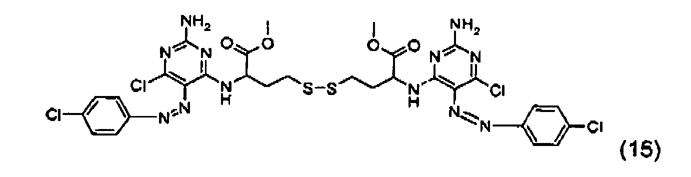 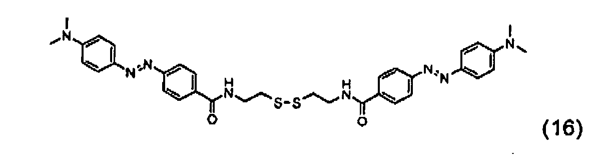 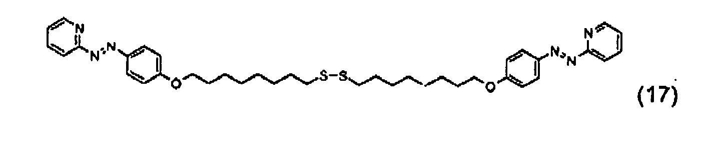 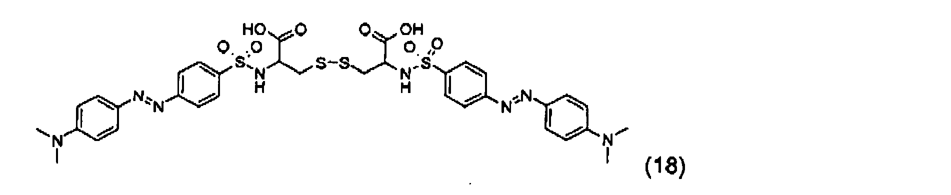 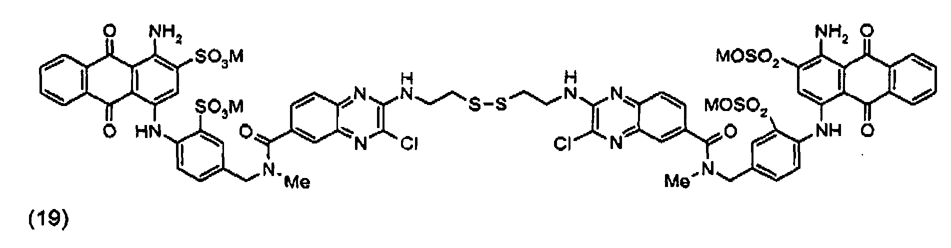 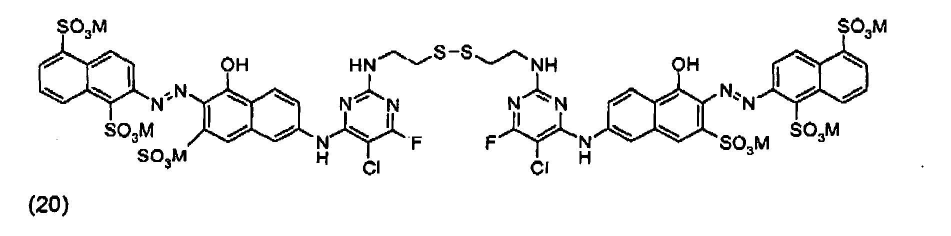 M représentant un atome d'hydrogène, un métal alcalin ou un groupement ammonium ou un groupement ammonium substitué par un ou plusieurs radicaux alkyle, identiques ou non, linéaires ou ramifiés, en C₁-C₁₀ éventuellement porteurs d'au moins un hydroxyle; et les composés suivants, leurs sels, hydrates et solvates :

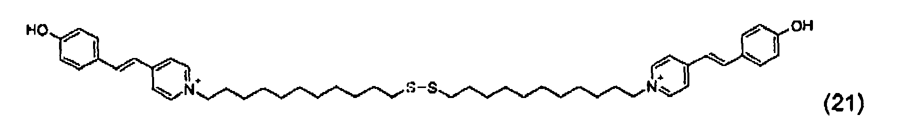

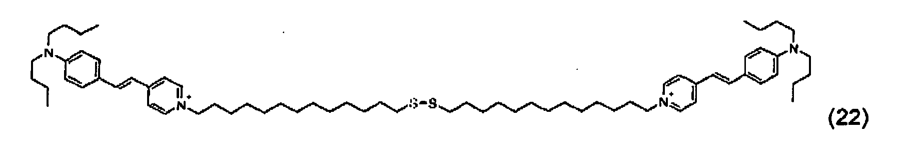

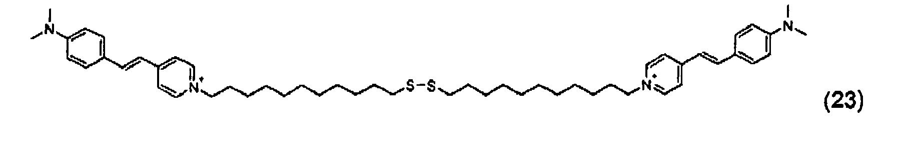

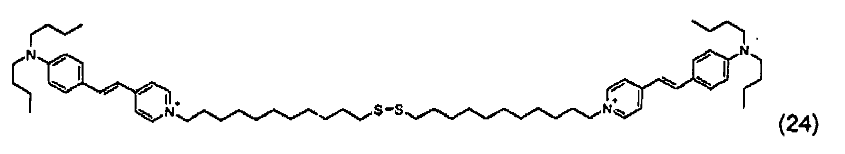

5. Composition selon les revendications 1 à 3, **caractérisée en ce que** le chromophore cationique comprend un radical cationique qui est un ammonium quaternaire.

6. Composition selon la revendication 1, **caractérisée en ce que** le colorant direct disulfure est un colorant de formule (I), (II) ou (III) dans lequel A et/ou A', identiques ou différents, représentent un radical, contenant un ou plusieurs chromophores fluorescents cationiques ou non.

7. Composition selon l'une quelconque des revendications 1 à 3 et 5 à 6, **caractérisée en ce que** X et X', identiques ou différents, représentent un groupement -N(R)-.

8. Composition selon l'une quelconque des revendications 5 à 7, **caractérisée en ce que** le colorant direct disulfure est tel que v ou v' est égal à 0, et A et/ou A', identiques ou différents, plus préférentiellement identiques, représentent W-N=N-Ar- ou -W-N=N-Ar ou W-C(R^{c})=C(R^{d})-Ar- ou -W-C(R^{c})=C(R^{d})-Ar, avec W représentant un hétérocycle, aromatique ou non, condensé ou non, comprenant un ammonium quaternaire ; Ar représente un radical (hétéro)aryle à 5 ou 6 chaînons, ou un bicycle (hétéro)aromatique de type naphtyle, benzopyridinium, indolinyle ou benzoindolinyle, éventuellement substitués par un ou plusieurs atomes d'halogène ; par un ou plusieurs groupements alkyle ; par un ou plusieurs groupements hydroxyle ; par un ou plusieurs groupements alcoxy, par un ou plusieurs groupements hydroxyalkyle, par un ou plusieurs groupements amino ou (di)alkylamino, par un ou plusieurs groupements acylamino ; par un ou plusieurs groupements hétérocycloalkyle ou hétéroaryle à 5 ou 6 chaînons ; R^{c}, R^{d}, identiques ou différents, représentent un atome d'hydrogène ou un groupement alkyle en C₁-C₄.

9. Composition selon l'une quelconque des revendications 1 à 5 et 7 à 8, **caractérisée en ce que** le colorant direct disulfure est choisi parmi : 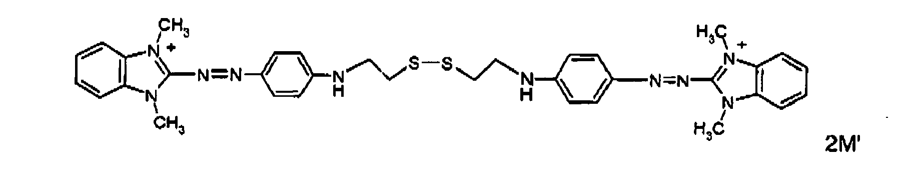 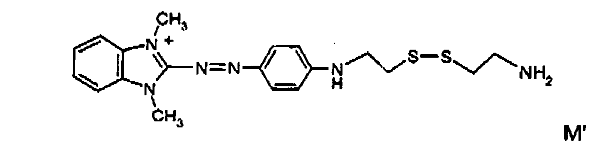 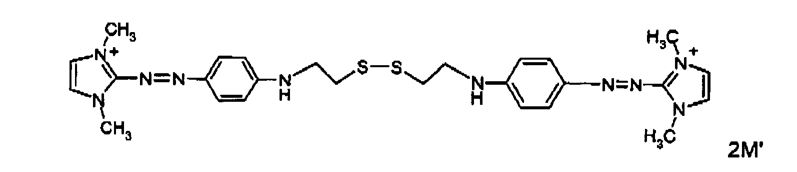 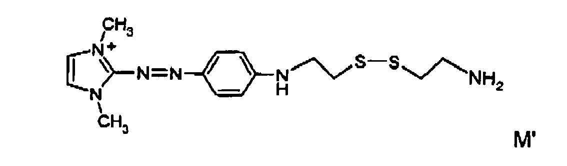 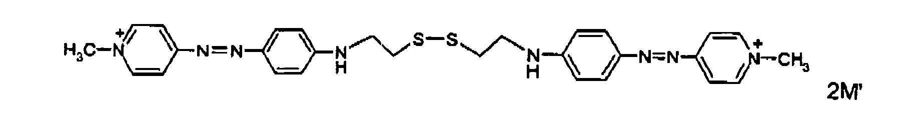 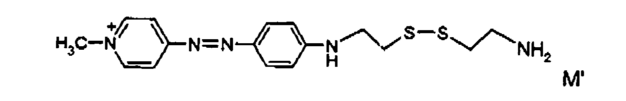 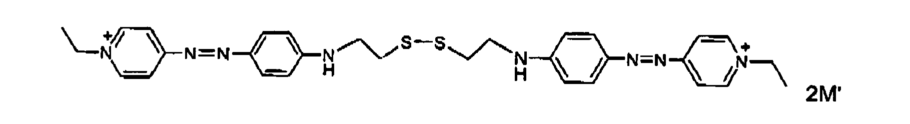 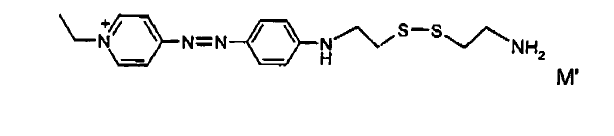 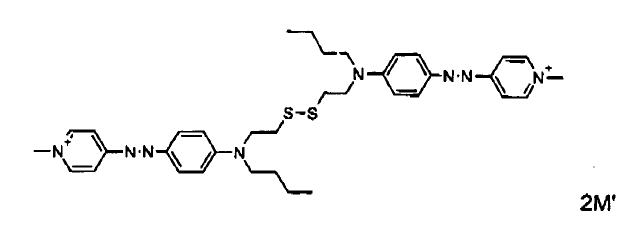 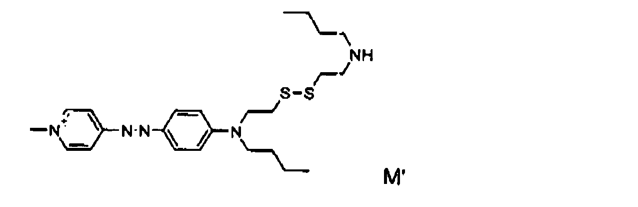 M' représentant un sel d'acide organique ou minéral.

10. Composition selon l'une quelconque des revendications 1 et 6 à 8, **caractérisée en ce que** le colorant direct disulfure est choisi parmi les composés (III) à (XII): 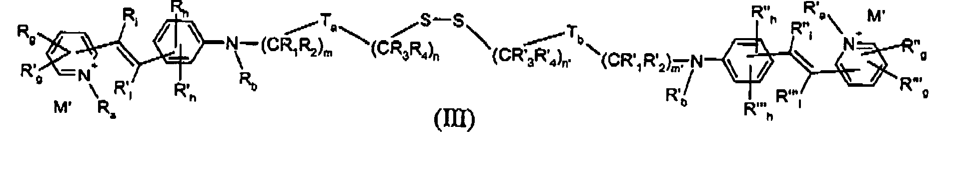 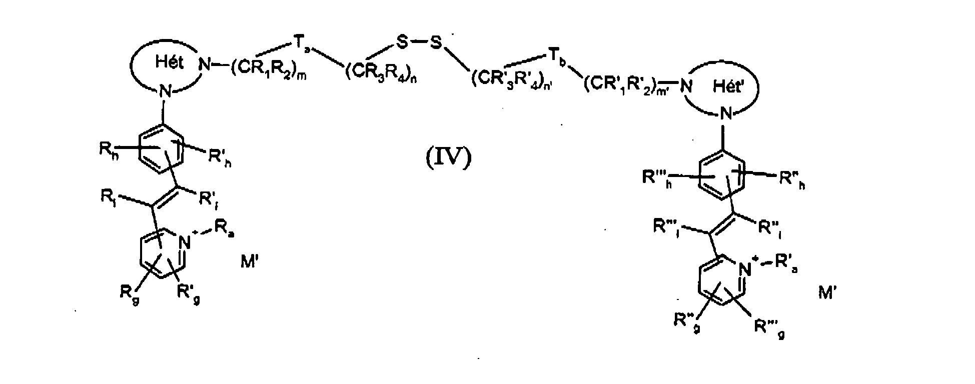 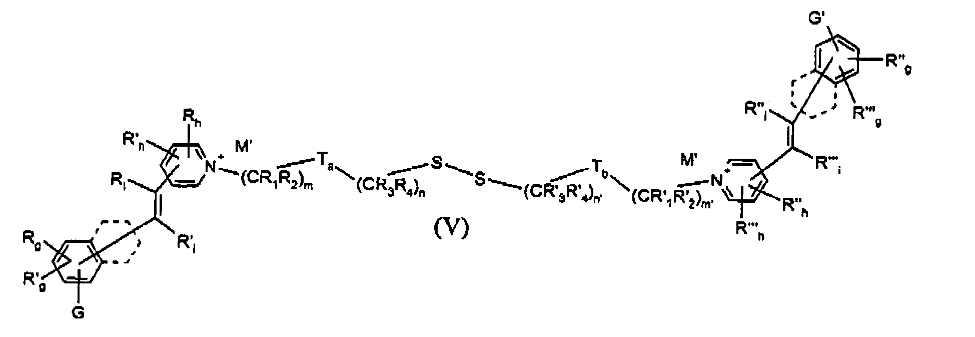 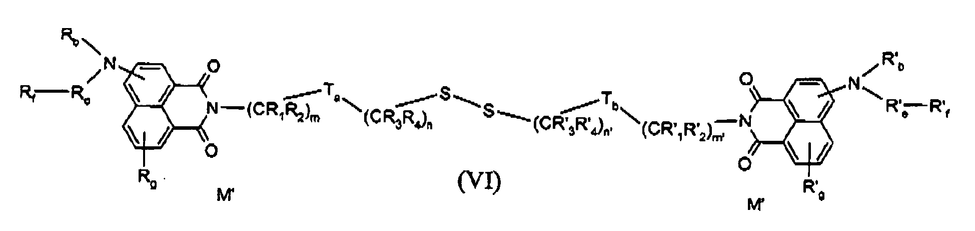 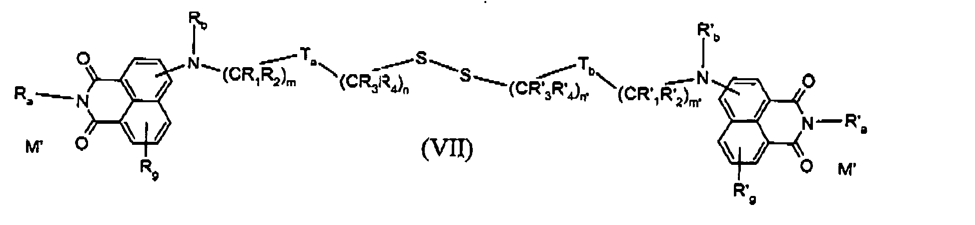 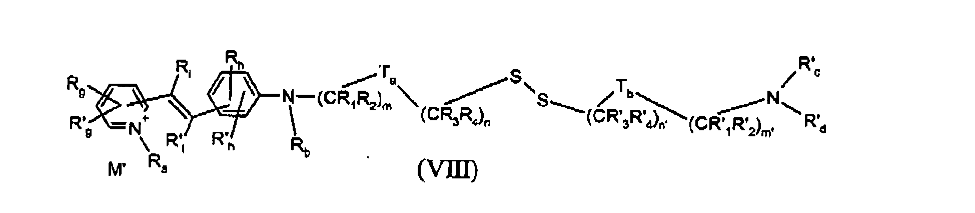 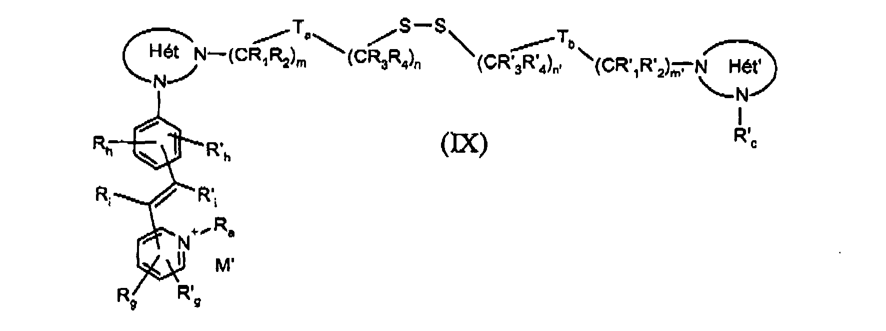 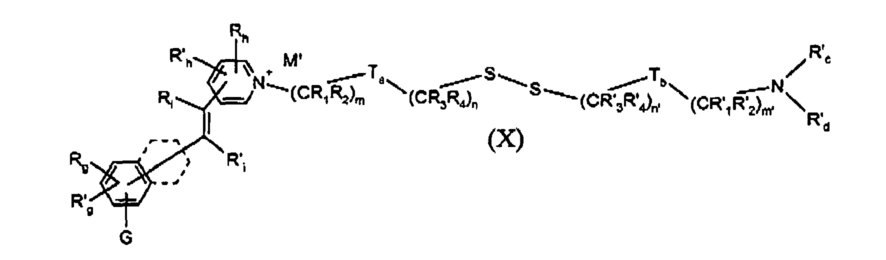 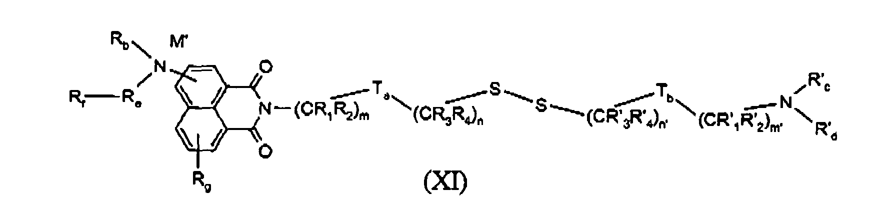 et 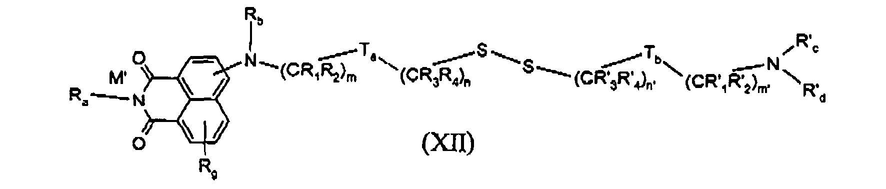 dans lequel
• G et G', identiques ou différents, représentent un groupement -NR_{c}R_{d}, -NR'_{c}R'_{d}, ou C₁-C₆ alkoxy éventuellement substitué; préférentiellement G et G' représentent un groupement -NR_{c}R_{d} et -NR'_{c}R'_{d} respectivement ;
• Rₐ et R'ₐ, identiques ou différents, un groupement aryl(C₁-C₄)alkyle ou un groupement C₁-C₆ alkyle éventuellement substitué par un groupement hydroxyle ou amino, C₁-C₄ alkylamino, C₁-C₄ dialkylamino, lesdits radicaux alkyles pouvant former avec l'atome d'azote qui les portent un hétérocyle comprenant de 5 à 7 chaînons, comprenant éventuellement un autre hétéroatome différent ou non de l'azote ;
• R_{b}, R'_{b}, identiques ou différents, représentent un atome d'hydrogène, un groupement aryl(C₁-C₄)alkyle ou un groupement C₁-C₆ alkyle éventuellement substitué;
• R_{c}, R'_{c}, R_{d} et R'_{d}, identiques ou différents, représentent un atome d'hydrogène, un groupement aryl(C₁-C₄)alkyle, C₁-C₆ alcoxy ou un groupement C₁-C₆ alkyle éventuellement substitué ;
ou alors deux radicaux adjacents R_{c} et R_{d}, R'_{c} et R'_{d} portés par le même atome d'azote forment ensemble un groupe hétérocyclique ou hétéroaryle ;
• Rₑ et R'ₑ, identiques ou différents, représentent une chaîne hydrocarbonée en C₁-C₆ alkylènyle divalente, linéaire ou ramifiée, éventuellement insaturée ;
• R_{f} et R'_{f}, identiques ou différents, représentent un groupement ammonium quaternaire (R")(R"')(R"")N⁺- où R", R'" et R"", identiques ou différents, représentent un atome d'hydrogène ou un groupement C₁-C₄ alkyle ou alors (R")(R"')(R"")N⁺- représente un groupement hétéroaryle cationique éventuellement substitué préférentiellement un groupement imidazolinium éventuellement substitué par un groupement C₁-C₃ alkyle ;
• R_{g}, R'_{g}, R"_{g}, R"'_{g}, Rₕ, R'ₕ, R"ₕ, et R"'ₕ, identiques ou différents, représentent un atome d'hydrogène, un atome d'halogène, un groupement amino, C₁-C₄ alkylamino, C₁-C₄ dialkylamino, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle, alkylsulfonylamino, un radical amino-sulfonyle, ou un radical C₁-C₁₆ alkyle éventuellement substitué par un groupement choisi parmi C₁-C₁₂ alcoxy, hydroxy, cyano, carboxy, amino, C₁-C₄ alkylamino et C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ;
ou alors deux groupements R_{g} et R'_{g} ; R"_{g} et R"'_{g} ; Rₕ, et R'ₕ ; R"ₕ et R"'ₕ portés par deux atomes de carbone adjacents, forment ensembles une cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, C₁-C₄ alkylamino, C₁-C₄ dialkylamino, nitro, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle, alkylsulfonylamino, un radical amino-sulfonyle, ou un radical C₁-C₁₈ alkyle éventuellement substitué par un groupement choisi parmi C₁-C₁₂ alcoxy, hydroxy, cyano, carboxy, amino, C₁-C₄ alkylamino, C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ;
• ou alors lorsque G représente -NR_{c}R_{d} et G' représente -NR'_{c}R'_{d}, deux groupements R_{c} et R'_{g} ; R'_{c} et R"_{g} ; R_{d} et R_{g} ; R'_{d} et R"'_{g} forment ensemble un hétéroaryle ou hétérocycle saturé, éventuellement substitué par un groupement C₁-C₆ alkyle;
• Rₗ, R'ₗ, R"ₗ, et R"'ₗ, identiques ou différents, représentent un atome d'hydrogène, ou un groupement C₁-C₄ alkyle ;
• R₁, R₂, R₃, R₄, R'₁, R'₂, R'₃ et R'₄, identiques ou différents, représentent un atome d'hydrogène ou un groupement C₁-C₄ alkyle, C₁-C₁₂ alcoxy, hydroxy, cyano, carboxy, amino, C₁-C₄ alkylamino, C₁-C₄ dialkylamino, lesdits radicaux alkyles pouvant former avec l'atome d'azote qui les portent un hétérocyle comprenant de 5 à 7 chaînons, comprenant éventuellement un autre hétéroatome différent ou non de l'azote ;
• Tₐ et T_{b}, identiques ou différents, représentent i) soit une liaison covalent σ, ii) soit un ou plusieurs radicaux ou leurs combinaisons choisis parmi -SO₂-, -O-,-S-, -N(R)-, -N⁺(R)(R°), -CO-, avec R, R°, identiques ou différents, représentant un atome d'hydrogène, un radical alkyle en C₁-C₄, hydroxyalkyle en C₁-C₄; ou un aryl(C₁-C₄)alkyle, iii) soit un radical hétérocycloalkyle ou hétéroaryle, cationique ou non;
• 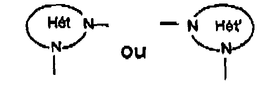 identiques ou différents, représentent une groupement hétérocyclique éventuellement substitué ;
• 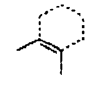 représentent une groupement aryle ou hétéroaryle fusionné au cycle
- phényle ; ou alors est absent du cycle phényle;
• m, m', n et n', identiques ou différents, représentent un entier compris inclusivement entre 0 et 6 avec m+n, m'+n', identiques ou différents, représentent un entier compris inclusivement entre 1 et 10;
M' représentant un sel d'acide organique ou minéral.

11. Composition selon l'une des revendications 1, 6 à 8 et 10, **caractérisée en ce que** le colorant direct disulfure est choisi parmi : 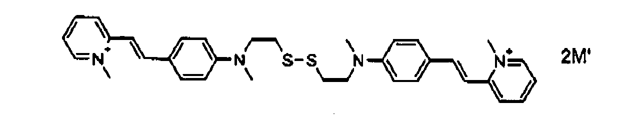 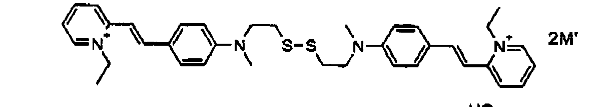 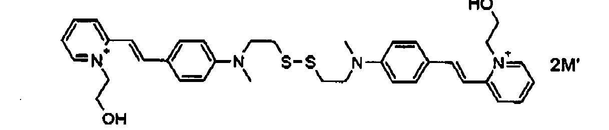 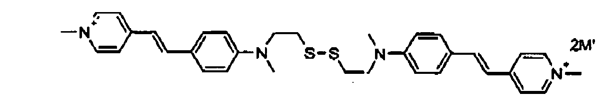 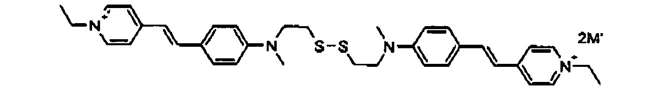 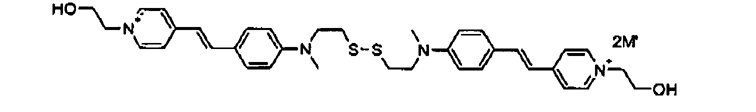 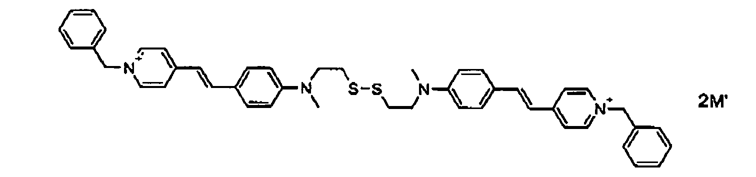 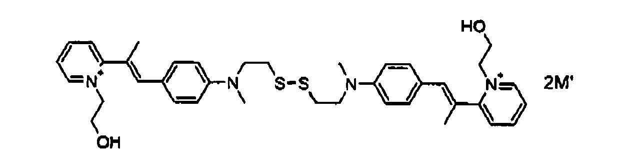 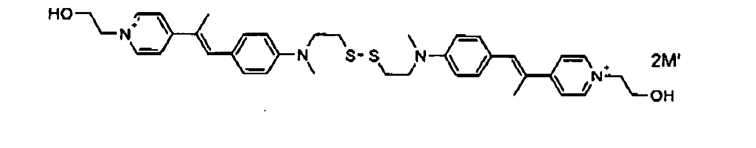 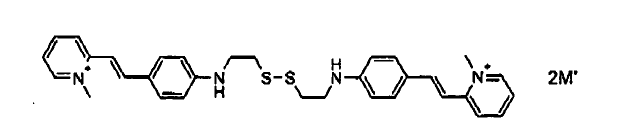 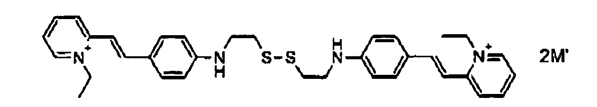 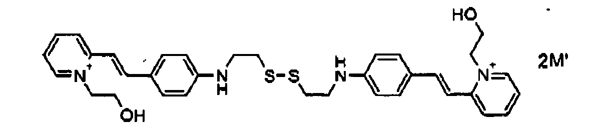 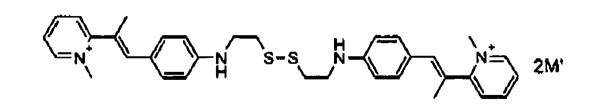 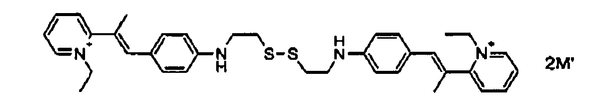 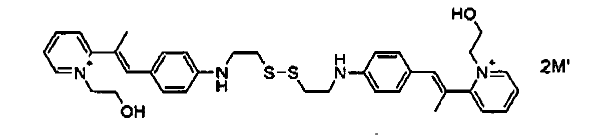 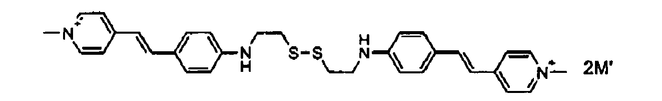 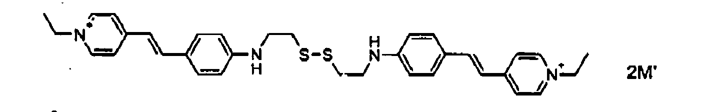 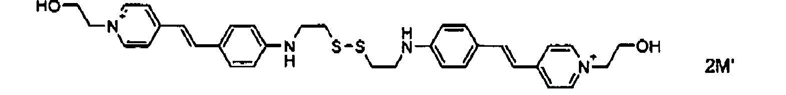 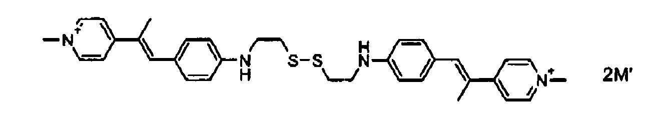 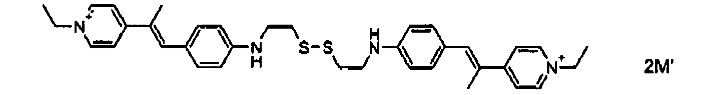 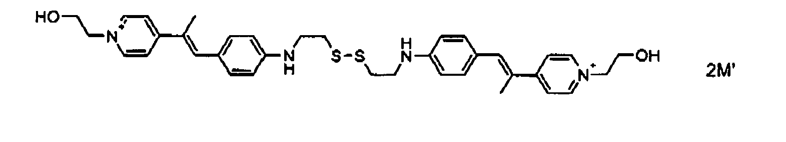 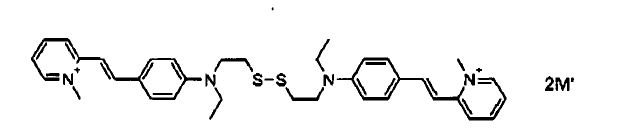 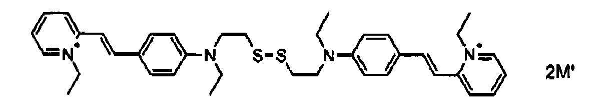 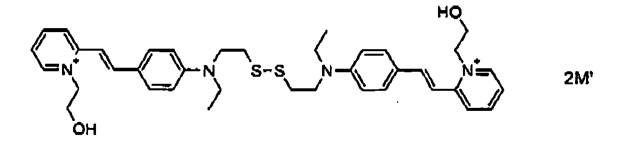 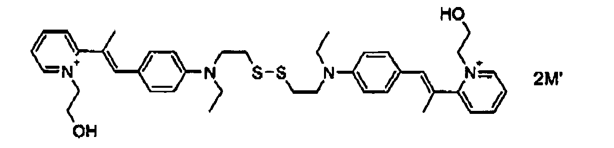 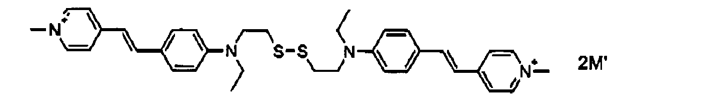 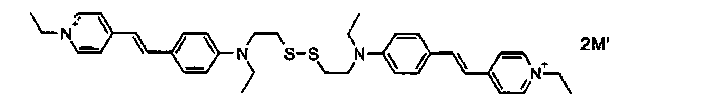 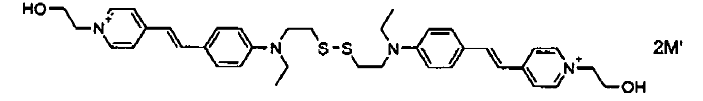 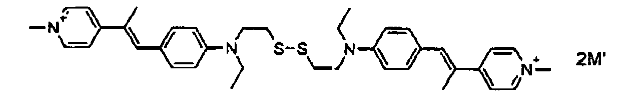 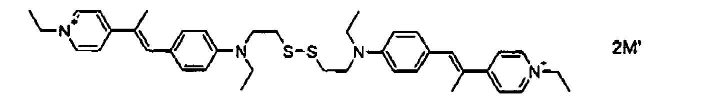 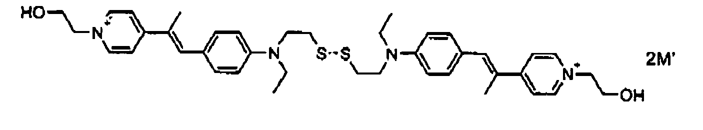 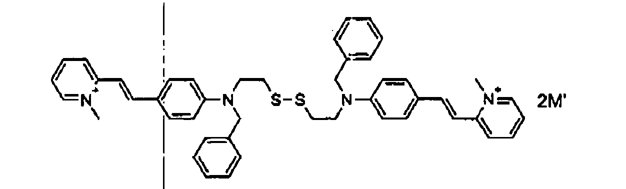 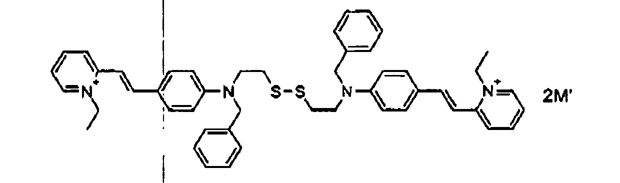 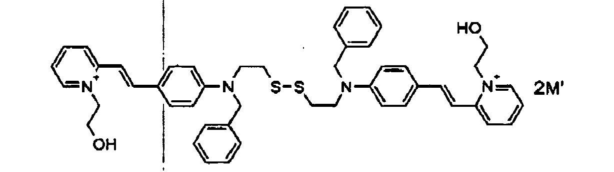 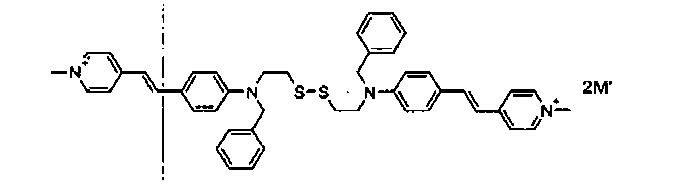 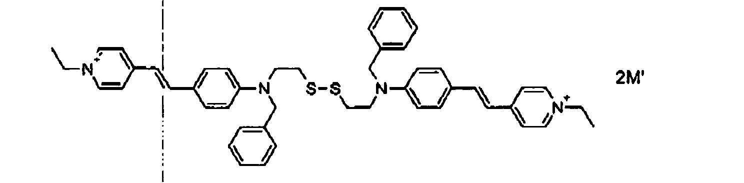 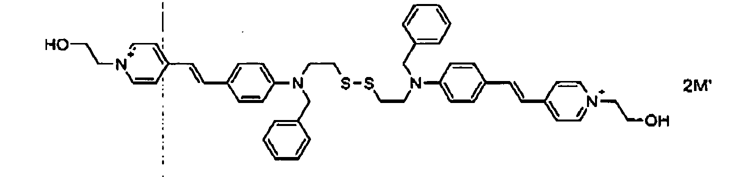 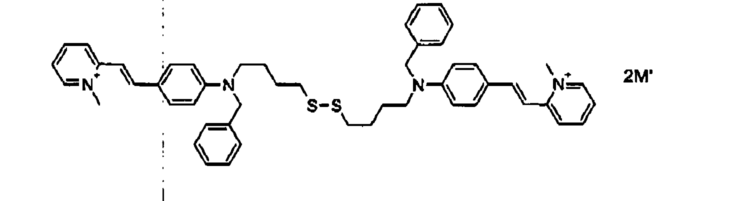 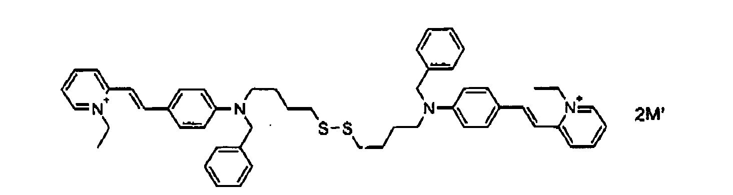 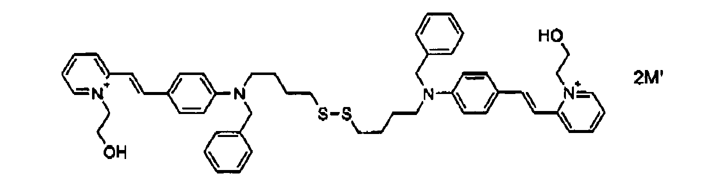 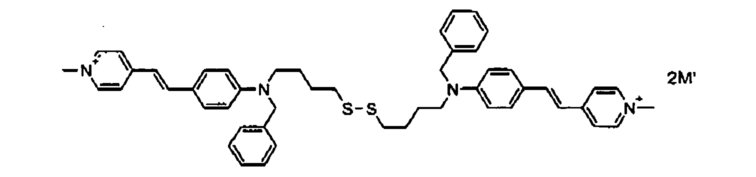 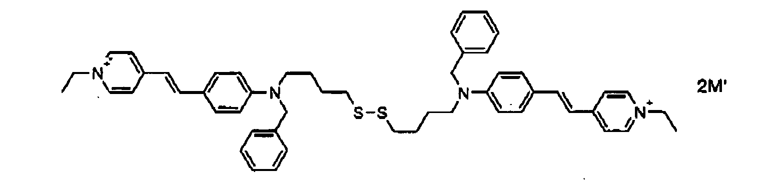 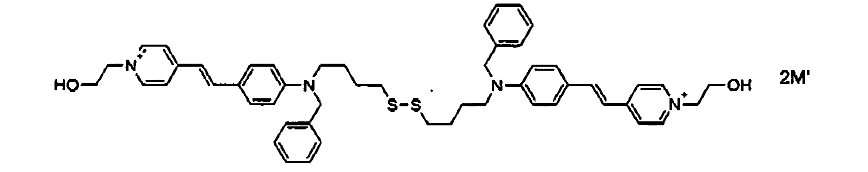 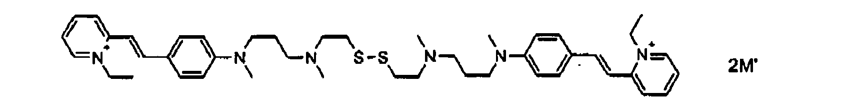 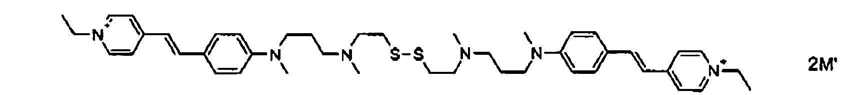 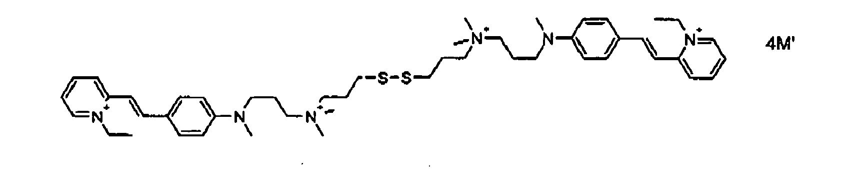 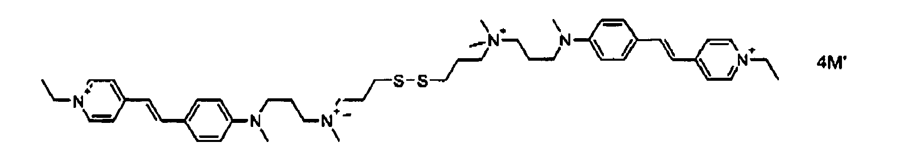 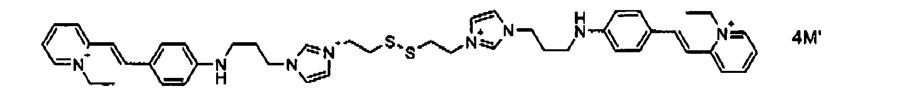 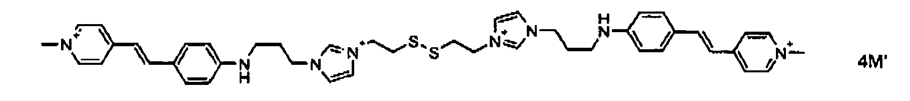 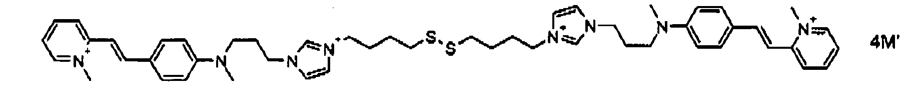 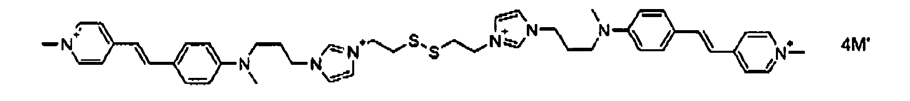 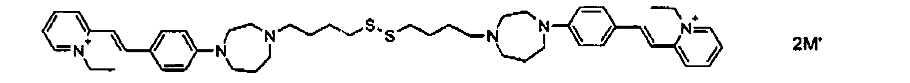 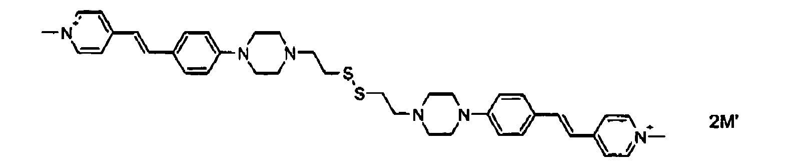 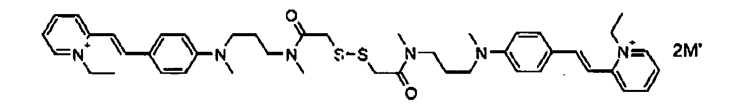 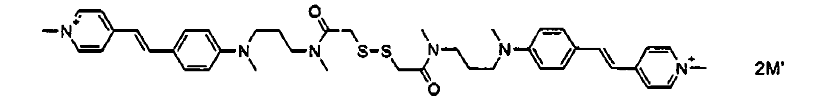 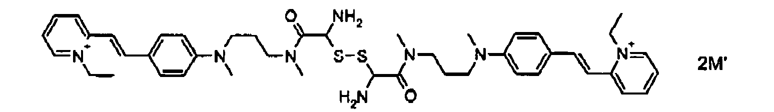 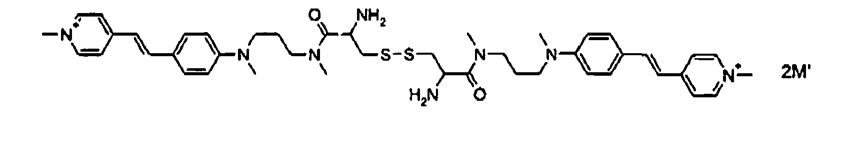 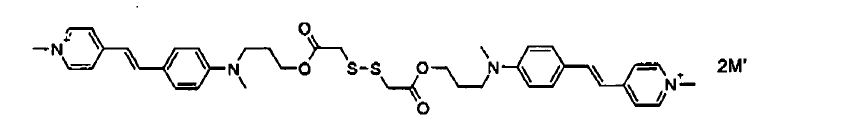 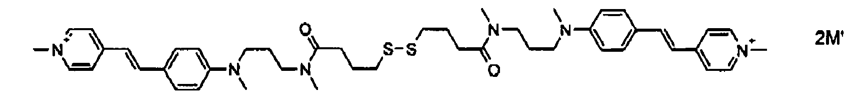 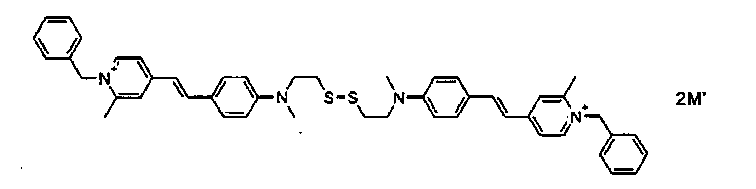 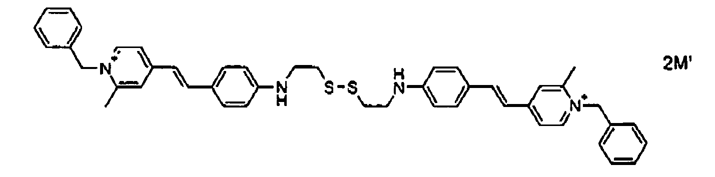 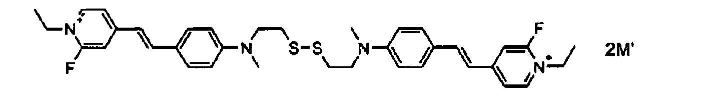 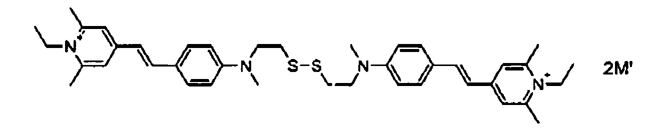 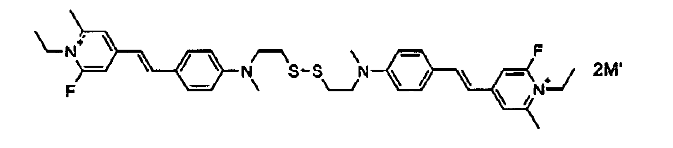 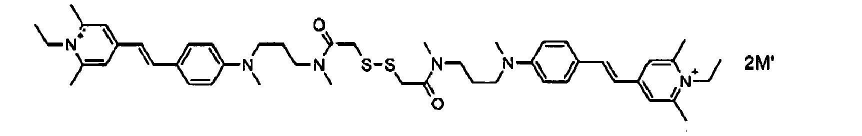 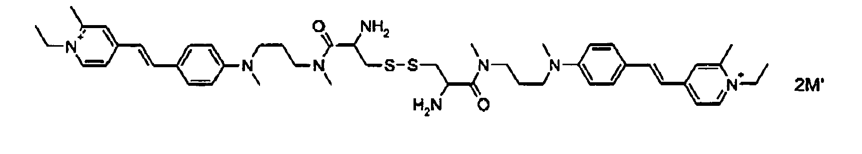 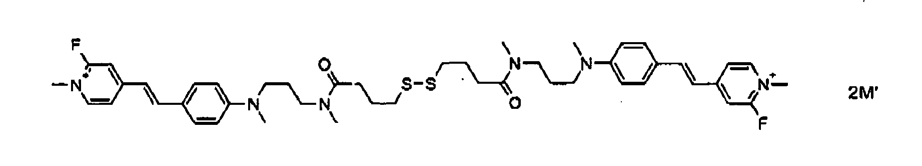 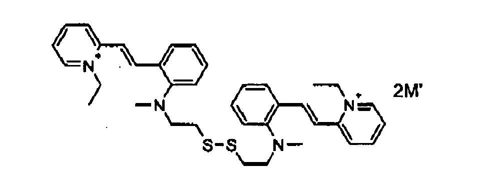 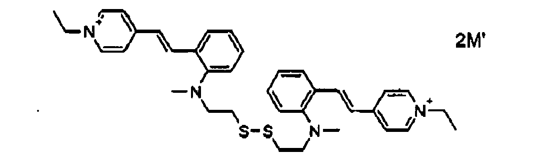 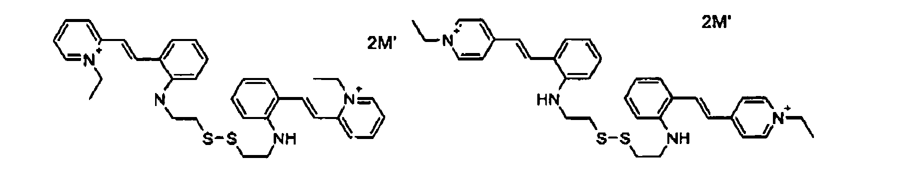 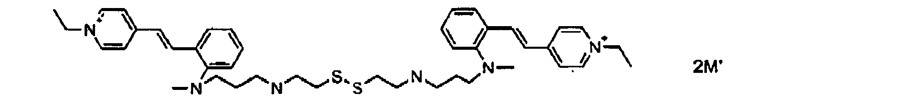 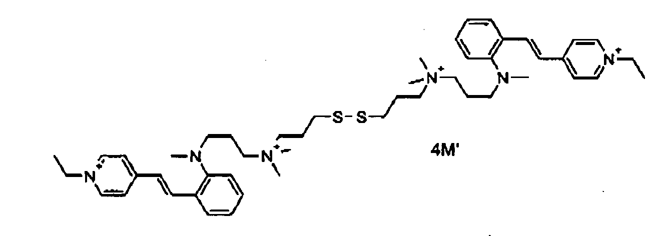 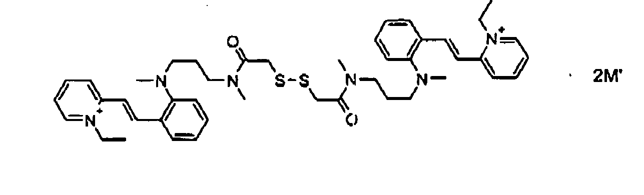 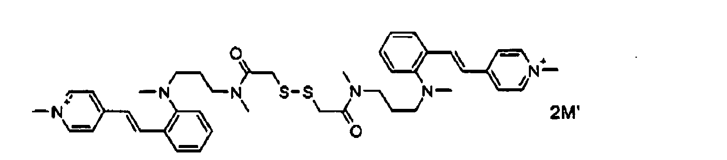 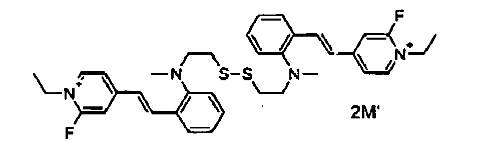 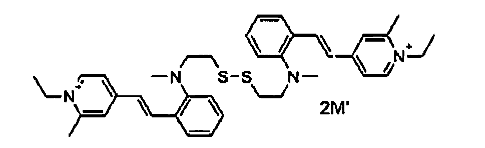 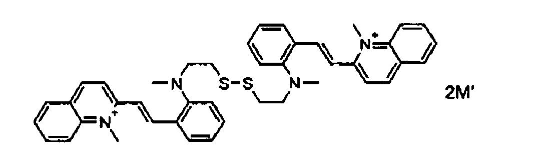 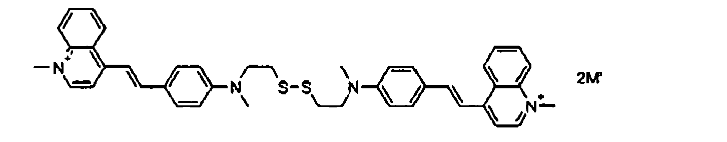 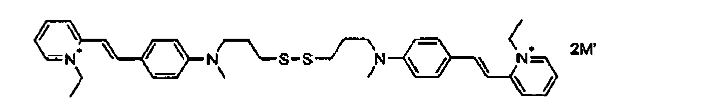 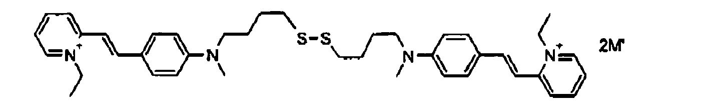 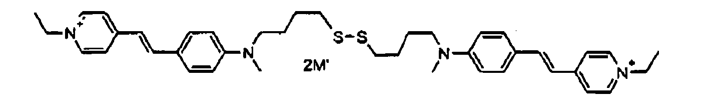 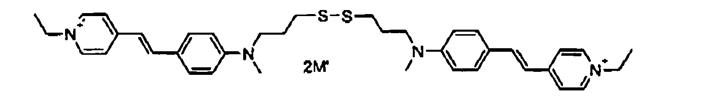 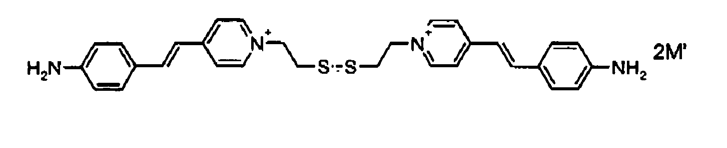 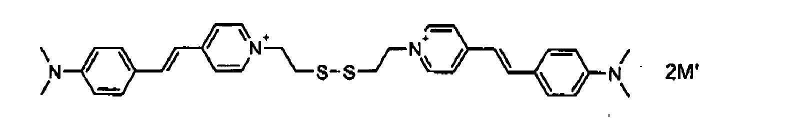 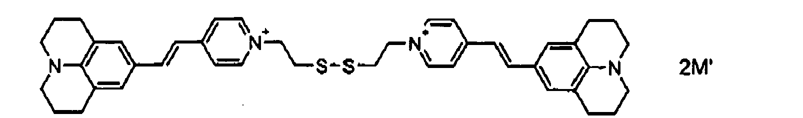 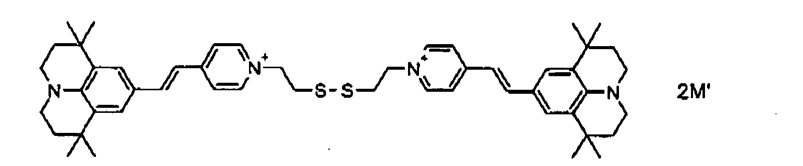 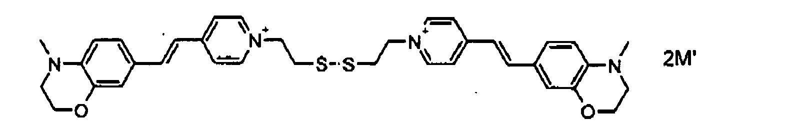 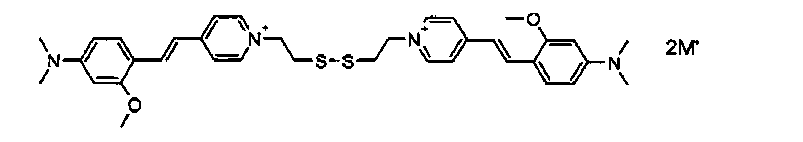 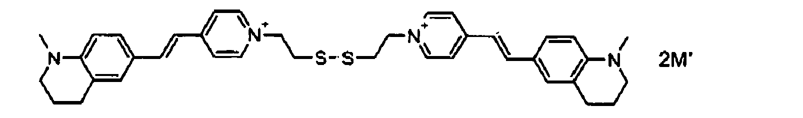 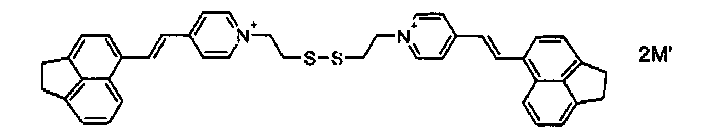 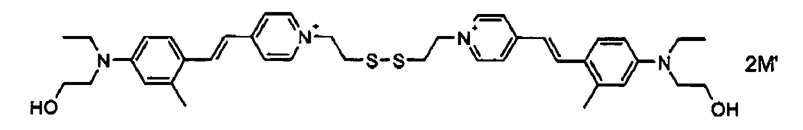 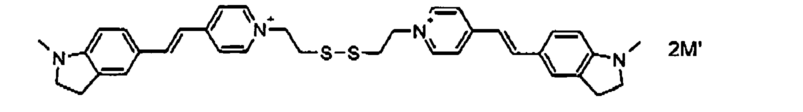 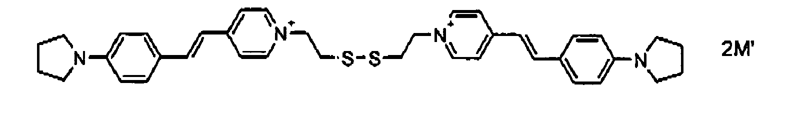 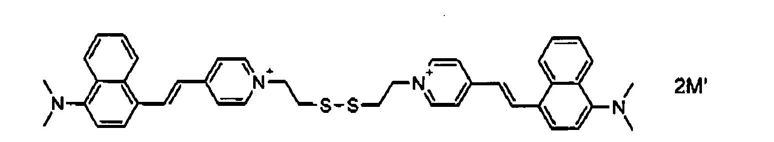 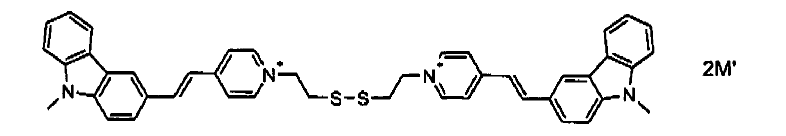 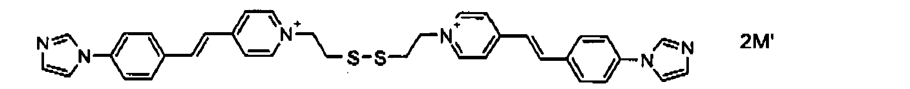 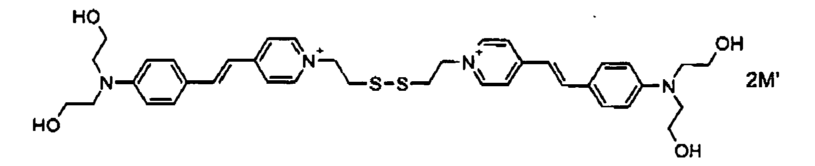 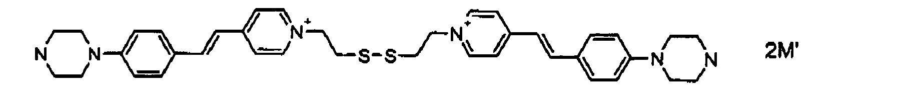 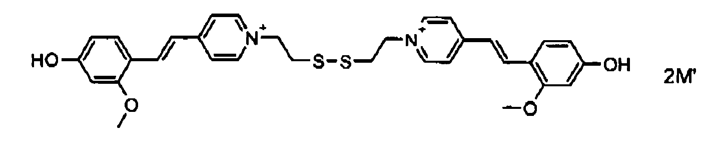 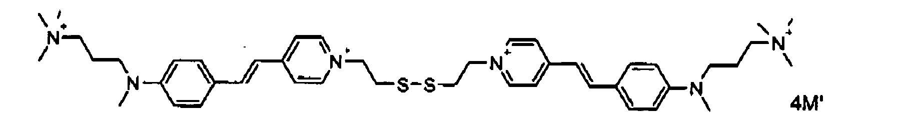 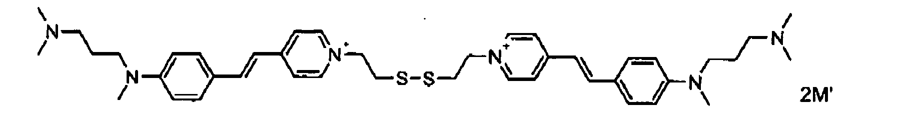 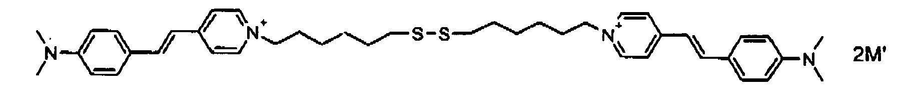 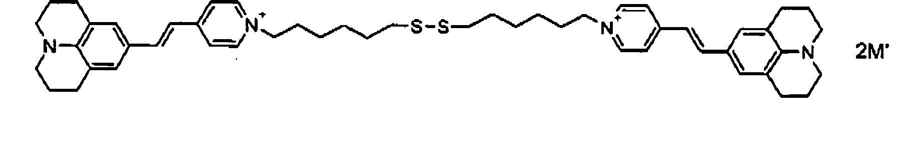 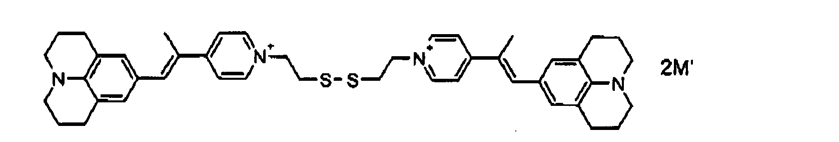 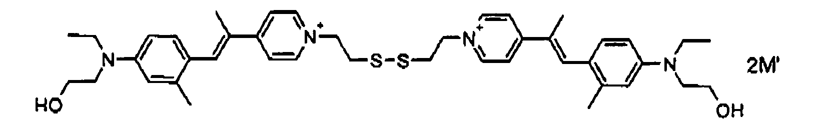 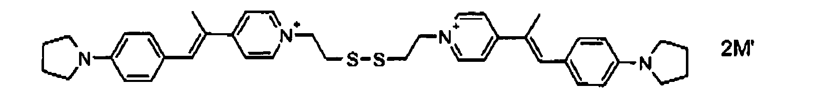 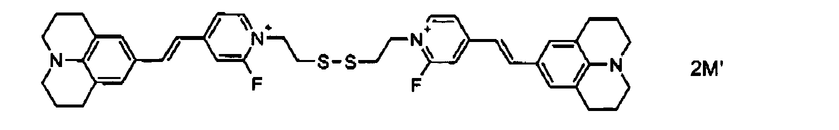 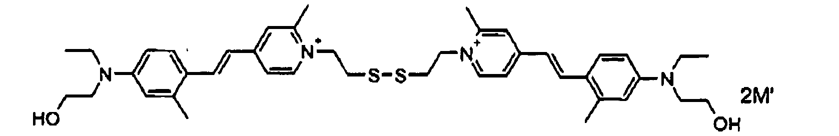 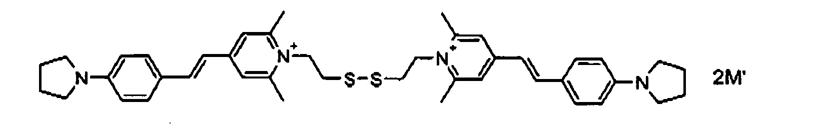 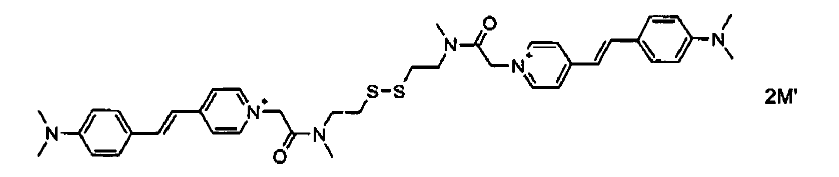 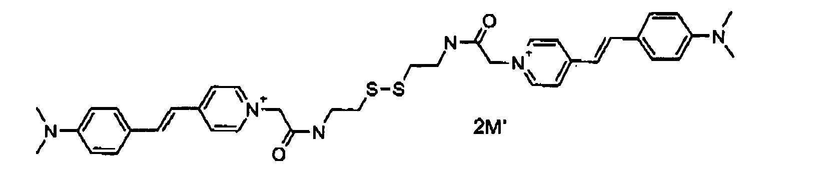 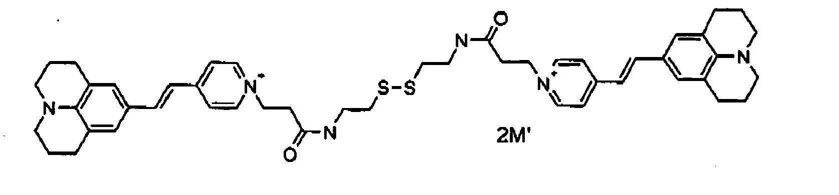 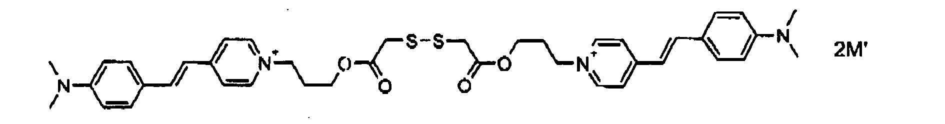 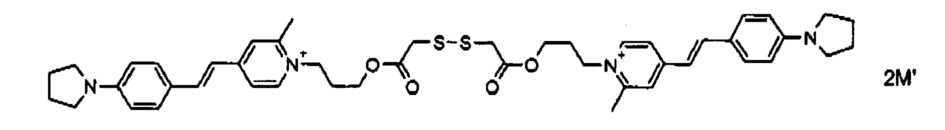 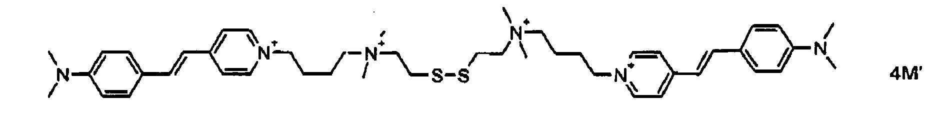 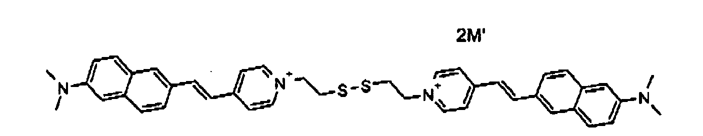 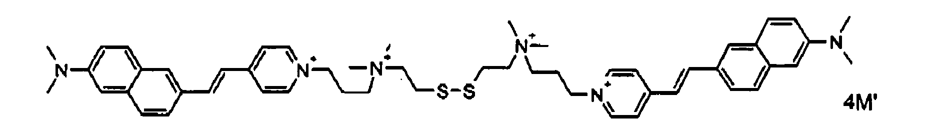 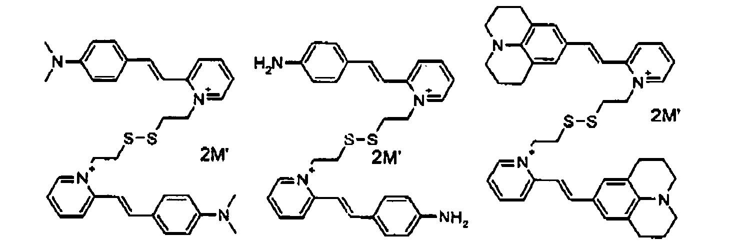 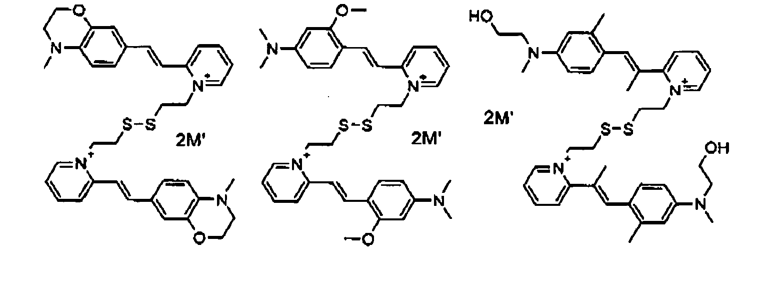 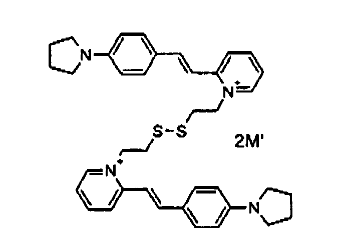 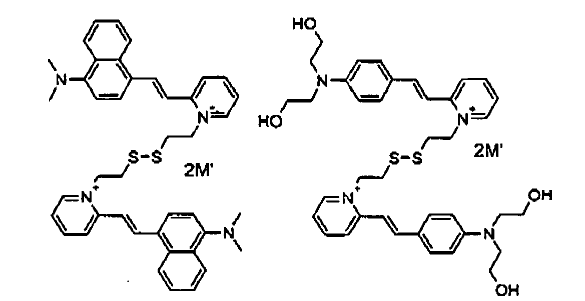 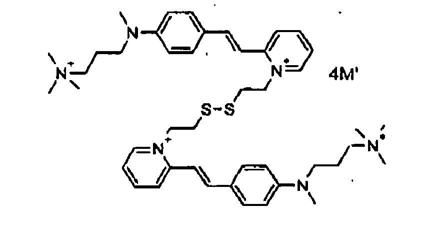 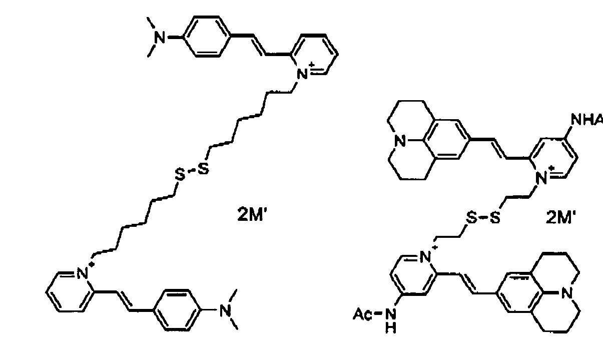 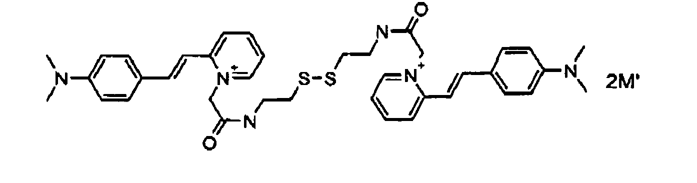 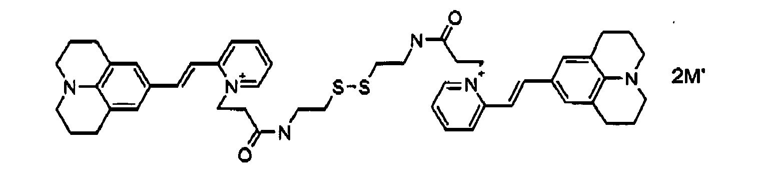 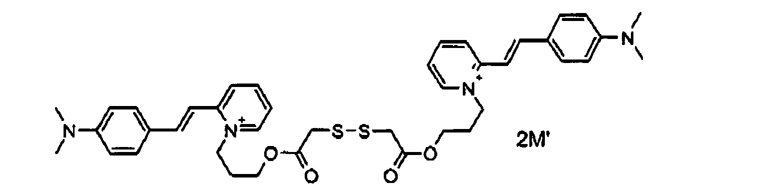 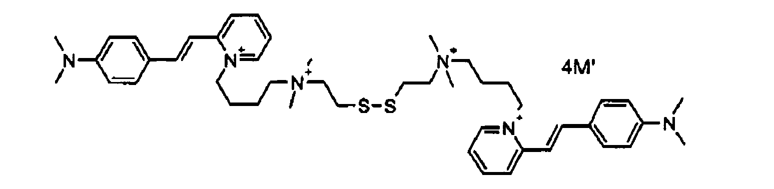 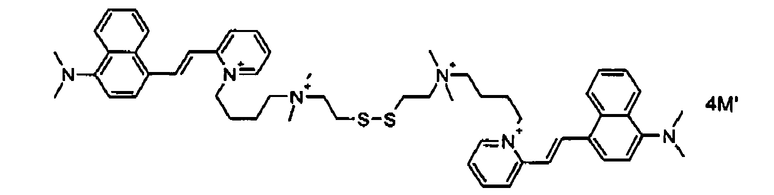 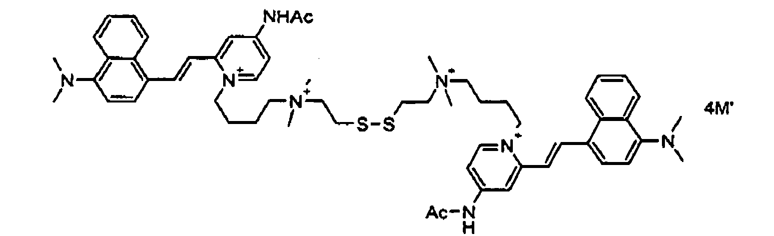 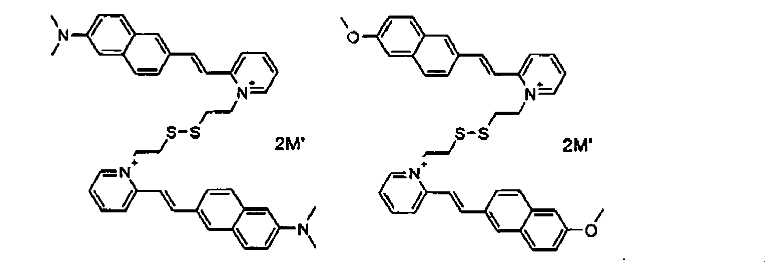 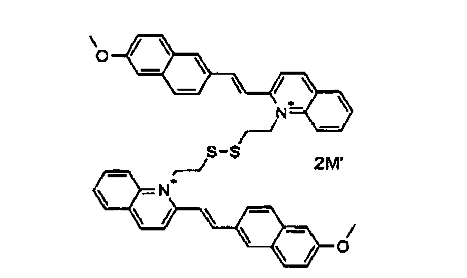 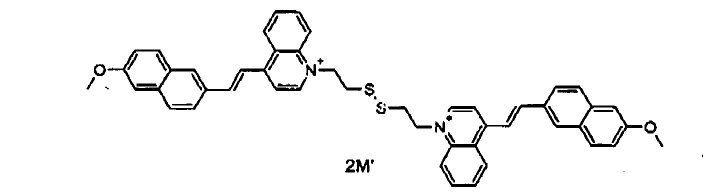 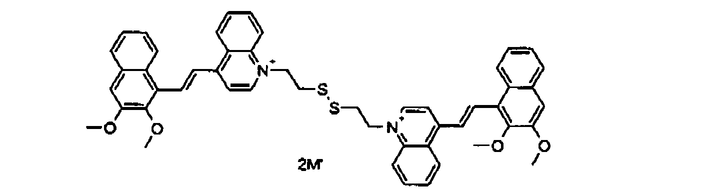 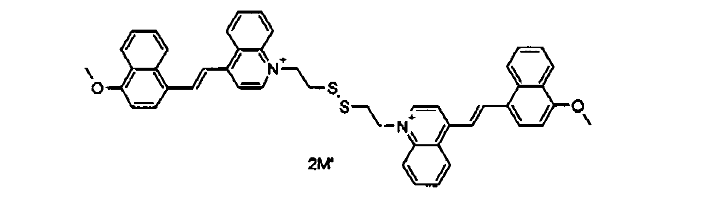 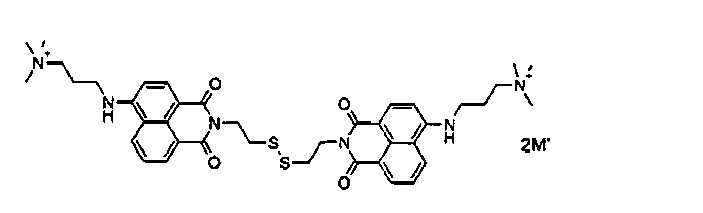 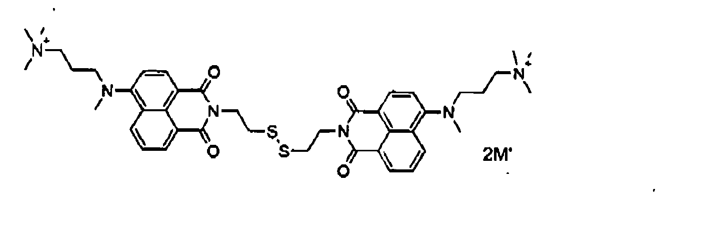 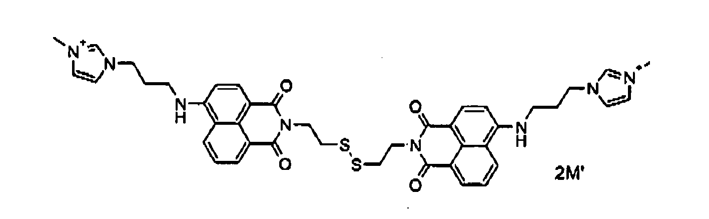 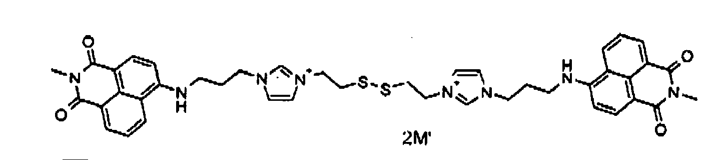 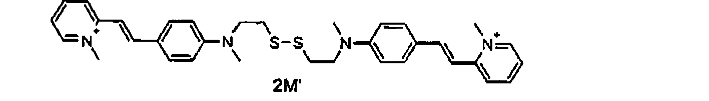 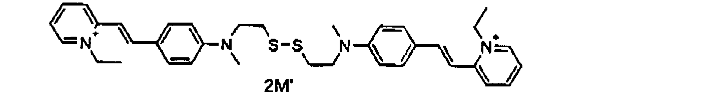 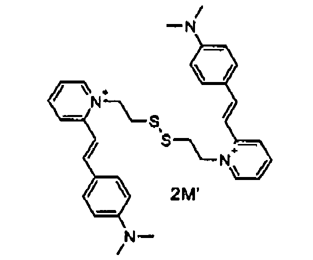 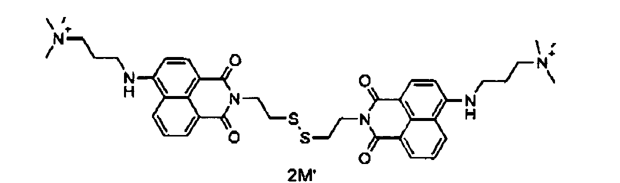 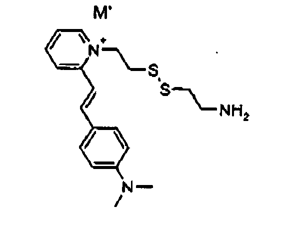 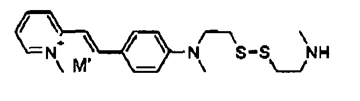 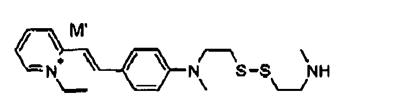 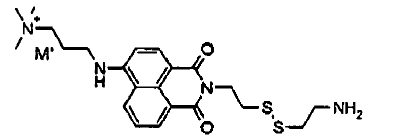 et 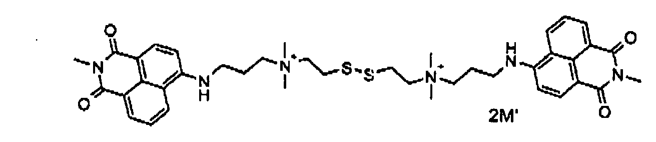 avec M' représentant un sel d'acide organique ou minéral.

12. Composition selon l'une quelconque des revendications précédentes, **caractérisée en ce que** l'agent alcalin de type hydroxyde minéral ou organique est choisi parmi les hydroxydes de métaux alcalins, alcalino-terreux, de métaux de transition, en particulier des groupes IIIB, IVB, VB et VIB, de lanthanides ou d'actinides, les hydroxydes d'ammonium, de guanidine ou leurs mélanges.

13. Procédé de mise en forme, de coloration et/ou d'éclaircissement simultanés des fibres kératiniques, **caractérisé en ce que** l'on applique la composition tinctoriale telle que définie selon l'une quelconque des revendications 1 à 12 sur les fibres kératiniques pendant un temps suffisant pour obtenir la mise en forme et la coloration, et/ou l'éclaircissement souhaités, puis on rince éventuellement les fibres kératiniques et on les lave au shampoing.

14. Dispositif à plusieurs compartiments, **caractérisé par le fait qu'**il comporte un premier compartiment (i) comprenant un ou plusieurs agents alcalins de type hydroxyde minéral ou organique selon la revendication 12, et un deuxième compartiment (ii) comprenant une composition contenant un ou plusieurs colorants directs disulfures selon l'une des revendications 1 à 11, le pH du mélange du contenu des différents compartiments étant compris entre 10 et 14.

## Patentansprüche

1. Zusammensetzung zur Behandlung von Keratinfasern, die in einem kosmetisch unbedenklichen Medium Folgendes umfasst:
* einen oder mehrere Disulfid-Direktfarbstoffe, ausgewählt aus Verbindungen der folgenden Formeln (I), (II) oder (III): 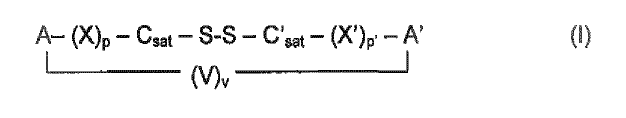 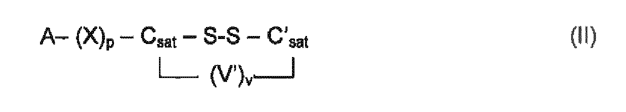
A- (X)p - Cₛₐₜ - S-S - C'ₛₐₜ - (X')_{p'} - D (III)
deren Salzen, optischen Isomeren, geometrischen Isomeren und Solvaten wie Hydraten,
wobei in den Formeln:
• A und A' gleich oder verschieden sind und für einen Rest mit einem oder mehreren kationischen oder nichtkationischen Chromophoren stehen;
• V und V' gleich oder verschieden sind und für eine Brückengruppe stehen;
• v und v' gleich oder verschieden sind und für 0 oder 1 stehen;
• die Koeffizienten p und p' gleich oder verschieden sind und für eine ganze Zahl mit einem Wert von 0 oder 1 stehen;
• X und X' gleich oder verschieden sind und für Folgendes stehen:
1) eine gesättigte oder ungesättigte, lineare oder verzweigte C₁-C₃₀-Kohlenwasserstoffkette, die gegebenenfalls durch eine oder mehrere zweiwertige Gruppen oder Kombinationen davon, die aus:
-N(R)-, -N⁺(R) (R') -, -O-, -S-, -CO- und -SO₂-, wobei R und R' gleich oder verschieden sind und aus einem Wasserstoff und einem C₁-C₄-Alkyl-, Hydroxyalkyl- und Aminoalkylrest,
einem kondensierten oder nichtkondensierten, gesättigten oder ungesättigten, aromatischen oder nichtaromatischen (hetero)cyclischen Rest, der gegebenenfalls ein oder mehrere gleiche oder verschiedene Heteroatome enthält und gegebenenfalls substituiert ist,
ausgewählt sind, unterbrochen und/oder an einem oder beiden ihrer Enden durch diese Gruppen oder Kombinationen davon terminiert ist;
oder
2) die folgende Sequenz:
-(T)ₜ-(Y)y-(Z)_{z}-
wobei die Sequenz in den Formeln (I), (II) oder (III) folgendermaßen gebunden ist:
-Cₛₐₜ(bzw. C'ₛₐₜ) - (T)ₜ - (Y)_{y} - (Z)_{z} - A (bzw. A');
worin:
(i) T für einen oder mehrere Reste oder Kombinationen davon, die aus -SO₂-, -O-, -S-, -N (R) -, -N⁺ (R) (R') - und -CO- ausgewählt sind, wobei R und R' gleich oder verschieden sind und für ein Wasserstoffatom oder einen C₁-C₄-Alkyl- oder C₁-C₄-Hydroxyalkylrest stehen, steht;
der Koeffizient t gleich 0 oder 1 ist;
(ii) Y für:
- einen Rest, der aus -CO-, -(CH₂)-SO₂, (CH₂)₂- So₂-; -CH₂-CHR-CO-NR'- ausgewählt ist, wobei R und R' gleich oder verschieden sind und für ein Wasserstoffatom oder einen C₁-C₄-Alkylrest stehen;
- eine Gruppe der Formel (a), (a') oder (a''): 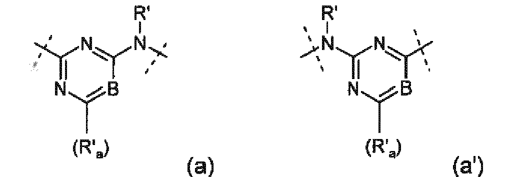 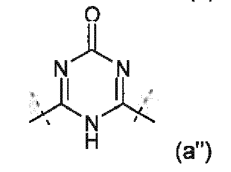
worin:
• B für -N- oder -CRₐ- steht, wobei
Rₐ für
- ein Wasserstoffatom,
- ein Halogenatom, das aus Chlor oder Fluor ausgewählt ist,
- eine Nitrogruppe,
- eine gegebenenfalls substituierte Pyridiniumgruppe,
- eine Hydroxylgruppe,
- eine Amino-, Alkylamino- oder Dialkylaminogruppe, wobei die gleichen oder verschiedenen linearen oder verzweigten C₁-C₁₈-Alkylgruppen gegebenenfalls durch ein Heteroatom, das aus N und O ausgewählt ist, unterbrochen und gegebenenfalls durch eine oder mehrere Hydroxylgruppen substituiert sind,
- eine NHNHCOR-Gruppe, wobei R für eine lineare oder verzweigte C₁-C₁₀-Alkylgruppe steht,
steht;
• R' die gleiche Definition wie oben hat;
• R' a für :
- ein Wasserstoffatom,
- ein Chloratom oder ein Fluoratom,
- eine Pyridiniumgruppe, die gegebenenfalls durch mindestens eine Gruppe R_{c} substituiert ist, wobei E_{c} für eine C₁-C₄-Alkylgruppe, ein Halogenatom, eine Carboxylgruppe -COOM (wobei M für ein Wasserstoffatom, ein Alkalimetall, eine Ammoniumgruppe oder eine durch einen oder mehrere gleiche oder verschiedene lineare oder verzweigte C₁-C₁₈-Alkylreste, die gegebenenfalls mindestens ein Hydroxyl tragen, substituierte Ammoniumgruppe steht), eine Estergruppe -COOR_{d}, wobei R_{d} für einen C₁-C₄-Alkylrest steht, eine Amidgruppe -CON(R_{d})₂, wobei die R_{d}-Gruppen gleich oder verschieden sind und für ein Wasserstoffatom oder einen C₁-C₄-Alkylrest stehen, stehen kann,
- eine Hydroxylgruppe,
- eine Amino-, Alkylamino- oder Dialkylaminogruppe, wobei die gleichen oder verschiedenen linearen oder verzweigten C₁-C₁₈-Alkylgruppen gegebenenfalls durch ein Heteroatom, das aus N und O ausgewählt ist, unterbrochen und gegebenenfalls durch eine oder mehrere Hydroxylgruppen substituiert sind,
- eine NHNHCOR-Gruppe, wobei R für eine lineare oder verzweigte C₁-C₁₀-Alkylgruppe steht,
steht,
- eine Gruppe der folgenden Formel (b): 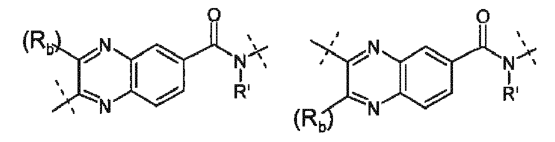 worin
• R' die gleiche Definition wie oben hat;
• R_{b} für:
- ein Chloratom,
- eine Amino-, Alkylamino- oder Dialkylaminogruppe, wobei die gleichen oder verschiedenen linearen oder verzweigten C₁-C₁₈-Alkylgruppen gegebenenfalls durch ein Heteroatom, das aus N, O und S ausgewählt ist, unterbrochen und gegebenenfalls durch eine oder mehrere Hydroxylgruppen substituiert sind,
- einen gesättigten oder ungesättigten stickstoffhaltigen Heterocyclus, der substituiert sein kann,
- eine Arylaminogruppe, wobei der Arylrest vorzugsweise C₆ ist, steht,
steht;
y gleich 0 oder 1 ist;
(iii) Z für:
- - (CH2)ₘ-, wobei m für eine ganze Zahl zwischen 1 und 8 steht,
- - (CH₂CH₂O)_{q}- oder - (OCH₂CH₂)_{q}-, worin q für eine ganze Zahl zwischen 1 und 15 steht,
- einen Aryl-, Alkylaryl- oder Arylalkylrest, dessen Alkylrest C₁-C₄ ist und dessen Arylrest vorzugsweise C₆ ist und der gegebenenfalls durch mindestens eine SO₃M-Gruppe substituiert ist, wobei M für ein Wasserstoffatom, ein Alkalimetall oder eine durch einen oder mehrere gleiche oder verschiedene lineare oder verzweigte C₁-C₁₈-Alkylreste, die gegebenenfalls mindestens ein Hydroxyl tragen, substituierte Ammoniumgruppe steht,
steht,
z für 0 oder 1 steht;
• Cₛₐₜ und C'ₛₐₜ gleich oder verschieden sind und für eine gegebenenfalls cyclische, gegebenenfalls substituierte, lineare oder verzweigte C₁-C₁₈-Alkylenkette stehen;
• D einem aus Hydroxy-, Hydroxyalkyl-, Alkoxy-, Carboxyl-, Carboxylat-, Amino-, Alkylamino- und Dialkylaminoresten ausgewählten Rest entspricht,
und
* ein oder mehrere alkalische Mittel vom Typ anorganisches oder organisches Hydroxid in einer solchen Menge, dass der pH-Wert der Zusammensetzung zwischen 10 und 14 liegt.

2. Zusammensetzung nach Anspruch 1, **dadurch gekennzeichnet, dass** in den Formeln (I), (II) oder (III) dann, wenn p und/oder p' respektive gleich 1 sind, X und/oder X' respektive für die folgende Sequenz stehen:
[-(T)ₜ-(Y)_{y}-(Z)_{z}-
wobei die Sequenz in den Formeln (I), (II) oder (III) folgendermaßen gebunden ist: -Cₛₐₜ (bzw. C'ₛₐₜ) - (T)ₜ-(Y)_{y} - (Z)_{z}- A (bzw. A');
worin:
T für einen oder mehrere Reste oder Kombinationen davon, die aus -SO₂, -O-, -S-, -N(R)-, -N⁺(R)(R')- und -CO- ausgewählt sind, wobei R und R' gleich oder verschieden sind und für ein Wasserstoffatom oder einen C₁-C₄-Alkyl- oder C₁-C₄-Hydroxyalkylrest stehen, steht; der Koeffizient t gleich 0 oder 1, ist;
Y für:
- einen Rest, der aus -CO-, - (CH2) -SO₂, - (CH2)₂- SO₂-; -CH₂-CHR-CO-NR'- ausgewählt ist, wobei R und R' gleich oder verschieden sind und für ein Wasserstoffatom oder einen C₁-C₄-Alkylrest stehen;
- eine Gruppe der Formel (a), (a') oder (a"): 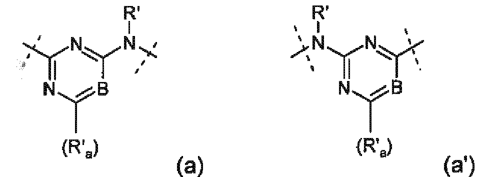 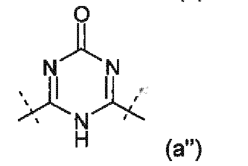
worin:
• B für -N- oder -CRₐ- steht, wobei
Rₐ für
- ein Wasserstoffatom,
- ein Halogenatom, das aus Chlor oder Fluor ausgewählt ist,
- eine Nitrogruppe,
- eine gegebenenfalls substituierte Pyridiniumgruppe,
- eine Hydroxylgruppe,
- eine Amino-, Alkylamino- oder Dialkylaminogruppe, wobei die gleichen oder verschiedenen linearen oder verzweigten C₁-C₁₈-Alkylgruppen gegebenenfalls durch ein Heteroatom, das aus N und O ausgewählt ist, unterbrochen und gegebenenfalls durch eine oder mehrere Hydroxylgruppen substituiert sind,
- eine NHNHCOR-Gruppe, wobei R für eine lineare oder verzweigte C₁-C₁₀-Alkylgruppe steht,
steht;
• R' die gleiche Definition wie oben hat;
• R'ₐ für:
- ein Wasserstoffatom,
- ein Chloratom oder ein Fluoratom,
- eine Pyridiniumgruppe, die gegebenenfalls durch mindestens eine Gruppe R_{c} substituiert ist, wobei R_{c} für eine C₁-C₄-Alkylgruppe, ein Halogenatom, eine Carboxylgruppe -COOM (wobei M für ein Wasserstoffatom, ein Alkalimetall, eine Ammoniumgruppe oder eine durch einen oder mehrere gleiche oder verschiedene lineare oder verzweigte C₁-C₁₈-Alkylreste, die gegebenenfalls mindestens ein Hydroxyl tragen, substituierte Ammoniumgruppe steht), eine Estergruppe -COOR_{d}, wobei R_{d} für einen C₁-C₄-Alkylrest steht, eine Amidgruppe
- CON(R_{d})₂, wobei die R_{d}-Gruppen gleich oder verschieden sind und für ein Wasserstoffatom oder einen C₁-C₄-Alkylrest stehen, stehen kann,
- eine Hydroxylgruppe,
- eine Amino-, Alkylamino- oder Dialkylaminogruppe, wobei die gleichen oder verschiedenen linearen oder verzweigten C₁-C₁₈-Alkylgruppen gegebenenfalls durch ein Heteroatom, das aus N und O ausgewählt ist, unterbrochen und gegebenenfalls durch eine oder mehrere Hydroxylgruppen substituiert sind,
- eine NHNHCOR-Gruppe, wobei R für eine lineare oder verzweigte C₁-C₁₀-Alkylgruppe steht,
steht,
- eine Gruppe der folgenden Formel (b): 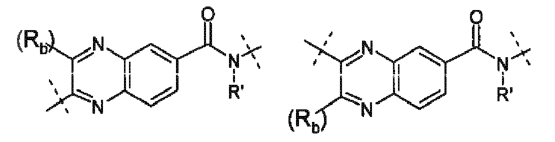
worin
• R' die gleiche Definition wie oben hat;
• R'ₐ für:
- ein Chloratom,
- eine Amino-, Alkylamino- oder Dialkylaminogruppe, wobei die gleichen oder verschiedenen linearen oder verzweigten C₁-C₁₈-Alkylgruppen gegebenenfalls durch ein Heteroatom, das aus N, O und S ausgewählt ist, unterbrochen und gegebenenfalls durch eine oder mehrere Hydroxylgruppen substituiert sind,
- einen gesättigten oder ungesättigten stickstoffhaltigen Heterocyclus, der substituiert sein kann,
- eine Arylaminogruppe, wobei der Arylrest vorzugsweise C₆ ist, steht,
steht;
y gleich 0 oder 1 ist;
Z für:
- -(cm2)ₘ-, wobei m für eine ganze Zahl zwischen 1 und 8 steht,
- -(CH₂CH₂O)_{q}- oder - (OCH₂CH₂)_{q}-, worin q für eine ganze Zahl zwischen 1 und 15 steht,
- einen Aryl-, Alkylaryl- oder Arylalkylrest, dessen Alkylrest C₁-C₄ ist und dessen Arylrest vorzugsweise C₆ ist und der gegebenenfalls durch mindestens eine SO₃M-Gruppe substituiert ist, wobei M für ein Wasserstoffatom, ein Alkalimetall oder eine durch einen oder mehrere gleiche oder verschiedene lineare oder verzweigte C₁-C₁₈-Alkylreste, die gegebenenfalls mindestens ein Hydroxyl tragen, substituierte Ammoniumgruppe steht,
steht,
z für 0 oder 1 steht;
• Cₛₐₜ und C'ₛₐₜ gleich oder verschieden sind und für eine gegebenenfalls cyclische, gegebenenfalls substituierte, lineare oder verzweigte C₁-C₁₈-Alkylenkette stehen;
• D einem aus Hydroxy-, Hydroxyalkyl-, Alkoxy-, Carboxyl-, Carboxylat-, Amino-, Alkylamin- und Dialkylaminoresten ausgewählten Rest entspricht.

3. Zusammensetzung nach Anspruch 1 oder 2, **dadurch gekennzeichnet, dass** in den Formeln (I), (II) oder (III) A und/oder A' für einen Rest mit mindestens einem Chromophor, das sich von Farbstoffen vom Acridin-, Acridon-, Anthranthron-, Anthrapyrimidin-, Anthrachinon-, Azin-, Azo-, Azomethin-, Benzanthron-, Benzimidazol-, Benzimidazolon-, Benzindol-, Benzoxazol-, Benzopyran-, Benzothiazol-, Benzochinon-, Bisazin-, Bisisoindolin-, Carboxanilid-, Cumarin-, Cyanin-, Diazin-, Diketopyrrolopyrrol-, Dioxazin-, Diphenylamin-, Diphenylmethan-, Dithiazin-, Flavonoid-, Fluorindin-, Formazan-, Hydrazon-, Hydroxyketon-, Indamin-, Indanthron-, Indigoid- und Pseudoindigoid-, Indophenol-, Indoanilin-, Isoindolin-, Isoindolinon-, Isoviolanthron-, Lacton-, Methin-, Naphthalimid-, Naphthanilid-, Naphtholactam-, Naphthochinon-, Nitro-,
Oxadiazol-, Oxazin-, Perilon-, Perinon-, Perylen-, Phenazin-, Phenothiazin-, Phthalocyanin-, Polyen/Carotenoid-, Porphyrin-, Pyranthron-, Pyrazolanthron-, Pyrazolon-, Pyrimidinoanthron-, Pyronin-, Chinacridon-, Chinolin-, Chinophthalon-, Squaran-, Stilben-; Tetrazolium-, Thiazin-, Thioindigo-, Thiopyronin-, Triarylmethan- und Xanthen-Typ ableitet, stehen.

4. Zusammensetzung nach einem der Ansprüche 1 bis 3, wobei der Disulfid-Direktfarbstoff ausgewählt ist aus 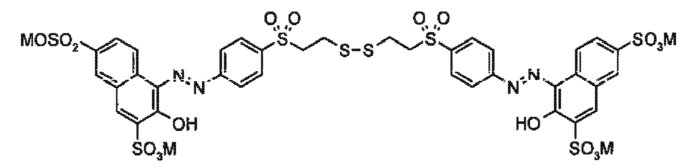 sowie den folgenden Verbindungen in saurer, basischer oder neutralisierter Form: 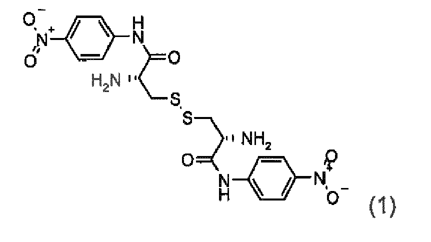 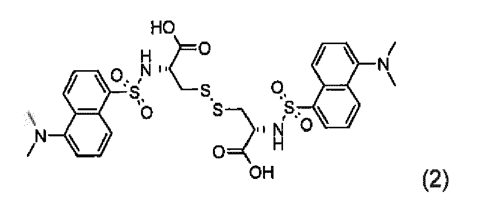 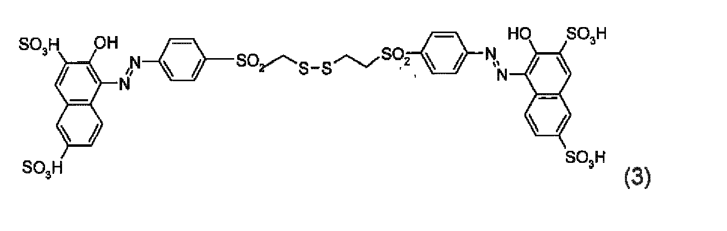 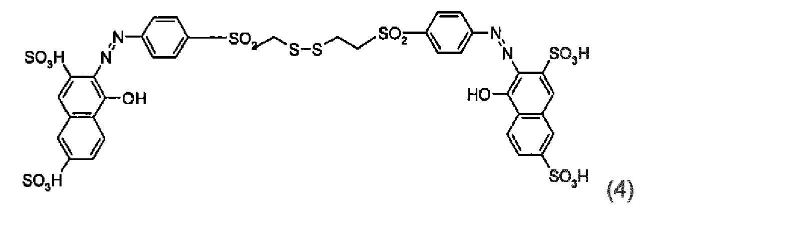 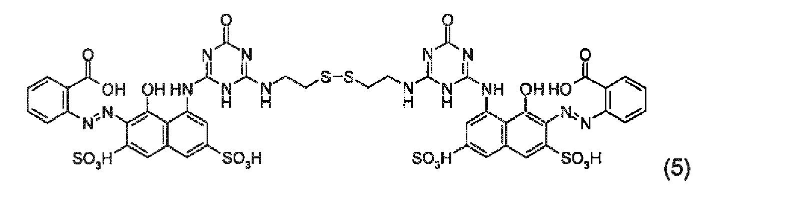 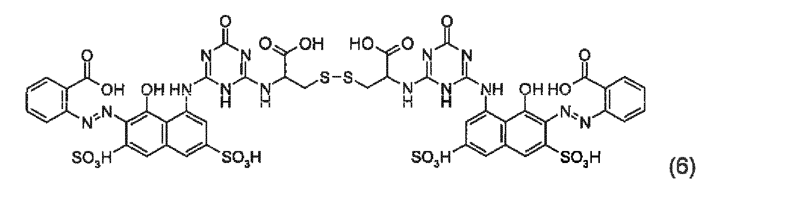 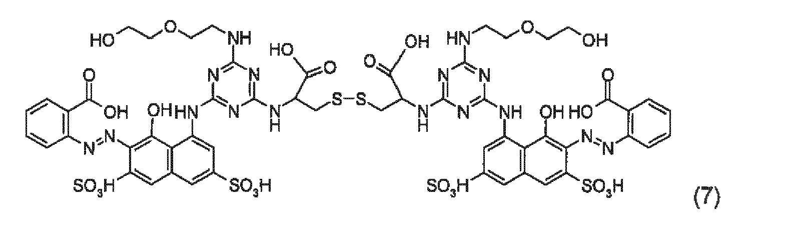 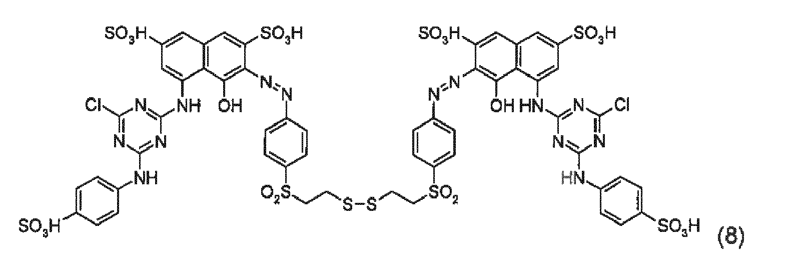 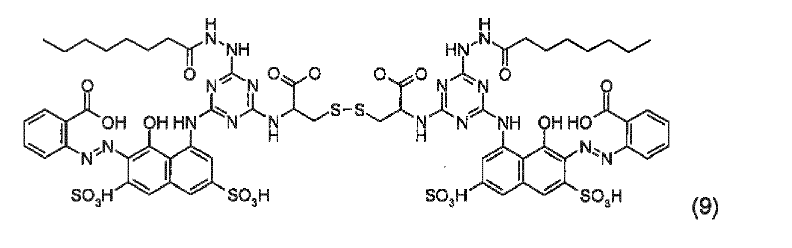 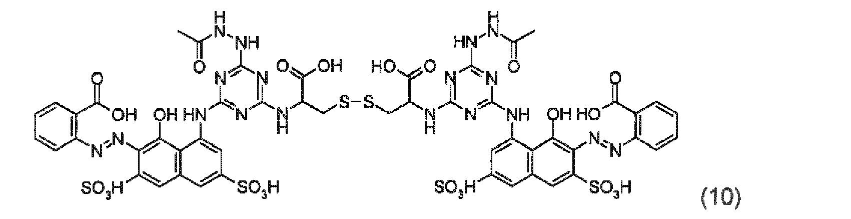 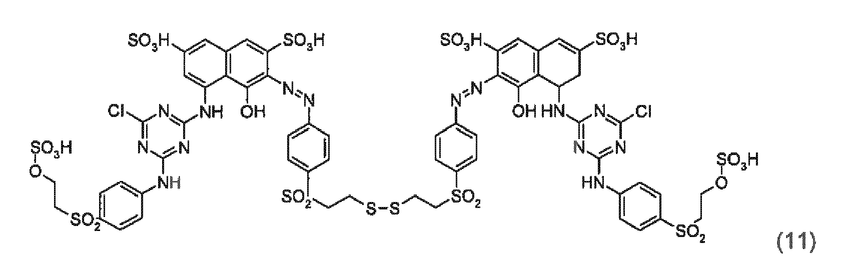 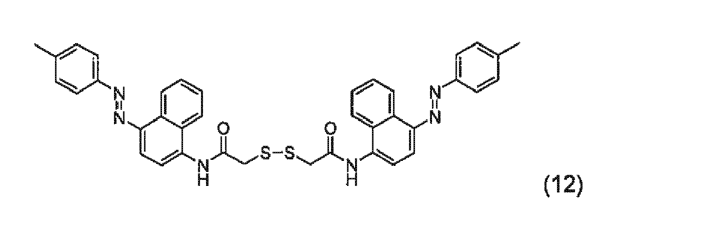 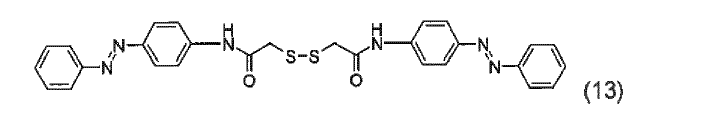 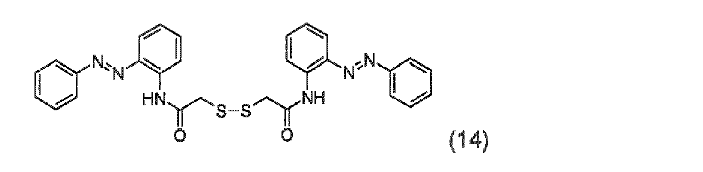 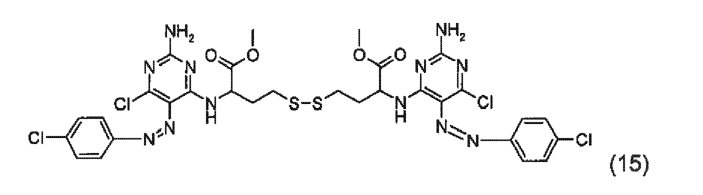 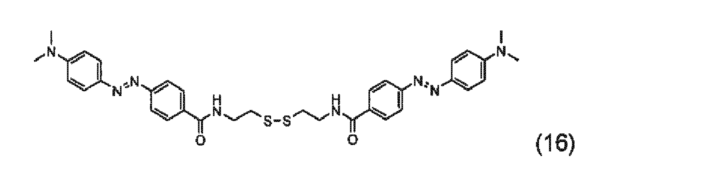 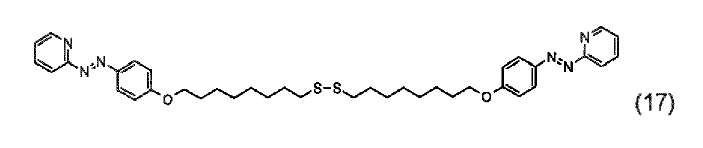 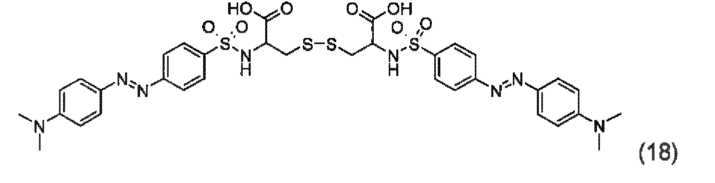 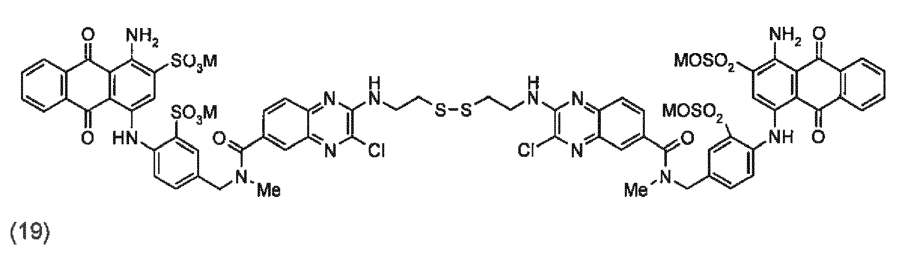 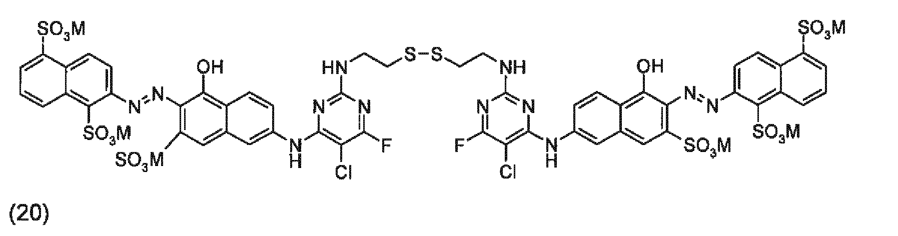 wobei M für ein Wasserstoffatom, ein Alkalimetall, eine Ammoniumgruppe oder eine durch einen oder mehrere gleiche oder verschiedene lineare oder verzweigte C₁-C₁₀-Alkylreste, die gegebenenfalls mindestens ein Hydroxyl tragen, substituierte Ammoniumgruppe steht; und den folgenden Verbindungen, ihren Salzen, Hydraten und Solvaten:

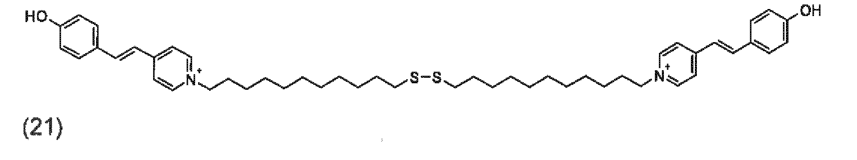

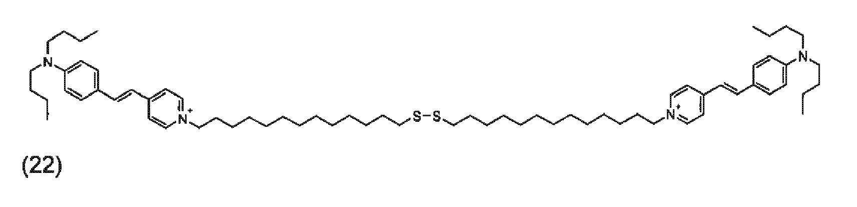

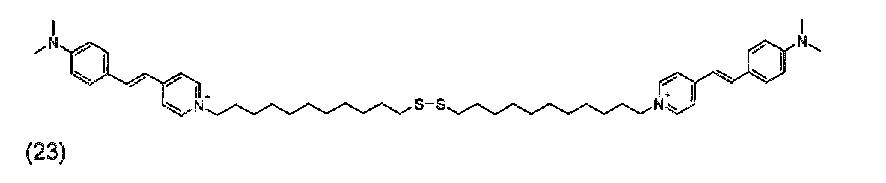

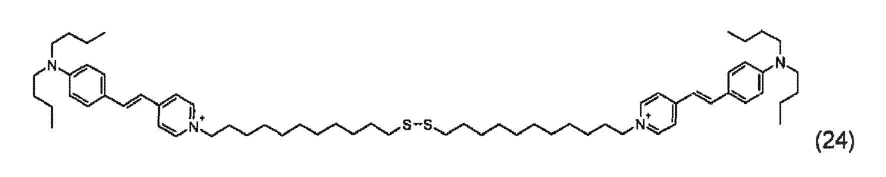

5. Zusammensetzung nach den Ansprüchen 1 bis 3, **dadurch gekennzeichnet, dass** das kationische Chromophor einen kationischen Rest, bei dem es sich um ein quaternäres Ammonium handelt, umfasst.

6. Zusammensetzung nach Anspruch 1, **dadurch gekennzeichnet, dass** es sich bei dem Disulfid-Direktfarbstoff um einen Farbstoff der Formel (I), (II) oder (III), worin A und/oder A' gleich oder verschieden sind und für einen Rest mit einem oder mehreren kationischen oder nichtkationischen fluoreszierenden Chromophoren stehen, handelt.

7. Zusammensetzung nach einem der Ansprüche 1 bis 3 und 5 bis 6, **dadurch gekennzeichnet, dass** X und X' gleich oder verschieden sind und für eine -N(R)-Gruppe stehen.

8. Zusammensetzung nach einem der Ansprüche 5 bis 7, **dadurch gekennzeichnet, dass** der Disulfid-Direktfarbstoff so beschaffen ist, dass v oder v' gleich 0 ist und A und/oder A' gleich oder verschieden sind und vorzugsweise gleich sind und für W-N=N-Ar- oder -W-N=N-Ar oder W-C(R^{c})=C(R^{d})-Ar- oder -W-C(R^{c})=C(R^{d})-Ar stehen, wobei W für einen kondensierten oder nichtkondensierten, aromatischen oder nicht aromatischen Heterocyclus mit einem quaternären Ammonium steht; Ar für einen 5- oder 6-gliedrigen (Hetero)arylrest oder einen (hetero)aromatischen bicylischen Ring vom Naphthyl-, Benzopyridinium-, Indolinyl- oder Benzoindolinyltyp, der gegebenenfalls durch ein oder mehrere Halogenatome; durch eine oder mehrere Alkylgruppen; durch eine oder mehrere Hydroxylgruppen; durch eine oder mehrere Alkoxygruppen, durch eine oder mehrere Hydroxyalkylgruppen, durch eine oder mehrere Amino- oder (Di)alkylaminogruppen, durch eine oder mehrere Acylaminogruppen; durch eine oder mehrere 5- oder 6-gliedrige Heterocycloalkyl- oder Heteroarylgruppen substituiert ist, steht; R^{c} und R^{d} gleich oder verschieden sind und für ein Wasserstoffatom oder eine C₁-C₄-Alkylgruppe stehen.

9. Zusammensetzung nach einem der Ansprüche 1 bis 5 und 7 bis 8, **dadurch gekennzeichnet, dass** der Disulfid-Direktfarbstoff ausgewählt ist aus: 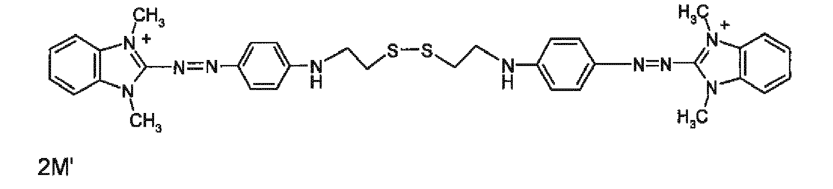 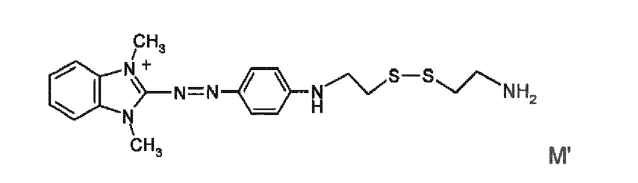 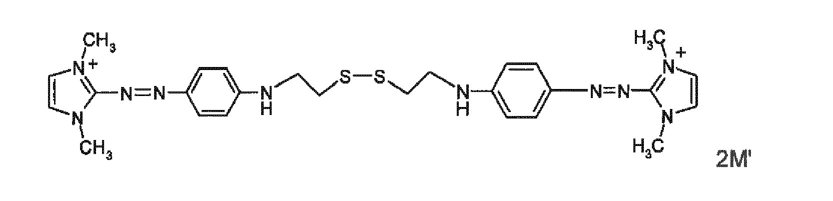 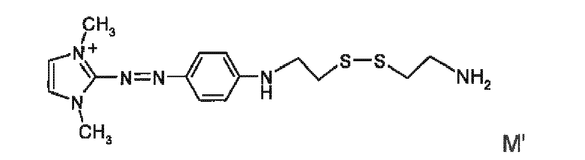 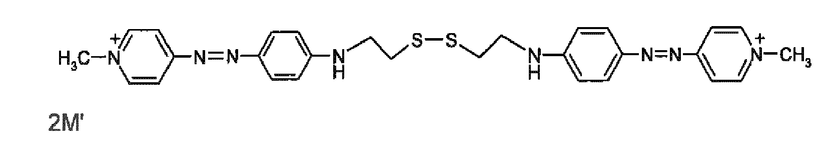 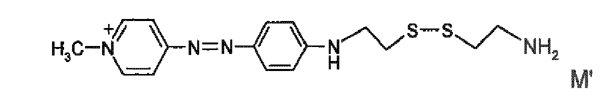 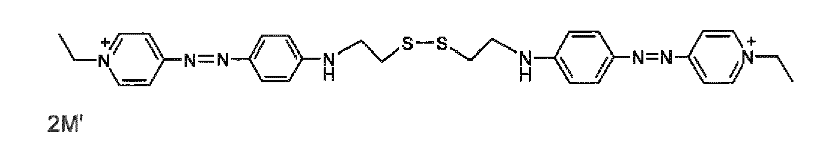 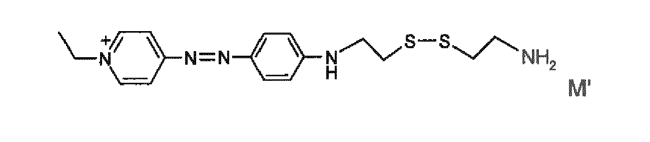 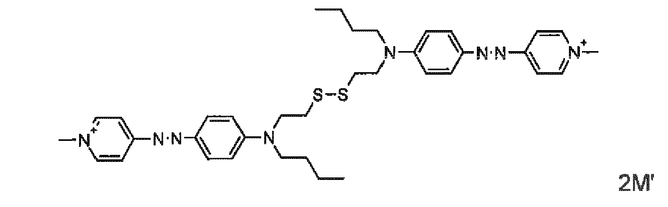 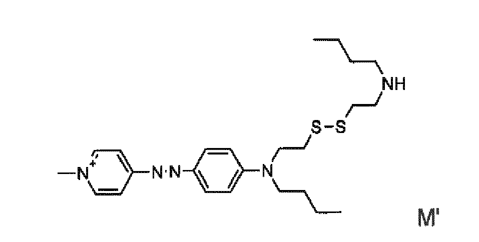 wobei M' für ein organisches oder anorganisches Säuresalz steht.

10. Zusammensetzung nach einem der Ansprüche 1 und 6 bis 8, **dadurch gekennzeichnet, dass** der Disulfid-Direktfarbstoff aus den Verbindungen (III) bis (XII) ausgewählt ist: 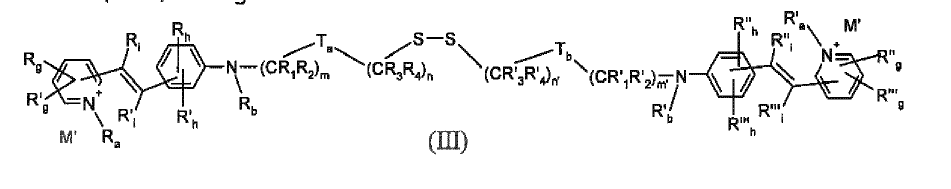 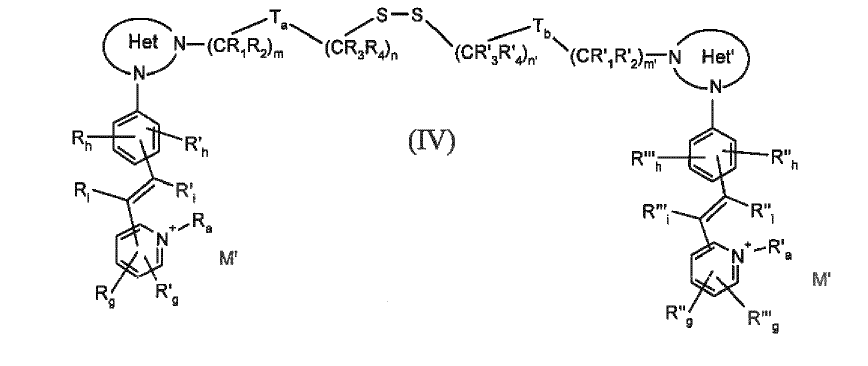 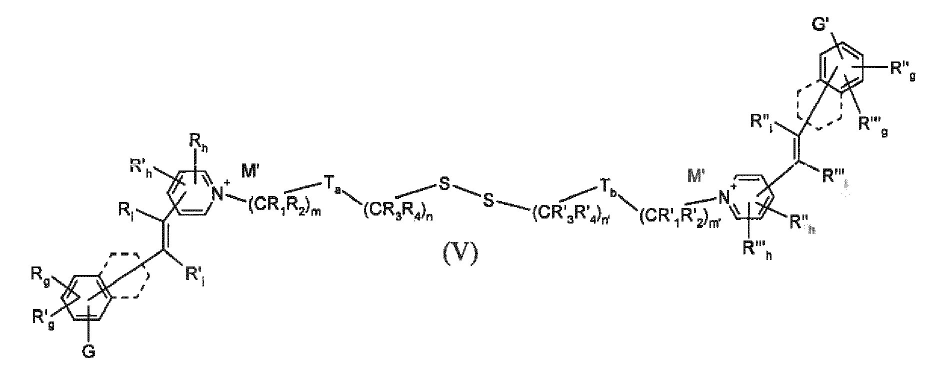 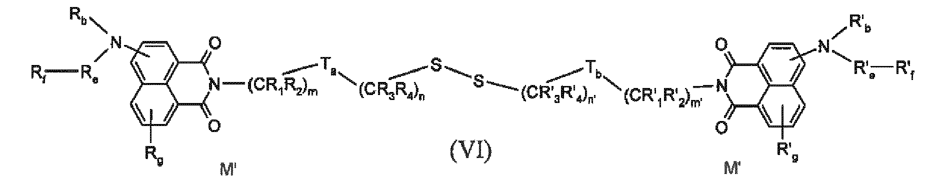 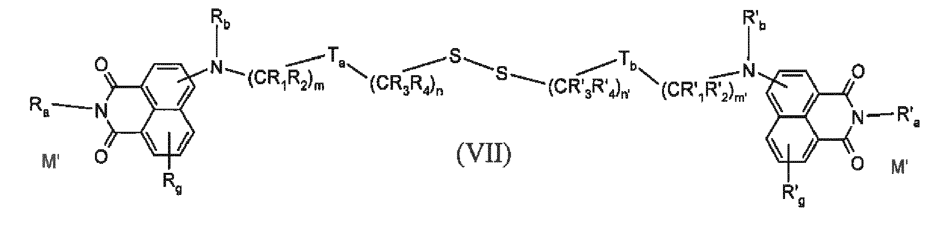 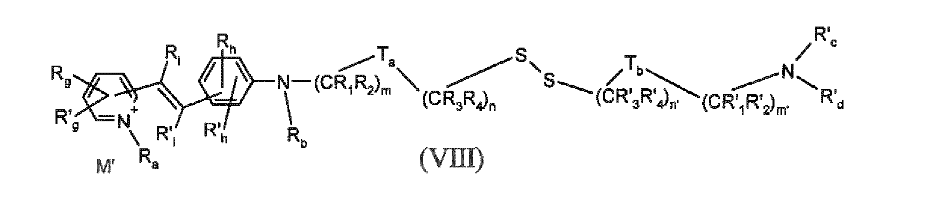 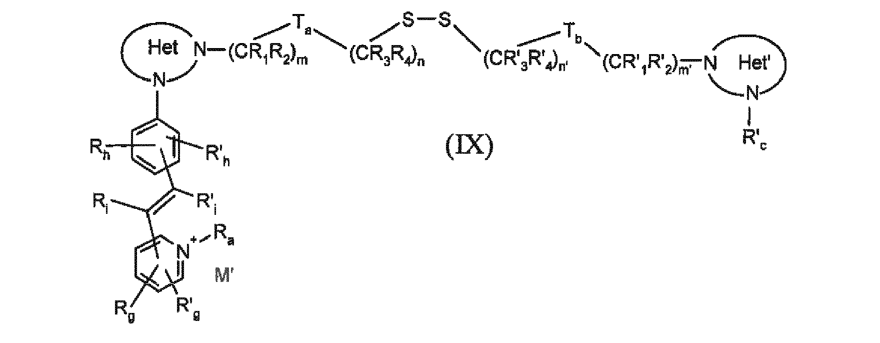 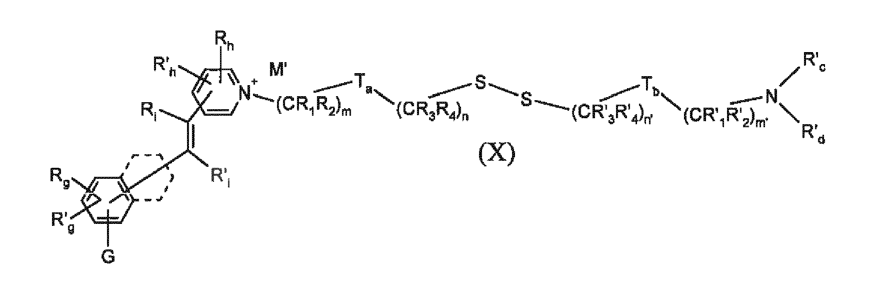 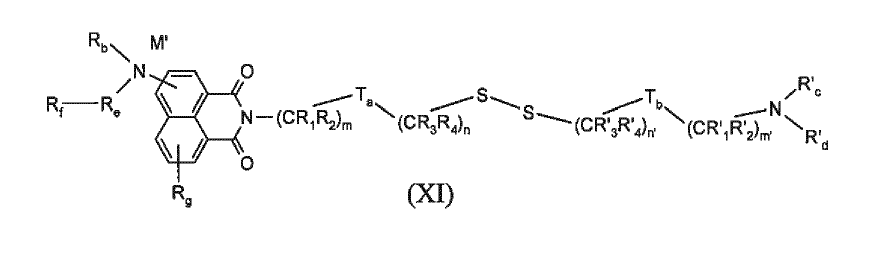 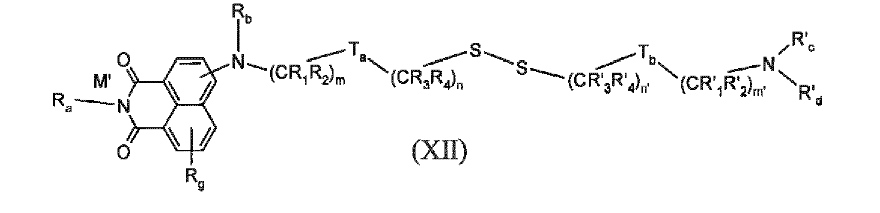 worin
• G und G' gleich oder verschieden sind und für eine -NR_{c}R_{d}-, -NR'_{c}R'_{d}- oder C₁-C₆-Alkoxygruppe, die gegebenenfalls substituiert ist, stehen; G und G' vorzugsweise für eine -NR_{c}R_{d}- bzw. -NR'_{c}R'_{d}-Gruppe stehen;
• Rₐ und R'ₐ gleich oder verschieden sind und für eine Aryl(C₁-C₄)alkylgruppe oder eine C₁-C₆-Alkylgruppe, die gegebenenfalls durch eine Hydroxylgruppe oder eine Amino-, C₁-C₄-Alkylamino- oder C₁-C₄-Dialkylaminogruppe substituiert ist, stehen, wobei die Alkylreste mit dem Stickstoffatom, an das sie gebunden sind, einen 5- bis 7-gliedrigen Heterocyclus bilden können, der gegebenenfalls ein weiteres Heteroatom, das mit Stickstoff identisch oder davon verschieden ist, enthält;
• R_{b} und R'_{b} gleich oder verschieden sind und für ein Wasserstoffatom, eine Aryl(C₂-C₄)alkylgruppe oder eine C₁-C₆-Alkylgruppe, die gegebenenfalls substituiert ist, stehen;
• R_{c}, R'_{c}, R_{d} und R'_{d} gleich oder verschieden sind und für ein Wasserstoffatom, eine Aryl(C₁-C₄) alkylgruppe, eine C₁-C₆-Alkoxygruppe oder eine C₁-C₆-Alkylgruppe, die gegebenenfalls substituiert ist, stehen;
oder auch zwei benachbarte Reste R_{c} und R_{d} oder R'_{c} und R'_{d}, die an dasselbe Stickstoffatom gebunden sind, eine heterocyclische Gruppe oder Heteroarylgruppe bilden;
• Rₑ und R'ₑ gleich oder verschieden sind und für eine gegebenenfalls ungesättigte, lineare oder verzweigte, zweiwertige C₁-C₆-Alkylenyl-Kohlenwasserstoffkette stehen;
• R_{f} und R'_{f} gleich oder verschieden sind und für eine quaternäre Ammoniumgruppe (R'') (R''') (R'''')N⁺- stehen, wobei R'', R''' und R'''' gleich oder verschieden sind und für ein Wasserstoffatom oder eine C₁-C₄-Alkylgruppe stehen oder (R'') (R''') (R'''')N⁺- für eine gegebenenfalls substituierte kationische Heteroarylgruppe, vorzugsweise eine gegebenenfalls durch eine C₁-C₃-Alkylgruppe substituierte Imidazoliniumgruppe, steht;
• R_{g}, R'_{g}, R"_{g}, R"'_{g}, Rₕ, R'ₕ, R''ₕ und R'''ₕ gleich oder verschieden sind und für ein Wasserstoffatom, ein Halogenatom, eine Amino-, C₁-C₄-Alkylamino-, C₁-C₄-Dialkylamino-, Cyano-, Carboxy-, Hydroxy- oder Trifluormethylgruppe, einen Acylamino-, C₁-C₄-Alkoxy-, (Poly)hydroxy-(C₂-C₄)alkoxy-, Alkylcarbonyloxy-, Alkoxycarbonyl- oder Alkylcarbonylaminorest, einen Acylamino-, Carbamoyl- oder Alkylsulfonylaminorest, einen Aminosulfonylrest oder einen C₁-C₁₆-Alkylrest, der gegebenenfalls durch eine aus C₁-C₁₂-Alkoxy, Hydroxy, Cyano, Carboxy, Amino, C₁-C₄-Alkylamino und C₁-C₄-Dialkylamino ausgewählte Gruppe substituiert ist, stehen oder auch die beiden Alkylreste, die an das Stickstoffatom der Aminogruppe gebunden sind, einen 5- bis 7-gliedrigen Heterocyclus bilden, der gegebenenfalls ein weiteres Heteroatom, das mit demjenigen des Stickstoffatoms identisch oder davon verschieden ist, enthält;
oder auch zwei Gruppen R_{g} und R'_{g}; R"_{g} und R'''_{g}; Rₕ und R'ₕ; R''ₕ und R'''ₕ, die an zwei benachbarte Kohlenstoffatome gebunden sind, zusammen einen Benzo- oder Indenoring oder eine kondensierte Heterocycloalkyl- oder kondensierte Heteroarylgruppe bilden; wobei der Benzo-, Indeno-, Heterocycloalkyl- oder Heteroarylring gegebenenfalls durch ein Halogenatom, eine Amino-, C₁-C₄-Alkylamino-, C₁-C₄-Dialkylamino-, Nitro-, Cyano-, Carboxy-, Hydroxy- oder Trifluormethylgruppe, einen Acylamino-, C₁-C₄-Alkoxy-, (Poly)hydroxy(C₂-C₄)alkoxy-, Alkylcarbonyloxy-, Alkoxycarbonyl- oder Alkylcarbonylaminorest, einen Acylamino-, Carbamoyl- oder Alkylsulfonylaminorest, einen Aminosulfonylrest oder einen C₁-C₁₆-Alkylrest, der gegebenenfalls durch eine aus C₁-C₁₂-Alkoxy, Hydroxy, Cyano, Carboxy, Amino, C₁-C₄-Alkylamino und C₁-C₄-Dialkylamino ausgewählte Gruppe substituiert ist, substituiert ist oder auch die beiden Alkylreste, die an das Stickstoffatom der Aminogruppe gebunden sind, einen 5-bis 7-gliedrigen Heterocyclus bilden, der gegebenenfalls ein weiteres Heteroatom, das mit demjenigen des Stickstoffatoms identisch oder davon verschieden ist, enthält;
• oder auch dann, wenn G für -NR_{c}R_{d} steht und G¹ für -NR'_{c}R'_{d} steht, zwei Gruppen R_{c} und R'_{g}; R'_{c} und R''_{g}; R_{d} und R_{g}; R'_{d} und R'''_{g} zusammen ein Heteroaryl oder einen gesättigten Heterocyclus, das bzw. der gegebenenfalls durch eine C₁-C₆-Alkylgruppe substituiert ist;
• Rᵢ, R'ᵢ, R''ᵢ und R'''ᵢ gleich oder verschieden sind und für ein Wasserstoffatom oder eine C₁-C₄-Alkylgruppe stehen;
• R₁, R₂, R₃, R₄, R'₁, R'₂, R'₃ und R'₄ gleich oder verschieden sind und für ein Wasserstoffatom oder eine C₁-C₄-Alkyl-, C₁-C₁₂-Alkoxy-, Hydroxy-, Cyano-, Carboxy-, Amino-, C₁-C₄-Alkylamino- oder C₁-C₄-Dialkylaminogruppe stehen, wobei die Alkylreste mit dem Stickstoffatom, an das sie gebunden sind, einen 5- bis 7-gliedrigen Heterocyclus bilden können, der gegebenenfalls ein weiteres Heteroatom, das mit Stickstoff identisch oder davon verschieden ist, enthält;
• Tₐ und T_{b} gleich oder verschieden sind und für i) eine kovalente σ-Bindung, ii) einen oder mehrere Reste oder Kombinationen davon, die aus -SO₂-, -O-, -S-, -N(R)-, -N⁺(R) (R°) - und -CO-, wobei R und R° gleich oder verschieden sind und für ein Wasserstoffatom oder einen C₁-C₄-Alkyl-, C₁-C₄-Hydroxyalkyl- oder Aryl(C₁-C₄) -alkylrest stehen, ausgewählt sind; iii) oder einen kationischen oder nichtkationischen Heterocycloalkyl- oder Heteroarylrest stehen;
• 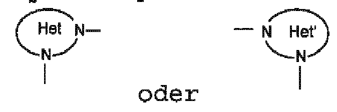 gleich oder verschieden sind und für eine gegebenenfalls substituierte heterocyclische Gruppe stehen;
• 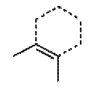 für eine mit einem Phenylring kondensierte Aryl- oder Heteroarylgruppe steht oder auch an dem Phenylring fehlt;
• m, m', n und n' gleich oder verschieden sind und für eine ganze Zahl zwischen 0 und 6 inklusive stehen, wobei m+n, m'+n' gleich oder verschieden sind und für eine ganze Zahl zwischen 1 und 10 inklusive stehen;
wobei M' für ein organisches oder anorganisches Säuresalz steht.

11. Zusammensetzung nach einem der Ansprüche 1, 6 bis 8 und 10, **dadurch gekennzeichnet, dass** der Disulfid-Direktfarbstoff ausgewählt ist aus: 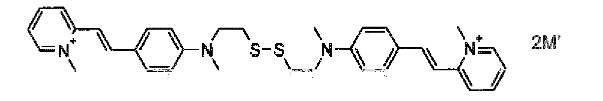 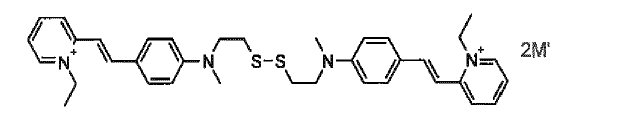 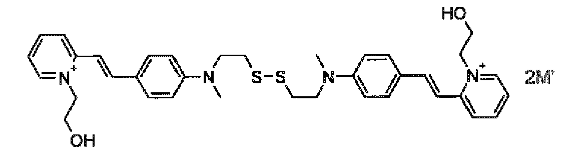 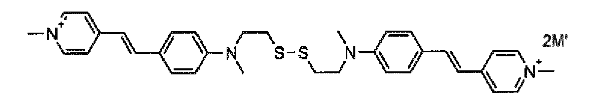 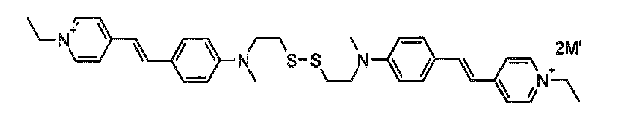 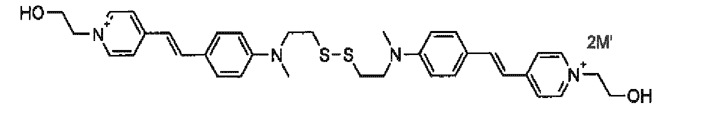 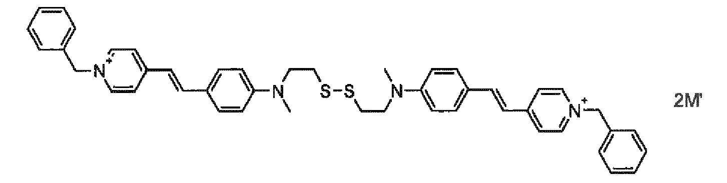 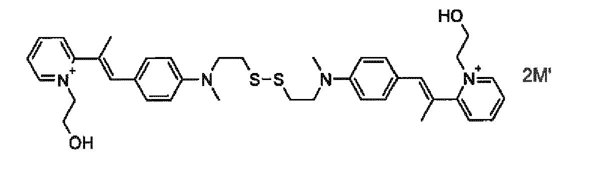 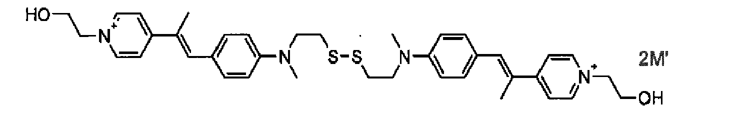 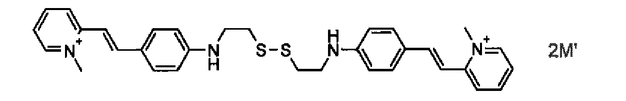 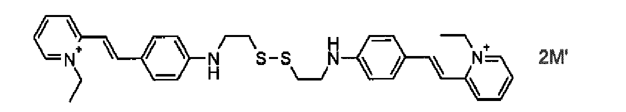 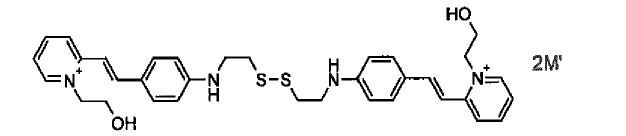 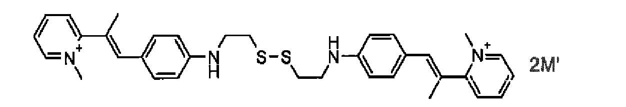 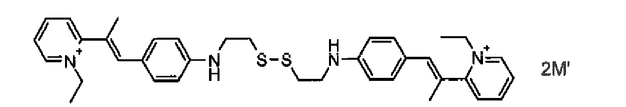 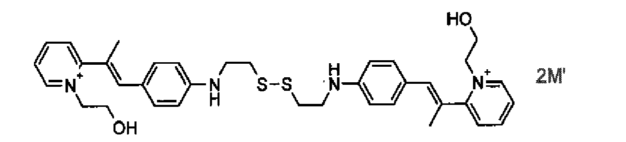 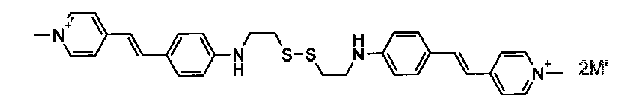 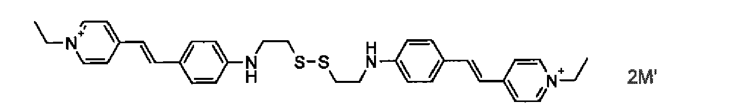 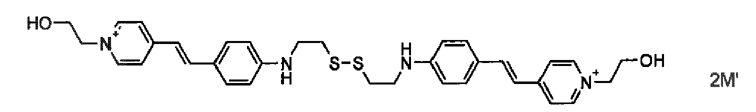 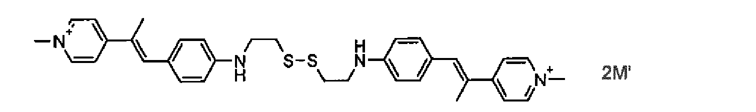 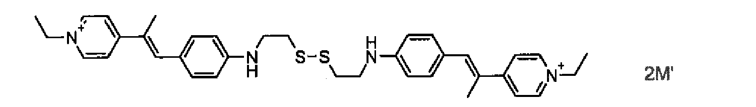 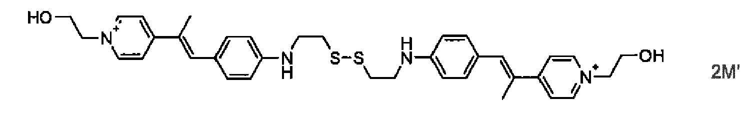 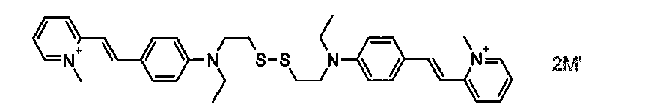 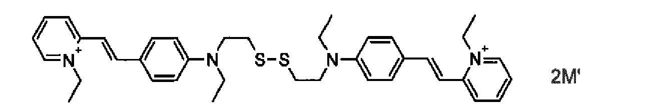 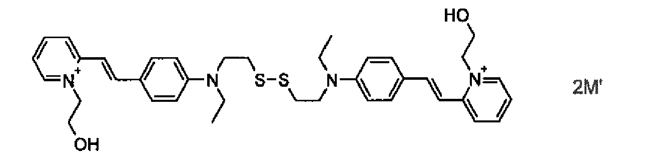 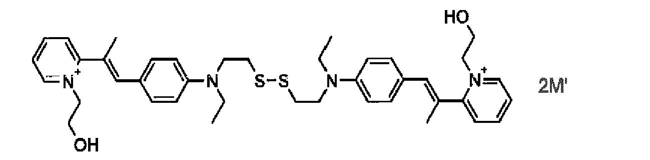 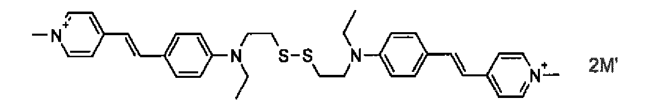 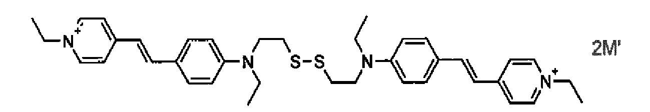 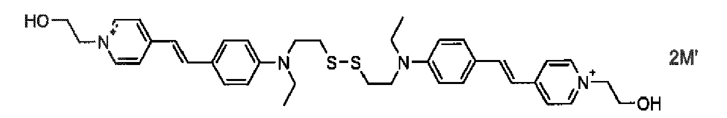 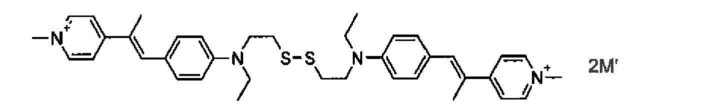 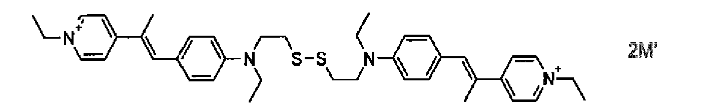 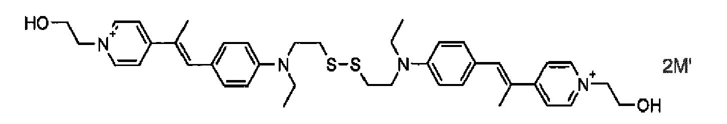 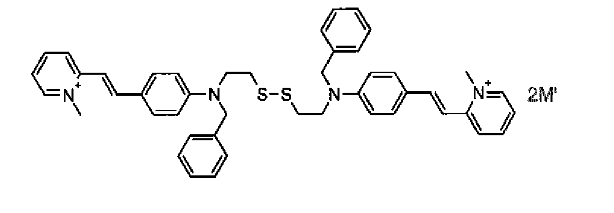 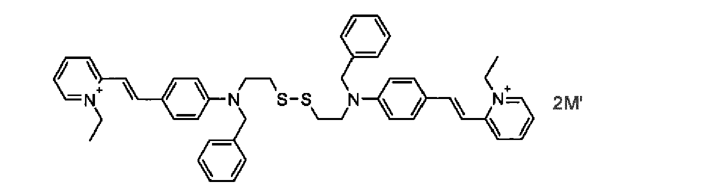 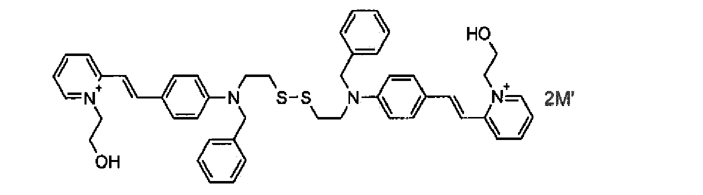 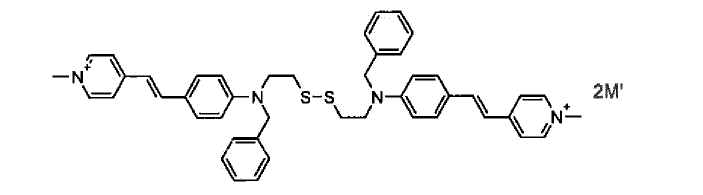 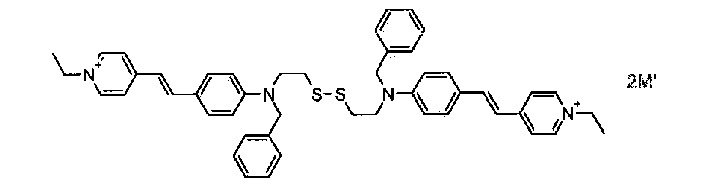 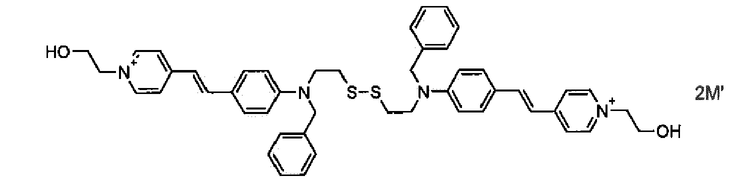 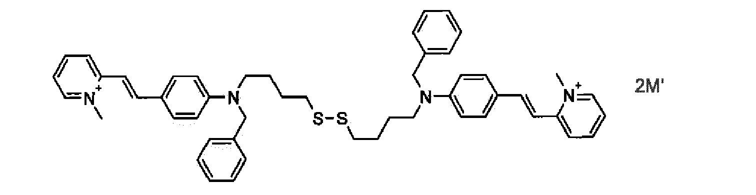 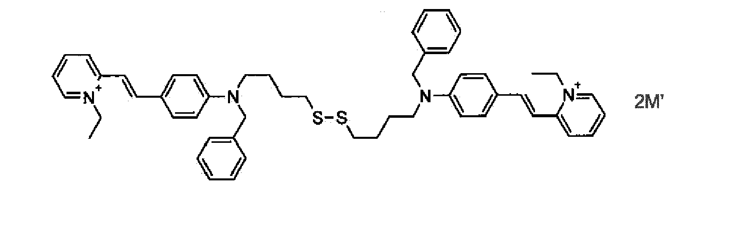 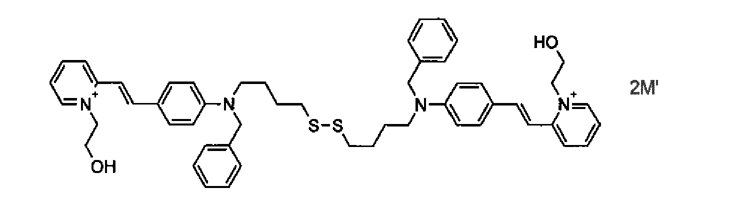 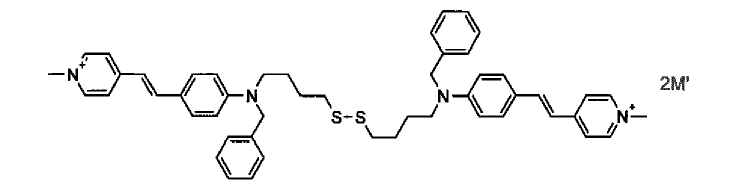 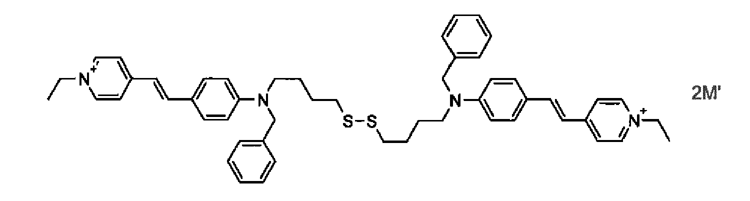 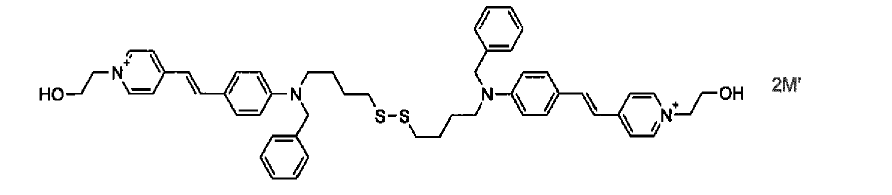 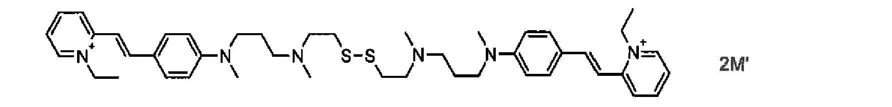 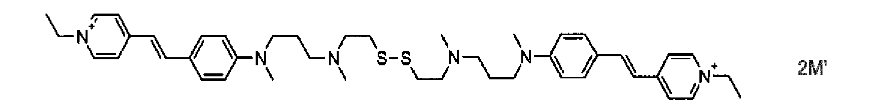 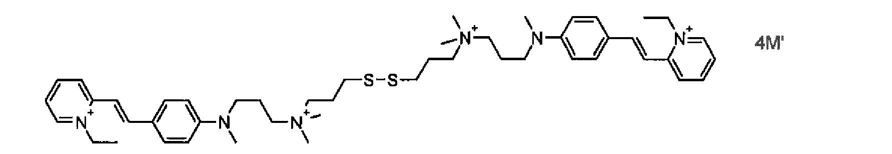 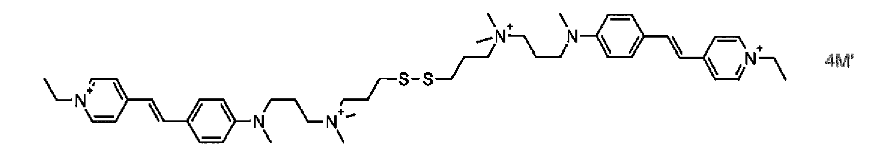 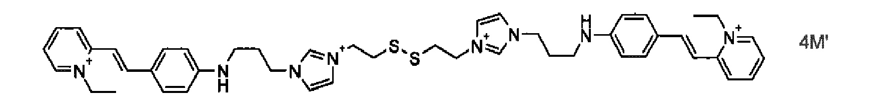 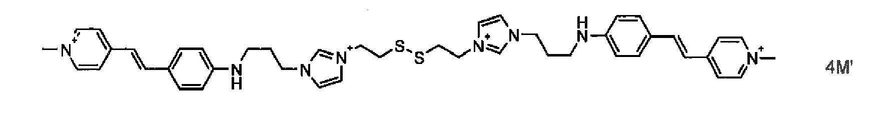 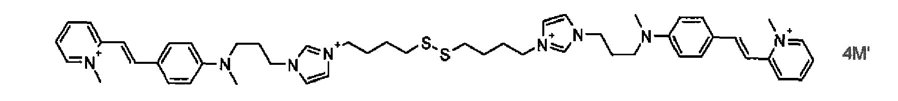 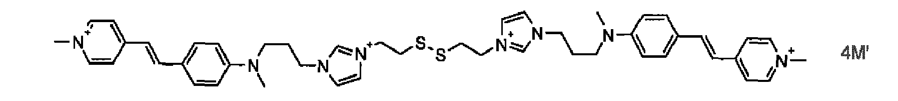 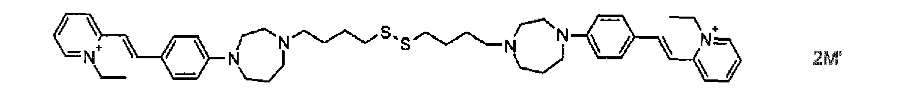 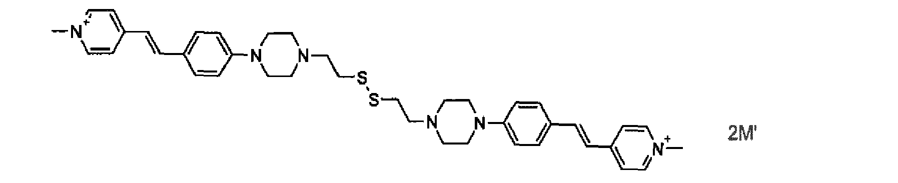 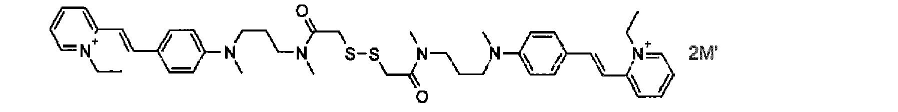 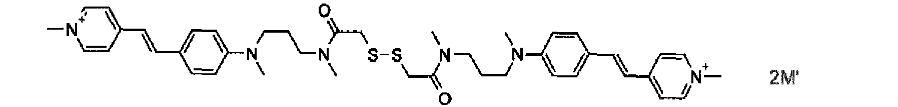 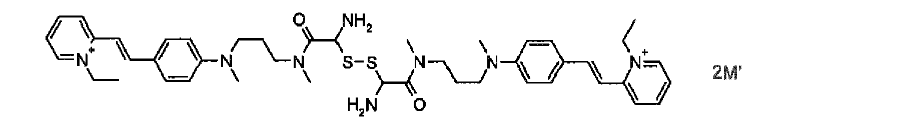 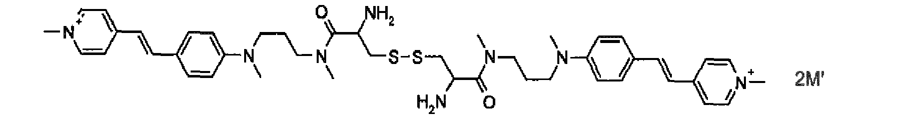 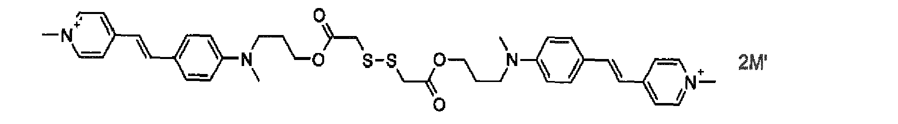 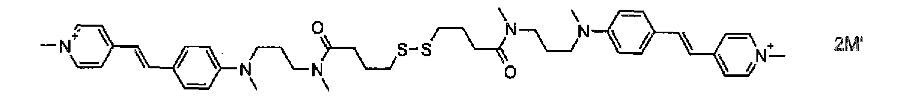 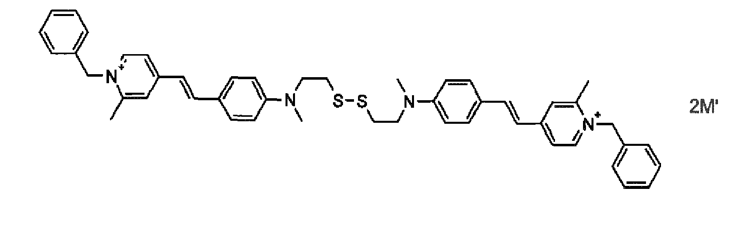 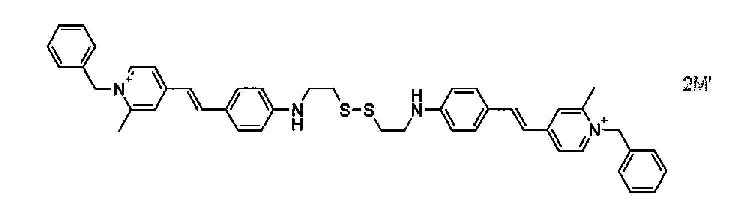 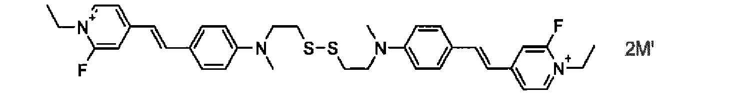 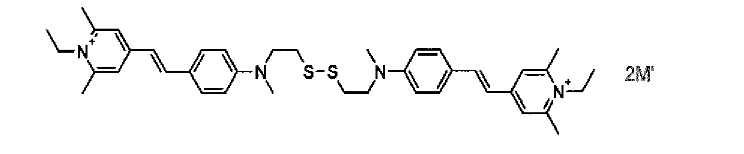 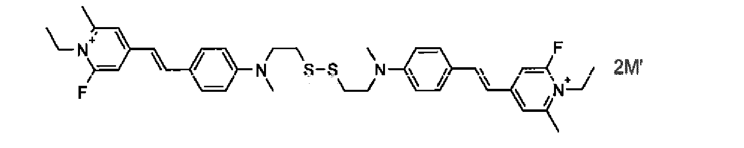 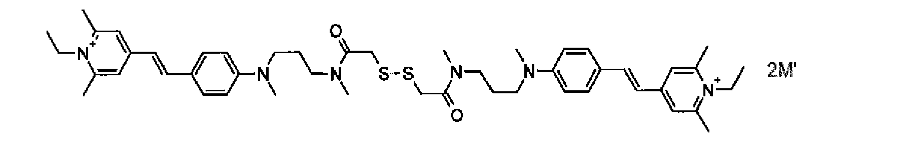 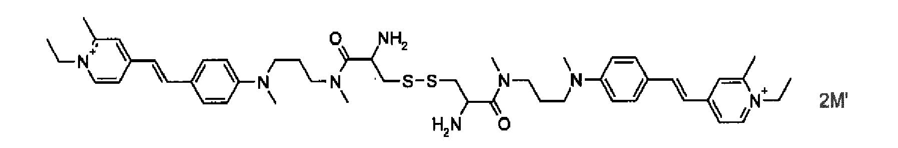 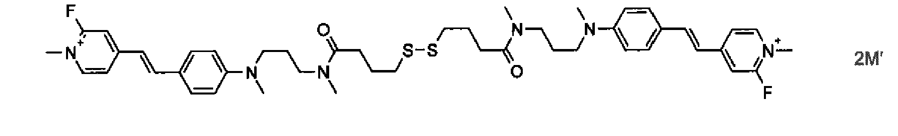 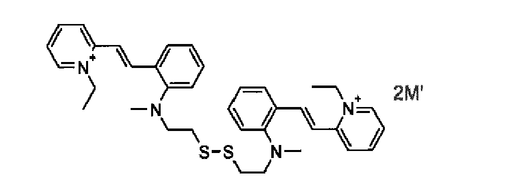 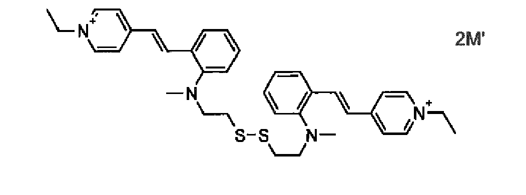 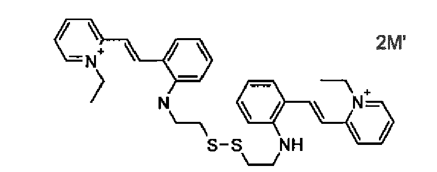 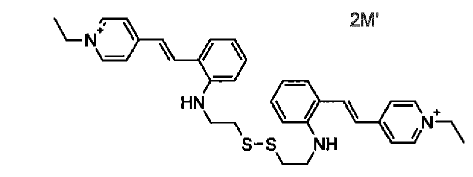 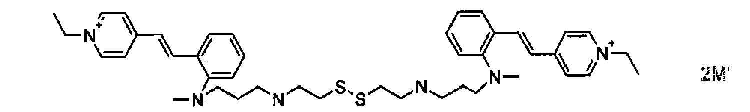 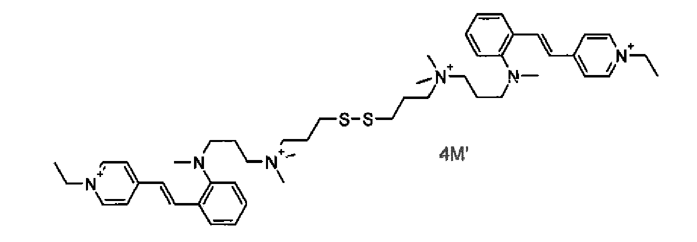 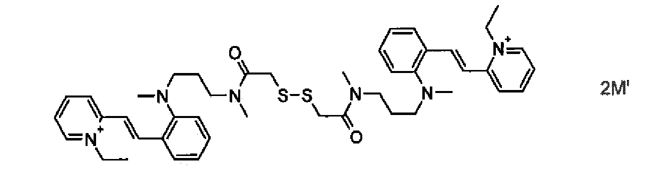 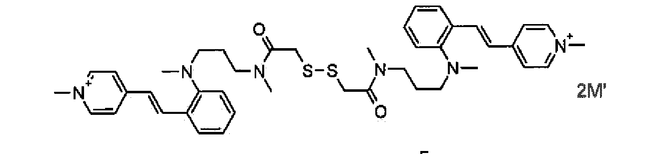 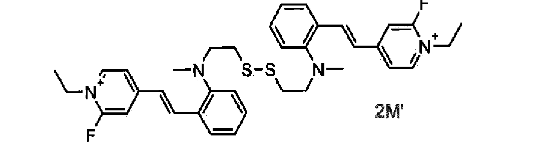 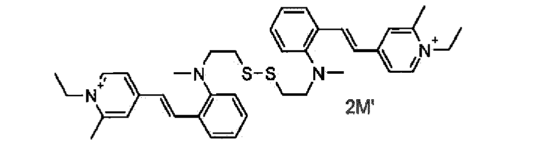 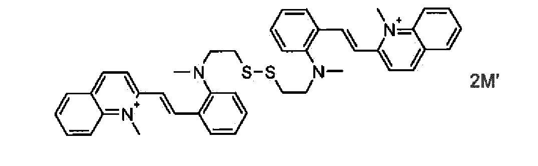 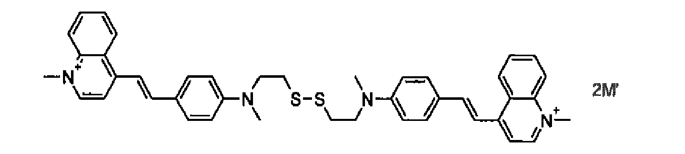 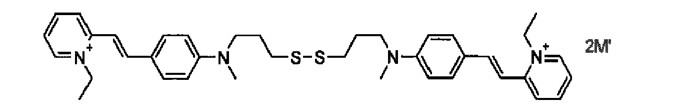 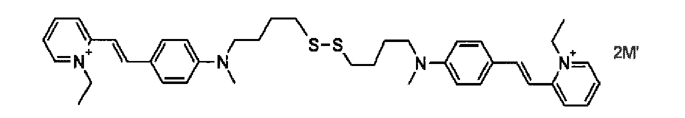 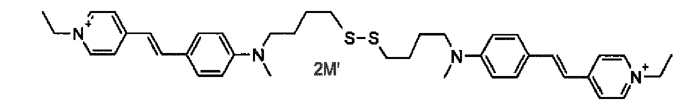 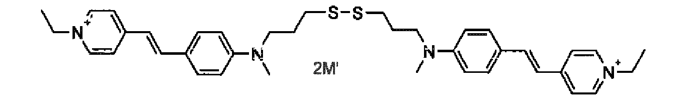 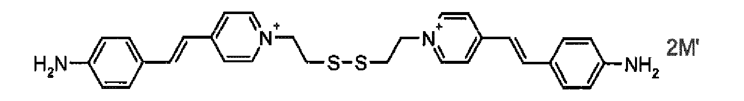 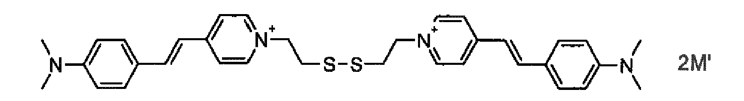 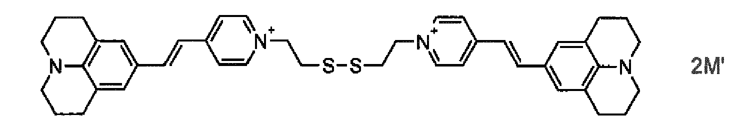 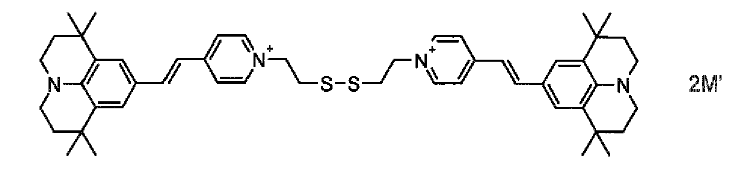 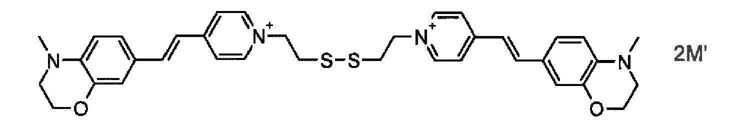 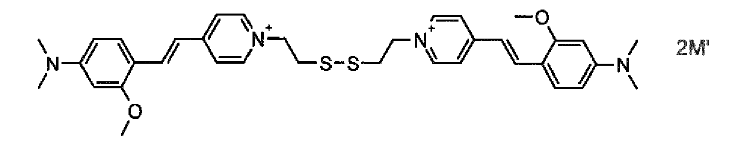 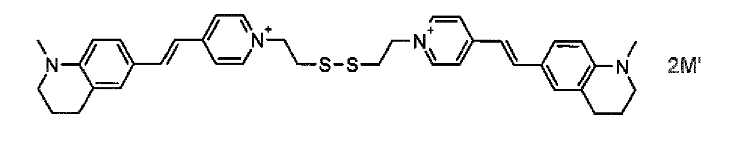 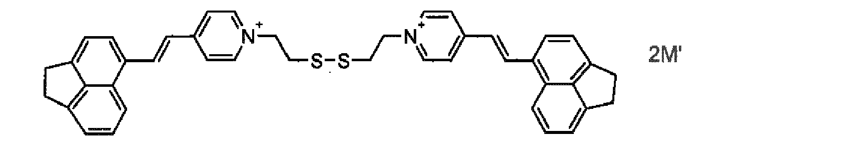 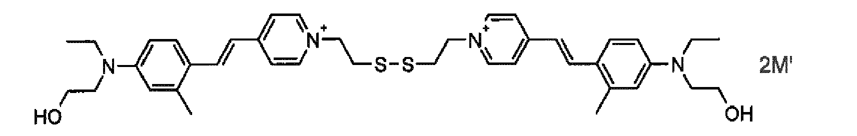 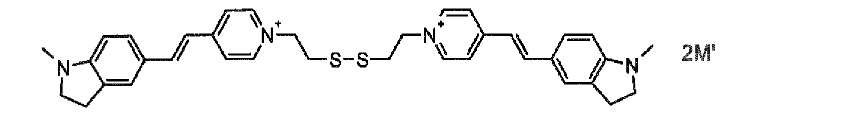 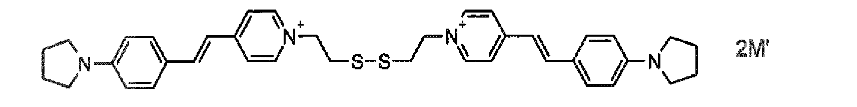 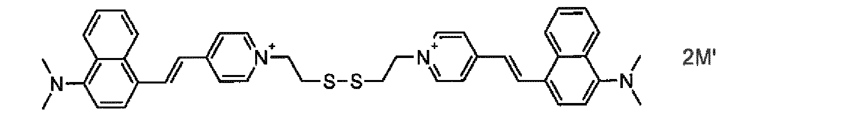 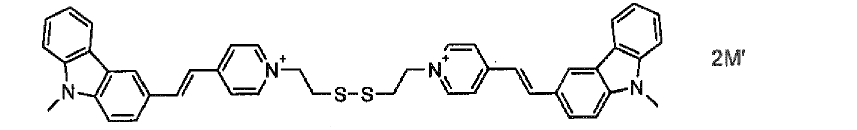 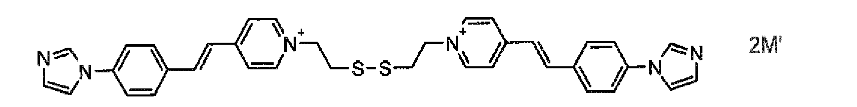 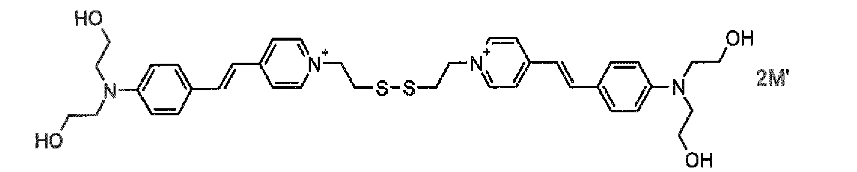 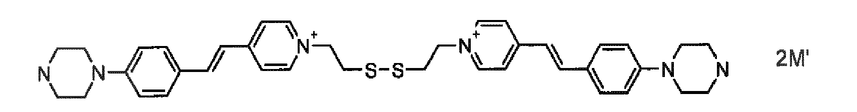 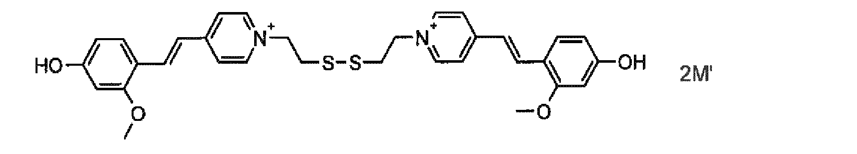 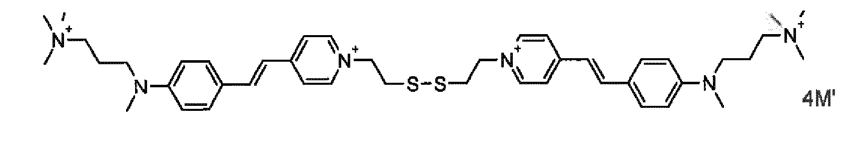 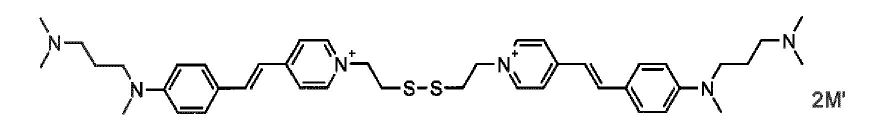 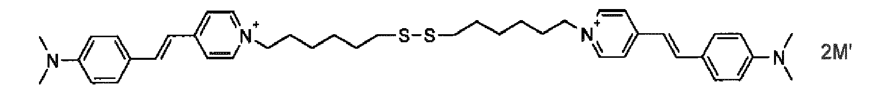 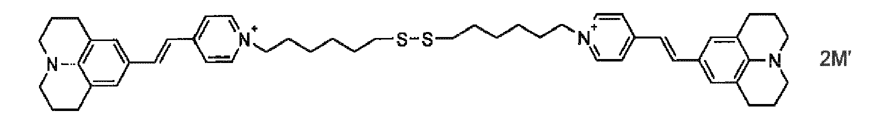 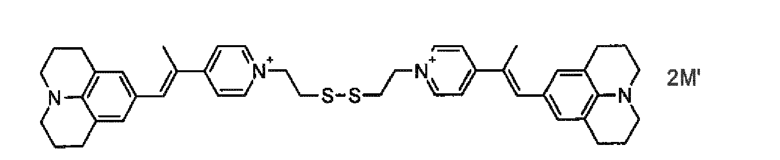 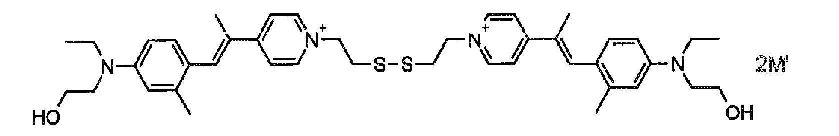 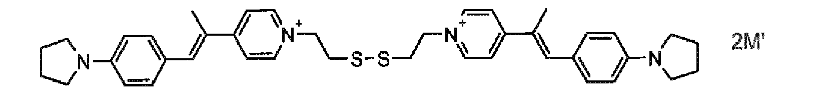 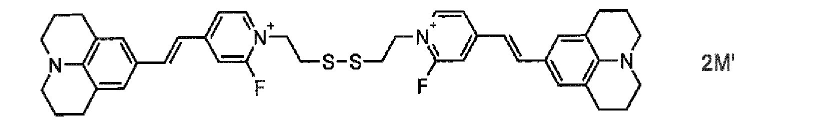 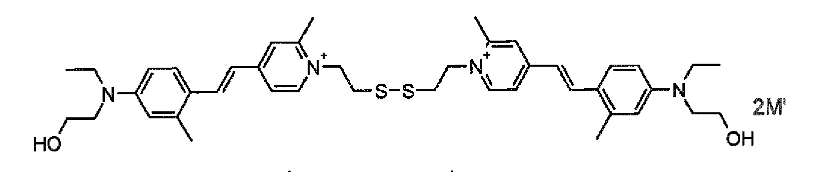 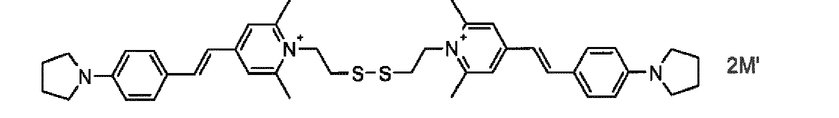 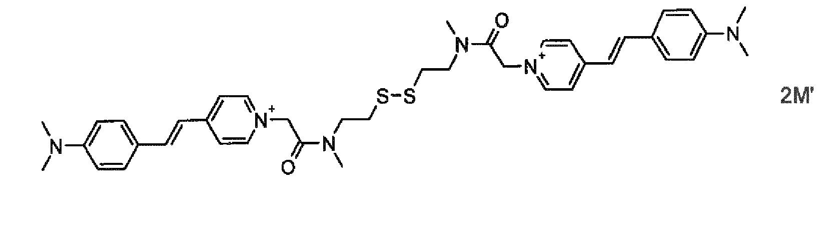 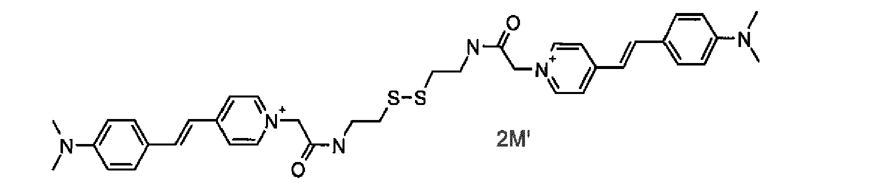 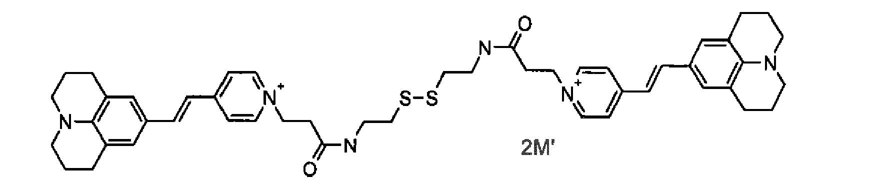 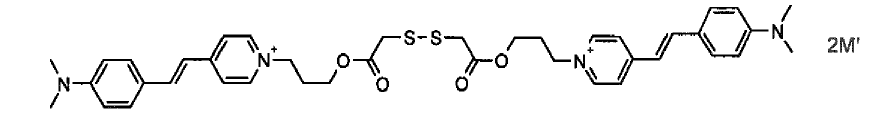 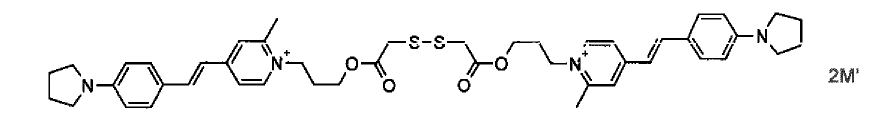 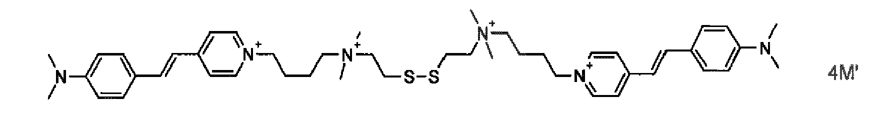 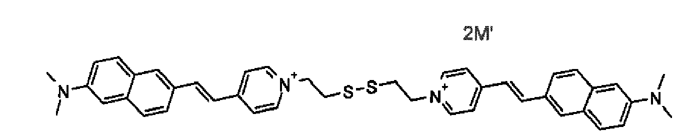 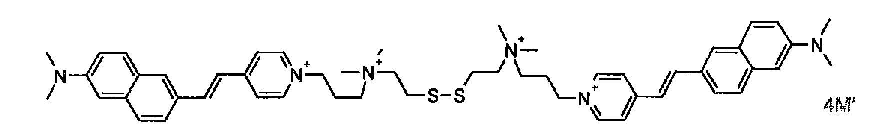 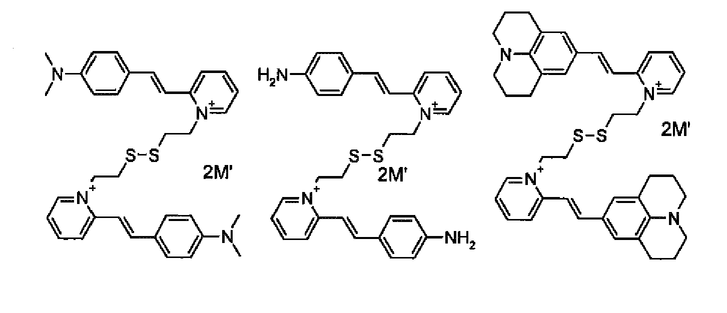 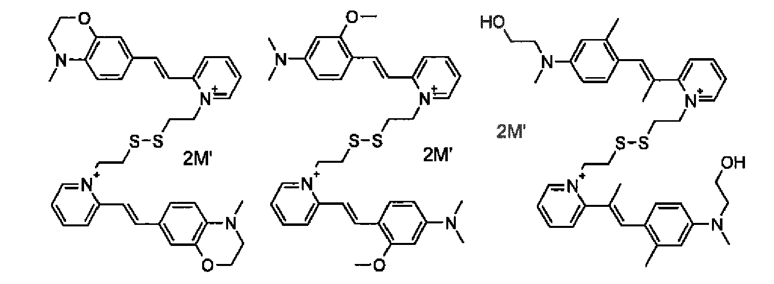 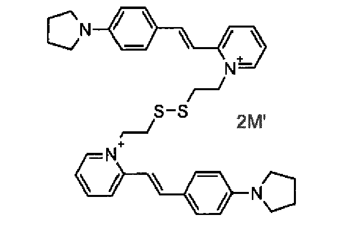 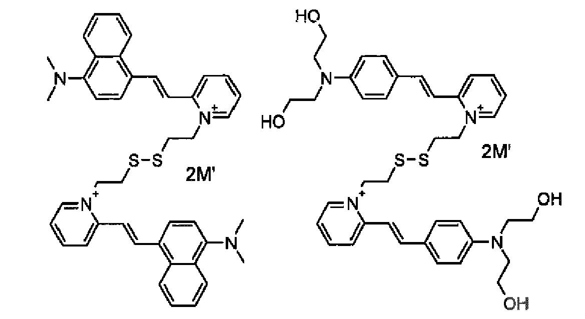 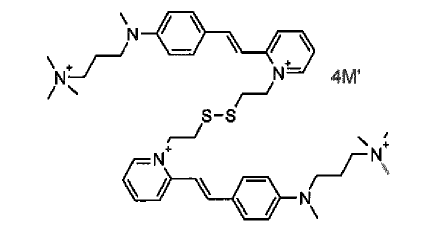 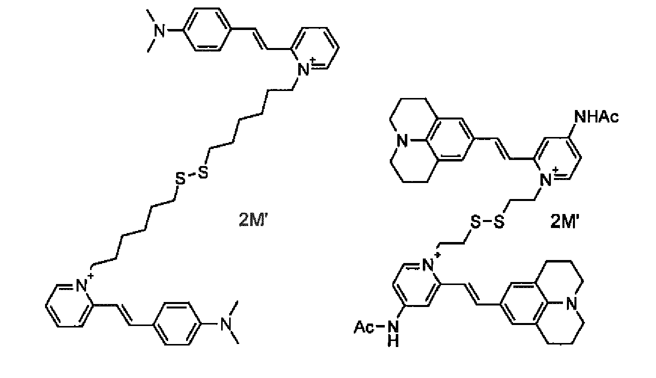 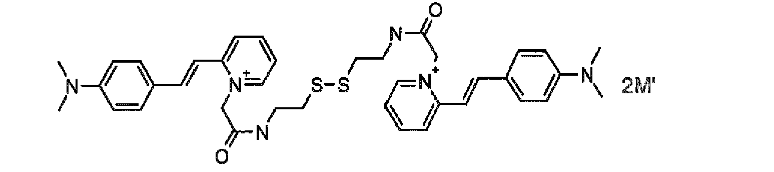 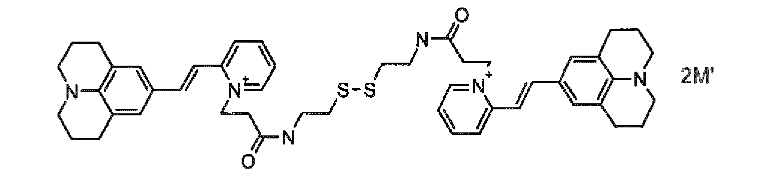 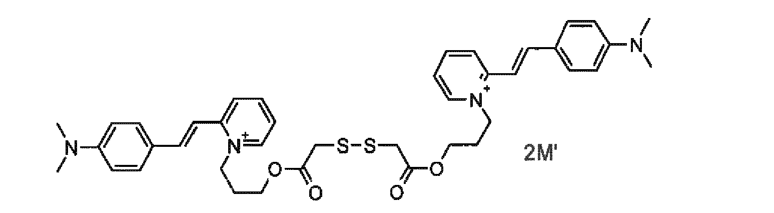 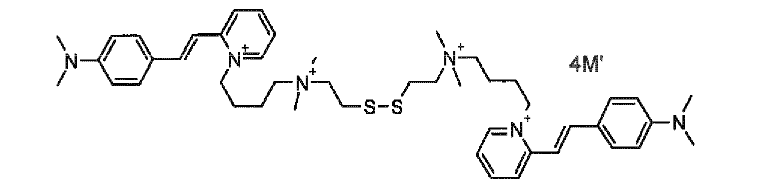 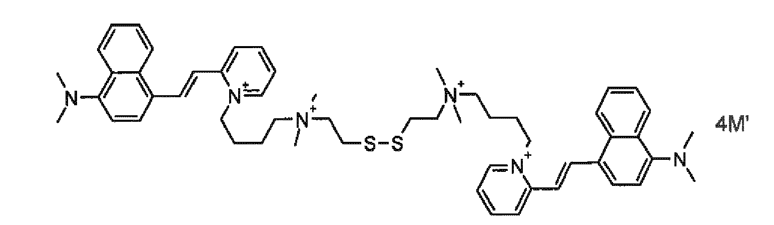 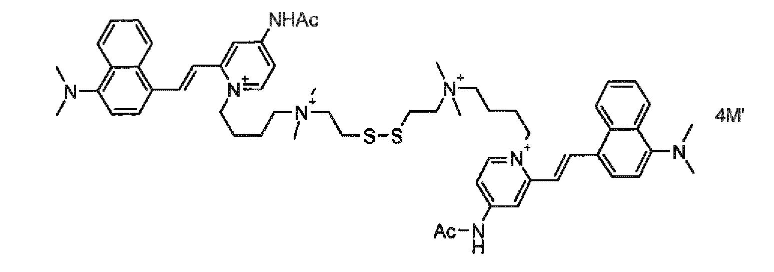 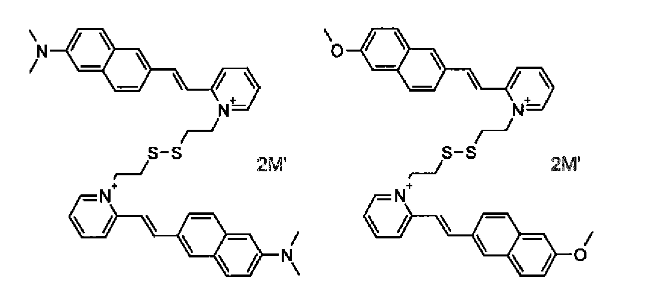 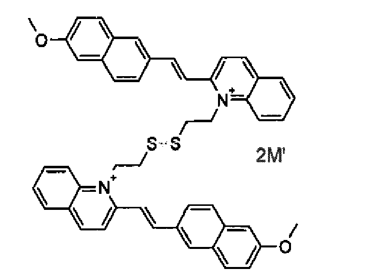 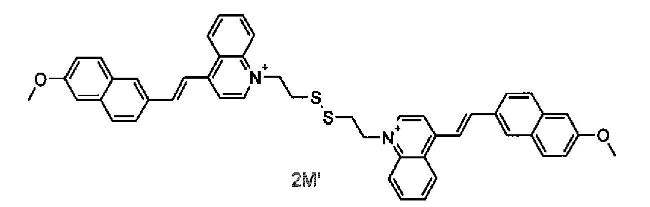 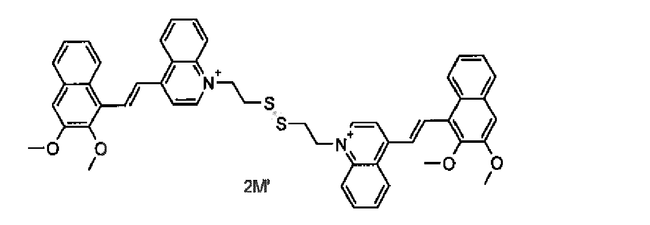 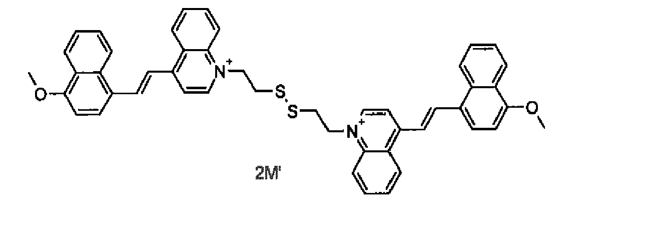 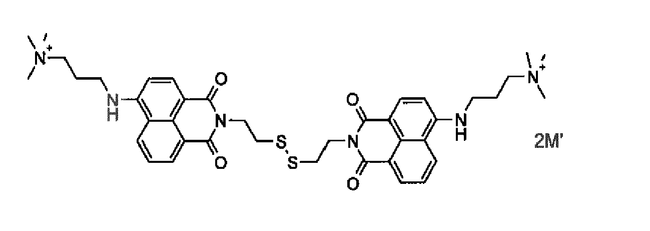 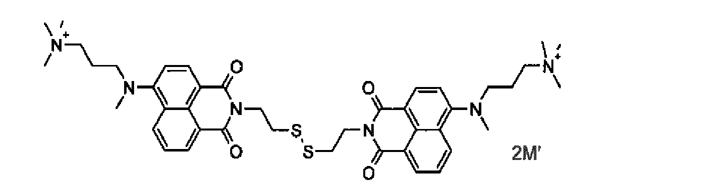 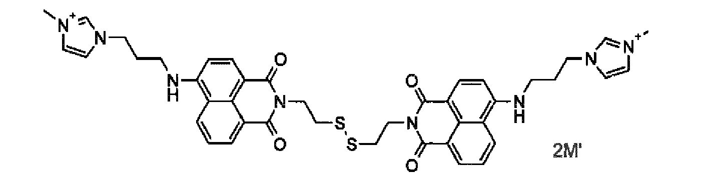 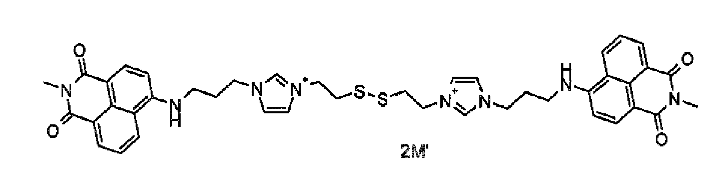 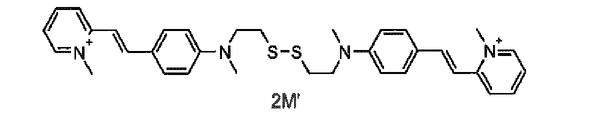 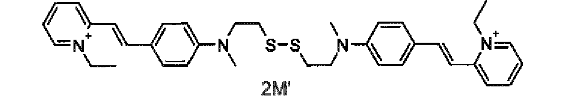 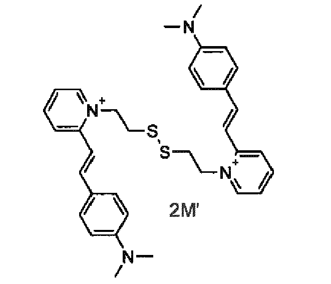 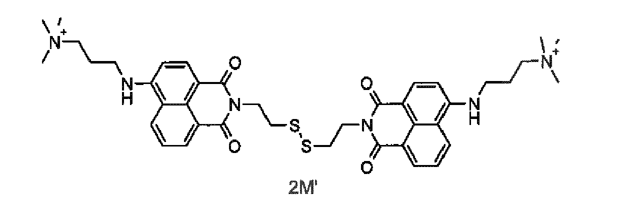 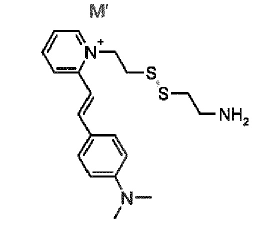 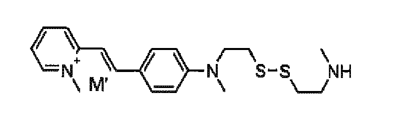 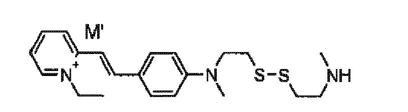 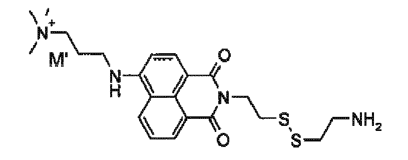 und 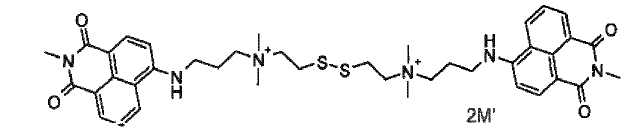 wobei M' für ein organisches oder anorganisches Säuresalz steht,

12. Zusammensetzung nach einem der vorhergehenden Ansprüche, **dadurch gekennzeichnet, dass** das alkalische Mittel vom Typ anorganisches oder organisches Hydroxid aus Hydroxiden von Alkalimetallen, Erdalkalimetallen, Übergangsmetallen, insbesondere der Gruppen IIIB, IVB, VB und VIB, Lanthaniden oder Actiniden, Ammonium- und Guanidinhydroxiden oder Mischungen davon ausgewählt ist.

13. Verfahren zum gleichzeitigen Formen, Färben und/oder Aufhellen von Keratinfasern, **dadurch gekennzeichnet, dass** man die Färbezusammensetzung gemäß einem der Ansprüche 1 bis 12 über einen zum Erhalt der gewünschten Formung, Färbung und/oder Aufhellung ausreichenden Zeitraum auf die Keratinfasern aufbringt und dann die Keratinfasern gegebenenfalls spült und mit Shampoo wäscht.

14. Vorrichtung mit mehreren Kompartimenten, **dadurch gekennzeichnet, dass** sie ein erstes Kompartiment (i), das ein oder mehrere alkalische Mittel vom Typ anorganisches oder organisches Hydroxid nach Anspruch 12 umfasst, und ein zweites Kompartiment (ii), das eine Zusammensetzung, die einen oder mehrere Disulfid-Direktfarbstoffe nach einem der Ansprüche 1 bis 11 enthält, umfasst, umfasst, wobei der pH-Wert der Mischung des Inhalts der verschiedenen Kompartimente zwischen 10 und 14 liegt.

## Claims

1. Composition for treating keratin fibres comprising, in a cosmetically acceptable medium:
* one or more disulfide direct dyes chosen from the compounds of formulae (I), (II) or (III) below: 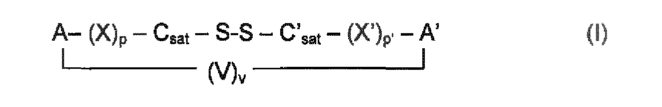 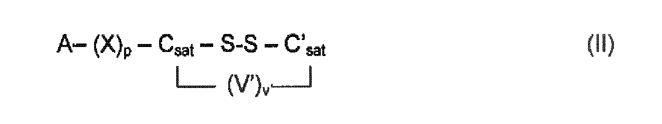
A- (X)ₚ- Cₛₐₜ - S-S - C'ₛₐₜ - (X')_{p'} - D (III)
their salts, optical isomers, geometrical isomers and solvates such as hydrates,
in which formulae:
• A and A', which are identical or different, represent a radical containing one or more cationic or non-cationic chromophores;
• V and V', which are identical or different, represent a bridging group;
• v and v', which are identical or different, represent 0 or 1;
• the coefficients p and p', which are identical or different, represent an integer equal to 0 or 1;
• X and X', which are identical or different, represent:
1) a saturated or unsaturated, linear or branched C₁-C₃₀ hydrocarbon chain optionally interrupted and/or optionally terminated at one or both of its ends by one or more divalent groups or combinations thereof, chosen from: -N(R)-, -N⁺(R)(R)-, -O-, -S-, -CO-, -SO₂- with R and R', which are identical or different, being chosen from hydrogen, a C₁-C₄ alkyl, hydroxyalkyl or aminoalkyl radical,
an optionally substituted, saturated or unsaturated, fused or non-fused, aromatic or non-aromatic (hetero)cyclic radical optionally comprising one or more identical or different heteroatoms; or
2) the following sequence:
- (T)ₜ - (Y)_{y} - (Z)_{z}-
said sequence being linked in formulae (I), (II) or (III) as follows:
- Cₛₐₜ (or C'ₛₐₜ respectively) - (T)ₜ- (Y)_{y} - (Z)_{z} - (A or A' respectively);
in which:
(i) T represents one or more radicals or combinations thereof, chosen from -SO₂-, -O-, - S-, -N(R)-, -N⁺(R)(R') -CO-, where R and R', which are identical or different, represent a hydrogen atom, a C₁-C₄ alkyl radical or a C₁-C₄ hydroxyalkyl radical;
the coefficient t is equal to 0 or 1;
(ii) Y represents:
- a radical chosen from -CO-, (CH₂)-SO₂-, - (CH₂)₂-SO₂-; -CH₂-CHR-CO-NR'- with R and R', which are identical or different, representing a hydrogen atom, a C₁-C₄ alkyl radical;
- a group of formula (a), (a') or (a'''): 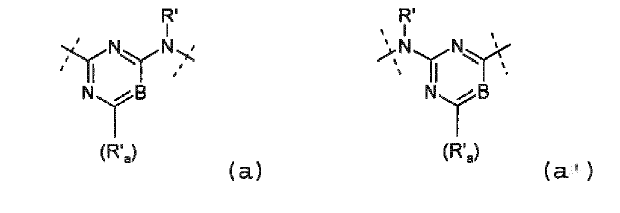 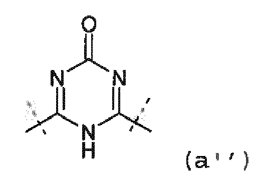
in which:
• B represents -N-, -CRₐ, with
Rₐ representing
- a hydrogen atom,
- a halogen atom chosen from chlorine or fluorine,
- a nitro group,
- a pyridinium group which is optionally substituted,
- a hydroxyl group;
- an amino, alkylamino or dialkylamino group, the identical or different C₁-C₁₈ alkyl groups being linear or branched, optionally interrupted by a heteroatom chosen from N, O, which are optionally substituted with one or more hydroxyl groups;
- a group NHNHCOR where R represents a linear or branched C₁-C₁₀ alkyl group;
• R' has the same definition as above
• R'ₐ represents:
- a hydrogen atom
- a chlorine atom or a fluorine atom
- a pyridinium group which is optionally substituted with at least one group R_{c}, it being possible for R_{c} to be a C₁-C₄ alkyl group, a halogen atom, a carboxyl group -COOM (with M representing a hydrogen atom, an alkali metal, an ammonium group or an ammonium group substituted with one or more linear or branched, identical or different C₁-C₁₈ alkyl radicals, optionally bearing at least one hydroxyl); an ester group -COOR_{d} with R_{d} representing a C₁-C₄ alkyl radical; an amide group -CON(R_{d})₂ with the R_{d} groups which are identical or different, representing a hydrogen atom or a C₁-C₄ alkyl radical;
- a hydroxyl group;
- an amino, alkylamino or dialkylamino group, the identical or different C₁-C₁₈ alkyl groups being linear or branched, optionally interrupted by a heteroatom chosen from N, O, which are optionally substituted with one or more hydroxyl groups;
- a group NHNHCOR where R represents a linear or branched C₁-C₁₀ alkyl group;
- a group of the following formula (b): 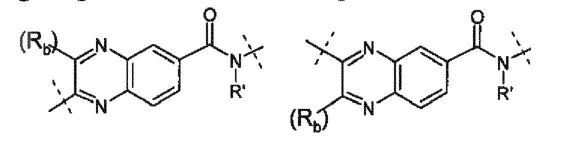
in which:
• R' has the same definition as above
• R_{b} represents
- a chlorine atom
- an amino, alkylamino or dialkylamino group, the identical or different C₁-C₁₈ alkyl groups being linear or branched, optionally interrupted by a heteroatom chosen from N, O, S, which are optionally substituted with one or more hydroxyls
- a saturated or unsaturated nitrogen-containing heterocycle which may be substituted
- an arylamino group in which the aryl radical is preferably a C₆ aryl radical;
y is equal to 0 or 1;
(iii) Z represents
- -(CH₂)ₘ- with m being an integer between 1 and 8 - -(CH2_{C}H₂O)_{q}- or - -(OCH₂CH₂)_{q}- in which q is an integer between 1 and 15
- an aryl, alkylaryl or arylalkyl radical of which the alkyl radical is a C₁-C₄ alkyl radical and the aryl radical is preferably a C₆ aryl radical being optionally substituted with at least one group SO₃M with M representing a hydrogen atom, an alkali metal or an ammonium group substituted with one or more identical or different, linear or branched C₁-C₁₈ alkyl radicals optionally bearing at least one hydroxyl
z is equal to 0 or 1;
• Cₛₐₜ and C'ₛₐₜ, which are identical or different, represent an optionally substituted, optionally cyclic, linear or branched C₁-C₁₈ alkylene chain;
• D corresponds to a radical chosen from hydroxyl, hydroxyalkyl, alkoxy, carboxyl, carboxylate, amino, alkylamino and dialkylamino radicals; and
* one or more alkaline agents of mineral or organic hydroxide type in amount such that the pH of the composition is between 10 and 14.

2. Composition according to Claim 1, **characterized in that**, in formulae (I), (II) or (III), when p and/or p' respectively are equal to 1, X and/or X' respectively represent the following sequence:
-(T)ₜ-(Y)y-(Z)_{z}-
said sequence being linked in formulae (I), (II) or (III) as follows:
- Cₛₐₜ (or C'ₛₐₜ respectively) - (T)ₜ- (Y) y- (Z)_{z}- (A or A' respectively);
in which:
T represents one or more radicals or combinations thereof, chosen from -SO₂-, -O-, -S-, -N(R)-, -N⁺(R)(R') -CO-, where R and R', which are identical or different, represent a hydrogen atom, a C₁-C₄ alkyl radical or a C₁-C₄ hydroxyalkyl radical;
the coefficient t is equal to 0 or 1;
Y represents:
- a radical chosen from -CO-, - (CH₂) -SO₂, - (CH2)₂- SO₂-; -CH₂-CHR-CO-NR'- with R and R', which are identical or different, representing a hydrogen atom, a C₁-C₄ alkyl radical;
- a group of formula (a), (a') or (a''') : 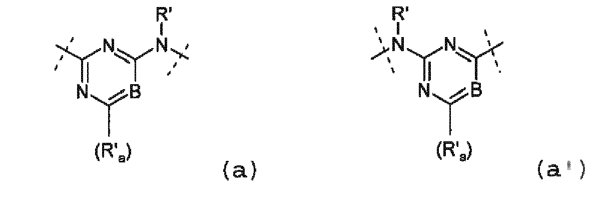 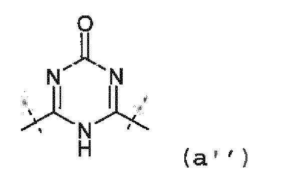
in which:
• B represents -N-, -CRₐ, with
- Rₐ representing
- a hydrogen atom,
- a halogen atom chosen from chlorine or fluorine,
- a nitro group,
- a pyridinium group which is optionally substituted,
- a hydroxyl group;
- an amino, alkylamino or dialkylamino group, the identical or different C₁-C₁₈ alkyl groups being linear or branched, optionally interrupted by a heteroatom chosen from N, O, which are optionally substituted with one or more hydroxyl groups;
- a group NHNHCOR where R represents a linear or branched C₁-C₁₀ alkyl group;
• R' has the same definition as above
• R'ₐ represents:
- a hydrogen atom
- a chlorine atom or a fluorine atom
- a pyridinium group which is optionally substituted with at least one group R_{c}, it being possible for R_{c} to be a C₁-C₄ alkyl group, a halogen atom, a carboxyl group -COOM (with M representing a hydrogen atom, an alkali metal, an ammonium group or an ammonium group substituted with one or more linear or branched, identical or different C₁-C₁₈ alkyl radicals, optionally bearing at least one hydroxyl); an ester group -COOR_{d} with R_{d} representing a C₁-C₄ alkyl radical; an amide group -CON(R_{d})₂ with the R_{d} groups which are identical or different, representing a hydrogen atom or a C₁-C₄ alkyl radical;
- a hydroxyl group;
- an amino, alkylamino or dialkylamino group, the identical or different C₁-C₁₈ alkyl groups being linear or branched, optionally interrupted by a heteroatom chosen from N, O, which are optionally substituted with one or more hydroxyl groups;
- a group NHNHCOR where R represents a linear or branched C₁-C₁₀ alkyl group;
- a group of the following formula (b): 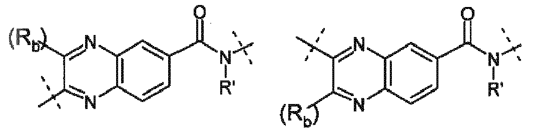
in which:
• R' has the same definition as above
• R_{b} represents
- a chlorine atom
- an amino, alkylamino or dialkylamino group, the identical or different C₁-C₁₈ alkyl groups being linear or branched, optionally interrupted by a heteroatom chosen from N, O, S, which are optionally substituted with one or more hydroxyls
- a saturated or unsaturated nitrogen-containing heterocycle which may be substituted
- an arylamino group in which the aryl radical is preferably a C₆ aryl radical;
y is equal to 0 or 1;
Z represents
- -(CH2)ₘ- with m being an integer between 1 and 8
- -(CH₂CH₂O)_{q}- or -(OCH₂CH₂)_{q}- in which q is an integer between 1 and 15
- an aryl, alkylaryl or arylalkyl radical of which the alkyl radical is a C₁-C₄ alkyl radical and the aryl radical is preferably a C₆ aryl radical being optionally substituted with at least one group SO₃M with M representing a hydrogen atom, an alkali metal or an ammonium group substituted with one or more identical or different, linear or branched C₁-C₁₈ alkyl radicals optionally bearing at least one hydroxyl
z is equal to 0 or 1;
• Cₛₐₜ and C'ₛₐₜ, which are identical or different, represent an optionally substituted, optionally cyclic, linear or branched C₁-C₁₈ alkylene chain;
• D corresponds to a radical chosen from hydroxyl, hydroxyalkyl, alkoxy, carboxyl, carboxylate, amino, alkylamino and dialkylamino radicals.

3. Composition according to Claim 1 or 2, **characterized in that** in formulae (I), (II) or (III), A and A' represent a radical comprising at least one chromophore derived from dyes of the following type: acridines, acridones, anthranthrones, anthrapyrimidines, anthraquinones, azines, azos, azomethines, benzanthrones, benzimidazoles, benzimidazolones, benzindoles, benzoxazoles, benzopyrans, benzothiazoles, benzoquinones, bisazines, bisisoindolines, carboxanilides, coumarins, cyanins, diazines, diketopyrrolopyrroles, dioxazines, diphenylamines, diphenylmethanes, dithiazines, flavonoids, fluorindines, formazans, hydrazones, hydroxy ketones, indamines, indanthrones, indigoids and pseudo-indigoids, indophenols, indoanilines, isoindolines, isoindolinones, isoviolanthrones, lactones, methines, naphthalimides, naphthanilides, naphtholactams, naphthoquinones, nitro dyes, oxadiazoles, oxazines, perilones, perinones, perylenes, phenazines, phenothiazines, phthalocyanin, polyenes/carotenoids, porphyrins, pyranthrones, pyrazolanthrones, pyrazolones, pyrimidinoanthrones, pyronines, quinacridones, quinolines, quinophthalones, squaranes, stilbenes, tetrazoliums, thiazines, thioindigo, thiopyronines, triarylmethanes and xanthenes.

4. Composition according to any one of Claims 1 to 3, in which the disulfide direct dye is chosen from: 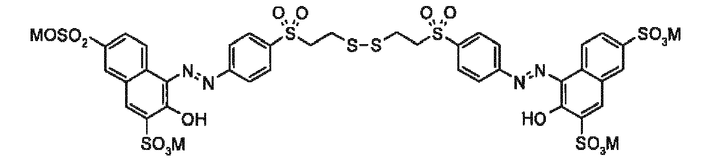 and also the following compounds, in acidic, basic or neutralized form: 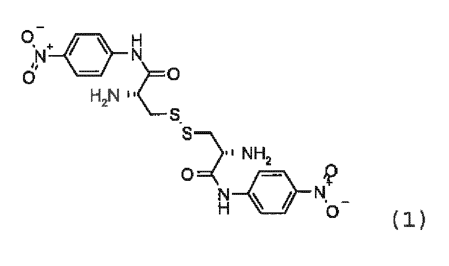 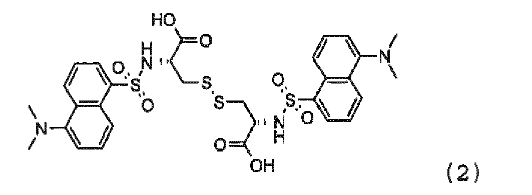 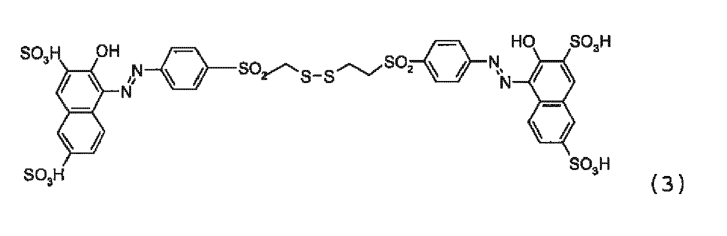 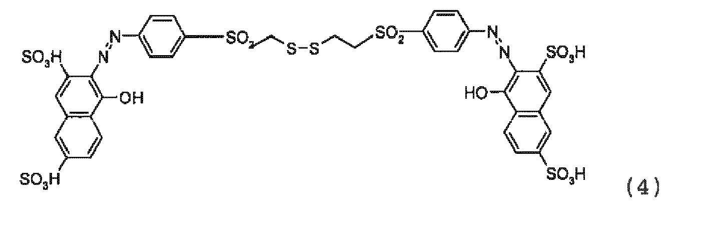 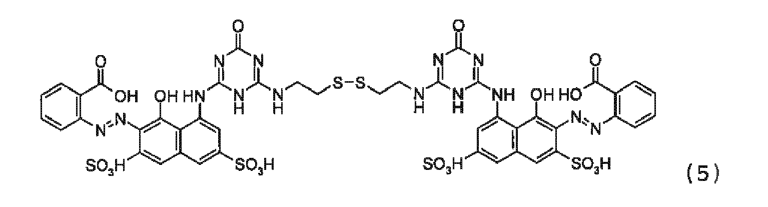 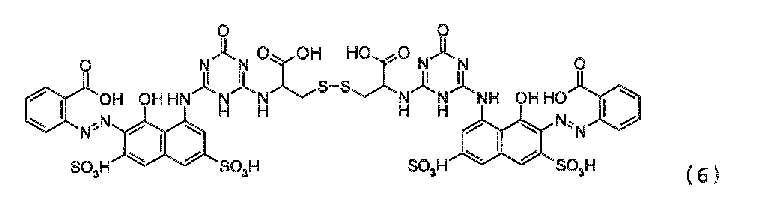 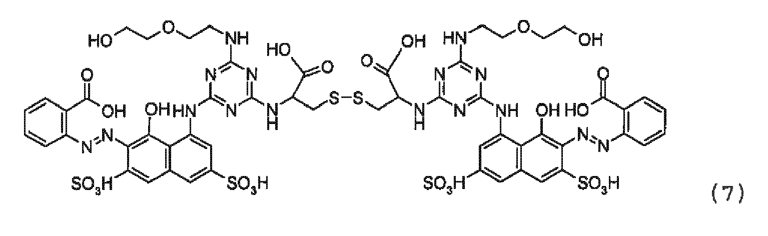 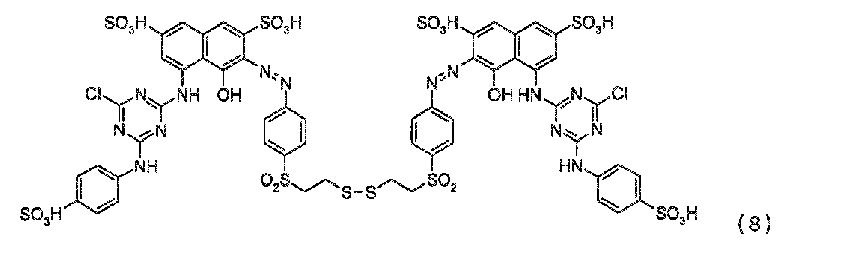 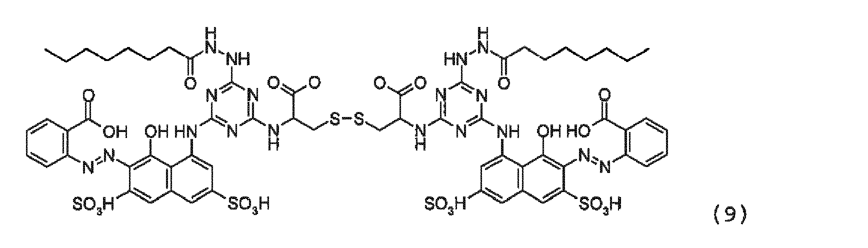 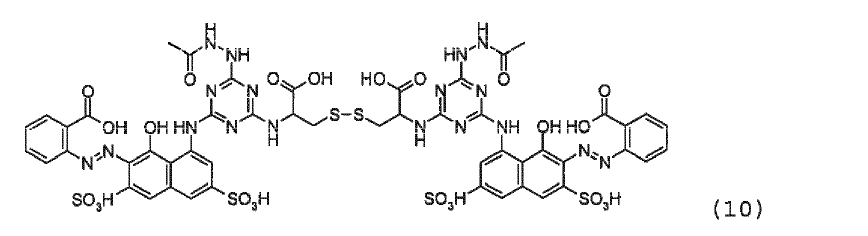 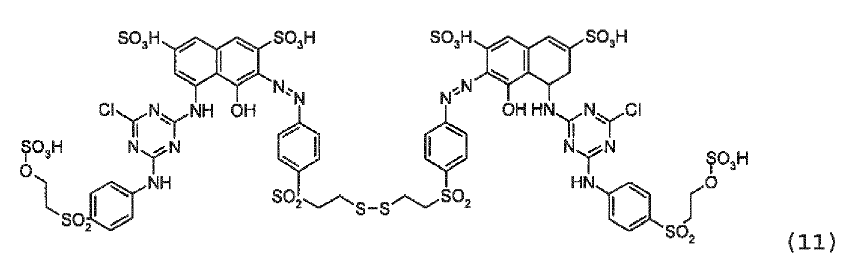 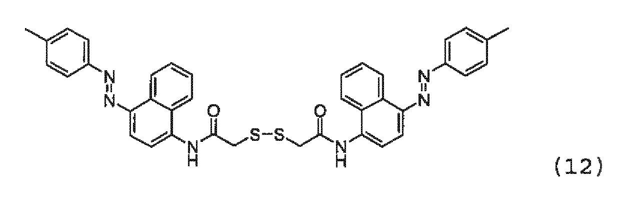 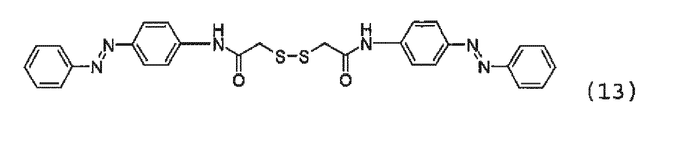 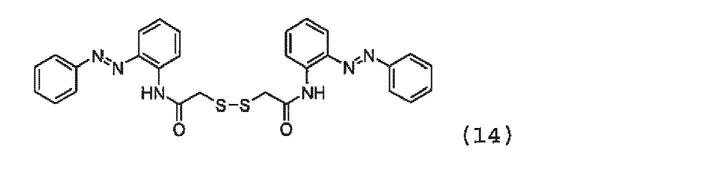 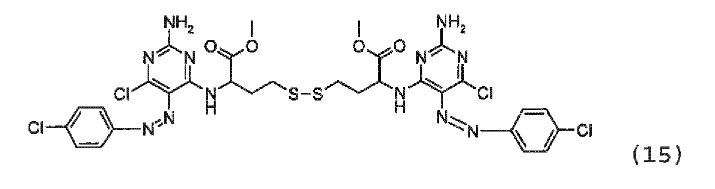 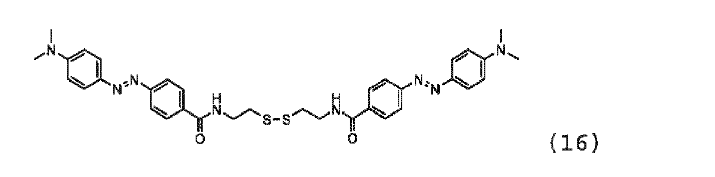 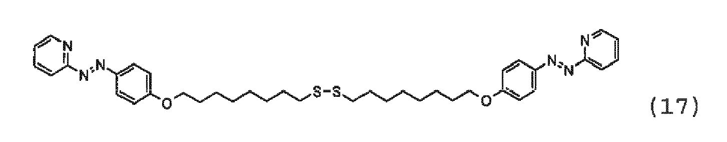 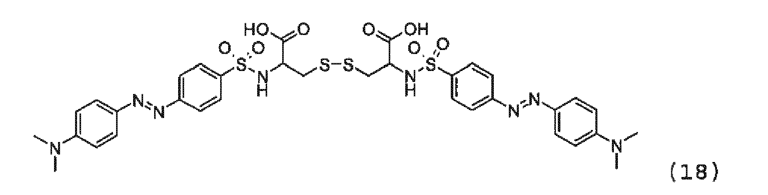 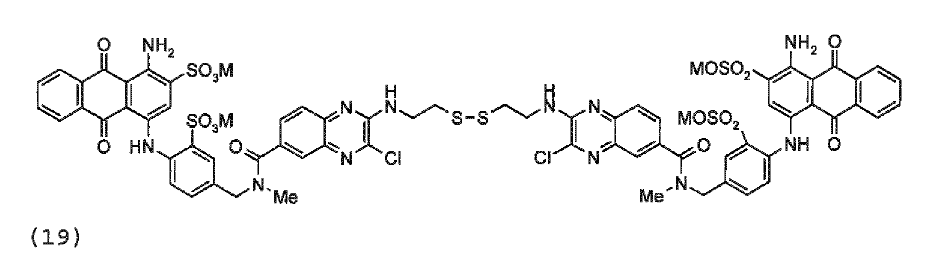 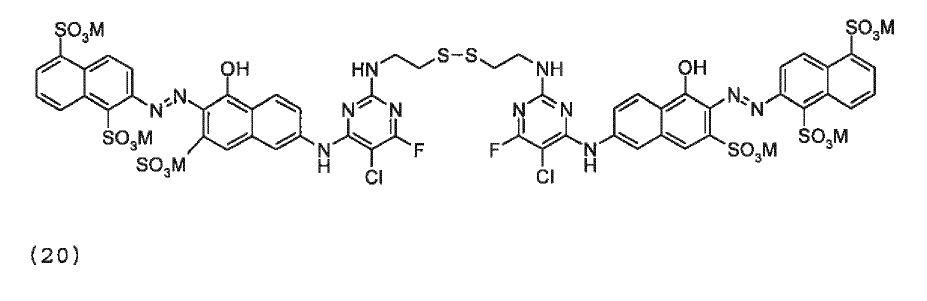 M representing a hydrogen atom, an alkali metal or an ammonium group or an ammonium group substituted with one or more identical or different, linear or branched C₁-C₁₀ alkyl radicals optionally bearing at least one hydroxyl; and the following compounds, their salts, hydrates and solvates:

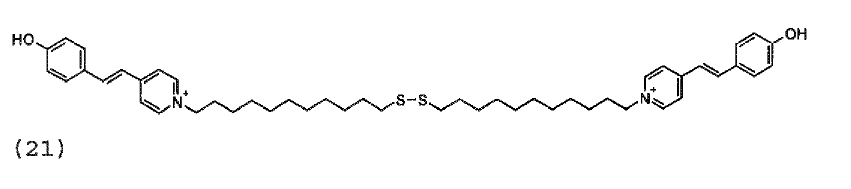

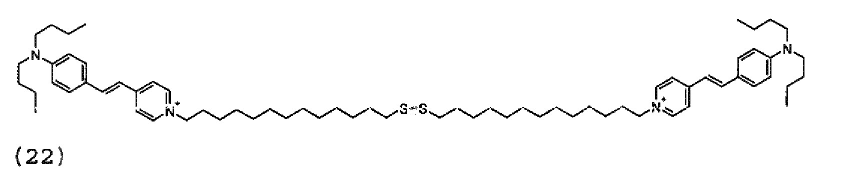

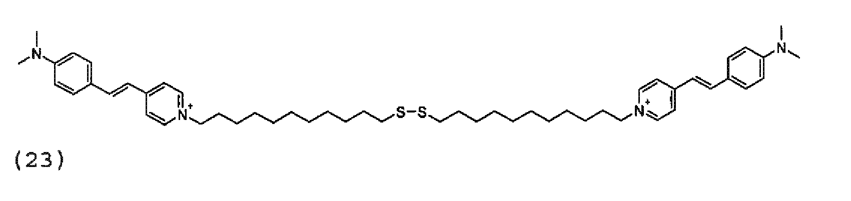

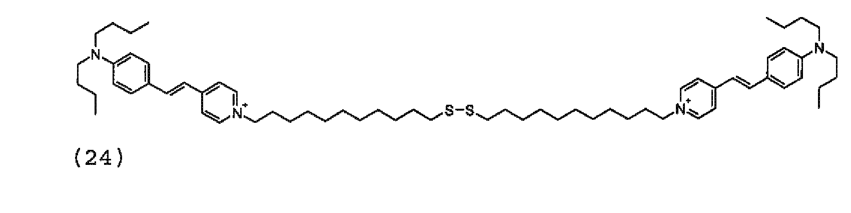

5. Composition according to Claims 1 to 3, **characterized in that** the cationic chromophore comprises a cationic radical which is a quaternary ammonium.

6. Composition according to Claim 1, **characterized in that** the disulfide direct dye is a dye of formula (I), (II) or (III) in which A and/or A', which are identical or different, represent a radical containing one or more cationic or non-cationic fluorescent chromophores.

7. Composition according to any one of Claims 1 to 3 and 5 to 6, **characterized in that** X and X', which are identical or different, represent an -N(R)-group.

8. Composition according to any one of Claims 5 to 7, **characterized in that** the disulfide direct dye is such that v or v' is equal to 0, and A and/or A', which are identical or different, and more preferably are identical, represent W-N=N-Ar- or - W-N=N-Ar or W-C(R^{c})=C(R^{d})-Ar- or -W-C(R^{c})=C(R^{d}) -Ar, with W representing a fused or non-fused, aromatic or non-aromatic heterocycle comprising a quaternary ammonium; Ar represents a (hetero)aryl radical having 5 or 6 ring members, or a (hetero)aromatic bicyclic ring of naphthyl, benzopyridinium, indolinyl or benzoindolinyl type, optionally substituted with one or more halogen atoms; with one or more alkyl groups; with one or more hydroxyl groups; with one or more alkoxy groups, with one or more hydroxyalkyl groups, with one or more amino or (di)alkylamino groups, with one or more acylamino groups; with one or more heterocycloalkyl or heteroaryl groups having 5 or 6 ring members; R^{c} and R^{d}, which are identical or different, represent a hydrogen atom or a C₁-C₄ alkyl group.

9. Composition according to any one of Claims 1 to 5 and 7 to 8, **characterized in that** the disulfide direct dye is chosen from: 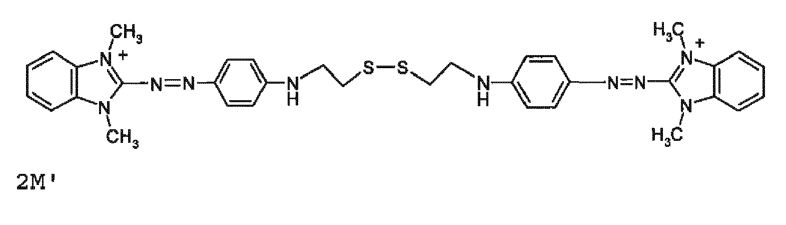 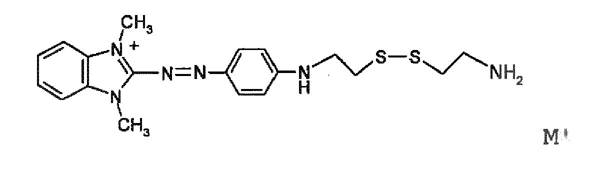 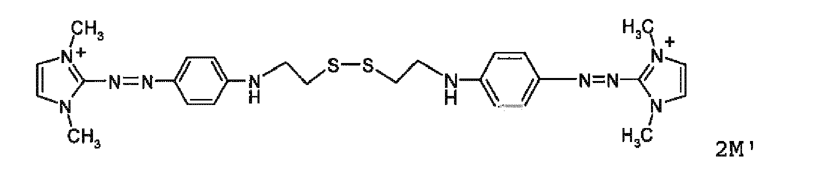 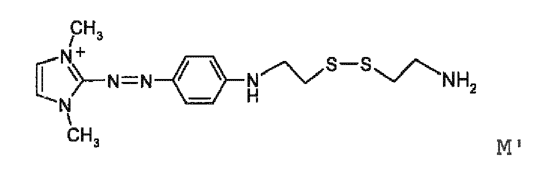 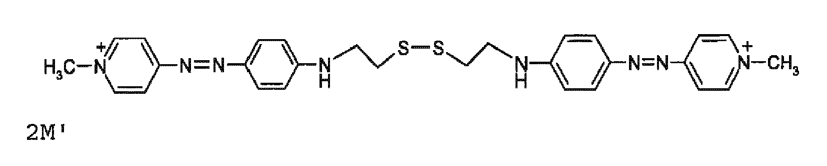 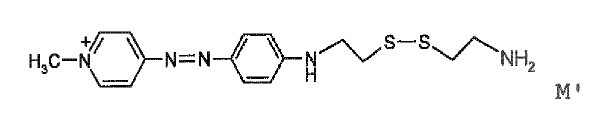 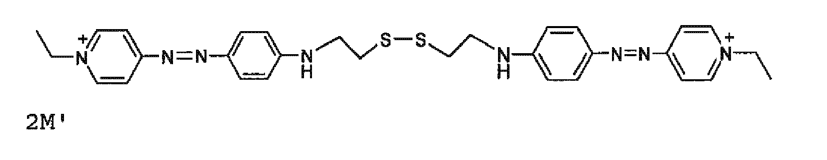 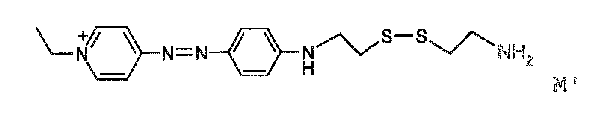 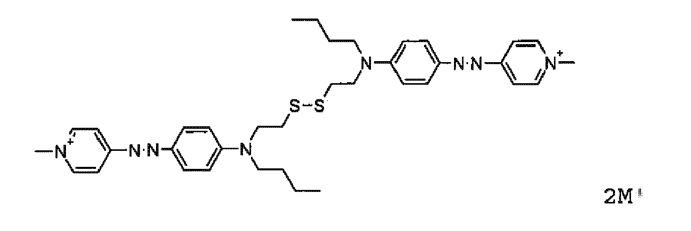 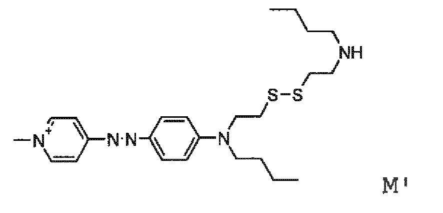
M' representing an organic or mineral acid salt.

10. Composition according to any one of Claims 1 and 6 to 8, **characterized in that** the disulfide direct dye is chosen from the compounds (III) to (XII): 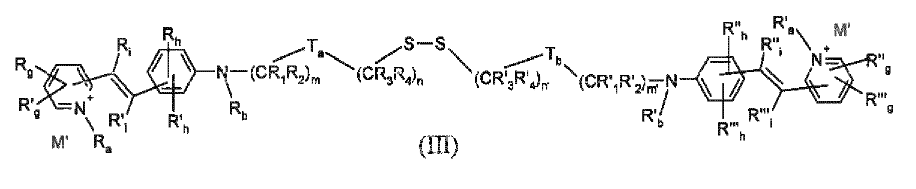 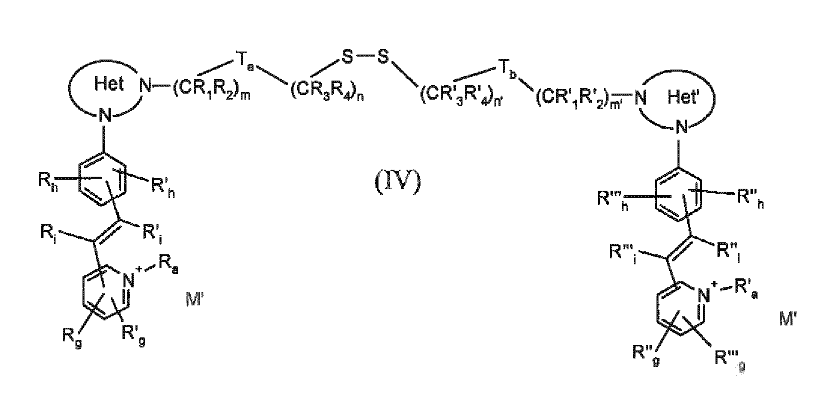 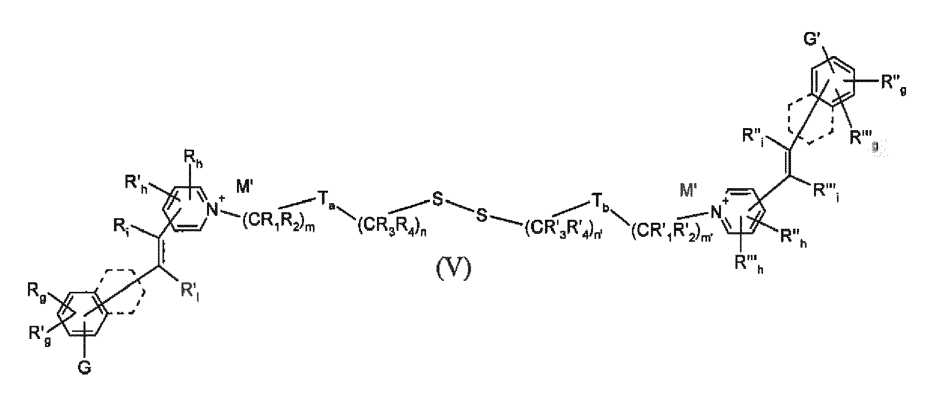 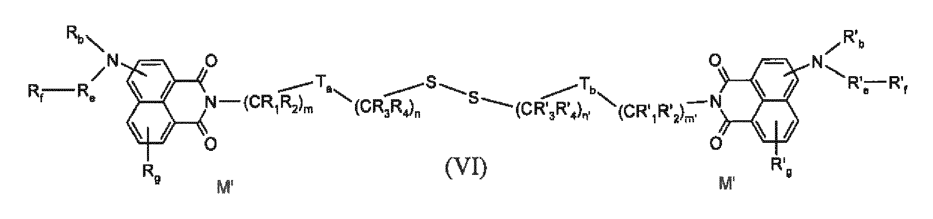 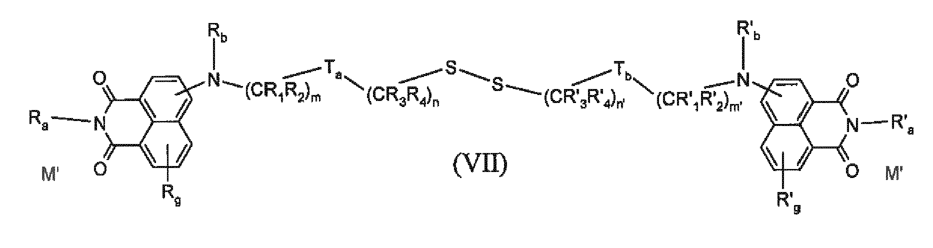 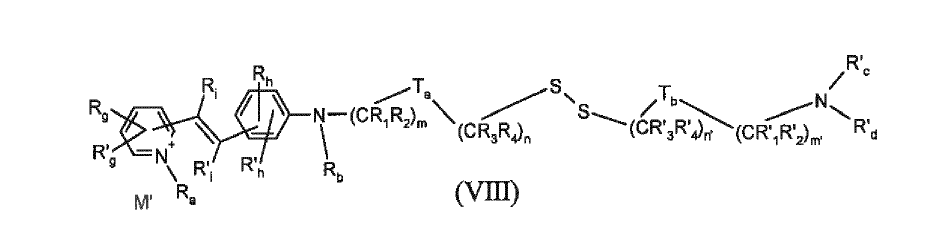 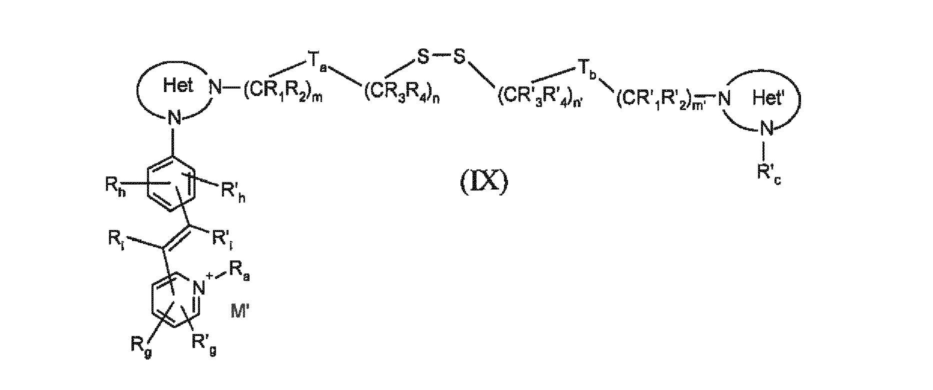 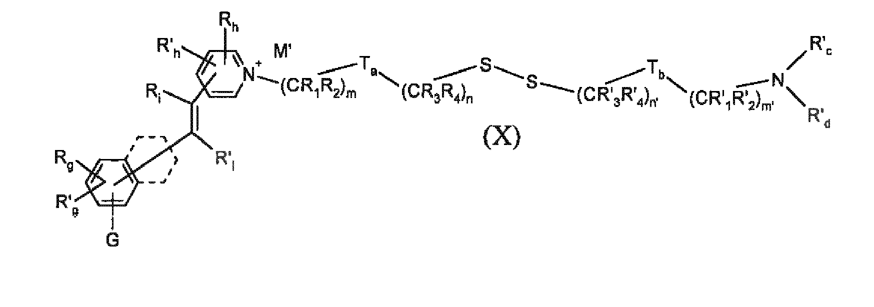 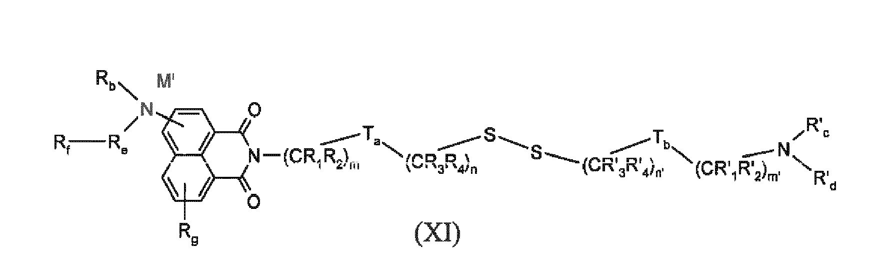 and 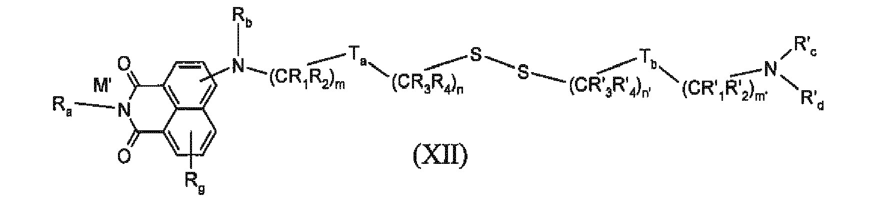 in which:
• G and G', which are identical or different, represent an optionally substituted -NR_{c}R_{d}, - NR'_{c}R'_{d}, or C₁-C₆ alkoxy group; preferably G and G' represent an -NR_{c}R_{d} and -NR'_{c}R'_{d} group respectively;
• Rₐ and R'ₐ, which are identical or different, represent an aryl(C₁-C₄)alkyl group or a C₁-C₆ alkyl group optionally substituted with a hydroxyl, amino, C₁-C₄ alkylamino or C₁-C₄ dialkylamino group, said alkyl radicals possibly forming with the nitrogen atom which bears them, a heterocycle comprising from 5 to 7 ring members, optionally comprising another heteroatom which may or may not be different from nitrogen;
• R_{b}, R'_{b}, which are identical or different, represent a hydrogen atom, an aryl(C₁-C₄)alkyl group or a C₁-C₆ alkyl group which is optionally substituted;
• R_{c}, R'_{c}, R_{d} and R'_{d}, which are identical or different, represent a hydrogen atom, an aryl(C₁-C₄) alkyl, C₁-C₆ alkoxy group or an optionally substituted C₁-C₆ alkyl group;
or else two adjacent radicals R_{c} and R_{d}, or R'_{c} and R'_{d} borne by the same nitrogen atom together form a heterocyclic or heteroaryl group;
• Rₑ and R'ₑ, which are identical or different, represent an optionally unsaturated, linear or branched, divalent C₁-C₆ alkylenyl hydrocarbon chain;
• R_{f} and R'_{f}, which are identical or different, represent a quaternary ammonium group (R'')(R''')(R'''')N⁺- where R'', R''' and R'''', which are identical or different, represent a hydrogen atom or a C₁-C₄ alkyl group or else (R'') (R''') (R'''')N⁺- represents an optionally substituted cationic heteroaryl group, preferably an imidazolinium group optionally substituted with a C₁-C₃ alkyl group;
• R_{g}, R'_{g}, R''_{g}, R'''_{g}, Rₕ, R'ₕ, R''ₕ, and R'''ₕ, which are identical or different, represent a hydrogen atom, a halogen atom, an amino, C₁-C₄ alkylamino, C₁-C₄ dialkylamino, cyano, carboxy, hydroxyl, or trifluoromethyl group, an acylamino, C₁-C₄ alkoxy, a (poly)hydroxy(C₂-C₄)alkoxy, alkylcarbonyloxy, alkoxycarbonyl or alkylcarbonylamino radical, an acylamino, carbamoyl or alkylsulfonylamino radical, an aminosulfonyl radical, or a C₁-C₁₆ alkyl radical optionally substituted with a group chosen from C₁-C₁₂ alkoxy, hydroxy, cyano, carboxy, amino, C₁-C₄ alkylamino and C₁-C₄ dialkylamino groups, or else the two alkyl radicals borne by the nitrogen atom of the amino group form a heterocycle comprising from 5 to 7 ring members and optionally comprising another heteroatom which is identical or different to that of the nitrogen atom;
or else two groups R_{g} and R'_{g}; R''_{g} and R'''_{g}; Rₕ, and R'ₕ; R''ₕ and R'''ₕ borne by two adjacent carbon atoms together form a benzo or indeno ring, or a fused heterocycloalkyl or fused heteroaryl group; the benzo, indeno, heterocycloalkyl or heteroaryl ring optionally being substituted with a halogen atom, an amino, C₁-C₄ alkylamino, C₁-C₄ dialkylamino, nitro, cyano, carboxy, hydroxyl, or trifluoromethyl group, an acylamino, C₁-C₄ alkoxy, a (poly) hydroxy(C₂-C₄) alkoxy, alkylcarbonyloxy, alkoxycarbonyl or alkylcarbonylamino radical, an acylamino, carbamoyl or alkylsulfonylamino radical, an aminosulfonyl radical, or a C₁-C₁₆ alkyl radical optionally substituted with a group chosen from C₁-C₁₂ alkoxy, hydroxy, cyano, carboxy, amino, C₁-C₄ alkylamino and C₁-C₄ dialkylamino groups, or else the two alkyl radicals borne by the nitrogen atom of the amino group form a heterocycle comprising from 5 to 7 ring members and optionally comprising another heteroatom which is identical or different to that of the nitrogen atom;
• or else when G represents -NR_{c}R_{d} and G' represents -NR'_{c}R'_{d}, two groups R_{c} and R'_{g}; R'_{c} and R''_{g}; R_{d} and R_{g}; or R'_{d} and R'''_{g} together form a saturated heteroaryl or heterocycle, optionally substituted with a C₁-C₆ alkyl group;
• Rᵢ, R'ᵢ, R''ᵢ, and R'''ᵢ, which are identical or different, represent a hydrogen atom or a C₁-C₄ alkyl group;
• R₁, R₂, R₃, R₄, R'ᵢ, R'₂, R'₃ and R'₄, which are identical or different, represent a hydrogen atom or a C₁-C₄ alkyl, C₁-C₁₂ alkoxy, hydroxy, cyano, carboxy, amino, C₁-C₄ alkylamino or C₁-C₄ dialkylamino group, said alkyl radicals possibly forming with the nitrogen atom that bears them, a heterocycle comprising from 5 to 7 ring members, optionally comprising another heteroatom that may or may not be different from nitrogen;
• Tₐ and T_{b}, which are identical or different, represent i) either a covalent bond σ, ii) or one or more radicals or combinations thereof chosen from -SO₂-, -O-, -S-, -N(R)-, -N⁺(R)(R°)- , -CO-, with R and R°, which are identical or different, representing a hydrogen atom, a C₁-C₄alkyl or C₁-C₄ hydroxyalkyl radical; or an aryl(C₁-C₄)alkyl radical, iii) or a cationic or non-cationic heterocycloalkyl or heteroaryl radical;
• 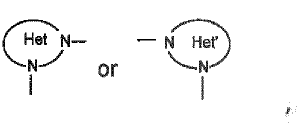 which are identical or different, represent an optionally substituted heterocyclic group;
• 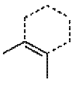 represent an aryl or heteroaryl group fused to the phenyl ring; or else that is lacking the phenyl ring;
• m, m', n and n', which are identical or different, represent an integer between 0 and 6 inclusive with m+n and m'+n', which are identical or different, representing an integer between 1 and 10 inclusive;
M' representing an organic or mineral acid salt.

11. Composition according to one of Claims 1, 6 to 8 and 10, **characterized in that** the disulfide direct dye is chosen from: 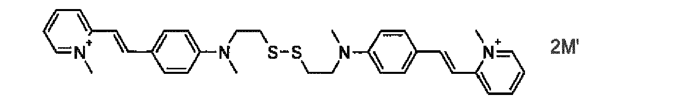 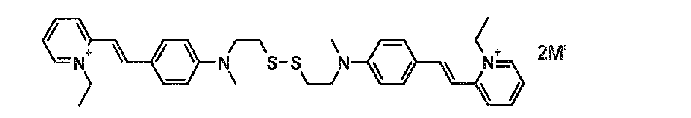 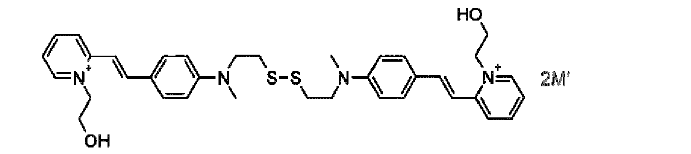 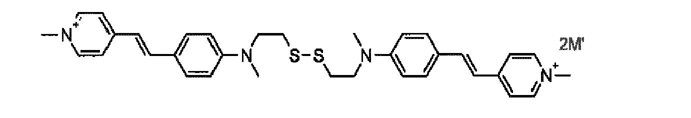 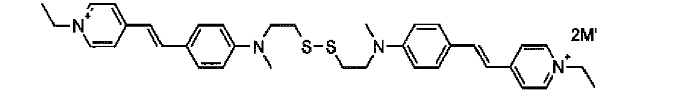 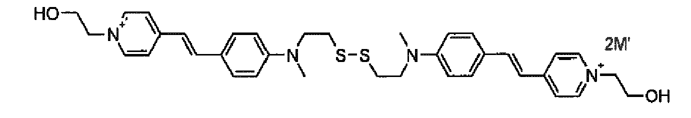 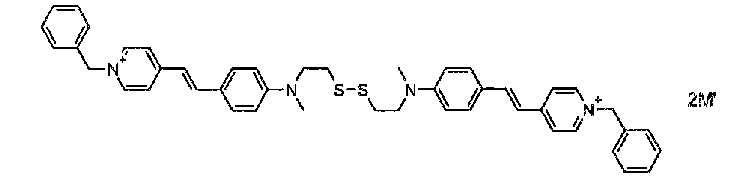 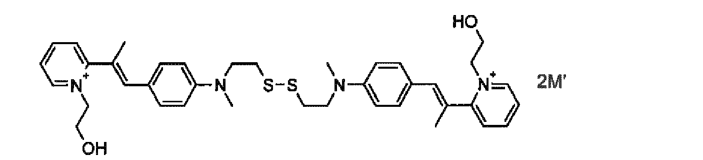 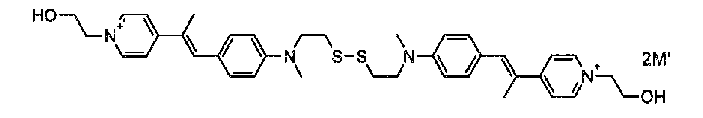 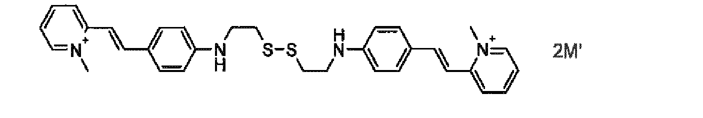 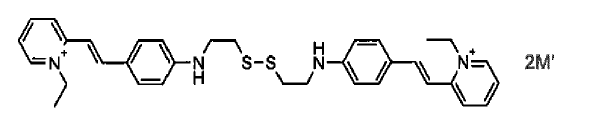 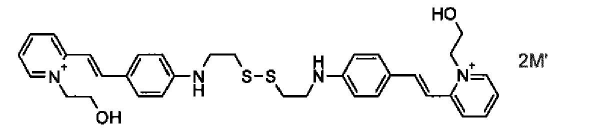 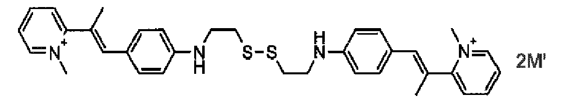 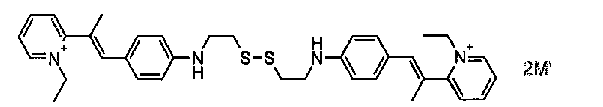 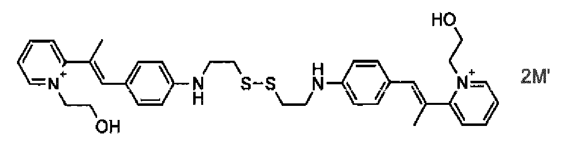 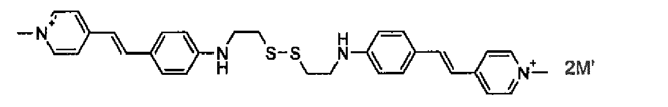 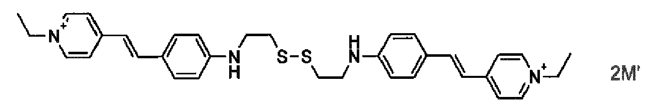 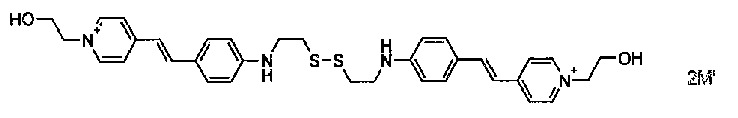 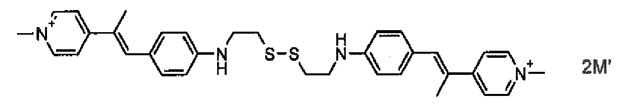 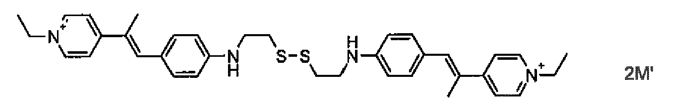 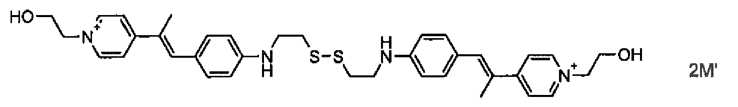 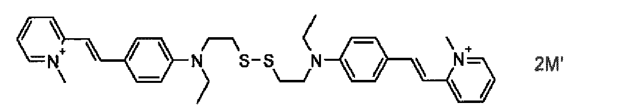 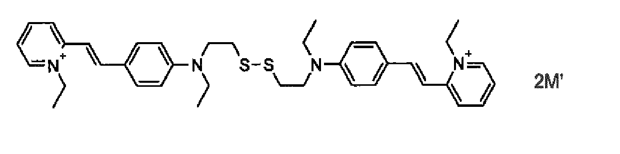 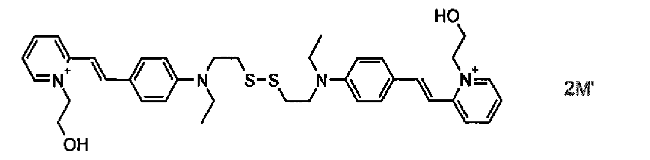 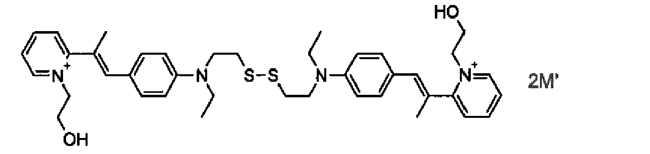 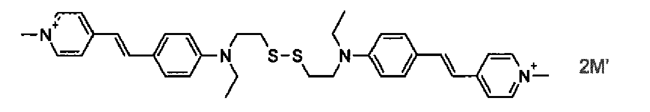 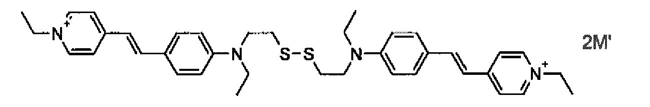 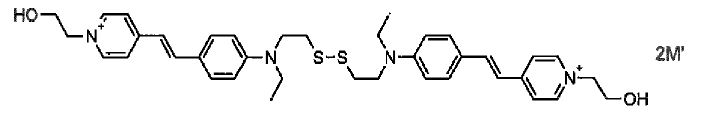 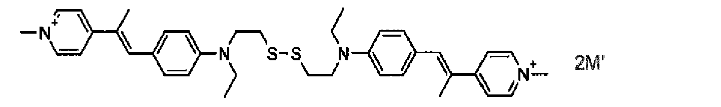 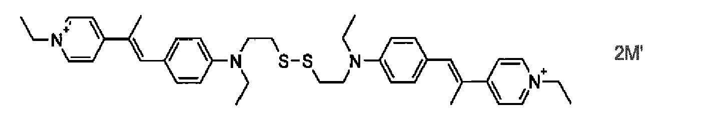 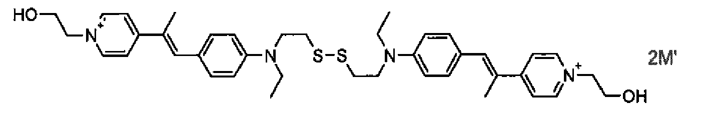 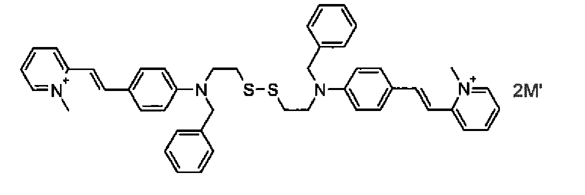 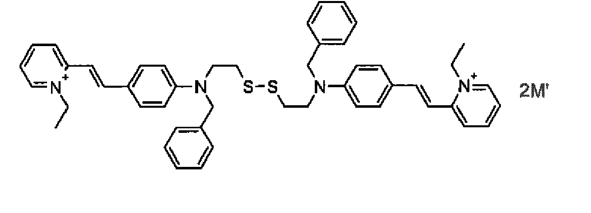 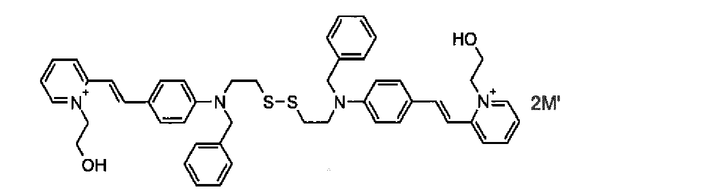 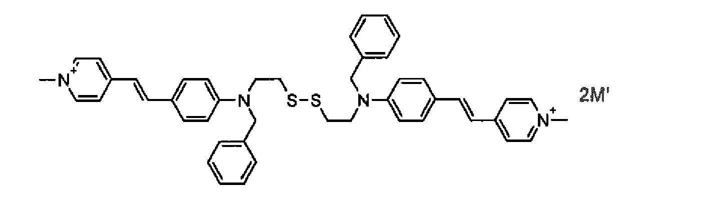 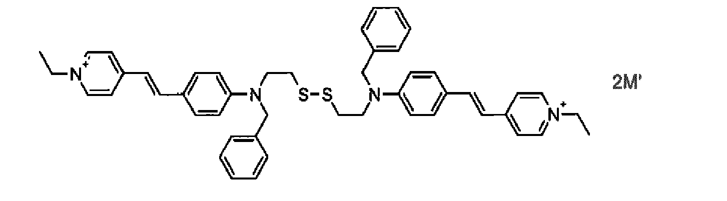 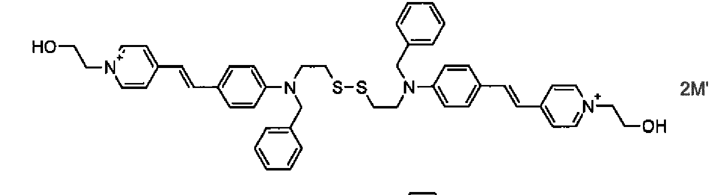 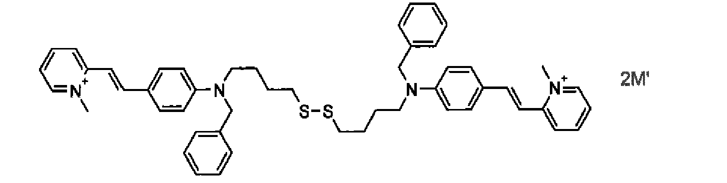 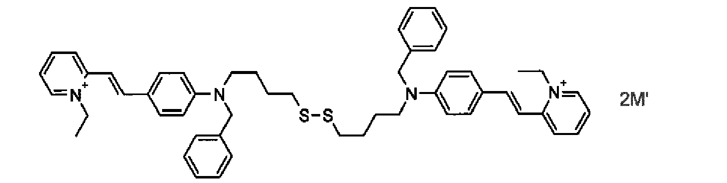 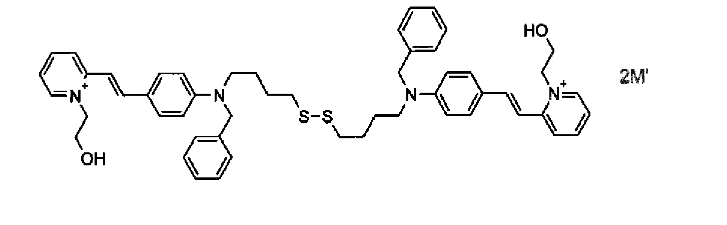 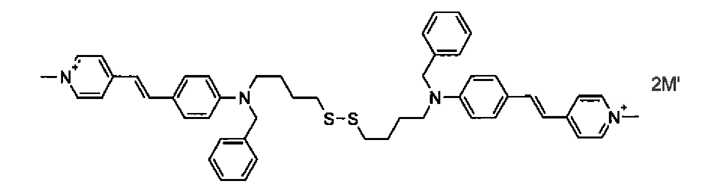 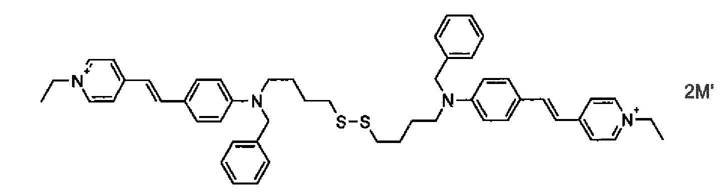 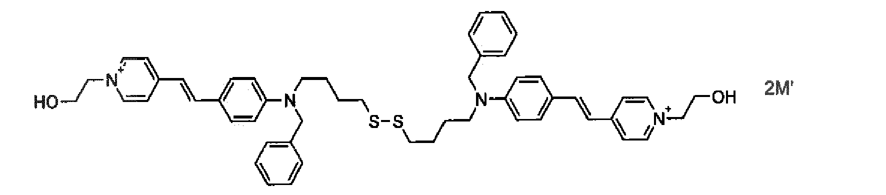 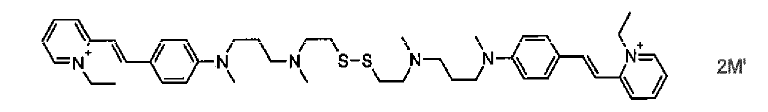 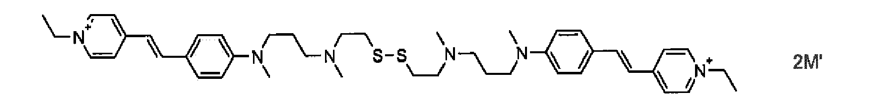 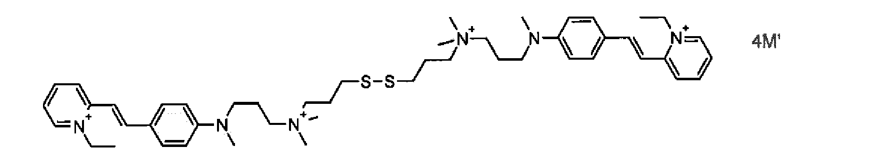 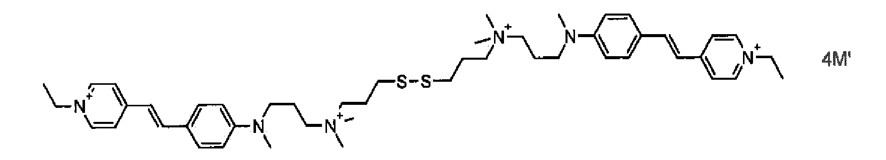 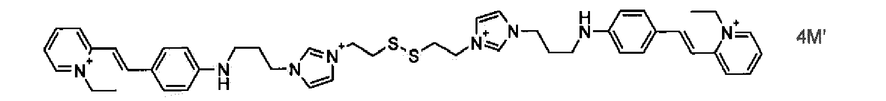 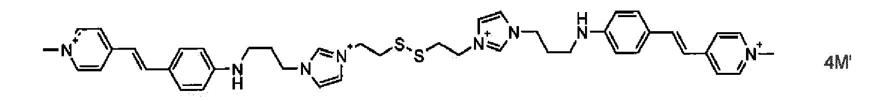 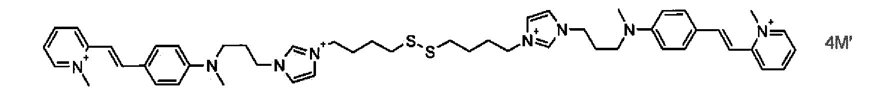 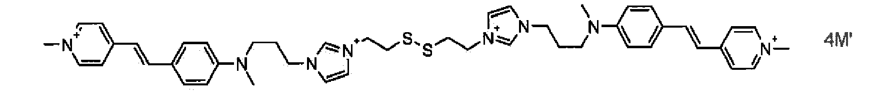 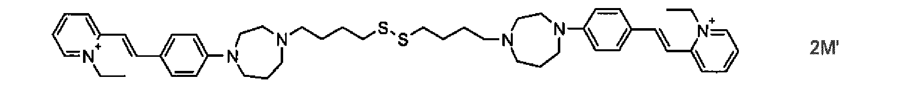 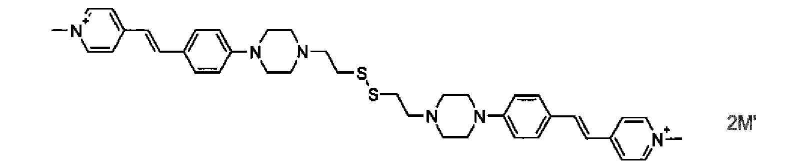 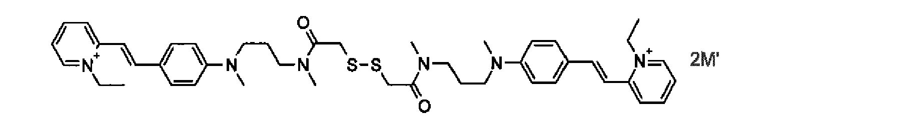 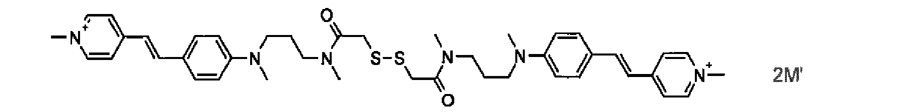 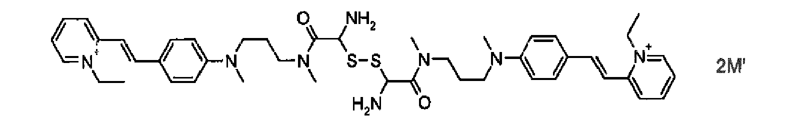 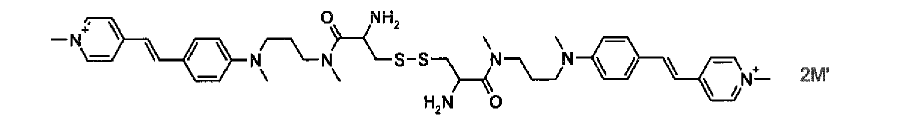 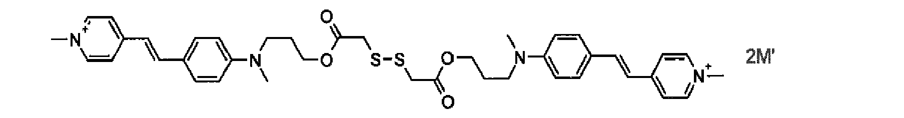 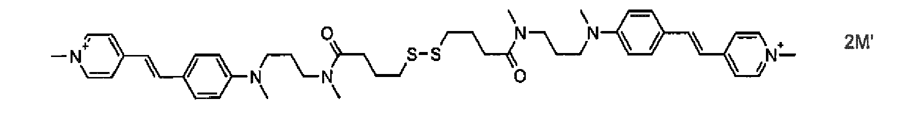 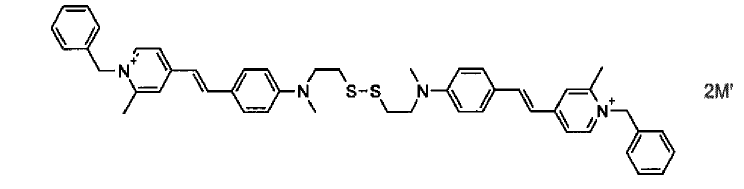 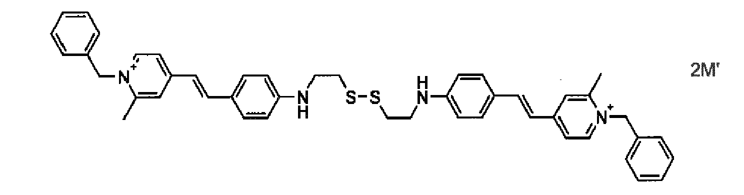 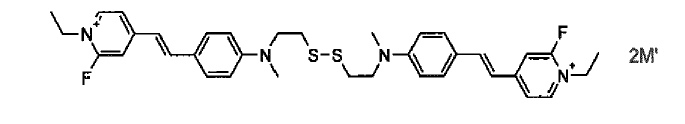 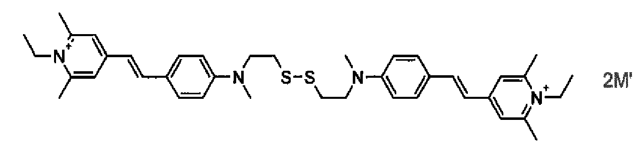 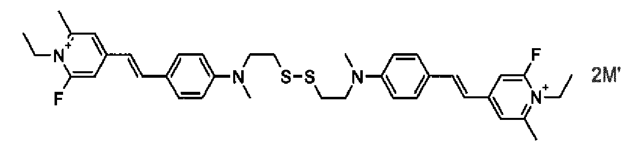 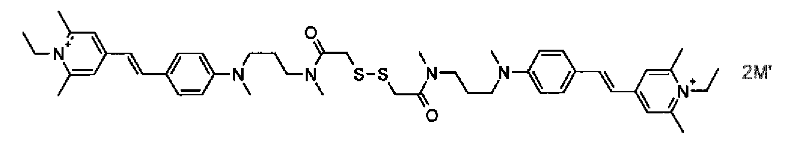 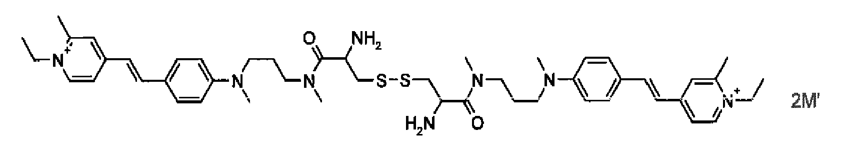 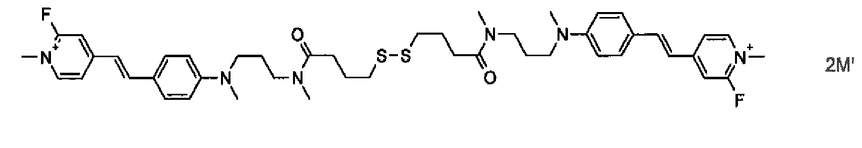 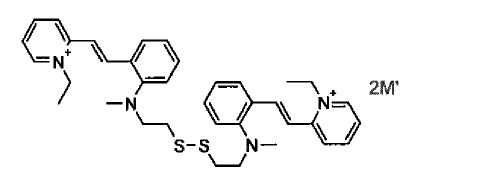 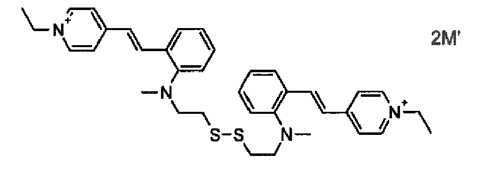 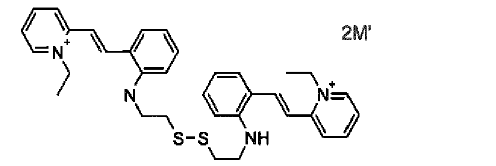 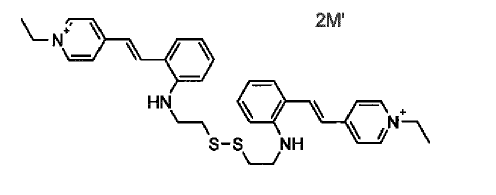 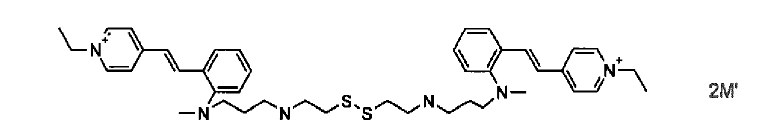 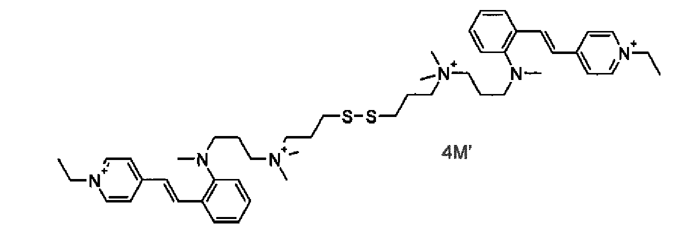 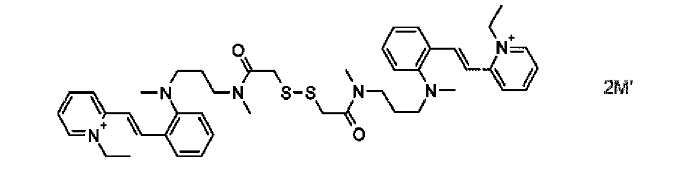 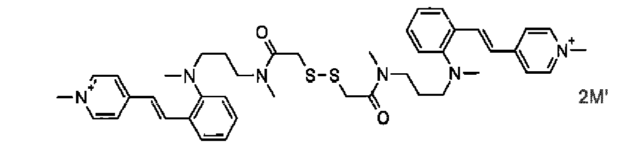 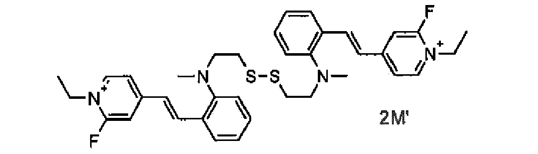 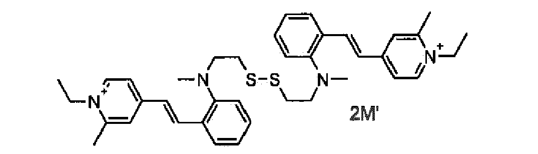 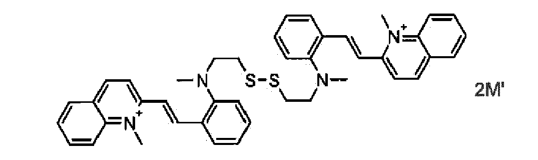 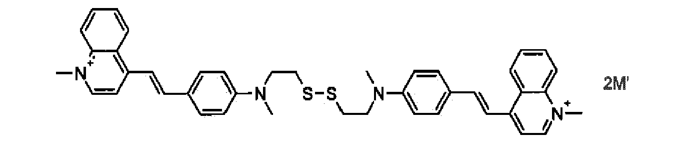 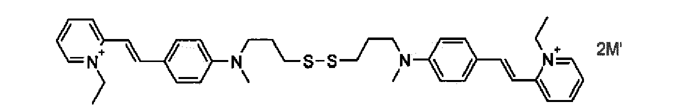 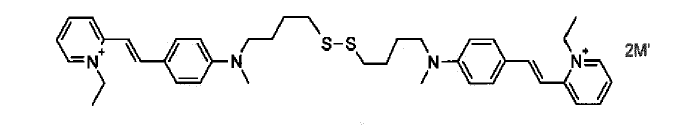 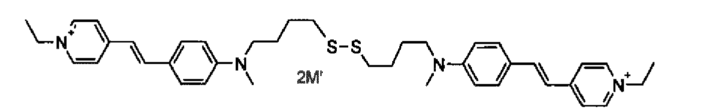 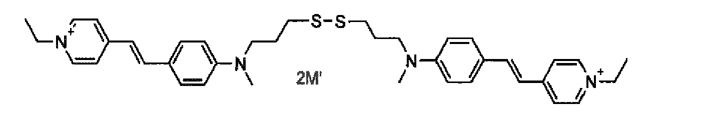 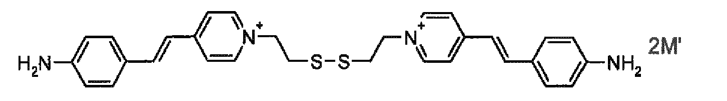 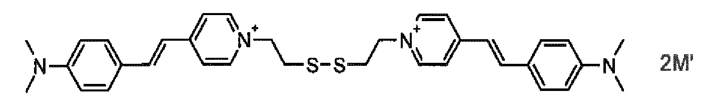 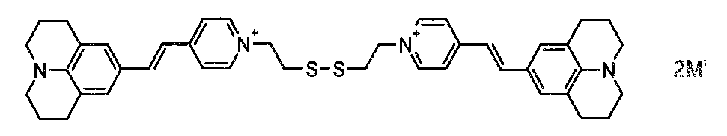 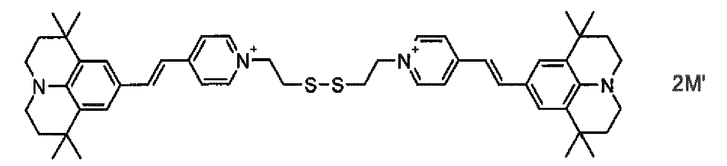 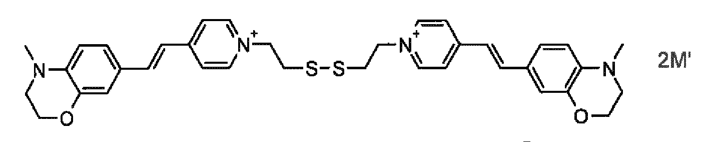 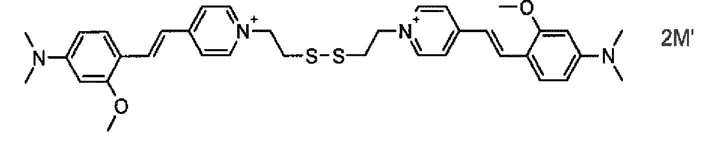 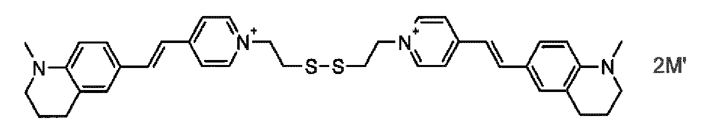 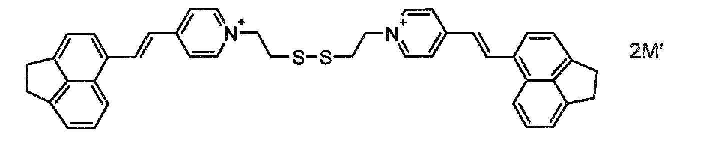 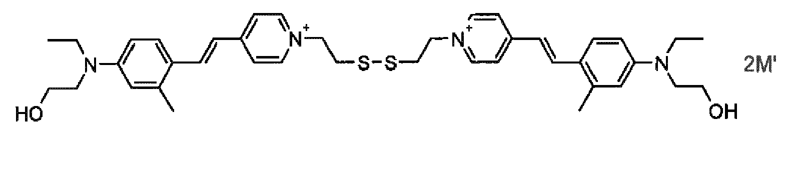 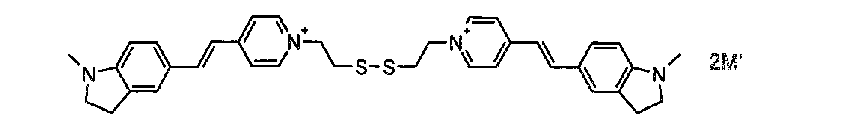 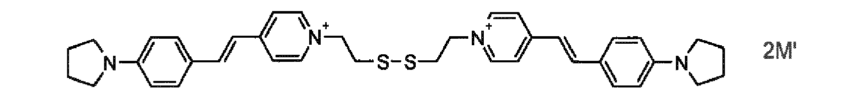 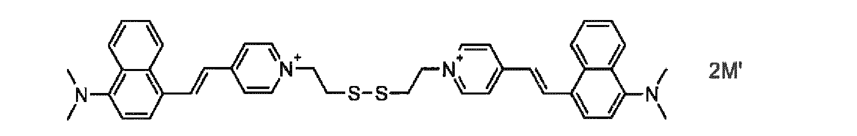 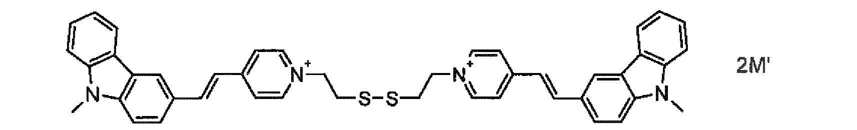 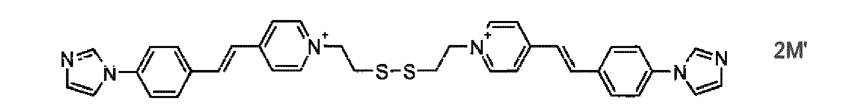 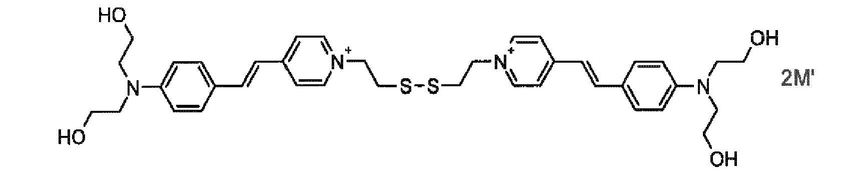 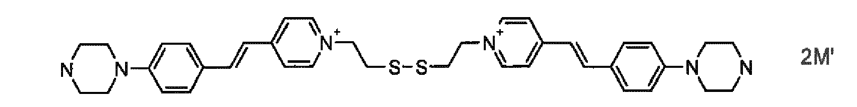 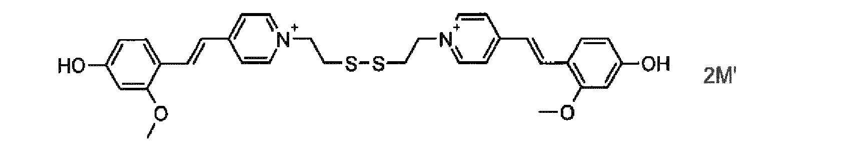 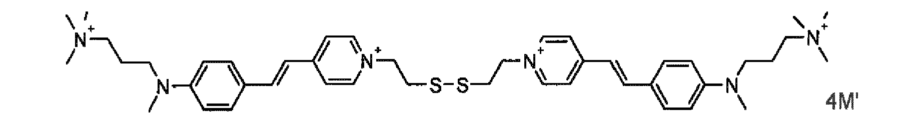 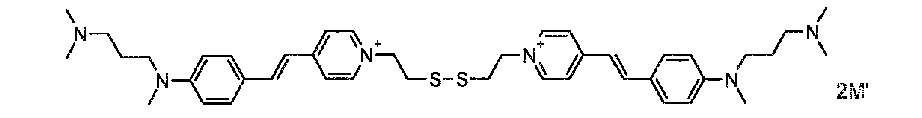 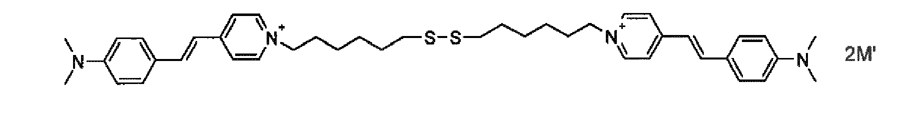 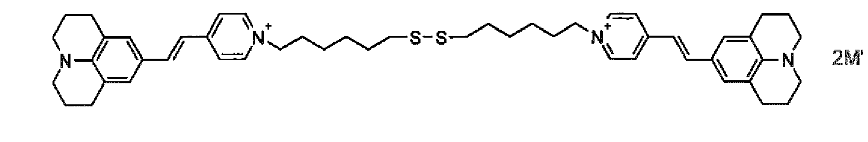 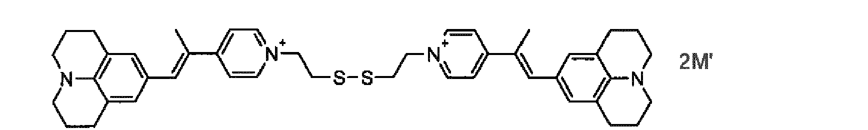 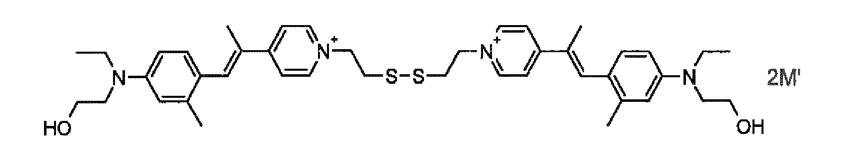 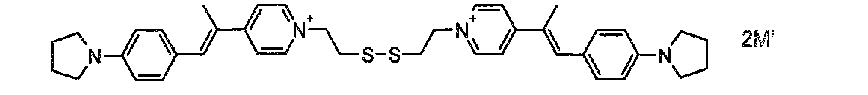 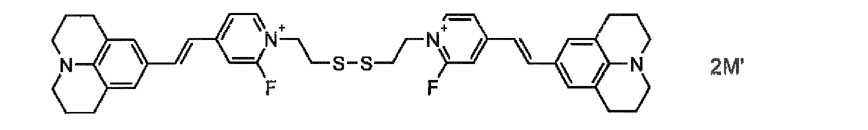 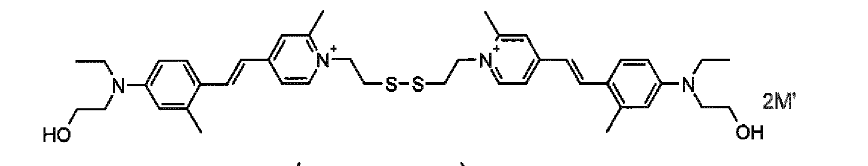 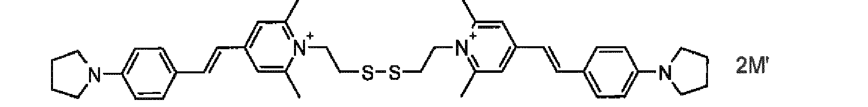 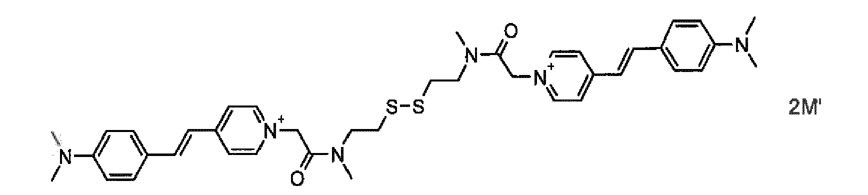 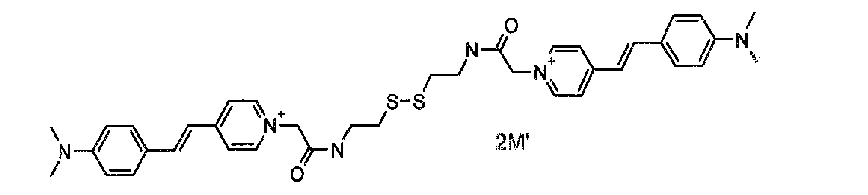 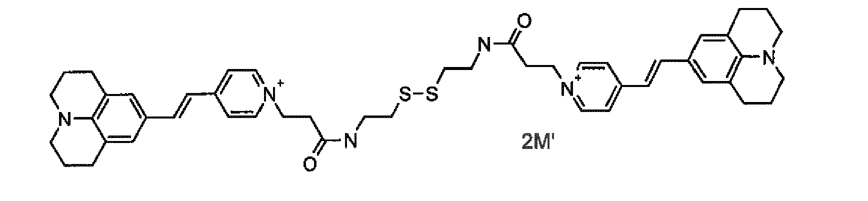 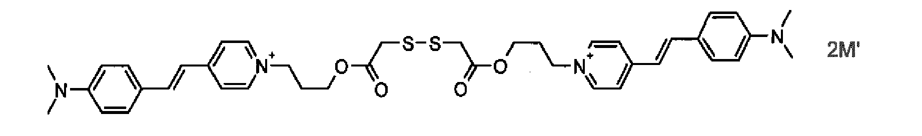 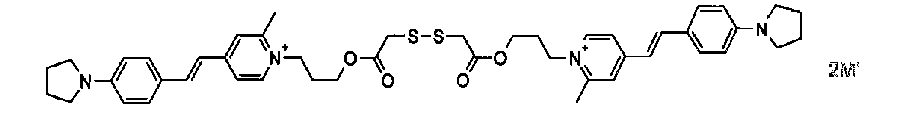 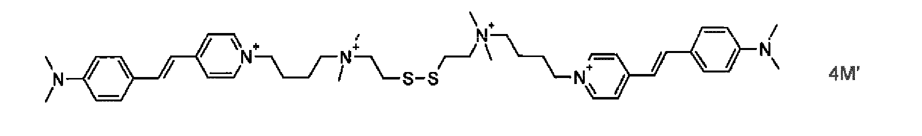 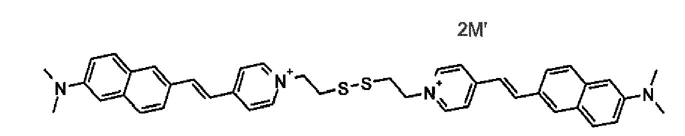 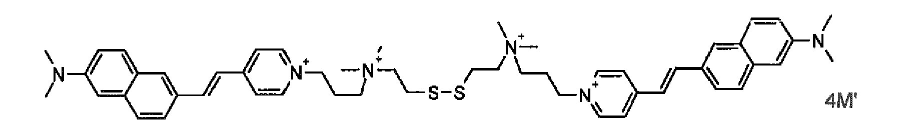 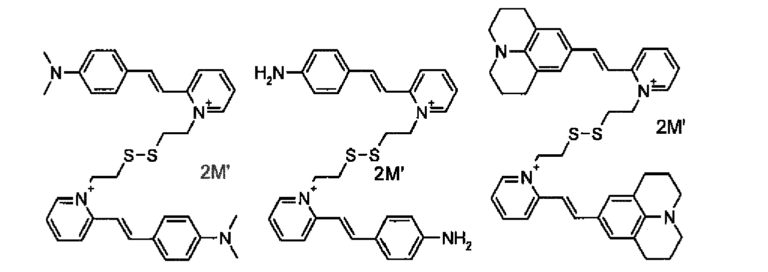 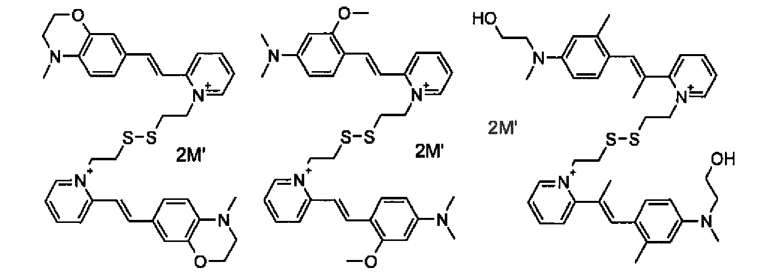 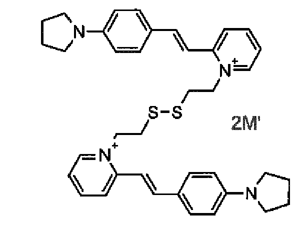 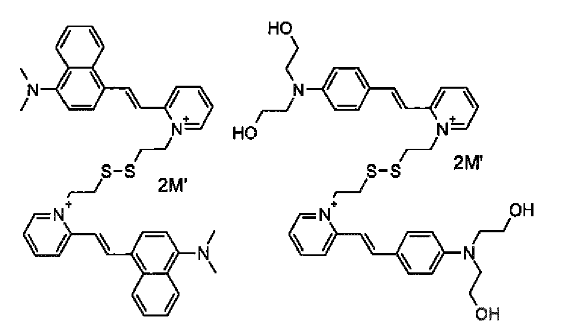 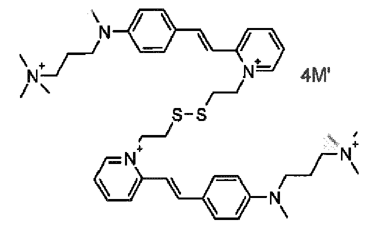 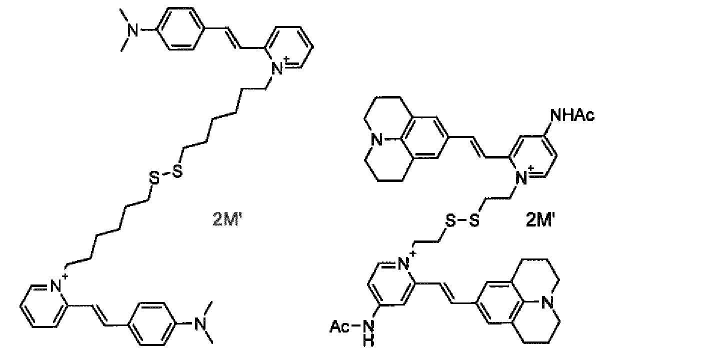 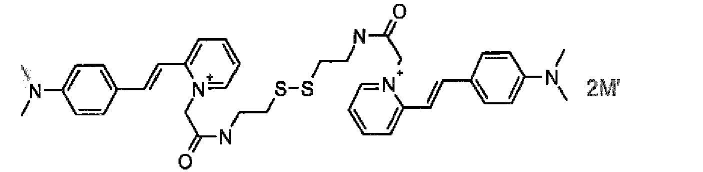 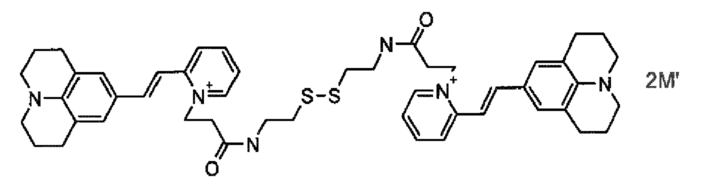 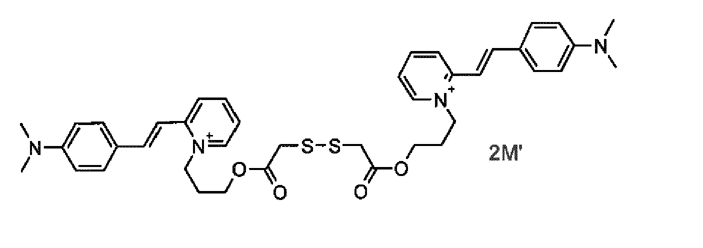 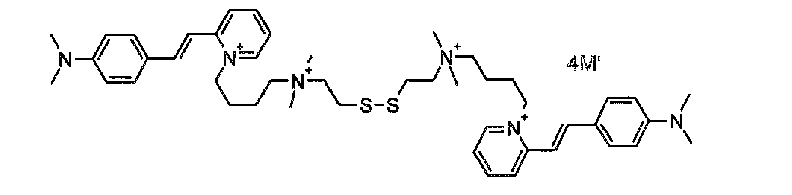 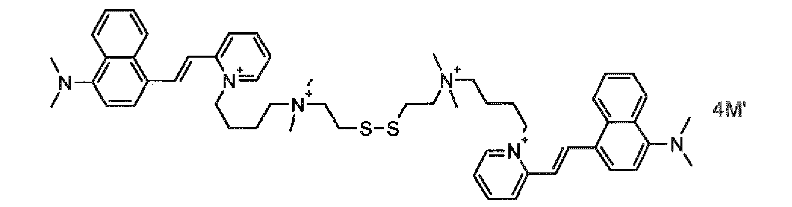 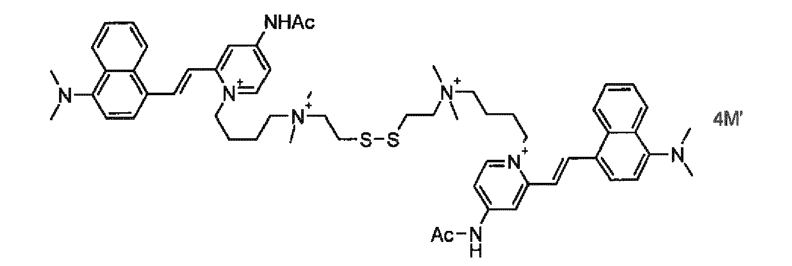 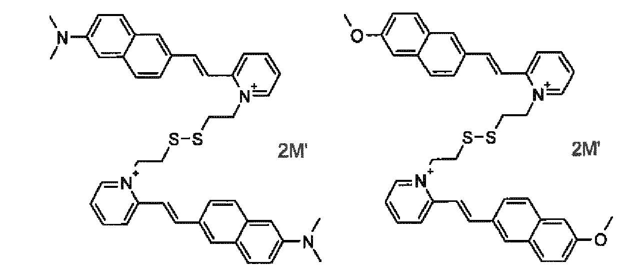 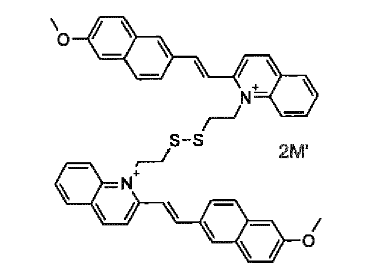 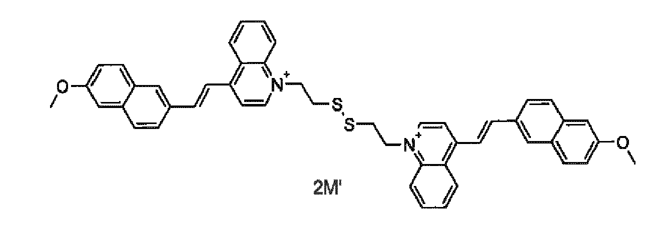 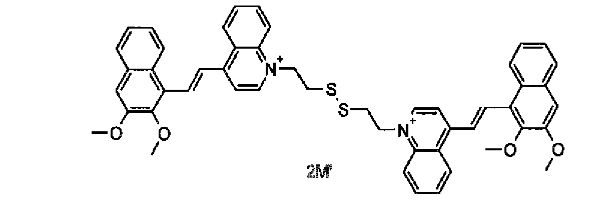 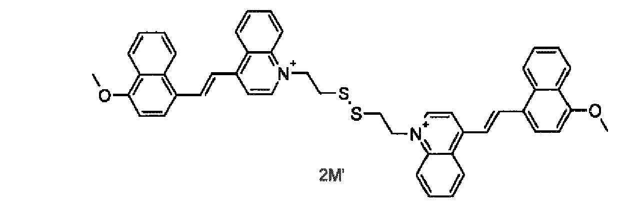 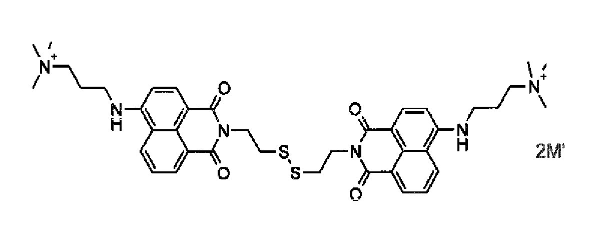 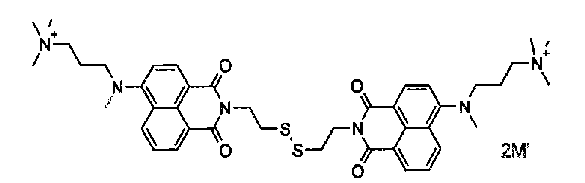 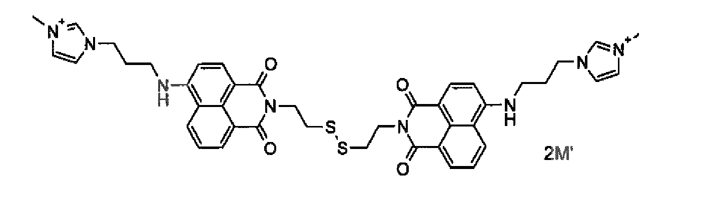 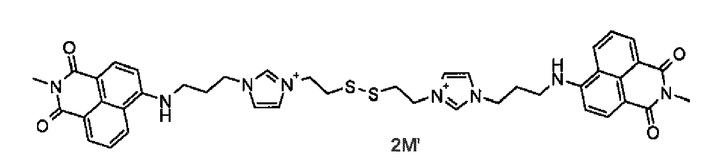 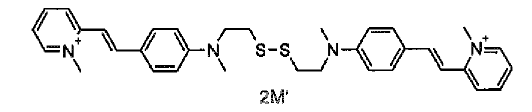 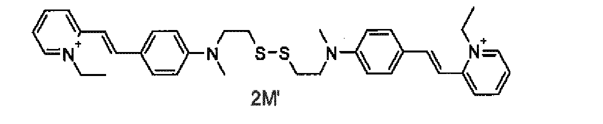 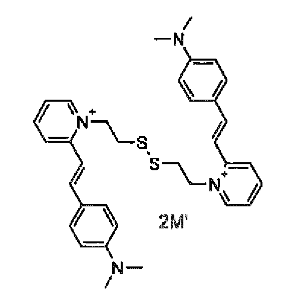 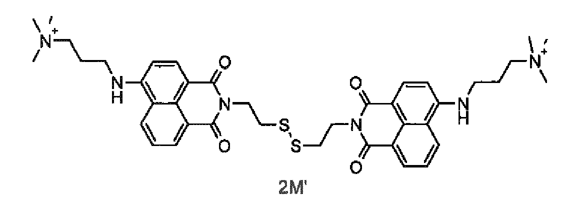 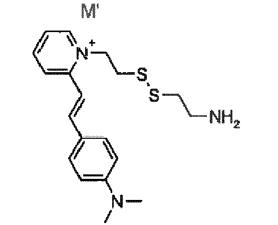 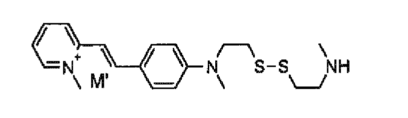 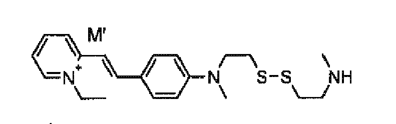 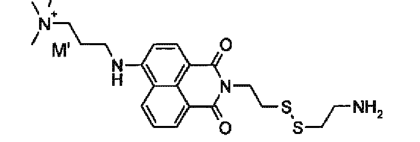 and 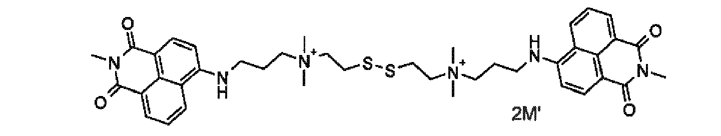 with M' representing an organic or mineral acid salt.

12. Composition according to any one of the preceding claims, **characterized in that** the alkaline agent of mineral or organic hydroxide type is chosen from the hydroxides of alkali metals, alkaline-earth-metals, transition metals, in particular from groups IIIB, IVB, VB and VIB, of lanthanides or of actinides, ammonium and guanidine hydroxides, or mixtures thereof.

13. Process for the simultaneous shaping, dyeing and/or lightening of keratin fibres, **characterized in that** the dyeing composition as defined according to any one of Claims 1 to 12 is applied to the keratin fibres for a sufficient time to obtain the desired shaping and dyeing, and/or lightening, then the keratin fibres are optionally rinsed and washed with shampoo.

14. Multi-compartment device, **characterized in that** it comprises a first compartment (i) comprising one or more alkaline agents of mineral or organic hydroxide type according to Claim 12, and a second compartment (ii) comprising a composition containing one or more disulfide direct dyes according to one of Claims 1 to 11, the pH of the mixture of the contents of the various compartments being between 10 and 14.
